# EUROPEAN PATENT SPECIFICATION

(11) **EP 4 522 277 B1**
(45) Date of publication and mention of the grant of the patent: **28.10.2026**
(21) Application number: 23728523.4
(22) Date of filing: 10.05.2023
(51) Int. Cl.: A61P 35/00, C07D 495/02, A61K 31/4985

(54) **PYRAZINE DERIVATIVES AND USES THEREOF**
PYRAZINDERIVATE UND VERWENDUNGEN DAVON
DÉRIVÉS DE PYRAZINE ET LEURS UTILISATIONS

(30) Priority: 10.05.2022 US 202263340308 P
(43) Date of publication of application: 19.03.2025
(73) Proprietor: Foghorn Therapeutics Inc., Cambridge, MA 02139 (US)
(72) Inventor: NETHERTON, Matthew, Cambridge, Massachusetts 02139 (US); DENG, Jing, Cambridge, Massachusetts 02139 (US); BRUCELLE, Francois, Cambridge, Massachusetts 02139 (US); WILSON, Kevin J., Cambridge, Massachusetts 02139 (US); VOIGT, Johannes H., Cambridge, Massachusetts 02139 (US)
(74) Representative: J A Kemp LLP
(86) International application number: PCT/US2023/021676
(87) International publication number: WO 2023/220137

(56) References cited:
- WO-A1-2021/207291
- WO-A1-2022/109396
- WO-A1-2022/109426
- FARNABY WILLIAM ET AL: "BAF complex vulnerabilities in cancer demonstrated via structure-based PROTAC design", NATURE CHEMICAL BIOLOGY, NATURE PUBLISHING GROUP US, NEW YORK, vol. 15, no. 7, 10 June 2019 (2019-06-10), pages 672 - 680, XP036824718, ISSN: 1552-4450, [retrieved on 20190610], DOI: 10.1038/S41589-019-0294-6

## Description

### Background

The invention relates to compounds useful for modulating BRG1- or BRM-associated factors (BAF) complexes. In particular, the invention relates to compounds useful for treatment of disorders associated with BAF complex function.

Chromatin regulation is essential for gene expression, and ATP-dependent chromatin remodeling is a mechanism by which such gene expression occurs. The human Switch/Sucrose Non-Fermentable (SWI/SNF) chromatin remodeling complex, also known as BAF complex, has two SWI2-like ATPases known as BRG1 (Brahma-related gene-1) and BRM (Brahma). The transcription activator BRG1, also known as ATP-dependent chromatin remodeler SMARCA4, is encoded by the SMARCA4 gene on chromosome 19. BRG1 is overexpressed in some cancer tumors and is needed for cancer cell proliferation. BRM, also known as probable global transcription activator SNF2L2 and/or ATP-dependent chromatin remodeler SMARCA2, is encoded by the SMARCA2 gene on chromosome 9 and has been shown to be essential for tumor cell growth in cells characterized by loss of BRG1 function mutations. Deactivation of BRG and/or BRM results in downstream effects in cells, including cell cycle arrest and tumor suppression.

WO 2022/109396, WO 2022/109426 and WO 2021/207291 are concerned with various compounds useful for the treatment of BAF complex-related disorders.

Farnaby et al. (Nature Chemical Biology 15, 672-680 (2019)) describes a study in which proteolysis targeting chimera (PROTAC) degraders of the BAF ATPase subunits SMARCA2 and SMARCA4 were developed using a bromodomain ligand and recruitment of the E3 ubiquitin ligase VHL.

### Summary

The present invention features compounds useful for modulating a BAF complex, as defined in the appended set of claims. In some embodiments, the compounds are useful for the treatment of disorders associated with an alteration in a BAF complex, e.g., a disorder associated with an alteration in one or both of the BRG1 and BRM proteins. The compounds of the invention, alone or in combination with other pharmaceutically active agents, can be used for treating such disorders.

In an aspect, the invention features a compound, or a pharmaceutically acceptable salt thereof, having the structure of **Formula** I **or II:** 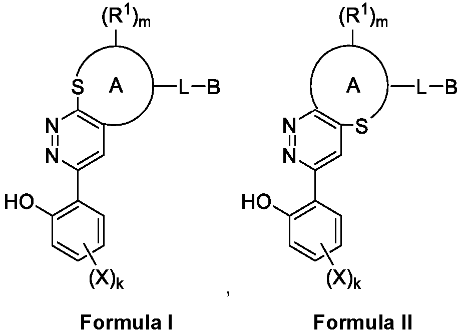 wherein
ring system A is a 5 to 9-membered heterocyclyl or heteroaryl;
m is 0, 1, 2, or 3;
k is 0, 1, or 2;
each R¹ is, independently, halo, optionally substituted C₁-C₆ alkyl, optionally substituted C₁-C₆ heteroalkyl, optionally substituted C₂-C₉ heterocyclyl, optionally substituted C₃-C₈ cycloalkyl or optionally substituted CH₂-C₃-C₈ cycloalkyl;
each X is, independently, halo;
L is a linker of Formula III:

   A¹-(B¹)_{f}-(C¹)_{g}-(B²)ₕ-(B³)ᵢ-(C²)ⱼ-(B⁴)ₖ-A², **Formula III**
wherein
   A¹ is a bond between the linker and ring system A;
   A² is a bond between the degradation moiety and the linker;
   each of B¹, B², B³, and B⁴ is, independently, optionally substituted ethynyl, optionally substituted C₆-C₁₀ aryl, optionally substituted C₃-C₁₀ cycloalkyl, optionally substituted C₂-C₉ heterocyclyl, optionally substituted C₂-C₉ heteroaryl, O, S, S(O)₂, or NR^{N};
   each R^{N} is, independently, H, optionally substituted C₁₋₄ alkyl, optionally substituted C₂₋₄ alkenyl, optionally substituted C₂₋₄ alkynyl, optionally substituted C₂₋₆ heterocyclyl, optionally substituted C₆₋₁₂ aryl, or optionally substituted C₁₋₇ heteroalkyl;
   each of C¹ and C² is, independently, carbonyl, thiocarbonyl, sulphonyl, or phosphoryl; and
   each of f, g, h, i, j, and k is, independently, 0 or 1; and
B is a degradation moiety, wherein the degradation moiety has the structure of Formula C: 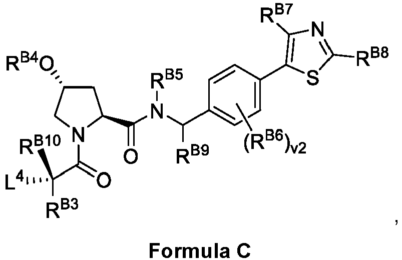
wherein
L⁴ is -N(R^{B1})(R^{B2}), 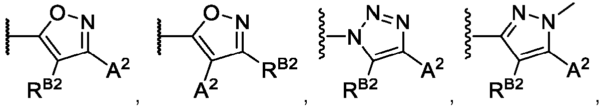 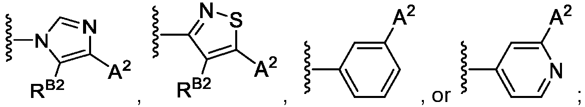
R^{B1} is H, A², optionally substituted C₁-C₆ alkyl, or optionally substituted C₁-C₆ heteroalkyl;
R^{B2} is H, optionally substituted C₁-C₆ alkyl, or optionally substituted C₁-C₆ heteroalkyl;
R^{B3} is A², optionally substituted C₁-C₆ alkyl, optionally substituted C₁-C₆ heteroalkyl, optionally substituted C₃-C₁₀ carbocyclyl, optionally substituted C₆-C₁₀ aryl, optionally substituted C₁-C₆ alkyl C₃-C₁₀ carbocyclyl, or optionally substituted C₁-C₆ alkyl C₆-C₁₀ aryl;
R^{B4} is H, optionally substituted C₁-C₆ alkyl, optionally substituted C₃-C₁₀ carbocyclyl, optionally substituted C₆-C₁₀ aryl, optionally substituted C₁-C₆ alkyl C₃-C₁₀ carbocyclyl, or optionally substituted C₁-C₆ alkyl C₆-C₁₀ aryl;
R^{B5} is H, optionally substituted C₁-C₆ alkyl, or optionally substituted C₁-C₆ heteroalkyl;
v2 is 0, 1, 2, 3, or 4;
each R^{B6} is, independently, A², halogen, optionally substituted C₁-C₆ alkyl, optionally substituted C₂-C₆ alkynyl, optionally substituted C₁-C₆ heteroalkyl, optionally substituted C₃-C₁₀ carbocyclyl, optionally substituted C₂-C₉ heterocyclyl, optionally substituted C₆-C₁₀ aryl, optionally substituted C₂-C₉ heteroaryl, optionally substituted C₂-C₆ alkenyl, optionally substituted C₂-C₆ heteroalkenyl, hydroxy, thiol, cyano, or optionally substituted amino;
each of R^{B7} and R^{B8} is, independently, H, halogen, optionally substituted C₁-C₆ alkyl, or optionally substituted C₆-C₁₀ aryl;
R^{B9} is H or optionally substituted C₁-C₆ alkyl;
R^{B10} is H or F; and
A² is a bond between the degradation moiety and the linker;
wherein one and only one of R^{B1}, R^{B3}, and R^{B6} is A²,
or a pharmaceutically acceptable salt thereof.

In some embodiments, each R' is independently halo, optionally substituted C₁-C₆ alkyl, optionally substituted C₁-C₆ heteroalkyl, optionally substituted C₃-C₈ cycloalkyl, or optionally substituted C₂-C₉ heterocyclyl.

In some embodiments, the compound has the structure of **Formula I-A or II-A:** 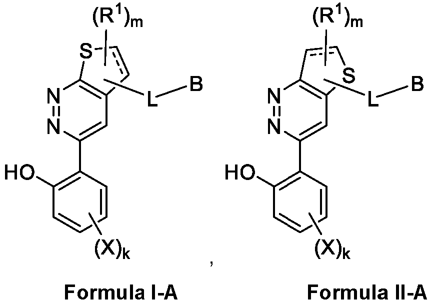
wherein the dashed bond represents a single or double bond,
or a pharmaceutically acceptable salt thereof.

In some embodiments, the compound has the structure of **Formula I-G or II-G:** 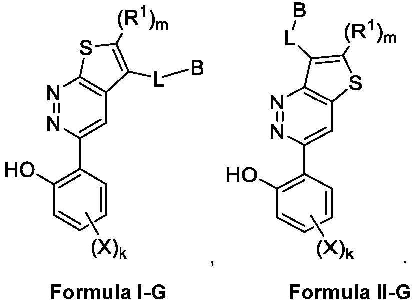 or a pharmaceutically acceptable salt thereof.

In some embodiments, the compound has the structure of **Formula I-H or II-H:** 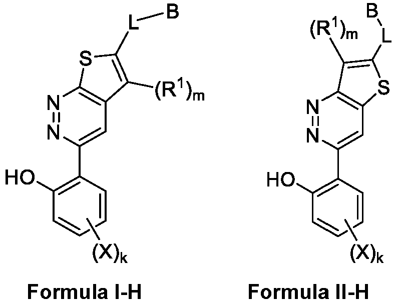 or a pharmaceutically acceptable salt thereof.

In some embodiments, m is 0 or 1.

In some embodiments, m is 1.

In some embodiments, R¹ is halo, optionally substituted C₁-C₆ alkyl or optionally substituted C₃-C₈ cycloalkyl.

In some embodiments, R' is methyl.

In some embodiments, R' is cyclopropane.

In some embodiments, m is 0.

In some embodiments, k is 1.

In some embodiments, X is Cl.

In some embodiments, k is 0.

In some embodiments, the degradation moiety has the structure of **Formula C:** 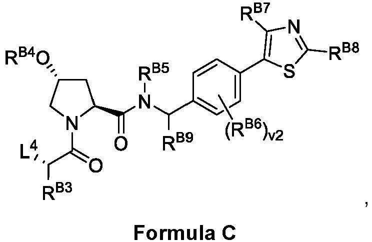 where
L⁴ is -N(R^{B1})(R^{B2}), 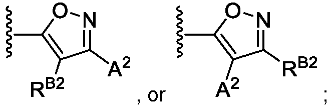
R^{B1} is H, A², optionally substituted C₁-C₆ alkyl, or optionally substituted C₁-C₆ heteroalkyl;
R^{B2} is H, optionally substituted C₁-C₆ alkyl, or optionally substituted C₁-C₆ heteroalkyl;
R^{B3} is A², optionally substituted C₁-C₆ alkyl, optionally substituted C₁-C₆ heteroalkyl, optionally substituted C₃-C₁₀ carbocyclyl, optionally substituted C₆-C₁₀ aryl, optionally substituted C₁-C₆ alkyl C₃-C₁₀ carbocyclyl, or optionally substituted C₁-C₆ alkyl C₆-C₁₀ aryl;
R^{B4} is H, optionally substituted C₁-C₆ alkyl, optionally substituted C₃-C₁₀ carbocyclyl, optionally substituted C₆-C₁₀ aryl, optionally substituted C₁-C₆ alkyl C₃-C₁₀ carbocyclyl, or optionally substituted C₁-C₆ alkyl C₆-C₁₀ aryl;
R^{B5} is H, optionally substituted C₁-C₆ alkyl, or optionally substituted C₁-C₆ heteroalkyl;
v2 is 0, 1, 2, 3, or 4;
each R^{B6} is, independently, A², halogen, optionally substituted C₁-C₆ alkyl, optionally substituted C₁-C₆ heteroalkyl, optionally substituted C₃-C₁₀ carbocyclyl, optionally substituted C₂-C₉ heterocyclyl, optionally substituted C₆-C₁₀ aryl, optionally substituted C₂-C₉ heteroaryl, optionally substituted C₂-C₆ alkenyl, optionally substituted C₂-C₆ heteroalkenyl, hydroxy, thiol, or optionally substituted amino;
each of R^{B7} and R^{B8} is, independently, H, halogen, optionally substituted C₁-C₆ alkyl, or optionally substituted C₆-C₁₀ aryl;
R^{B9} is H or optionally substituted C₁-C₆ alkyl; and
A² is a bond between the degradation moiety and the linker;
where one and only one of R^{B1}, R^{B3}, and R^{B6} is A²,
or a pharmaceutically acceptable salt thereof.

In some embodiments, the degradation moiety has the structure of **Formula C3.**

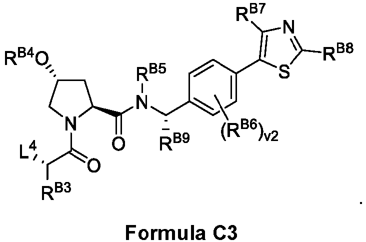

In some embodiments, the degradation moiety has the structure of **Formula C4.**

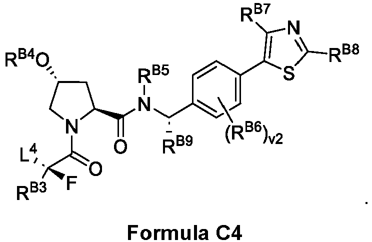

In some embodiments, the degradation moiety has the structure of **Formula C1:**

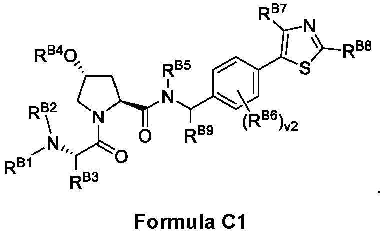

In some embodiments, the degradation moiety is

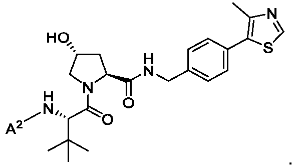

In some embodiments, the degradation moiety is

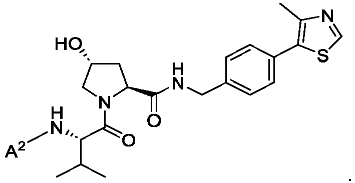

In some embodiments, the degradation moiety is

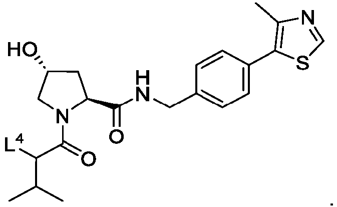

In some embodiments, the degradation moiety is

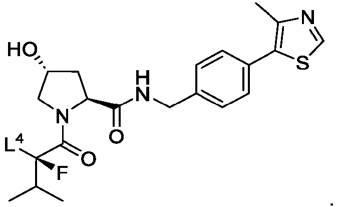

In some embodiments, the degradation moiety is

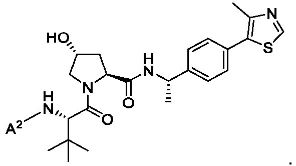

In some embodiments, the degradation moiety is

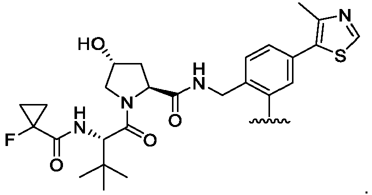

In some embodiments, the degradation moiety has the structure of **Formula C2:**

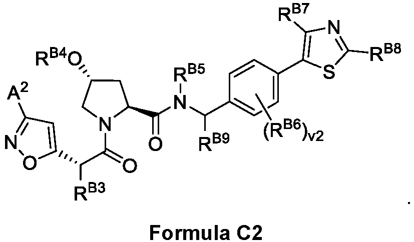

In some embodiments, R^{B9} is optionally substituted C₁-C₆ alkyl. In some embodiments, R^{B9} is methyl.

In some embodiments, R^{B9} is bonded to (S)-stereogenic center.

In some embodiments, v2 is 0. In some embodiments, R^{B4} is H. In some embodiments, R^{B5} is H. In some embodiments, R^{B7} is optionally substituted C₁-C₆ alkyl. In some embodiments, R^{B7} is methyl. In some embodiments, R^{B3} is optionally substituted C₁-C₆ alkyl. In some embodiments, R^{B3} is isopropyl. In some embodiments, R^{B8} is H. In some embodiments, R^{B2} is H.

In some embodiments, the degradation moiety is

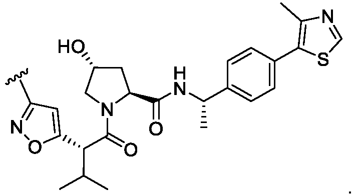

In some embodiments, the degradation moiety has the structure of **Formula Ca2:**

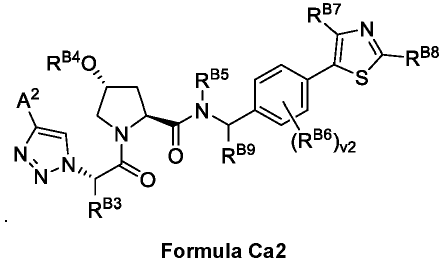

In some embodiments, the degradation moiety has the structure of **Formula Cb2:**

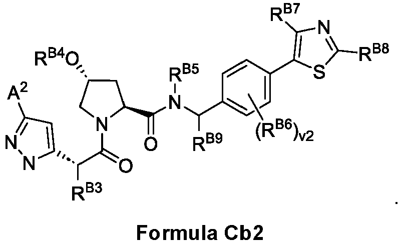

In some embodiments, the degradation moiety has the structure of **Formula Cc2:**

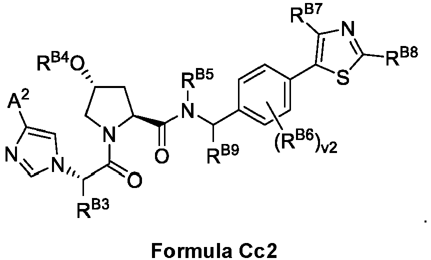

In some embodiments, the degradation moiety has the structure of **Formula Cd2:**

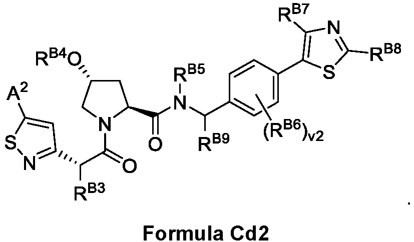

In some embodiments, the degradation moiety has the structure of **Formula Ce2:**

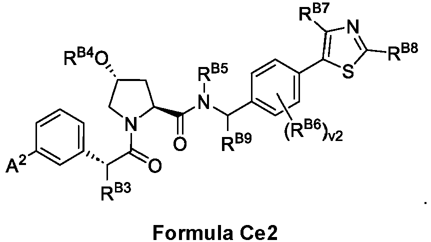

In some embodiments, the degradation moiety has the structure of **Formula Cf2:**

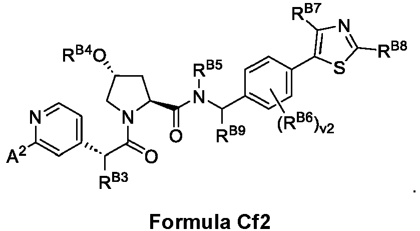

In some embodiments, R^{B9} is optionally substituted C₁-C₆ alkyl. In some embodiments, R^{B9} is methyl.

In some embodiments, R^{B9} is bonded to (S)-stereogenic center.

In some embodiments, v2 is 0. In some embodiments, R^{B4} is H. In some embodiments, R^{B5} is H. In some embodiments, R^{B7} is optionally substituted C₁-C₆ alkyl. In some embodiments, R^{B7} is methyl. In some embodiments, R^{B3} is optionally substituted C₁-C₆ alkyl. In some embodiments, R^{B3} is isopropyl. In some embodiments, R^{B3} is optionally substituted C₃-C₁₀ carbocyclyl. In some embodiments, R^{B3} is cyclopropane. In some embodiments, R^{B3} is cyclobutane. In some embodiments, R^{B3} is fluoro-2-methylpropane. In some embodiments, R^{B8} is H. In some embodiments, R^{B2} is H.

In some embodiments, the degradation moiety is

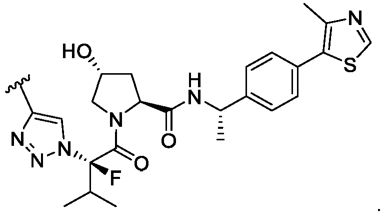

In some embodiments, the degradation moiety is

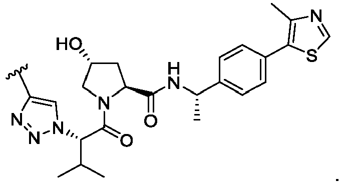

In some embodiments, the degradation moiety is

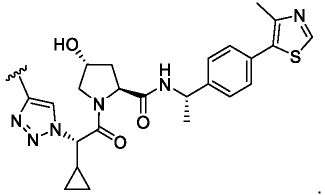

In some embodiments, the degradation moiety is

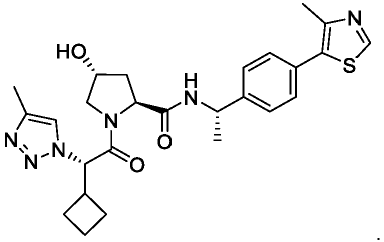

In some embodiments, the degradation moiety is

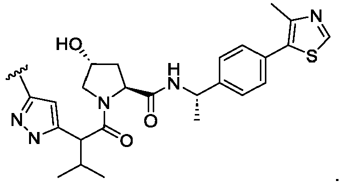

In some embodiments, the degradation moiety is

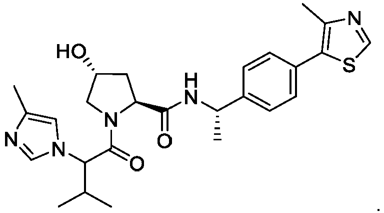

In some embodiments, the degradation moiety is

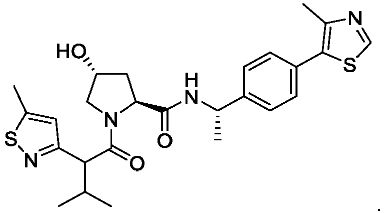

In some embodiments, the degradation moiety is

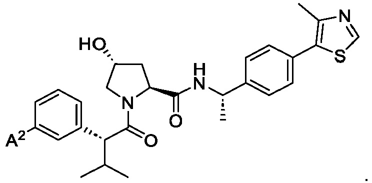

In some embodiments, the degradation moiety is

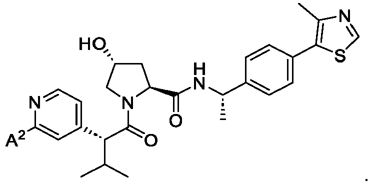

In some embodiments, the shortest chain of atoms connecting two valencies of the linker is 2 to 10 atoms long. In some embodiments, the shortest chain of atoms connecting two valencies of the linker is 6 atoms long.

In some embodiments, the linker has a structure of the linker in any one of compounds 1-121 in Table 1 (e.g., of any of the compounds with a ratio of BRG1 IC₅₀ to BRM IC₅₀ of at least 5 (e.g., at least 7, 10, 15, 20, 25, or 30)). In some embodiments, the linker has a structure of the linker in any one of compounds 1-121 in Table 1 (e.g., of any of the compounds with a BRM IC₅₀ of ++ or better (e.g., +++ or ++++ (e.g., ++++))). In some embodiments, the linker has a structure of the linker in any one of compounds 1-121 in Table 1 (e.g., of any of the compounds with a BRM IC₅₀ of ++ or better (e.g., +++ or ++++ (e.g., ++++)) and with a ratio of BRG1 IC₅₀ to BRM IC₅₀ of at least 5 (e.g., at least 7, 10, 15, 20, 25, or 30)).

In an aspect, the invention features a compound selected from the group consisting of 1-121 in Table 1 and pharmaceutically acceptable salts thereof. In some embodiments, the compound is any one of compounds 1-121 in Table 1 with a ratio of BRG1 IC₅₀ to BRM IC₅₀ of at least 5 (e.g., at least 7, 10, 15, 20, 25, or 30) or a pharmaceutically acceptable salt thereof. In some embodiments, the compound is any one of compounds 1-121 in Table 1 with a BRM IC₅₀ of ++ or better as found in Table 15 (e.g., +++ or ++++ (e.g., ++++)) or a pharmaceutically acceptable salt thereof. In some embodiments, the compound is any one of compounds 1-121 in Table 1 a BRM IC₅₀ of ++ or better as found in Table 15 (e.g., +++ or ++++ (e.g., ++++)) and with a ratio of BRG1 IC₅₀ to BRM IC₅₀ of at least 5 (e.g., at least 7, 10, 15, 20, 25, or 30) or a pharmaceutically acceptable salt thereof.

In an aspect, the invention features a compound selected from the group consisting of 1-103 in Table 1 and pharmaceutically acceptable salts thereof.

**Table 1. Compounds of the Invention**

| # | Compound |
|---|---|
| 1 | 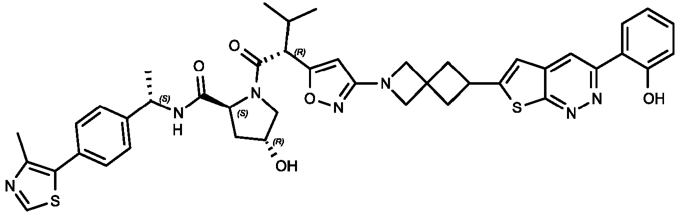 |
| 2 | 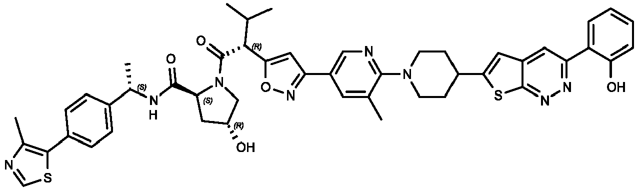 |
| 3 | 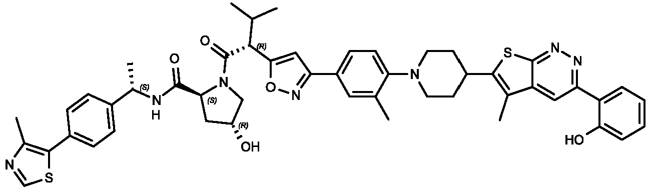 |
| 4 | 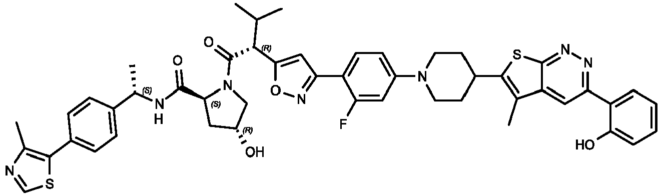 |
| 5 | 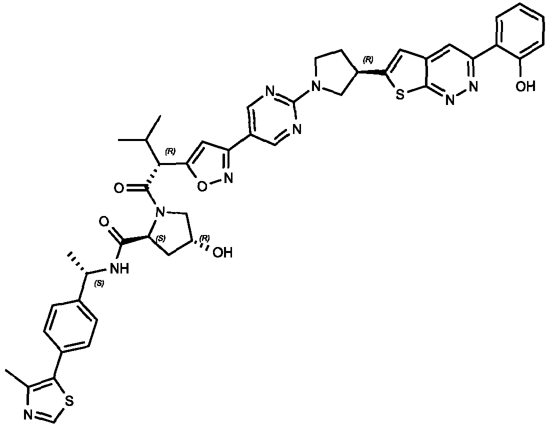 |
| 6 | 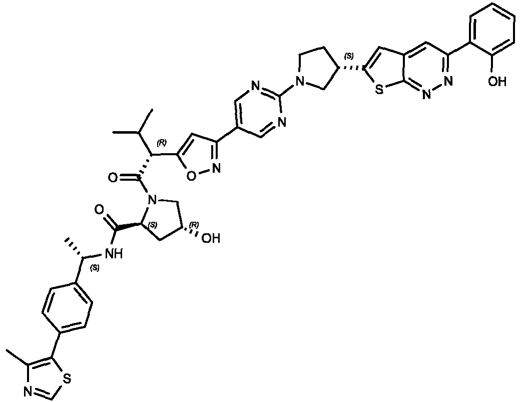 |
| 7 | 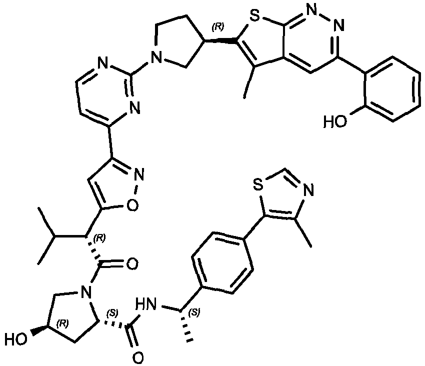 |
| 8 | 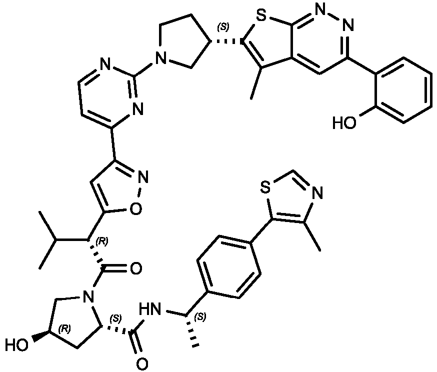 |
| 9 | 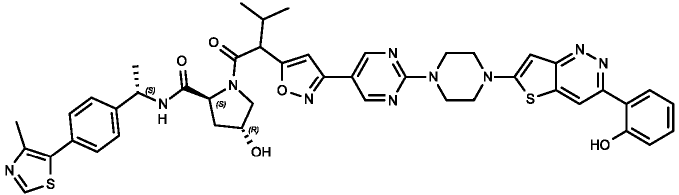 |
| 10 | 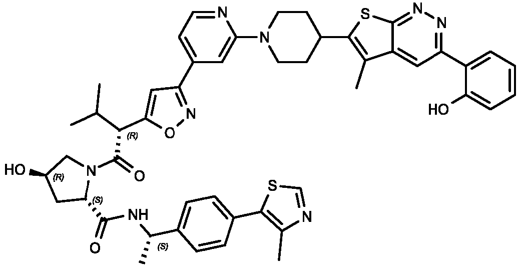 |
| 11 | 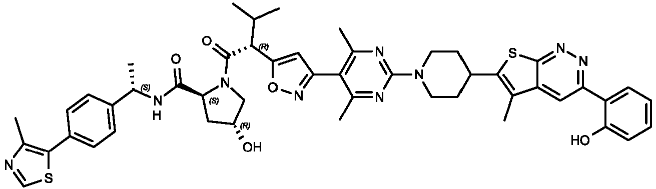 |
| 12 | 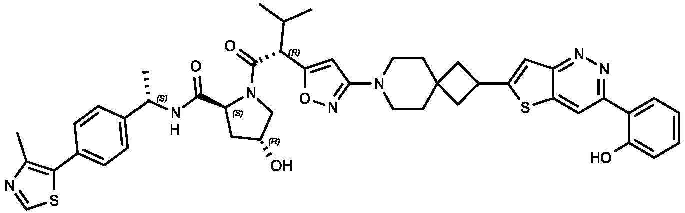 |
| 13 | 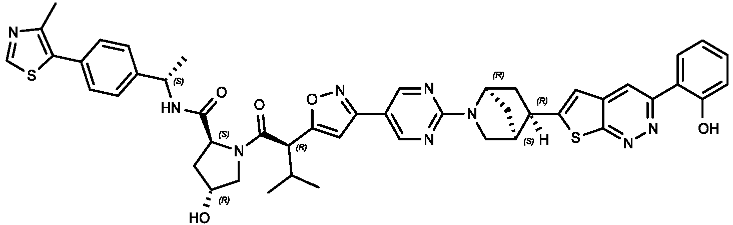 |
| 14 | 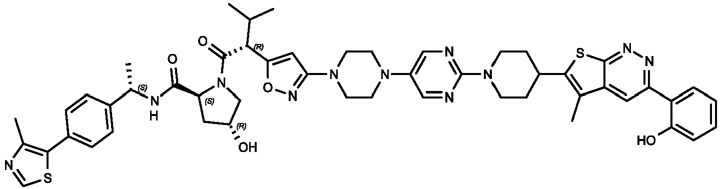 |
| 15 | 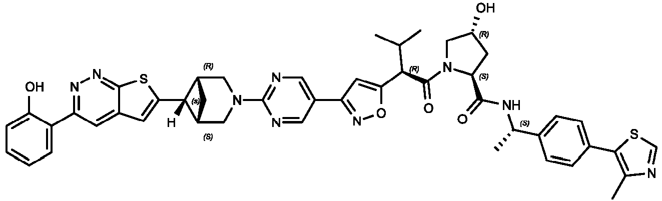 |
| 16 | 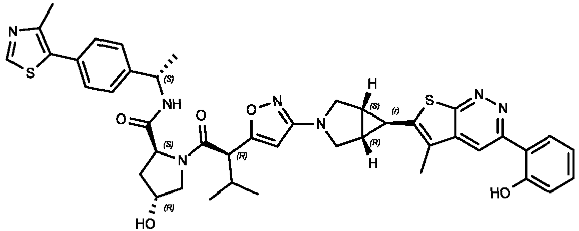 |
| 17 | 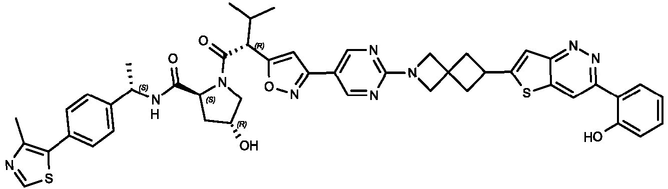 |
| 18 | 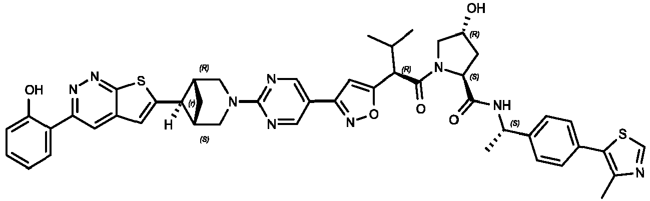 |
| 19 | 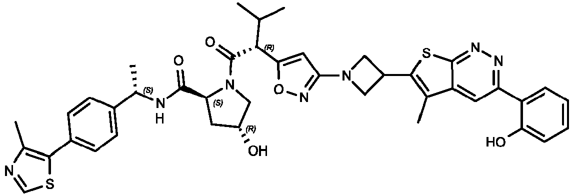 |
| 20 | 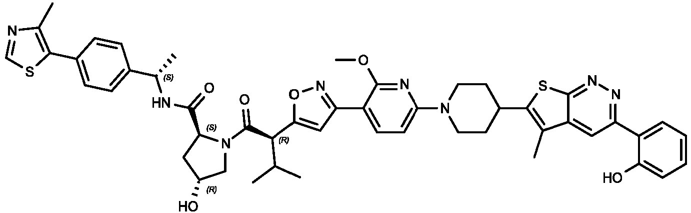 |
| 21 | 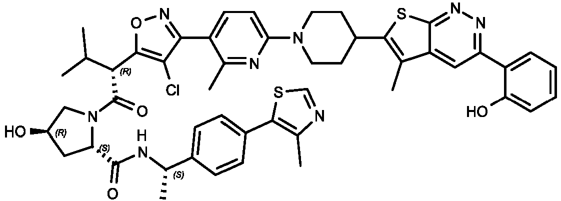 |
| 22 | 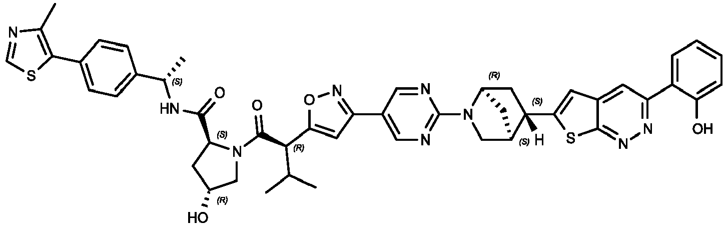 |
| 23 | 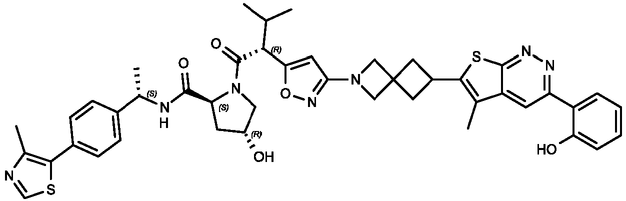 |
| 24 | 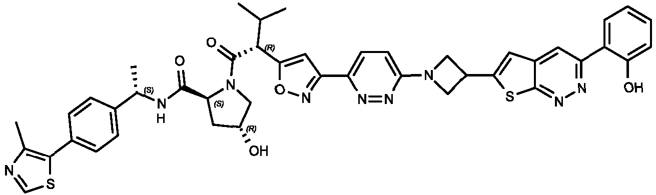 |
| 25 | 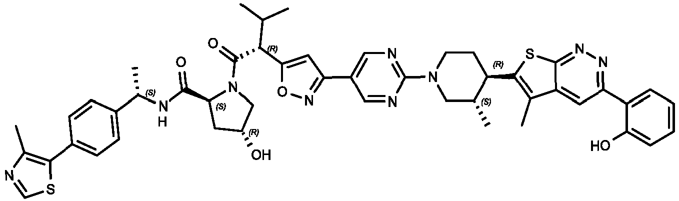 |
| 26 | 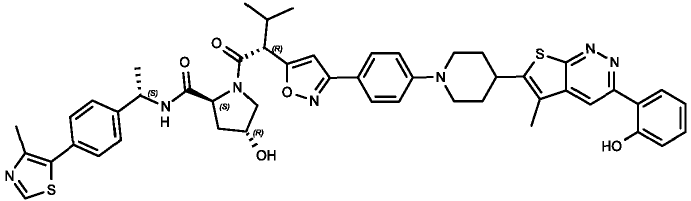 |
| 27 | 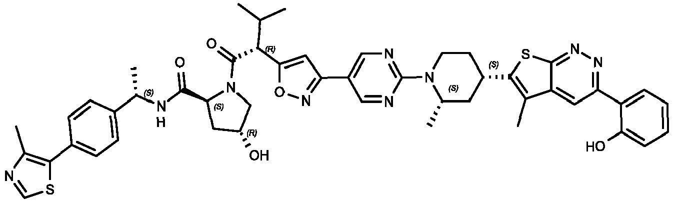 |
| 28 | 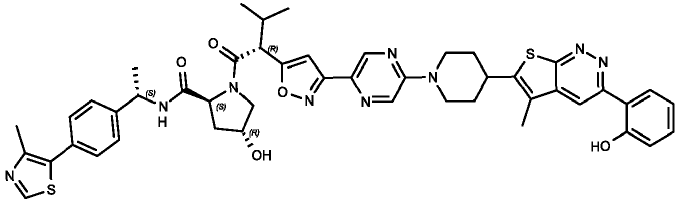 |
| 29 | 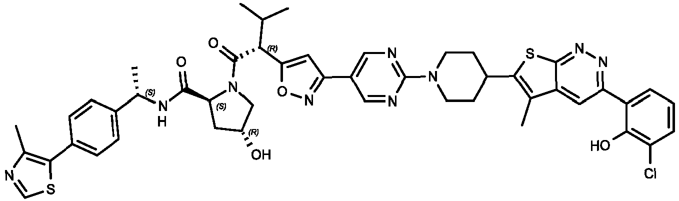 |
| 30 | 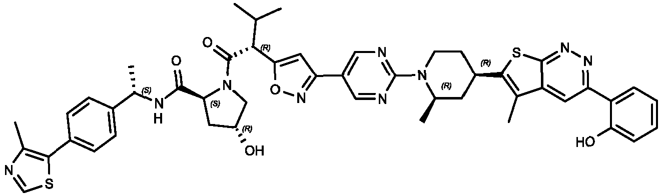 |
| 31 | 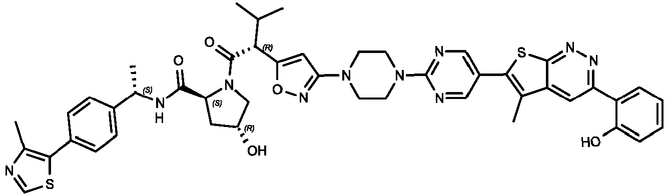 |
| 32 | 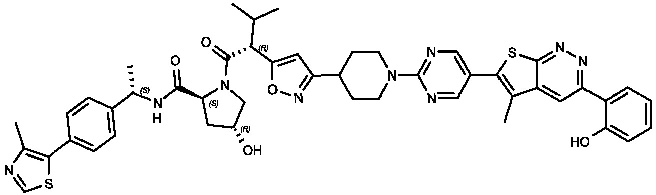 |
| 33 | 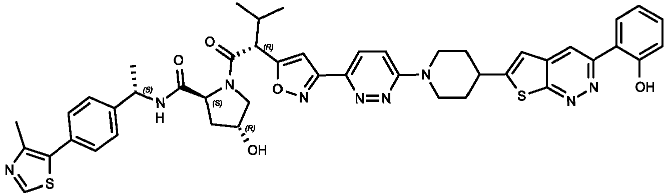 |
| 34 | 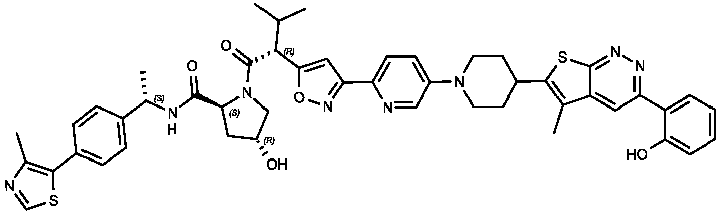 |
| 35 | 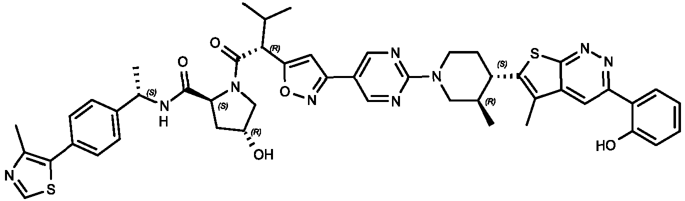 |
| 36 | 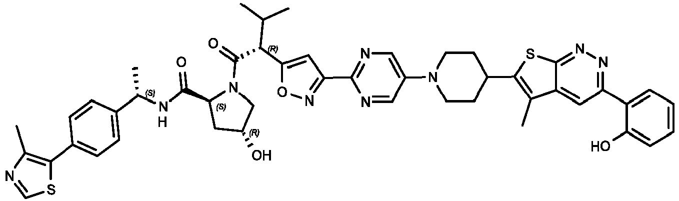 |
| 37 | 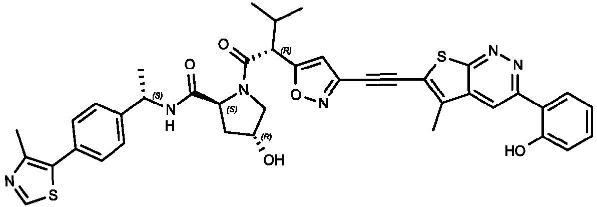 |
| 38 | 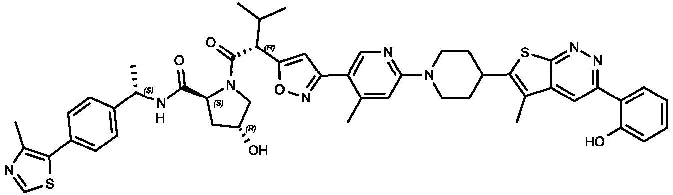 |
| 39 | 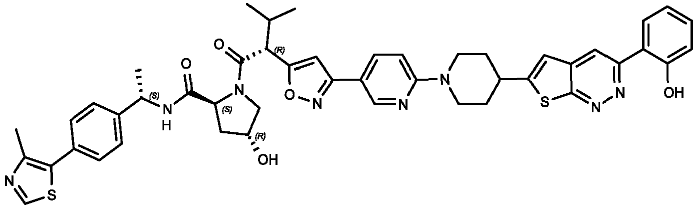 |
| 40 | 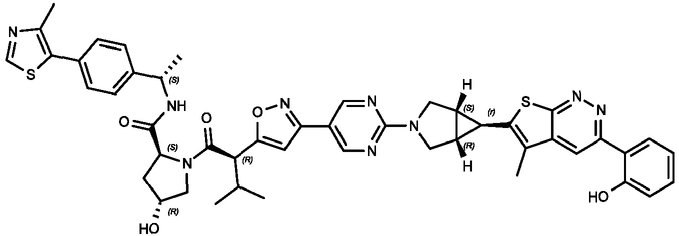 |
| 41 | 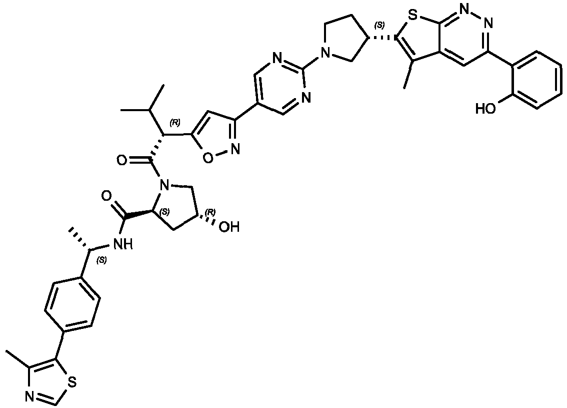 |
| 42 | 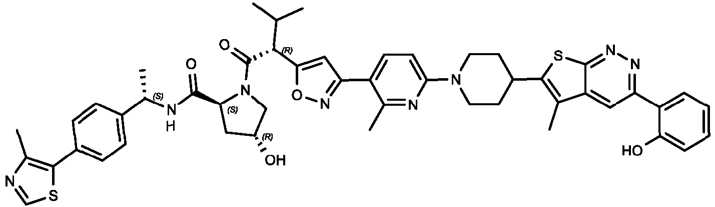 |
| 43 | 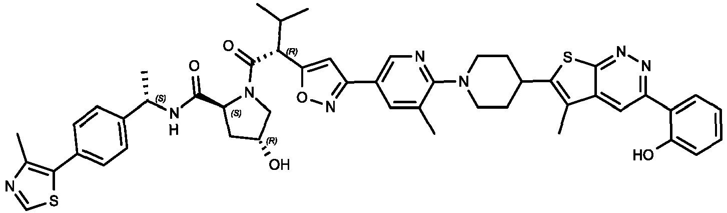 |
| 44 | 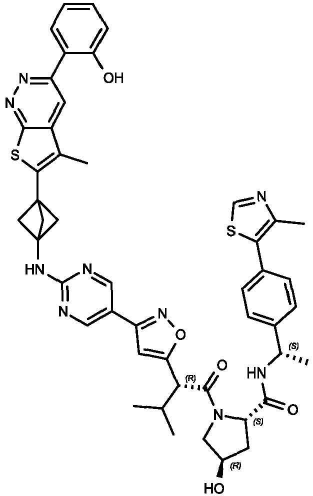 |
| 45 | 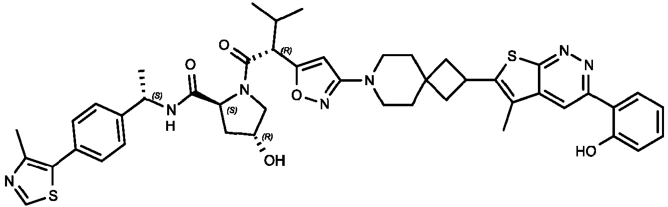 |
| 46 | 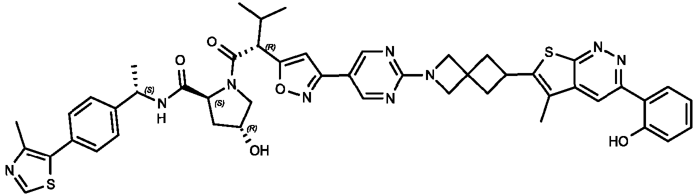 |
| 47 | 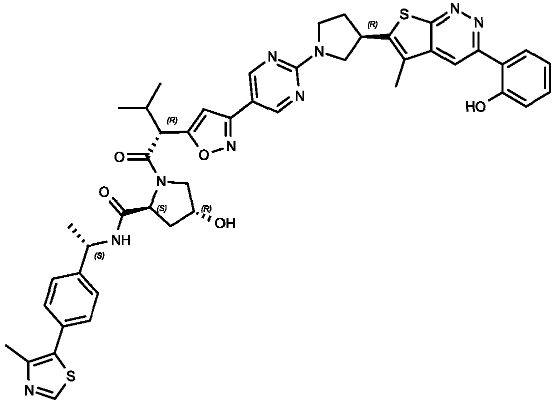 |
| 48 | 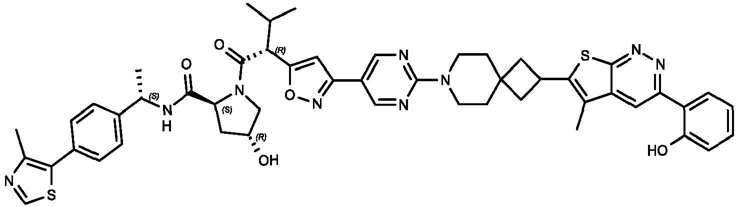 |
| 49 | 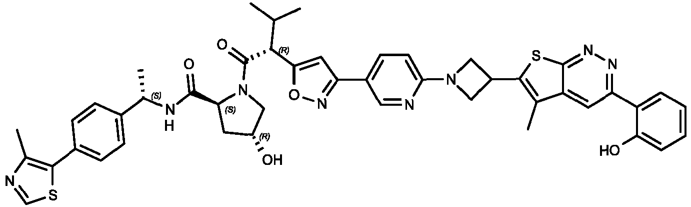 |
| 50 | 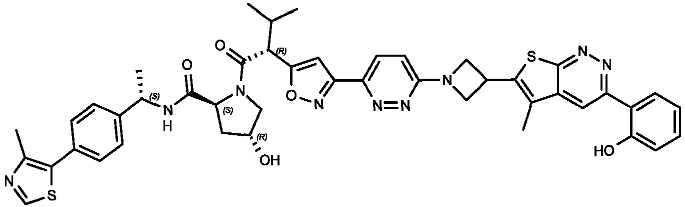 |
| 51 | 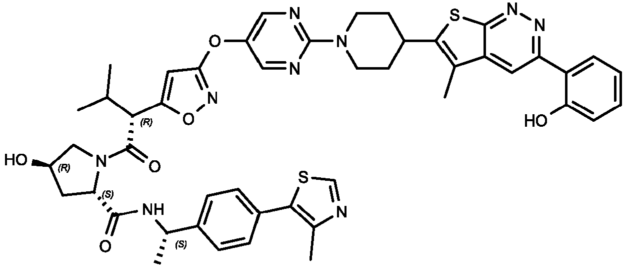 |
| 52 | 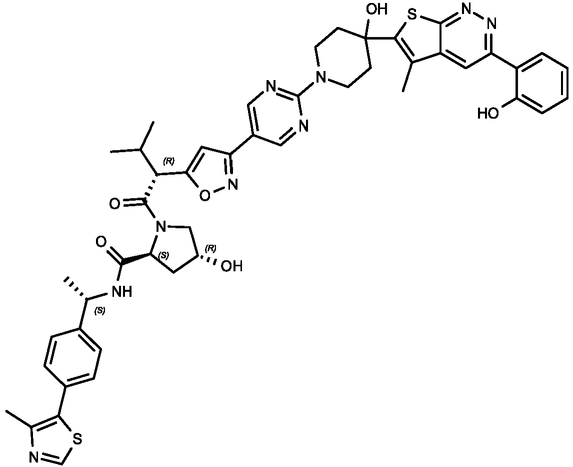 |
| 53 | 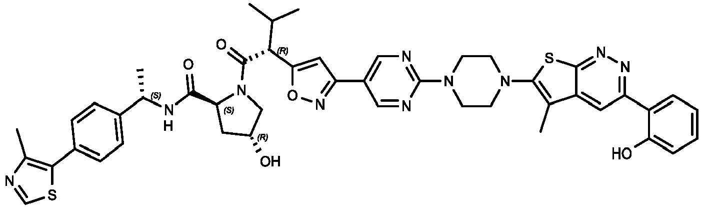 |
| 54 | 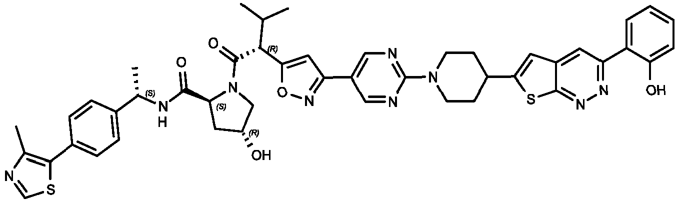 |
| 55 | 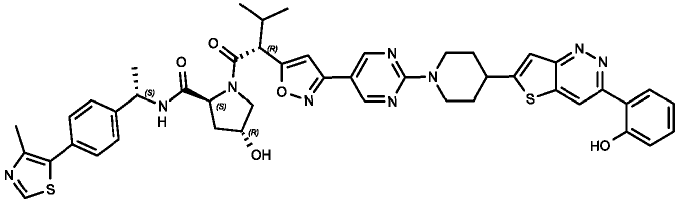 |
| 56 | 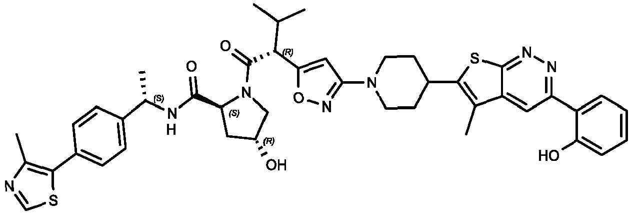 |
| 57 | 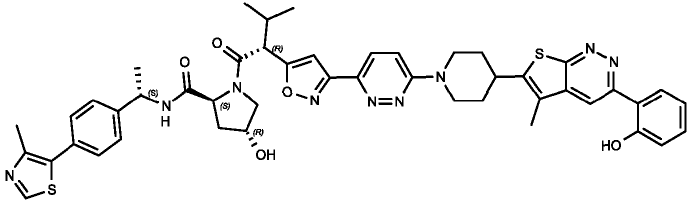 |
| 58 | 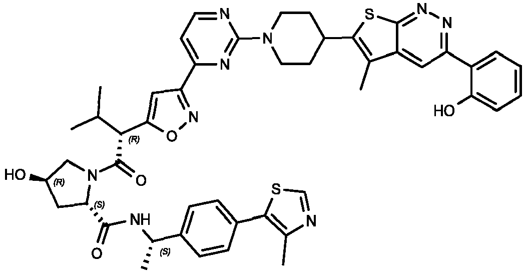 |
| 59 | 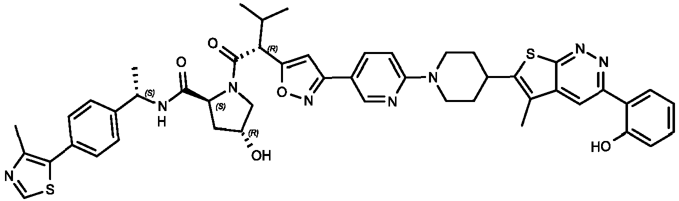 |
| 60 | 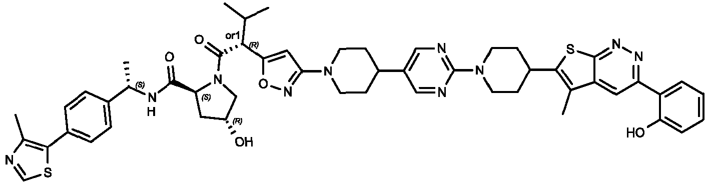 |
| 61 | 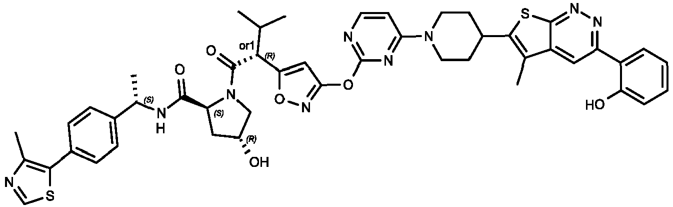 |
| 62 | 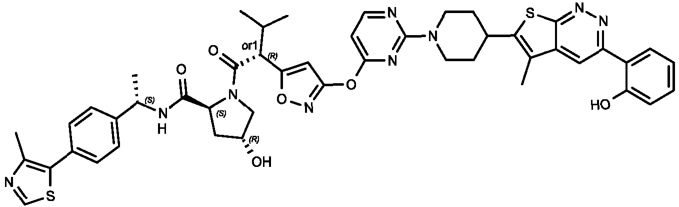 |
| 63 | 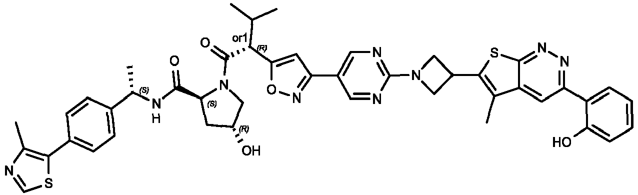 |
| 64 | 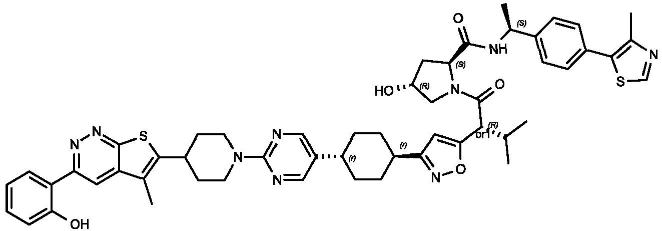 |
| 65 | 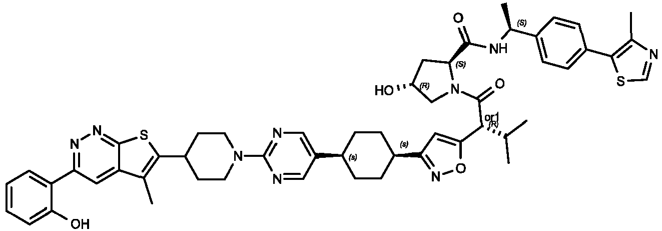 |
| 66 | 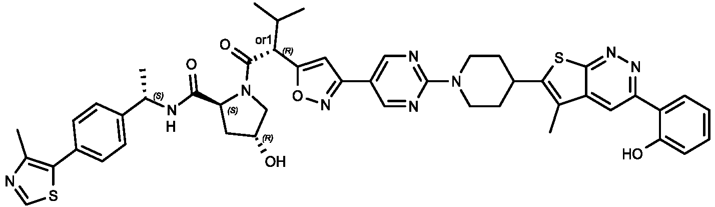 |
| 67 | 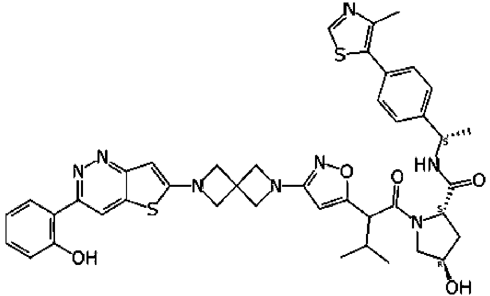 |
| 68 | 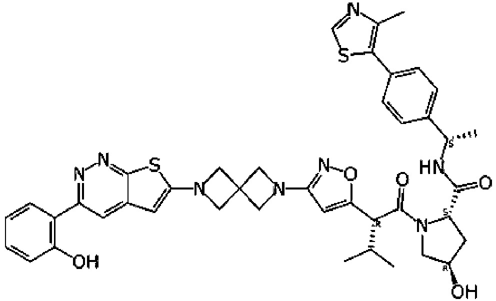 |
| 69 | 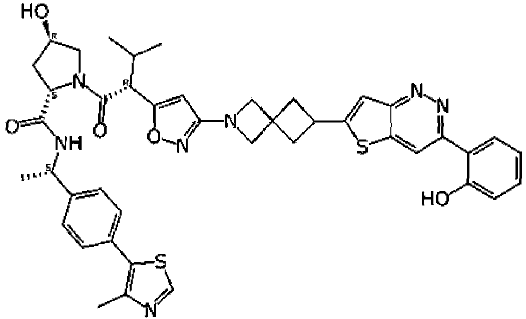 |
| 70 | 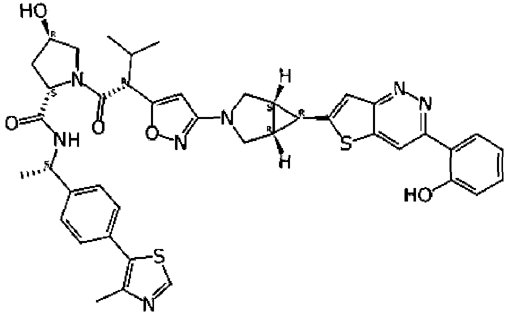 |
| 71 | 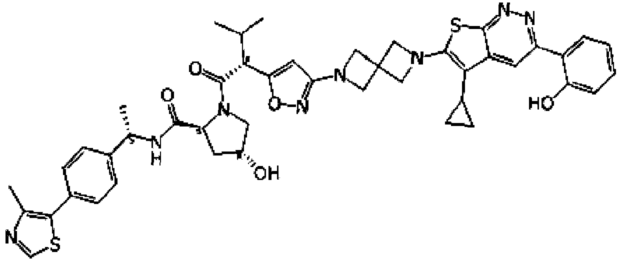 |
| 72 | 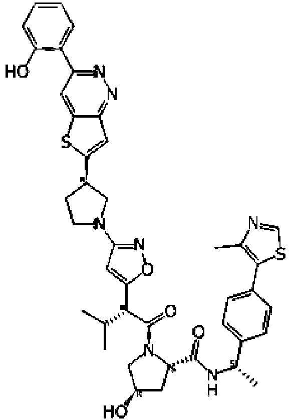 |
| 73 | 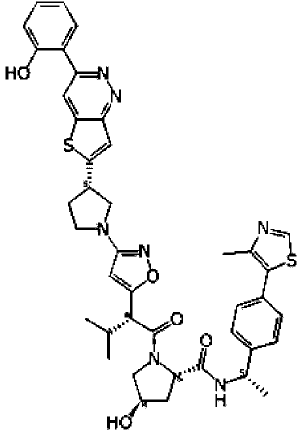 |
| 74 | 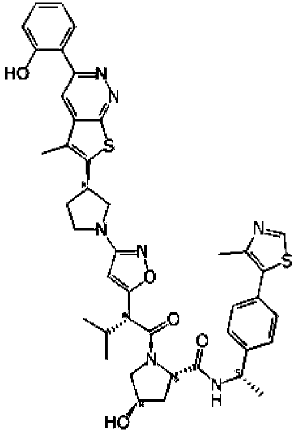 |
| 75 | 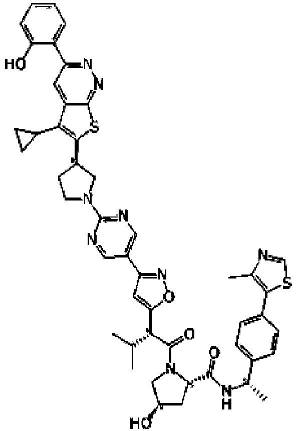 |
| 76 | 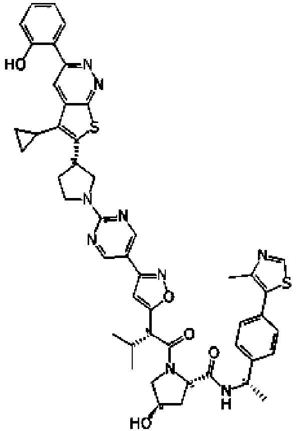 |
| 77 | 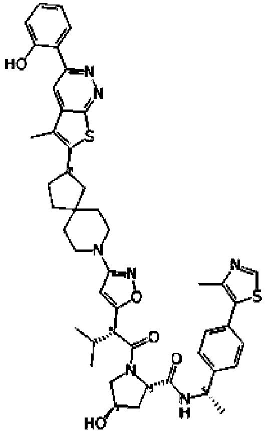 |
| 78 | 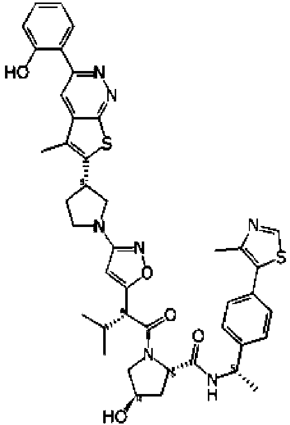 |
| 79 | 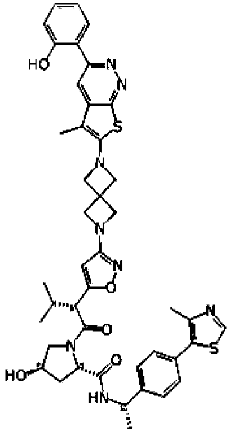 |
| 80 | 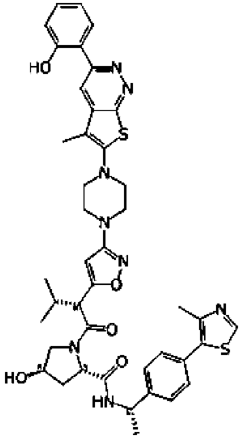 |
| 81 | 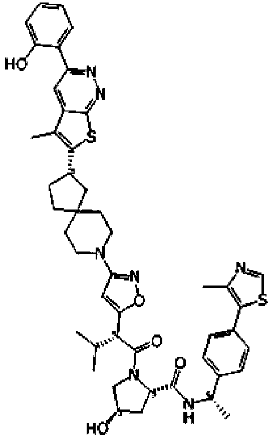 |
| 82 | 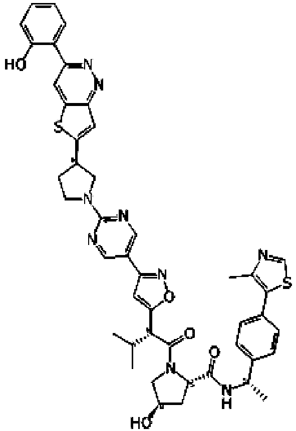 |
| 83 | 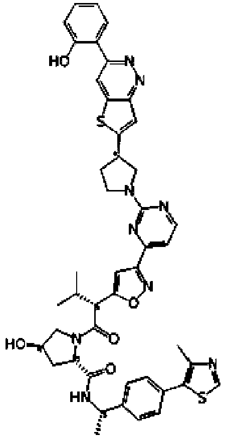 |
| 84 | 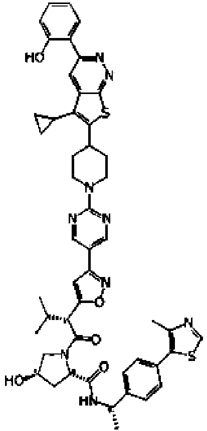 |
| 85 | 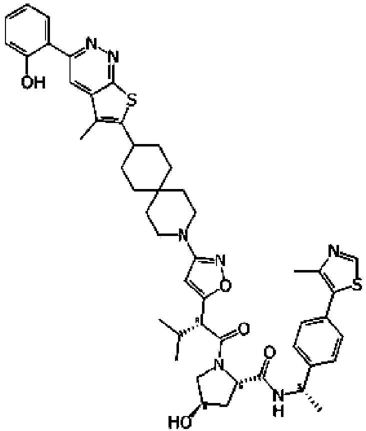 |
| 86 | 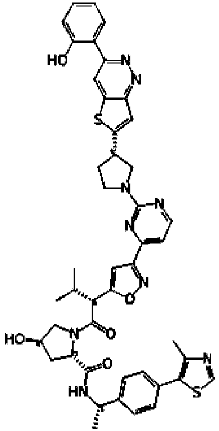 |
| 87 | 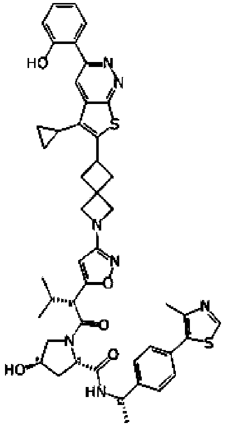 |
| 88 | 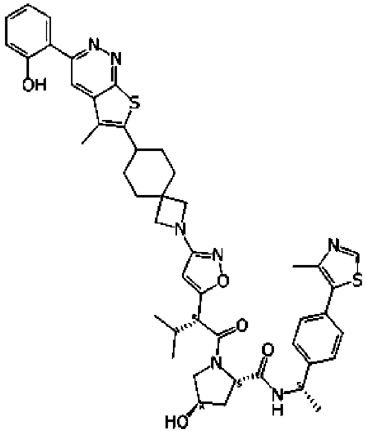 |
| 89 | 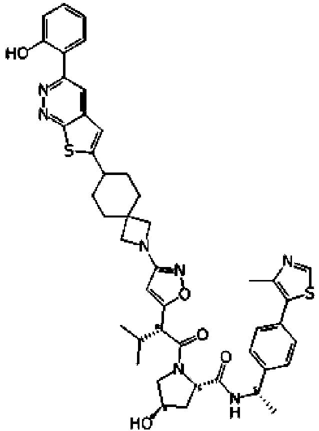 |
| 90 | 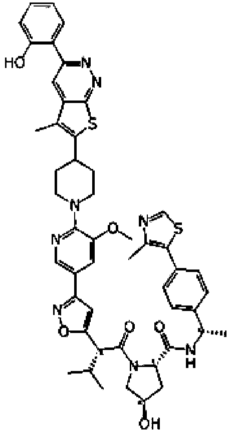 |
| 91 | 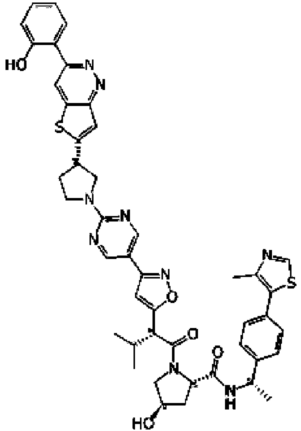 |
| 92 | 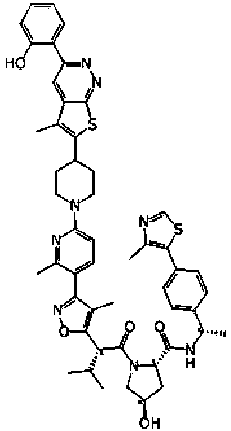 |
| 93 | 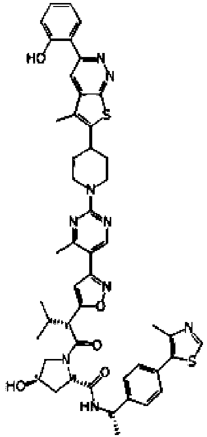 |
| 94 | 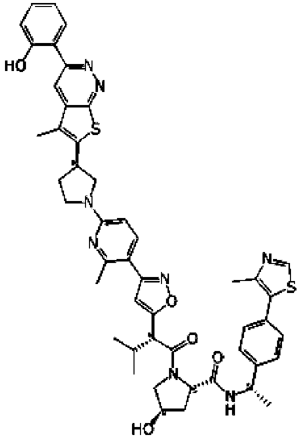 |
| 95 | 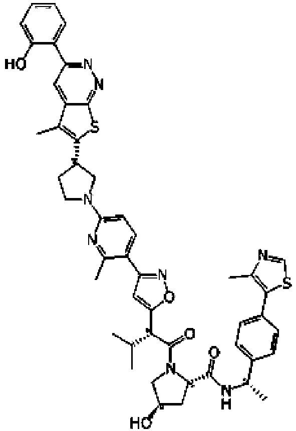 |
| 96 | 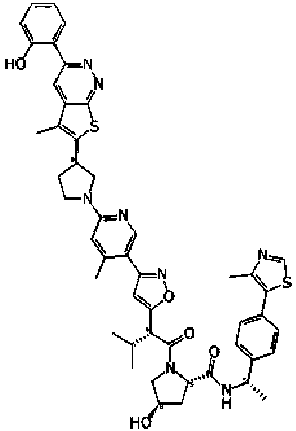 |
| 97 | 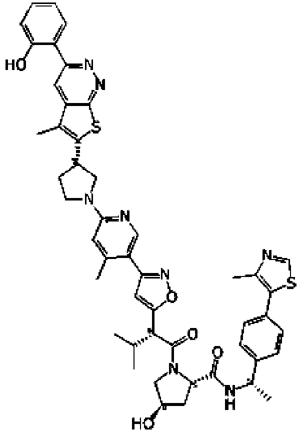 |
| 98 | 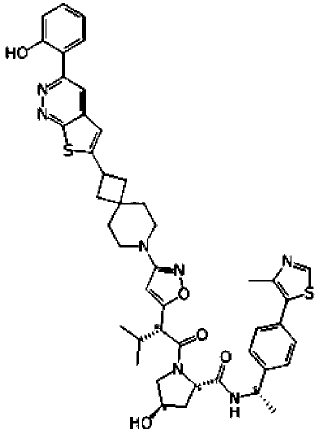 |
| 99 | 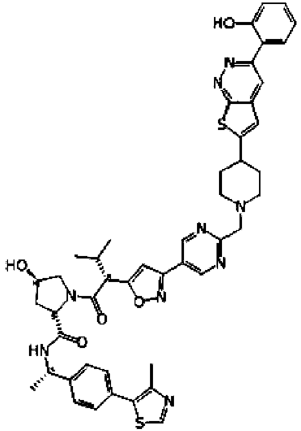 |
| 100 | 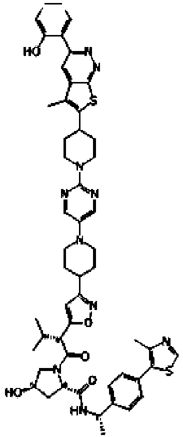 |
| 101 | 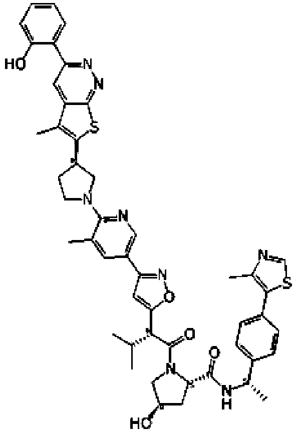 |
| 102 | 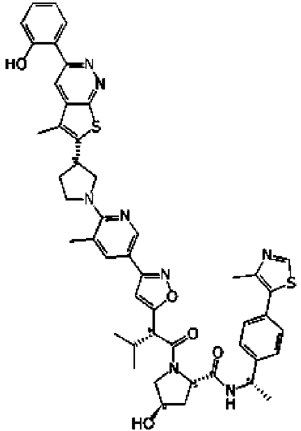 |
| 103 | 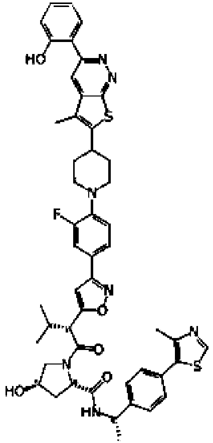 |

In some embodiments, the compound has a ratio of BRG1 IC₅₀ to BRM IC₅₀ of at least 5. In some embodiments, the compound has a ratio of BRG1 IC₅₀ to BRM IC₅₀ of at least 7. In some embodiments, the compound has a ratio of BRG1 IC₅₀ to BRM IC₅₀ of at least 10. In some embodiments, the compound has a ratio of BRG1 IC₅₀ to BRM IC₅₀ of at least 15. In some embodiments, the compound has a ratio of BRG1 IC₅₀ to BRM IC₅₀ of at least 20. In some embodiments, the compound has a ratio of BRG1 IC₅₀ to BRM IC₅₀ of at least 25. In some embodiments, the compound has a ratio of BRG1 IC₅₀ to BRM IC₅₀ of at least 30.

In an aspect, the invention features a pharmaceutical composition comprising any of the foregoing compounds and a pharmaceutically acceptable excipient.

For clarity, in the present disclosure all references to methods of medical treatment comprising administration of compounds or compositions are to be interpreted as being directed to the compounds or compositions for use in such methods of medical treatment.

The compounds and pharmaceutical compositions can be used in a method of decreasing the activity of a BAF complex in a cell, the method involving contacting the cell with an effective amount of any of the foregoing compounds or a pharmaceutical composition thereof.

In some instances, the cell is a cancer cell.

The compounds and pharmaceutical compositions can be used in a method of treating a BAF complex-related disorder in a subject in need thereof, the method involving administering to the subject an effective amount of any of the foregoing compounds (e.g., a BRM/BRG1 dual inhibitor compound or a BRM-selective compound) or a pharmaceutical composition thereof.

In some instances, the BAF complex-related disorder is cancer.

In an aspect, the present invention features any of the foregoing compounds or pharmaceutical compositions for use in therapy. In one particular such aspect, the present invention features any of the foregoing compounds or pharmaceutical compositions for use in treating cancer.

The compounds and pharmaceutical compositions can be used in a method of inhibiting BRM, the method involving contacting a cell with an effective amount of any of the foregoing compounds (e.g., a BRM/BRG1 dual inhibitor compound or a BRM-selective compound) or a pharmaceutical composition thereof.

In some instances, the cell is a cancer cell.

The compounds and pharmaceutical compositions can be used in a method of inhibiting BRG1, the method involving contacting the cell with an effective amount of any of the foregoing compounds or a pharmaceutical composition thereof.

In some instances, the cell is a cancer cell.

The compounds and pharmaceutical compositions can be used in a method of inhibiting BRM and BRG1, the method involving contacting the cell with an effective amount of any of the foregoing compounds or a pharmaceutical composition thereof.

In some instances, the cell is a cancer cell.

The compounds and pharmaceutical compositions can be used in a method of treating a disorder related to a BRG1 loss of function mutation in a subject in need thereof, the method involving administering to the subject an effective amount of any of the foregoing compounds (e.g., a BRM/BRG1 dual inhibitor compound or a BRM-selective compound) or a pharmaceutical composition thereof.

In some instances, the disorder related to a BRG1 loss of function mutation is cancer. In other instances, the subject is determined to have a BRG1 loss of function disorder, for example, is determined to have a BRG1 loss of function cancer (for example, the cancer has been determined to include cancer cells with loss of BRG1 function).

The compounds and pharmaceutical compositions can be used in a method of inducing apoptosis in a cell, the method involving contacting the cell with an effective amount of any of the foregoing compounds (e.g., a BRM/BRG1 dual inhibitor compound or a BRM-selective compound) or a pharmaceutical composition thereof.

In some instances, the cell is a cancer cell.

A method of treating cancer in a subject in need thereof can include administering to the subject an effective amount of any of the foregoing compounds (e.g., a BRM/BRG1 dual inhibitor compound or a BRM-selective compound) or a pharmaceutical composition thereof.

In some instances of any of the foregoing methods, and in some embodiments of the aspect of the invention featuring any of the foregoing compounds or a pharmaceutical composition thereof for use in treating cancer, the cancer is non-small cell lung cancer, colorectal cancer, bladder cancer, cancer of unknown primary, glioma, breast cancer, melanoma, non-melanoma skin cancer, endometrial cancer, esophagogastric cancer, pancreatic cancer, hepatobiliary cancer, soft tissue sarcoma, ovarian cancer, head and neck cancer, renal cell carcinoma, bone cancer, non-Hodgkin lymphoma, small-cell lung cancer, prostate cancer, embryonal tumor, germ cell tumor, cervical cancer, thyroid cancer, salivary gland cancer, gastrointestinal neuroendocrine tumor, uterine sarcoma, gastrointestinal stromal tumor, CNS cancer, thymic tumor, Adrenocortical carcinoma, appendiceal cancer, small bowel cancer, or penile cancer.

In some instances of any of the foregoing methods, and in some embodiments of the aspect of the invention featuring any of the foregoing compounds or a pharmaceutical composition thereof for use in treating cancer, the cancer is non-small cell lung cancer, colorectal cancer, bladder cancer, cancer of unknown primary, glioma, breast cancer, melanoma, non-melanoma skin cancer, endometrial cancer, or penile cancer.

In some instances of any of the foregoing methods, and in some embodiments of the aspect of the invention featuring any of the foregoing compounds or a pharmaceutical composition thereof for use in treating cancer, the cancer is a drug resistant cancer or has failed to respond to a prior therapy (e.g., vemurafenib, dacarbazine, a CTLA4 inhibitor, a PD1 inhibitor, interferon therapy, a BRAF inhibitor, a MEK inhibitor, radiotherapy, temozolomide, irinotecan, a CAR-T therapy, Herceptin^{®}, Perjeta^{®}, tamoxifen, Xeloda^{®}, docetaxol, platinum agents such as carboplatin, taxanes such as paclitaxel and docetaxel, ALK inhibitors, MET inhibitors, Alimta^{®}, Abraxane^{®}, Adriamycin^{®}, gemcitabine, Avastin^{®}, Halaven^{®}, neratinib, a PARP inhibitor, ARN810, an mTOR inhibitor, topotecan, Gemzar^{®}, a VEGFR2 inhibitor, a folate receptor antagonist, demcizumab, fosbretabulin, or a PDL1 inhibitor).

In some instances of any of the foregoing methods, and in some embodiments of the aspect of the invention featuring any of the foregoing compounds or a pharmaceutical composition thereof for use in treating cancer, the cancer has or has been determined to have BRG1 mutations. In some such instances and embodiments, the BRG1 mutations are homozygous. In some such instances and embodiments, the cancer does not have, or has been determined not to have, an epidermal growth factor receptor (EGFR) mutation. In some such instances and embodiments, the cancer does not have, or has been determined not to have, an anaplastic lymphoma kinase (ALK) driver mutation. In some such instances and embodiments methods, the cancer has, or has been determined to have, a KRAS mutation. In some such instances and embodiments, the BRG1 mutation is in the ATPase catalytic domain of the protein. In some such instances and embodiments, the BRG1 mutation is a deletion at the C-terminus of BRG1.

In another aspect, the disclosure provides a method treating a disorder related to BAF (e.g., cancer or viral infections) in a subject in need thereof. This method includes contacting a cell with an effective amount of any of the foregoing compounds (e.g., a BRM/BRG1 dual inhibitor compound or a BRM-selective compound), or pharmaceutically acceptable salts thereof, or any of the foregoing pharmaceutical compositions. In some instances, the disorder is a viral infection is an infection with a virus of the Retroviridae family such as the lentiviruses (e.g., Human immunodeficiency virus (HIV) and deltaretroviruses (e.g., human T cell leukemia virus I (HTLV-I), human T cell leukemia virus II (HTLV-II)), Hepadnaviridae family (e.g., hepatitis B virus (HBV)), Flaviviridae family (e.g., hepatitis C virus (HCV)), Adenoviridae family (e.g., Human Adenovirus), Herpesviridae family (e.g., Human cytomegalovirus (HCMV), Epstein-Barr virus, herpes simplex virus 1 (HSV-1), herpes simplex virus 2 (HSV-2), human herpesvirus 6 (HHV-6), Herpesvitus K*, CMV, varicella-zoster virus), Papillomaviridae family (e.g., Human Papillomavirus (HPV, HPV E1)), Parvoviridae family (e.g., Parvovirus B19), Polyomaviridae family (e.g., JC virus and BK virus), Paramyxoviridae family (e.g., Measles virus), Togaviridae family (e.g., Rubella virus). In some embodiments, the disorder is Coffin Siris, Neurofibromatosis (e.g., NF-1, NF-2, or Schwannomatosis), or Multiple Meningioma.

In another aspect, the disclosure provides a method for treating a viral infection in a subject in need thereof. This method includes administering to the subject an effective amount of any of the foregoing compounds (e.g., a BRM/BRG1 dual inhibitor compound or a BRM-selective compound), or pharmaceutically acceptable salts thereof, or any of the foregoing pharmaceutical compositions. In some instances, the viral infection is an infection with a virus of the Retroviridae family such as the lentiviruses (e.g., Human immunodeficiency virus (HIV) and deltaretroviruses (e.g., human T cell leukemia virus I (HTLV-I), human T cell leukemia virus II (HTLV-II)), Hepadnaviridae family (e.g., hepatitis B virus (HBV)), Flaviviridae family (e.g., hepatitis C virus (HCV)), Adenoviridae family (e.g., Human Adenovirus), Herpesviridae family (e.g., Human cytomegalovirus (HCMV), Epstein-Barr virus, herpes simplex virus 1 (HSV-1), herpes simplex virus 2 (HSV-2), human herpesvirus 6 (HHV-6), Herpesvitus K*, CMV, varicella-zoster virus), Papillomaviridae family (e.g., Human Papillomavirus (HPV, HPV E1)), Parvoviridae family (e.g., Parvovirus B19), Polyomaviridae family (e.g., JC virus and BK virus), Paramyxoviridae family (e.g., Measles virus), or Togaviridae family (e.g., Rubella virus).

In some instances of any of the foregoing aspects, the compound is a BRM-selective compound. In some instances, the BRM-selective compound inhibits the level and/or activity of BRM at least 10-fold greater than the compound inhibits the level and/or activity of BRG1 and/or the compound binds to BRM at least 10-fold greater than the compound binds to BRG1. For example, in some instances, a BRM-selective compound has an IC₅₀ or IP₅₀ that is at least 10-fold lower than the IC₅₀ or IP₅₀ against BRG1. In some instances of any of the foregoing aspects, the compound is a BRM/BRG1 dual inhibitor compound. In some instances, the BRM/BRG1 dual inhibitor compound has similar activity against both BRM and BRG1 (e.g., the activity of the compound against BRM and BRG1 with within 10-fold (e.g., less than 5-fold, less than 2-fold). In some instances, the activity of the BRM/BRG1 dual inhibitor compound is greater against BRM. In some embodiments, the activity of the BRM/BRG1 dual inhibitor compound is greater against BRG1. For example, in some instances, a BRM/BRG1 dual inhibitor compound has an IC₅₀ or IP₅₀ against BRM that is within 10-fold of the IC₅₀ or IP₅₀ against BRG1.

The compounds and pharmaceutical compositions can be used in a method of treating melanoma, prostate cancer, breast cancer, bone cancer, renal cell carcinoma, or a hematologic cancer in a subject in need thereof, the method including administering to the subject an effective amount of any of the foregoing compounds or pharmaceutical compositions thereof.

The compounds and pharmaceutical compositions can be used in a method of reducing tumor growth of melanoma, prostate cancer, breast cancer, bone cancer, renal cell carcinoma, or a hematologic cancer in a subject in need thereof, the method including administering to the subject an effective amount of any of the foregoing compounds or pharmaceutical compositions thereof.

The compounds and pharmaceutical compositions can be used in a method of suppressing metastatic progression of melanoma, prostate cancer, breast cancer, bone cancer, renal cell carcinoma, or a hematologic cancer in a subject, the method including administering an effective amount of any of the foregoing compounds or pharmaceutical compositions thereof.

The compounds and pharmaceutical compositions can be used in a method of suppressing metastatic colonization of melanoma, prostate cancer, breast cancer, bone cancer, renal cell carcinoma, or a hematologic cancer in a subject, the method including administering an effective amount of any of the foregoing compounds or pharmaceutical compositions thereof.

The compounds and pharmaceutical compositions can be used in a method of reducing the level and/or activity of BRG1 and/or BRM in a melanoma, prostate cancer, breast cancer, bone cancer, renal cell carcinoma, or hematologic cancer cell, the method including contacting the cell with an effective amount of any of the foregoing compounds or pharmaceutical compositions thereof.

In some instances of any of the above aspects, the melanoma, prostate cancer, breast cancer, bone cancer, renal cell carcinoma, or hematologic cell is in a subject.

In some instances of any of the above aspects, the effective amount of the compound reduces the level and/or activity of BRG1 by at least 5% (e.g., 6%, 7%, 8%, 9%, 10%, 15%, 20%, 25%, 30%, 35%, 40%, 45%, 50%, 55%, 60%, 65%, 70%, 75%, 80%, 85%, 90%, or 95%) as compared to a reference. In some instances, the effective amount of the compound that reduces the level and/or activity of BRG1 by at least 50% (e.g., 55%, 60%, 65%, 70%, 75%, 80%, 85%, 90%, or 95%) as compared to a reference. In some instances, the effective amount of the compound that reduces the level and/or activity of BRG1 by at least 90% (e.g., 91%, 92%, 93%, 94%, 95%, 96%, 97%, 98%, or 99%).

In some instances, the effective amount of the compound reduces the level and/or activity of BRG1 by at least 5% (e.g., 6%, 7%, 8%, 9%, 10%, 15%, 20%, 25%, 30%, 35%, 40%, 45%, 50%, 55%, 60%, 65%, 70%, 75%, 80%, 85%, 90%, or 95%) as compared to a reference for at least 12 hours (e.g., 14 hours, 16 hours, 18 hours, 20 hours, 22 hours, 24 hours, 30 hours, 36 hours, 48 hours, 72 hours, or more). In some instances, the effective amount of the compound that reduces the level and/or activity of BRG1 by at least 5% (e.g., 6%, 7%, 8%, 9%, 10%, 15%, 20%, 25%, 30%, 35%, 40%, 45%, 50%, 55%, 60%, 65%, 70%, 75%, 80%, 85%, 90%, or 95%) as compared to a reference for at least 4 days (e.g., 5 days, 6 days, 7 days, 14 days, 28 days, or more).

In some instances of any of the above aspects, the effective amount of the compound reduces the level and/or activity of BRM by at least 5% (e.g., 6%, 7%, 8%, 9%, 10%, 15%, 20%, 25%, 30%, 35%, 40%, 45%, 50%, 55%, 60%, 65%, 70%, 75%, 80%, 85%, 90%, or 95%) as compared to a reference. In some instances, the effective amount of the compound that reduces the level and/or activity of BRM by at least 50% (e.g., 55%, 60%, 65%, 70%, 75%, 80%, 85%, 90%, or 95%) as compared to a reference. In some instances, the effective amount of the compound that reduces the level and/or activity of BRM by at least 90% (e.g., 91%, 92%, 93%, 94%, 95%, 96%, 97%, 98%, or 99%).

In some instances, the effective amount of the compound reduces the level and/or activity of BRM by at least 5% (e.g., 6%, 7%, 8%, 9%, 10%, 15%, 20%, 25%, 30%, 35%, 40%, 45%, 50%, 55%, 60%, 65%, 70%, 75%, 80%, 85%, 90%, or 95%) as compared to a reference for at least 12 hours (e.g., 14 hours, 16 hours, 18 hours, 20 hours, 22 hours, 24 hours, 30 hours, 36 hours, 48 hours, 72 hours, or more). In some instances, the effective amount of the compound that reduces the level and/or activity of BRM by at least 5% (e.g., 6%, 7%, 8%, 9%, 10%, 15%, 20%, 25%, 30%, 35%, 40%, 45%, 50%, 55%, 60%, 65%, 70%, 75%, 80%, 85%, 90%, or 95%) as compared to a reference for at least 4 days (e.g., 5 days, 6 days, 7 days, 14 days, 28 days, or more).

In some instances, the subject has cancer. In some instances, the cancer expresses BRG1 and/or BRM protein and/or the cell or subject has been identified as expressing BRG1 and/or BRM. In some instances, the cancer expresses BRG1 protein and/or the cell or subject has been identified as expressing BRG1. In some instances, the cancer expresses BRM protein and/or the cell or subject has been identified as expressing BRM. In some instances, the cancer is melanoma (e.g., uveal melanoma, mucosal melanoma, or cutaneous melanoma). In some instances, the cancer is prostate cancer. In some instances, the cancer is a hematologic cancer, e.g., multiple myeloma, large cell lymphoma, acute T-cell leukemia, acute myeloid leukemia, myelodysplastic syndrome, immunoglobulin A lambda myeloma, diffuse mixed histiocytic and lymphocytic lymphoma, B-cell lymphoma, acute lymphoblastic leukemia (e.g., T-cell acute lymphoblastic leukemia or B-cell acute lymphoblastic leukemia), diffuse large cell lymphoma, or non-Hodgkin's lymphoma. In some instances, the cancer is breast cancer (e.g., an ER positive breast cancer, an ER negative breast cancer, triple positive breast cancer, or triple negative breast cancer). In some instances, the cancer is a bone cancer (e.g., Ewing's sarcoma). In some instances, the cancer is a renal cell carcinoma (e.g., a Microphthalmia Transcription Factor (MITF) family translocation renal cell carcinoma (tRCC)). In some instances, the cancer is metastatic (e.g., the cancer has spread to the liver). The metastatic cancer can include cells exhibiting migration and/or invasion of migrating cells and/or include cells exhibiting endothelial recruitment and/or angiogenesis. In other instances, the migrating cancer is a cell migration cancer. In still other instances, the cell migration cancer is a non-metastatic cell migration cancer. The metastatic cancer can be a cancer spread via seeding the surface of the peritoneal, pleural, pericardial, or subarachnoid spaces. Alternatively, the metastatic cancer can be a cancer spread via the lymphatic system, or a cancer spread hematogenously. In some instances, the effective amount of an agent that reduces the level and/or activity of BRG1 and/or BRM is an amount effective to inhibit metastatic colonization of the cancer to the liver.

In some instances the cancer harbors a mutation in GNAQ. In some instances the cancer harbors a mutation in GNA11. In some instances the cancer harbors a mutation in PLCB4. In some instances the cancer harbors a mutation in CYSLTR2. In some instances the cancer harbors a mutation in BAP1. In some instances the cancer harbors a mutation in SF3B1. In some instances the cancer harbors a mutation in EIF1AX. In some instances the cancer harbors a TFE3 translocation. In some instances the cancer harbors a TFEB translocation. In some instances the cancer harbors a MITF translocation. In some instances the cancer harbors an EZH2 mutation. In some instances the cancer harbors a SUZ12 mutation. In some instances the cancer harbors an EED mutation.

In some instances, the method further includes administering to the subject or contacting the cell with an anticancer therapy, e.g., a chemotherapeutic or cytotoxic agent, immunotherapy, surgery, radiotherapy, thermotherapy, or photocoagulation. In some instances, the anticancer therapy is a chemotherapeutic or cytotoxic agent, e.g., an antimetabolite, antimitotic, antitumor antibiotic, asparagine-specific enzyme, bisphosphonates, antineoplastic, alkylating agent, DNA-Repair enzyme inhibitor, histone deacetylase inhibitor, corticosteroid, demethylating agent, immunomodulatory, janus-associated kinase inhibitor, phosphinositide 3-kinase inhibitor, proteasome inhibitor, or tyrosine kinase inhibitor.

In some instances, the compound of the invention is used in combination with another anti-cancer therapy used for the treatment of uveal melanoma such as surgery, a MEK inhibitor, and/or a PKC inhibitor. For example, in some instances, the method further comprises performing surgery prior to, subsequent to, or at the same time as administration of the compound of the invention. In some instances, the method further comprises administration of a MEK inhibitor and/or a PKC inhibitor prior to, subsequent to, or at the same time as administration of the compound of the invention.

In some instances, the anticancer therapy and the compound of the invention are administered within 28 days of each other and each in an amount that together are effective to treat the subject.

In some instances, the subject or cancer has and/or has been identified as having a BRG1 loss of function mutation.

In some instances, the cancer is resistant to one or more chemotherapeutic or cytotoxic agents (e.g., the cancer has been determined to be resistant to chemotherapeutic or cytotoxic agents such as by genetic markers, or is likely to be resistant, to chemotherapeutic or cytotoxic agents such as a cancer that has failed to respond to a chemotherapeutic or cytotoxic agent). In some instances, the cancer has failed to respond to one or more chemotherapeutic or cytotoxic agents. In some instances, the cancer is resistant or has failed to respond to dacarbazine, temozolomide, cisplatin, treosulfan, fotemustine, IMCgp100, a CTLA-4 inhibitor (e.g., ipilimumab), a PD-1 inhibitor (e.g., Nivolumab or pembrolizumab), a PD-L1 inhibitor (e.g., atezolizumab, avelumab, or durvalumab), a mitogen-activated protein kinase (MEK) inhibitor (e.g., selumetinib, binimetinib, or tametinib), and/or a protein kinase C (PKC) inhibitor (e.g., sotrastaurin or IDE196).

In some instances, the cancer is resistant to or failed to respond to a previously administered therapeutic used for the treatment of uveal melanoma such as a MEK inhibitor or PKC inhibitor. For example, in some instances, the cancer is resistant to or failed to respond to a mitogen-activated protein kinase (MEK) inhibitor (e.g., selumetinib, binimetinib, or tametinib), and/or a protein kinase C (PKC) inhibitor (e.g., sotrastaurin or IDE196).

In an aspect, the invention provides a compound disclosed herein, or a pharmaceutically acceptable salt thereof, or a pharmaceutical composition disclosed herein, for use in therapy.

In some embodiments, the cancer is non-small cell lung cancer. In some embodiments, the cancer is soft tissue sarcoma.

In an aspect, a compound disclosed herein, or a pharmaceutically acceptable salt thereof, or a pharmaceutical composition disclosed herein, can be for use in treating a cancer selected from the group consisting of melanoma, prostate cancer, breast cancer, bone cancer, renal cell carcinoma, and a hematologic cancer.

In an aspect, a compound disclosed herein, or a pharmaceutically acceptable salt thereof, or a pharmaceutical composition disclosed herein, can be for use in reducing tumor growth of a

cancer selected from the group consisting of melanoma, prostate cancer, breast cancer, bone cancer, renal cell carcinoma, and a hematologic cancer.

In an aspect, a compound disclosed herein, or a pharmaceutically acceptable salt thereof, or a pharmaceutical composition disclosed herein, can be for use in suppressing metastatic progression of a cancer selected from the group consisting of melanoma, prostate cancer, breast cancer, bone cancer, renal cell carcinoma, and a hematologic cancer.

In an aspect, a compound disclosed herein, or a pharmaceutically acceptable salt thereof, or a pharmaceutical composition disclosed herein, can be for use in suppressing metastatic colonization of a cancer selected from the group consisting of melanoma, prostate cancer, breast cancer, bone cancer, renal cell carcinoma, and a hematologic cancer.

In an aspect, a compound disclosed herein, or a pharmaceutically acceptable salt thereof, or a pharmaceutical composition disclosed herein, can be for use in reducing the level and/or activity of BRM in a cancer cell selected from the group consisting of melanoma, prostate cancer, breast cancer, bone cancer, renal cell carcinoma, and hematologic cancer.

In some instances, the cell is in a subject.

In some instances, the cancer is metastatic.

In some instances, the use further includes an anticancer therapy.

In some instances, the anticancer therapy is a chemotherapeutic or cytotoxic agent, immunotherapy, surgery, radiotherapy, thermotherapy, or photocoagulation.

In some instances, the anticancer therapy is surgery.

In some instances, the anticancer therapy is a chemotherapeutic or cytotoxic agent.

In some instances, the chemotherapeutic or cytotoxic agent is an antimetabolite, antimitotic, antitumor antibiotic, asparagine-specific enzyme, bisphosphonates, antineoplastic, alkylating agent, DNA-Repair enzyme inhibitor, histone deacetylase inhibitor, corticosteroid, demethylating agent, immunomodulatory, janus-associated kinase inhibitor, phosphinositide 3-kinase inhibitor, proteasome inhibitor, or tyrosine kinase inhibitor.

In some instances, the one or more chemotherapeutic or cytotoxic agent is dacarbazine, temozolomide, cisplatin, treosulfan, fotemustine, IMCgp100, a CTLA-4 inhibitor, a PD-1 inhibitor, a PD-L1 inhibitor, a mitogen-activated protein kinase inhibitor, and/or a protein kinase C inhibitor.

In some instances, the anticancer therapy and the compound, or a pharmaceutical composition thereof, are administered within 28 days of each other and each in an amount that together are effective to treat the subject.

In some instances, the subject or cancer has and/or has been identified as having a BRG1 loss of function mutation.

In some instances, the cancer has failed to respond to or progressed after administration of one or more chemotherapeutic or cytotoxic agents.

In some instances, the cancer is resistant to, or predicted to be resistant to one or more chemotherapeutic agents.

In some instances, the one or more chemotherapeutic or cytotoxic agents is dacarbazine, temozolomide, cisplatin, treosulfan, fotemustine, IMCgp100, a CTLA-4 inhibitor, a PD-1 inhibitor, a PD-L1 inhibitor, a mitogen-activated protein kinase inhibitor, and/or a protein kinase C inhibitor.

In some embodiments, the cancer is melanoma. In some embodiments, the melanoma is uveal melanoma. In some embodiments, the melanoma is mucosal melanoma. In some embodiments, the melanoma is cutaneous melanoma. In some embodiments, the cancer is a hematologic cancer. In some embodiments, the hematologic cancer is multiple myeloma, large cell lymphoma, acute T-cell leukemia, acute myeloid leukemia, myelodysplastic syndrome, immunoglobulin A lambda myeloma, diffuse mixed histiocytic and lymphocytic lymphoma, B-cell lymphoma, acute lymphoblastic leukemia, diffuse large cell lymphoma, or non-Hodgkin's lymphoma. In some embodiments, the cancer is prostate cancer. In some embodiments, the cancer is breast cancer. In some embodiments, the breast cancer is an ER positive breast cancer, an ER negative breast cancer, triple positive breast cancer, or triple negative breast cancer. In some embodiments, the cancer is bone cancer. In some embodiments, the bone cancer is Ewing's sarcoma. In some embodiments, the cancer is renal cell carcinoma. In some embodiments, the renal cell carcinoma is Microphthalmia Transcription Factor (MITF) family translocation renal cell carcinoma.

A compound disclosed herein, or a pharmaceutically acceptable salt thereof, or a pharmaceutical composition disclosed herein, can be for use in treating a viral infection.

In some instances, the viral infection is an infection with a virus of the Retroviridae family, Hepadnaviridae family, Flaviviridae family, Adenoviridae family, Herpesviridae family, Papillomaviridae family, Parvoviridae family, Polyomaviridae family, Paramyxoviridae family, or Togaviridae family.

### Chemical Terms

The terminology employed herein is for the purpose of describing particular embodiments and is not intended to be limiting.

For any of the following chemical definitions, a number following an atomic symbol indicates that total number of atoms of that element that are present in a particular chemical moiety. As will be understood, other atoms, such as H atoms, or substituent groups, as described herein, may be present, as necessary, to satisfy the valences of the atoms. For example, an unsubstituted C₂ alkyl group has the formula -CH₂CH₃. When used with the groups defined herein, a reference to the number of carbon atoms includes the divalent carbon in acetal and ketal groups but does not include the carbonyl carbon in acyl, ester, carbonate, or carbamate groups. A reference to the number of oxygen, nitrogen, or sulfur atoms in a heteroaryl group only includes those atoms that form a part of a heterocyclic ring.

The term "acyl," as used herein, represents a H or an alkyl group that is attached to a parent molecular group through a carbonyl group, as defined herein, and is exemplified by formyl (i.e., a carboxaldehyde group), acetyl, trifluoroacetyl, propionyl, and butanoyl. Exemplary unsubstituted acyl groups include from 1 to 6, from 1 to 11, or from 1 to 21 carbons.

The term "alkyl," as used herein, refers to a branched or straight-chain monovalent saturated aliphatic hydrocarbon radical of 1 to 20 carbon atoms (e.g., 1 to 16 carbon atoms, 1 to 10 carbon atoms, 1 to 6 carbon atoms, or 1 to 3 carbon atoms).

An alkylene is a divalent alkyl group. The term "alkenyl," as used herein, alone or in combination with other groups, refers to a straight chain or branched hydrocarbon residue having a carbon-carbon double bond and having 2 to 20 carbon atoms (e.g., 2 to 16 carbon atoms, 2 to 10 carbon atoms, 2 to 6 carbon atoms, or 2 carbon atoms).

The term "alkynyl," as used herein, alone or in combination with other groups, refers to a straight chain or branched hydrocarbon residue having a carbon-carbon triple bond and having 2 to 20 carbon atoms (e.g., 2 to 16 carbon atoms, 2 to 10 carbon atoms, 2 to 6 carbon atoms, or 2 carbon atoms).

The term "amino," as used herein, represents -N(R^{N1})₂, wherein each R^{N1} is, independently, H, OH, NO₂, N(R^{N2})₂, SO₂OR^{N2}, SO₂R^{N2}, SOR^{N2}, an N-protecting group, alkyl, alkoxy, aryl, arylalkyl, cycloalkyl, acyl (e.g., acetyl, trifluoroacetyl, or others described herein), wherein each of these recited R^{N1} groups can be optionally substituted; or two R^{N1} combine to form an alkylene or heteroalkylene, and wherein each R^{N2} is, independently, H, alkyl, or aryl. The amino groups of the invention can be an unsubstituted amino (i.e., -NH₂) or a substituted amino (i.e., -N(R^{N1})₂).

The term "aryl," as used herein, refers to an aromatic mono- or polycarbocyclic radical of 6 to 12 carbon atoms having at least one aromatic ring. When polycyclic, the aryl group contains 2 or 3 rings. Examples of such groups include, but are not limited to, phenyl, naphthyl, 1,2,3,4-tetrahydronaphthyl, 1,2-dihydronaphthyl, indanyl, and 1H-indenyl.

The term "arylalkyl," as used herein, represents an alkyl group substituted with an aryl group. Unsubstituted arylalkyl groups contain from 7 to 30 carbons (e.g., from 7 to 16 or from 7 to 20 carbons, such as C₁-C₆ alkyl C₆-C₁₀ aryl, C₁-C₁₀ alkyl C₆-C₁₀ aryl, or C₁-C₂₀ alkyl C₆-C₁₀ aryl), such as, benzyl and phenethyl. In some embodiments, the alkyl and the aryl each are further substituted with 1, 2, 3, or 4 substituent groups, valency permitting, as defined herein for the respective groups.

The term "azido," as used herein, represents a -N₃ group.

The term "bridged polycycloalkyl," as used herein, refers to a bridged polycyclic group of 5 to 20 carbons, containing from 1 to 3 bridges. A bridged polycycloalkyl group may be unsubstituted or substituted as defined herein for cycloalkyl.

The term "cyano," as used herein, represents a -CN group.

The term "carbocyclyl," as used herein, refers to a non-aromatic C₃-C₁₂ monocyclic, bicyclic, or tricyclic structure in which the rings are formed by carbon atoms. Carbocyclyl structures include cycloalkyl groups and unsaturated carbocyclyl radicals.

The term "cycloalkyl," as used herein, refers to a saturated, non-aromatic, and monovalent mono- di-, or tricyclic radical of 3 to 10, preferably 3 to 6 carbon atoms. The cycloalkyl group may be fully saturated or contain 1 or more double or triple bonds, provided that no ring is aromatic. This term is further exemplified by radicals such as cyclopropyl, cyclobutyl, cyclopentyl, cyclohexyl, cycloheptyl, norbornyl, and adamantyl. The term "CH₂-cycloalkyl" as used herein, refers to cycloalkyl-CH₂- groups (e.g., cyclopropylmethyl and cyclobutylmethyl).

The term "halo," as used herein, means a fluorine (fluoro), chlorine (chloro), bromine (bromo), or iodine (iodo) radical.

The term "heteroalkyl," as used herein, refers to an alkyl group, as defined herein, in which one or more of the constituent carbon atoms have been replaced by nitrogen, oxygen, or sulfur. In some embodiments, the heteroalkyl group is further substituted with 1, 2, 3, or 4 substituent groups as described herein for alkyl groups. Examples of heteroalkyl groups are an "alkoxy" which, as used herein, refers alkyl-O- (e.g., methoxy and ethoxy). A heteroalkylene is a divalent heteroalkyl group. The term "heteroalkenyl," as used herein, refers to an alkenyl group, as defined herein, in which one or more of the constituent carbon atoms have been replaced by nitrogen, oxygen, or sulfur. In some embodiments, the heteroalkenyl group is further substituted with 1, 2, 3, or 4 substituent groups, valency permitting, as described herein for alkenyl groups. Examples of heteroalkenyl groups are an "alkenoxy" which, as used herein, refers alkenyl-O-. A heteroalkenylene is a divalent heteroalkenyl group. The term "heteroalkynyl," as used herein, refers to an alkynyl group, as defined herein, in which one or more of the constituent carbon atoms have been replaced by nitrogen, oxygen, or sulfur. In some embodiments, the heteroalkynyl group is further substituted with 1, 2, 3, or 4 substituent groups, valency permitting, as described herein for alkynyl groups. Examples of heteroalkynyl groups are an "alkynoxy" which, as used herein, refers alkynyl-O-. A heteroalkynylene is a divalent heteroalkynyl group.

The term "heteroaryl," as used herein, refers to a monocyclic, bicyclic, or tricyclic radical of 5 to 12 atoms having at least one aromatic ring and containing 1, 2, or 3 ring atoms selected from nitrogen, oxygen, and sulfur, with the remaining ring atoms being carbon. One or two ring carbon atoms of the heteroaryl group may be replaced with a carbonyl group. Examples of heteroaryl groups are pyridyl, pyrazoyl, benzooxazolyl, benzoimidazolyl, benzothiazolyl, imidazolyl, oxazolyl, and thiazolyl.

The term "heteroarylalkyl," as used herein, represents an alkyl group substituted with a heteroaryl group. Unsubstituted heteroarylalkyl groups contain from 7 to 30 carbons (e.g., from 7 to 16 or from 7 to 20 carbons, such as C₁-C₆ alkyl C₂-C₉ heteroaryl, C₁-C₁₀ alkyl C₂-C₉ heteroaryl, or C₁-C₂₀ alkyl C₂-C₉ heteroaryl). In some embodiments, the alkyl and the heteroaryl each are further substituted with 1, 2, 3, or 4 substituent groups, valency permitting, as defined herein for the respective groups.

The term "heterocyclyl," as used herein, refers a monocyclic, bicyclic, or tricyclic radical having 3 to 12 atoms having at least one ring containing 1, 2, 3, or 4 ring atoms selected from N, O or S, wherein no ring is aromatic. Examples of heterocyclyl groups include, but are not limited to, morpholinyl, thiomorpholinyl, furyl, piperazinyl, piperidinyl, pyranyl, pyrrolidinyl, tetrahydropyranyl, tetrahydrofuranyl, and 1,3-dioxanyl.

The term "heterocyclylalkyl," as used herein, represents an alkyl group substituted with a heterocyclyl group. Unsubstituted heterocyclylalkyl groups contain from 7 to 30 carbons (e.g., from 7 to 16 or from 7 to 20 carbons, such as C₁-C₆ alkyl C₂-C₉ heterocyclyl, C₁-C₁₀ alkyl C₂-C₉ heterocyclyl, or C₁-C₂₀ alkyl C₂-C₉ heterocyclyl). In some embodiments, the alkyl and the heterocyclyl each are further substituted with 1, 2, 3, or 4 substituent groups as defined herein for the respective groups.

The term "hydroxyalkyl," as used herein, represents an alkyl group substituted with an - OH group.

The term "hydroxyl," as used herein, represents an -OH group.

The term "*N*-protecting group," as used herein, represents those groups intended to protect an amino group against undesirable reactions during synthetic procedures. Commonly used *N*-protecting groups are disclosed in Greene, "Protective Groups in Organic Synthesis," 3rd Edition (John Wiley & Sons, New York, 1999). *N*-protecting groups include, but are not limited to, acyl, aryloyl, or carbamyl groups such as formyl, acetyl, propionyl, pivaloyl, t-butylacetyl, 2-chloroacetyl, 2-bromoacetyl, trifluoroacetyl, trichloroacetyl, phthalyl, o-nitrophenoxyacetyl, α-chlorobutyryl, benzoyl, 4-chlorobenzoyl, 4-bromobenzoyl, 4-nitrobenzoyl, and chiral auxiliaries such as protected or unprotected D, L, or D, L-amino acids such as alanine, leucine, and phenylalanine; sulfonyl-containing groups such as benzenesulfonyl, and p-toluenesulfonyl; carbamate forming groups such as benzyloxycarbonyl, p-chlorobenzyloxycarbonyl, p-methoxybenzyloxycarbonyl, p-nitrobenzyloxycarbonyl, 2-nitrobenzyloxycarbonyl, p-bromobenzyloxycarbonyl, 3,4-dimethoxybenzyloxycarbonyl, 3,5-dimethoxybenzyloxycarbonyl, 2,4- 20 dimethoxybenzyloxycarbonyl, 4-methoxybenzyloxycarbonyl, 2-nitro-4,5-dimethoxybenzyloxycarbonyl, 3,4,5-trimethoxybenzyloxycarbonyl, 1-(p-biphenylyl)-1-methylethoxycarbonyl, α,α-dimethyl-3,5-dimethoxybenzyloxycarbonyl, benzhydryloxy carbonyl, t-butyloxycarbonyl, diisopropylmethoxycarbonyl, isopropyloxycarbonyl, ethoxycarbonyl, methoxycarbonyl, allyloxycarbonyl, 2,2,2,-trichloroethoxycarbonyl, phenoxycarbonyl, 4-nitrophenoxy carbonyl, fluorenyl-9-methoxycarbonyl, cyclopentyloxycarbonyl, adamantyloxycarbonyl, cyclohexyloxycarbonyl, and phenylthiocarbonyl, arylalkyl groups such as benzyl, triphenylmethyl, and benzyloxymethyl, and silyl groups, such as trimethylsilyl. Preferred N-protecting groups are alloc, formyl, acetyl, benzoyl, pivaloyl, t-butylacetyl, alanyl, phenylsulfonyl, benzyl, t-butyloxycarbonyl (Boc), and benzyloxycarbonyl (Cbz).

The term "nitro," as used herein, represents an -NO₂ group.

The term "oxo," as used herein, represents a divalent oxygen atom (e.g., the structure of oxo may be shown as =O). For example, a carbonyl group is a carbon (e.g., alkyl carbon, alkenyl carbon, alkynyl carbon, heteroalkyl carbon, heteroalkenyl carbon, heteroalkynyl carbon, carbocyclyl carbon, etc.) substituted with oxo. Alternatively, sulfur may be substituted with one or two oxo groups (e.g., -SO- or -SO₂- within a substituted heteroalkyl, heteroalkenyl, heteroalkynyl, or heterocyclyl group).

The term "thiol," as used herein, represents an -SH group.

The alkyl, alkenyl, alkynyl, heteroalkyl, heteroalkenyl, heteroalkynyl, carbocyclyl (e.g., cycloalkyl), aryl, heteroaryl, and heterocyclyl groups may be substituted or unsubstituted. When substituted, there will be 1, 2, 3, 4, or 5 substituents present, valency permitting, unless otherwise specified. The 1 to 5 substituents are each, independently, selected from the group consisting of acyl, alkyl (e.g., unsubstituted and substituted, where the substituents include any group described herein, e.g., aryl, halo, hydroxy), alkenyl, alkynyl, aryl (e.g., substituted and unsubstituted phenyl), carbocyclyl (e.g., substituted and unsubstituted cycloalkyl), halo (e.g., fluoro), hydroxyl, heteroalkyl (e.g., substituted and unsubstituted methoxy, ethoxy, or thioalkoxy), heteroalkenyl, heteroalkynyl, heteroaryl, heterocyclyl, amino (e.g., NH₂ or mono- or dialkyl amino), azido, cyano, nitro, thiol, and oxo. Each of the substituents is unsubstituted or substituted with unsubstituted substituent(s) as defined herein for each respective group. In some embodiments, alkyl, alkenyl, alkynyl, heteroalkyl, heteroalkenyl, and heteroalkynyl are optionally substituted with 1, 2, 3, 4, or 5 substituents independently selected from the group consisting of aryl (e.g., substituted and unsubstituted phenyl), carbocyclyl (e.g., substituted and unsubstituted cycloalkyl), halo (e.g., fluoro), hydroxyl, heteroaryl, heterocyclyl, amino (e.g., NH₂ or mono- or dialkyl amino), azido, cyano, nitro, thiol, and oxo. Each of the substituents is unsubstituted or substituted with unsubstituted substituent(s) as defined herein for each respective group. In some embodiments, the substituents are themselves unsubstituted.

Compounds of the invention can have one or more asymmetric carbon atoms and can exist in the form of optically pure enantiomers, mixtures of enantiomers such as, for example, racemates, optically pure diastereoisomers, mixtures of diastereoisomers, diastereoisomeric racemates, or mixtures of diastereoisomeric racemates. The optically active forms can be obtained for example by resolution of the racemates, by asymmetric synthesis or asymmetric chromatography (chromatography with a chiral adsorbents or eluant). That is, certain of the disclosed compounds may exist in various stereoisomeric forms. Stereoisomers are compounds that differ only in their spatial arrangement. Enantiomers are pairs of stereoisomers whose mirror images are not superimposable, most commonly because they contain an asymmetrically substituted carbon atom that acts as a chiral center. "Enantiomer" means one of a pair of molecules that are mirror images of each other and are not superimposable. Diastereomers are stereoisomers that are not related as mirror images, most commonly because they contain two or more asymmetrically substituted carbon atoms and represent the configuration of substituents around one or more chiral carbon atoms. Enantiomers of a compound can be prepared, for example, by separating an enantiomer from a racemate using one or more well-known techniques and methods, such as, for example, chiral chromatography and separation methods based thereon. The appropriate technique and/or method for separating an enantiomer of a compound described herein from a racemic mixture can be readily determined by those of skill in the art. "Racemate" or "racemic mixture" means a compound containing two enantiomers, wherein such mixtures exhibit no optical activity; i.e., they do not rotate the plane of polarized light. "Geometric isomer" means isomers that differ in the orientation of substituent atoms in relationship to a carbon-carbon double bond, to a cycloalkyl ring, or to a bridged bicyclic system. Atoms (other than H) on each side of a carbon- carbon double bond may be in an E (substituents are on opposite sides of the carbon- carbon double bond) or Z (substituents are oriented on the same side) configuration. "R," "S," "S*," "R*," "E," "Z," "cis," and "trans," indicate configurations relative to the core molecule. Certain of the disclosed compounds may exist in atropisomeric forms. Atropisomers are stereoisomers resulting from hindered rotation about single bonds where the steric strain barrier to rotation is high enough to allow for the isolation of the conformers. The compounds of the invention may be prepared as individual isomers by either isomer-specific synthesis or resolved from an isomeric mixture. Conventional resolution techniques include forming the salt of a free base of each isomer of an isomeric pair using an optically active acid (followed by fractional crystallization and regeneration of the free base), forming the salt of the acid form of each isomer of an isomeric pair using an optically active amine (followed by fractional crystallization and regeneration of the free acid), forming an ester or amide of each of the isomers of an isomeric pair using an optically pure acid, amine or alcohol (followed by chromatographic separation and removal of the chiral auxiliary), or resolving an isomeric mixture of either a starting material or a final product using various well known chromatographic methods. When the stereochemistry of a disclosed compound is named or depicted by structure, the named or depicted stereoisomer is at least 60%, 70%, 80%, 90%, 99%, or 99.9% by weight relative to the other stereoisomers. When a single enantiomer is named or depicted by structure, the depicted or named enantiomer is at least 60%, 70%, 80%, 90%, 99%, or 99.9% by weight optically pure. When a single diastereomer is named or depicted by structure, the depicted or named diastereomer is at least 60%, 70%, 80%, 90%, 99%, or 99.9% by weight pure. Percent optical purity is the ratio of the weight of the enantiomer or over the weight of the enantiomer plus the weight of its optical isomer. Diastereomeric purity by weight is the ratio of the weight of one diastereomer or over the weight of all the diastereomers. When the stereochemistry of a disclosed compound is named or depicted by structure, the named or depicted stereoisomer is at least 60%, 70%, 80%, 90%, 99%, or 99.9% by mole fraction pure relative to the other stereoisomers. When a single enantiomer is named or depicted by structure, the depicted or named enantiomer is at least 60%, 70%, 80%, 90%, 99%, or 99.9% by mole fraction pure. When a single diastereomer is named or depicted by structure, the depicted or named diastereomer is at least 60%, 70%, 80%, 90%, 99%, or 99.9% by mole fraction pure. Percent purity by mole fraction is the ratio of the moles of the enantiomer or over the moles of the enantiomer plus the moles of its optical isomer. Similarly, percent purity by moles fraction is the ratio of the moles of the diastereomer or over the moles of the diastereomer plus the moles of its isomer. When a disclosed compound is named or depicted by structure without indicating the stereochemistry, and the compound has at least one chiral center, it is to be understood that the name or structure encompasses either enantiomer of the compound free from the corresponding optical isomer, a racemic mixture of the compound, or mixtures enriched in one enantiomer relative to its corresponding optical isomer. When a disclosed compound is named or depicted by structure without indicating the stereochemistry and has two or more chiral centers, it is to be understood that the name or structure encompasses a diastereomer free of other diastereomers, a number of diastereomers free from other diastereomeric pairs, mixtures of diastereomers, mixtures of diastereomeric pairs, mixtures of diastereomers in which one diastereomer is enriched relative to the other diastereomer(s), or mixtures of diastereomers in which one or more diastereomer is enriched relative to the other diastereomers. The invention embraces all of these forms.

Compounds of the present disclosure also include all of the isotopes of the atoms occurring in the intermediate or final compounds. "Isotopes" refers to atoms having the same atomic number but different mass numbers resulting from a different number of neutrons in the nuclei. For example, isotopes of hydrogen include tritium and deuterium.

Unless otherwise stated, structures depicted herein are also meant to include compounds that differ only in the presence of one or more isotopically enriched atoms. Exemplary isotopes that can be incorporated into compounds of the present invention include isotopes of hydrogen, carbon, nitrogen, oxygen, phosphorus, sulfur, fluorine, chlorine, and iodine, such as ²H, ³H, ¹¹C, ¹³C, ¹⁴C, ¹³N, ¹⁵N, ¹⁵O, ¹⁷O, ¹⁸O, ³²P, ³³P, ³⁵S, ¹⁸F, ³⁶Cl, ¹²³I and ¹²⁵I. Isotopically-labeled compounds (e.g., those labeled with ³H and ¹⁴C) can be useful in compound or substrate tissue distribution assays. Tritiated (i.e., ³H) and carbon-14 (i.e., ¹⁴C) isotopes can be useful for their ease of preparation and detectability. Further, substitution with heavier isotopes such as deuterium (i.e., ²H) may afford certain therapeutic advantages resulting from greater metabolic stability (e.g., increased in vivo half-life or reduced dosage requirements). In some embodiments, one or more hydrogen atoms are replaced by ²H or ³H, or one or more carbon atoms are replaced by ¹³C- or ¹⁴C-enriched carbon. Positron emitting isotopes such as ¹⁵O, ¹³N, ¹¹C, and ¹⁸F are useful for positron emission tomography (PET) studies to examine substrate receptor occupancy. Preparations of isotopically labelled compounds are known to those of skill in the art. For example, isotopically labeled compounds can generally be prepared by following procedures analogous to those disclosed for compounds of the present invention described herein, by substituting an isotopically labeled reagent for a non-isotopically labeled reagent.Unless otherwise defined, all technical and scientific terms used herein have the same meaning as commonly understood by one of ordinary skill in the art to which this invention belongs. Methods and materials are described herein for use in the present disclosure; other, suitable methods and materials known in the art can also be used. The materials, methods, and examples are

illustrative only and not intended to be limiting. In case of conflict, the present specification, including definitions, will control.

### Definitions

In this application, unless otherwise clear from context, the term "a" may be understood to mean "at least one", and the terms "comprising" and "including" may be understood to encompass itemized components or steps whether presented by themselves or together with one or more additional components or steps.

As used herein, the terms "about" and "approximately" refer to a value that is within 10% above or below the value being described. For example, the term "about 5 nM" indicates a range of from 4.5 to 5.5 nM.

As used herein, the term "administration" refers to the administration of a composition (e.g., a compound or a preparation that includes a compound as described herein) to a subject or system. Administration to an animal subject (e.g., to a human) may be by any appropriate route. For example, in some embodiments, administration may be bronchial (including by bronchial instillation), buccal, enteral, interdermal, intra-arterial, intradermal, intragastric, intramedullary, intramuscular, intranasal, intraperitoneal, intrathecal, intratumoral, intravenous, intraventricular, mucosal, nasal, oral, rectal, subcutaneous, sublingual, topical, tracheal (including by intratracheal instillation), transdermal, vaginal, and vitreal.

As used herein, the term "BAF complex" refers to the BRG1- or HRBM-associated factors complex in a human cell.

As used herein, the term "BAF complex-related disorder" refers to a disorder that is caused or affected by the level of activity of a BAF complex.

As used herein, the term "BRG1 loss of function mutation" refers to a mutation in BRG1 that leads to the protein having diminished activity (e.g., at least 1% reduction in BRG1 activity, for example 2%, 5%, 10%, 25%, 50%, or 100% reduction in BRG1 activity). Exemplary BRG1 loss of function mutations include, but are not limited to, a homozygous BRG1 mutation and a deletion at the C-terminus of BRG1.

As used herein, the term "BRG1 loss of function disorder" refers to a disorder (e.g., cancer) that exhibits a reduction in BRG1 activity (e.g., at least 1% reduction in BRG1 activity, for example 2%, 5%, 10%, 25%, 50%, or 100% reduction in BRG1 activity).

The term "cancer" refers to a condition caused by the proliferation of malignant neoplastic cells, such as tumors, neoplasms, carcinomas, sarcomas, leukemias, and lymphomas.

As used herein, a "combination therapy" or "administered in combination" means that two (or more) different agents or treatments are administered to a subject as part of a defined treatment regimen for a particular disease or condition. The treatment regimen defines the doses and periodicity of administration of each agent such that the effects of the separate agents on the subject overlap. In some embodiments, the delivery of the two or more agents is simultaneous or concurrent and the agents may be co-formulated. In some embodiments, the two or more agents are not co-formulated and are administered in a sequential manner as part of a prescribed regimen. In some embodiments, administration of two or more agents or treatments in combination is such that the reduction in a symptom, or other parameter related to the disorder is greater than what would be observed with one agent or treatment delivered alone or in the absence of the other. The effect of the two treatments can be partially additive, wholly additive, or greater than additive (e.g., synergistic). Sequential or substantially simultaneous administration of each therapeutic agent can be effected by any appropriate route including, but not limited to, oral routes, intravenous routes, intramuscular routes, and direct absorption through mucous membrane tissues. The therapeutic agents can be administered by the same route or by different routes. For example, a first therapeutic agent of the combination may be administered by intravenous injection while a second therapeutic agent of the combination may be administered orally.

By "determining the level" of a protein or RNA is meant the detection of a protein or an RNA, by methods known in the art, either directly or indirectly. "Directly determining" means performing a process (e.g., performing an assay or test on a sample or "analyzing a sample" as that term is defined herein) to obtain the physical entity or value. "Indirectly determining" refers to receiving the physical entity or value from another party or source (e.g., a third-party laboratory that directly acquired the physical entity or value). Methods to measure protein level generally include, but are not limited to, western blotting, immunoblotting, enzyme-linked immunosorbent assay (ELISA), radioimmunoassay (RIA), immunoprecipitation, immunofluorescence, surface plasmon resonance, chemiluminescence, fluorescent polarization, phosphorescence, immunohistochemical analysis, matrix-assisted laser desorption/ionization time-of-flight (MALDI-TOF) mass spectrometry, liquid chromatography (LC)-mass spectrometry, microcytometry, microscopy, fluorescence activated cell sorting (FACS), and flow cytometry, as well as assays based on a property of a protein including, but not limited to, enzymatic activity or interaction with other protein partners. Methods to measure RNA levels are known in the art and include, but are not limited to, quantitative polymerase chain reaction (qPCR) and Northern blot analyses.

By "decreasing the activity of a BAF complex" is meant decreasing the level of an activity related to a BAF complex, or a related downstream effect. A non-limiting example of decreasing an activity of a BAF complex is Sox2 activation. The activity level of a BAF complex may be measured using any method known in the art, e.g., the methods described in Kadoch et al. Cell, 2013, 153, 71-85.

As used herein, the term "degrader" refers to a small molecule compound including a degradation moiety, wherein the compound interacts with a protein (e.g., BRG1 and/or BRM) in a way which results in degradation of the protein, e.g., binding of the compound results in at least 5% reduction of the level of the protein, e.g., in a cell or subject.

As used herein, the term "degradation moiety" refers to a moiety whose binding results in degradation of a protein, e.g., BRG1 and/or BRM. In one example, the moiety binds to a protease or a ubiquitin ligase that metabolizes the protein, e.g., BRG1 and/or BRM.

By "modulating the activity of a BAF complex," is meant altering the level of an activity related to a BAF complex (e.g., GBAF), or a related downstream effect. The activity level of a BAF complex may be measured using any method known in the art, e.g., the methods described in Kadoch et al, Cell 153:71-85 (2013).

By "reducing the activity of BRG1 and/or BRM," is meant decreasing the level of an activity related to an BRG1 and/or BRM, or a related downstream effect. A non-limiting example of inhibition of an activity of BRG1 and/or BRM is decreasing the level of a BAF complex in a cell. The activity level of BRG1 and/or BRM may be measured using any method known in the art. In some embodiments, an agent which reduces the activity of BRG1 and/or BRM is a small molecule BRG1 and/or BRM degrader.

By "reducing the level of BRG1 and/or BRM," is meant decreasing the level of BRG1 and/or BRM in a cell or subject. The level of BRG1 and/or BRM may be measured using any method known in the art.

By "level" is meant a level of a protein, or mRNA encoding the protein, as compared to a reference. The reference can be any useful reference, as defined herein. By a "decreased level" or an "increased level" of a protein is meant a decrease or increase in protein level, as compared to a reference (e.g., a decrease or an increase by about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 100%, about 150%, about 200%, about 300%, about 400%, about 500%, or more; a decrease or an increase of more than about 10%, about 15%, about 20%, about 50%, about 75%, about 100%, or about 200%, as compared to a reference; a decrease or an increase by less than about 0.01-fold, about 0.02-fold, about 0.1-fold, about 0.3-fold, about 0.5-fold, about 0.8-fold, or less; or an increase by more than about 1.2-fold, about 1.4-fold, about 1.5-fold, about 1.8-fold, about 2.0-fold, about 3.0-fold, about 3.5-fold, about 4.5-fold, about 5.0-fold, about 10-fold, about 15-fold, about 20-fold, about 30-fold, about 40-fold, about 50-fold, about 100-fold, about 1000-fold, or more). A level of a protein may be expressed in mass/vol (e.g., g/dL, mg/mL, µg/mL, ng/mL) or percentage relative to total protein or mRNA in a sample.

As used herein, the term "inhibiting BRM" refers to blocking or reducing the level or activity of the ATPase catalytic binding domain or the bromodomain of the protein. BRM inhibition may be determined using methods known in the art, e.g., a BRM ATPase assay, a Nano DSF assay, or a BRM Luciferase cell assay.

The term "pharmaceutical composition," as used herein, represents a composition containing a compound described herein formulated with a pharmaceutically acceptable excipient and appropriate for administration to a mammal, for example a human. Typically, a pharmaceutical composition is manufactured or sold with the approval of a governmental regulatory agency as part of a therapeutic regimen for the treatment of disease in a mammal. Pharmaceutical compositions can be formulated, for example, for oral administration in unit dosage form (e.g., a tablet, capsule, caplet, gel cap, or syrup); for topical administration (e.g., as a cream, gel, lotion, or ointment); for intravenous administration (e.g., as a sterile solution free of particulate emboli and in a solvent system suitable for intravenous use); or in any other pharmaceutically acceptable formulation.

A "pharmaceutically acceptable excipient," as used herein, refers to any ingredient other than the compounds described herein (for example, a vehicle capable of suspending or dissolving the active compound) and having the properties of being substantially nontoxic and non-inflammatory in a patient. Excipients may include, for example: antiadherents, antioxidants, binders, coatings, compression aids, disintegrants, dyes (colors), emollients, emulsifiers, fillers (diluents), film formers or coatings, flavors, fragrances, glidants (flow enhancers), lubricants, preservatives, printing inks, sorbents, suspending or dispersing agents, sweeteners, and waters of hydration.

As used herein, the term "pharmaceutically acceptable salt" means any pharmaceutically acceptable salt of a compound, for example, any compound of **Formula I** or **II.** Pharmaceutically acceptable salts of any of the compounds described herein may include those that are within the scope of sound medical judgment, suitable for use in contact with the tissues of humans and animals without undue toxicity, irritation, allergic response and are commensurate with a reasonable benefit/risk ratio. Pharmaceutically acceptable salts are well known in the art. For example, pharmaceutically acceptable salts are described in: Berge et al., J. Pharmaceutical Sciences 66:1-19, 1977 and in Pharmaceutical Salts: Properties, Selection, and Use, (Eds. P.H. Stahl and C.G. Wermuth), Wiley-VCH, 2008. The salts can be prepared in situ during the final isolation and purification of the compounds described herein or separately by reacting a free base group with a suitable organic acid.

The compounds of the invention may have ionizable groups so as to be capable of preparation as pharmaceutically acceptable salts. These salts may be acid addition salts involving inorganic or organic acids or the salts may, in the case of acidic forms of the compounds of the invention be prepared from inorganic or organic bases. Frequently, the compounds are prepared or used as pharmaceutically acceptable salts prepared as addition products of pharmaceutically acceptable acids or bases. Suitable pharmaceutically acceptable acids and bases and methods for preparation of the appropriate salts are well-known in the art. Salts may be prepared from pharmaceutically acceptable non-toxic acids and bases including inorganic and organic acids and bases.

By a "reference" is meant any useful reference used to compare protein or RNA levels. The reference can be any sample, standard, standard curve, or level that is used for comparison purposes. The reference can be a normal reference sample or a reference standard or level. A "reference sample" can be, for example, a control, e.g., a predetermined negative control value such as a "normal control" or a prior sample taken from the same subject; a sample from a normal healthy subject, such as a normal cell or normal tissue; a sample (e.g., a cell or tissue) from a subject not having a disease; a sample from a subject that is diagnosed with a disease, but not yet treated with a compound of the invention; a sample from a subject that has been treated by a compound of the invention; or a sample of a purified protein or RNA (e.g., any described herein) at a known normal concentration. By "reference standard or level" is meant a value or number derived from a reference sample. A "normal control value" is a pre-determined value indicative of non-disease state, e.g., a value expected in a healthy control subject. Typically, a normal control value is expressed as a range ("between X and Y"), a high threshold ("no higher than X"), or a low threshold ("no lower than X"). A subject having a measured value within the normal control value for a particular biomarker is typically referred to as "within normal limits" for that biomarker. A normal reference standard or level can be a value or number derived from a normal subject not having a disease or disorder (e.g., cancer); a subject that has been treated with a compound of the invention. In preferred embodiments, the reference sample, standard, or level is matched to the sample subject sample by at least one of the following criteria: age, weight, sex, disease stage, and overall health. A standard curve of levels of a purified protein or RNA, e.g., any described herein, within the normal reference range can also be used as a reference.

As used herein, the term "subject" refers to any organism to which a composition in accordance with the invention may be administered, e.g., for experimental, diagnostic, prophylactic, and/or therapeutic purposes. Typical subjects include any animal (e.g., mammals such as mice, rats, rabbits, non-human primates, and humans). A subject may seek or be in need of treatment, require treatment, be receiving treatment, be receiving treatment in the future, or be a human or animal who is under care by a trained professional for a particular disease or condition.

As used herein, the terms "treat," "treated," or "treating" mean therapeutic treatment or any measures whose object is to slow down (lessen) an undesired physiological condition, disorder, or disease, or obtain beneficial or desired clinical results. Beneficial or desired clinical results include, but are not limited to, alleviation of symptoms; diminishment of the extent of a condition, disorder, or disease; stabilized (i.e., not worsening) state of condition, disorder, or disease; delay in onset or slowing of condition, disorder, or disease progression; amelioration of the condition, disorder, or disease state or remission (whether partial or total); an amelioration of at least one measurable physical parameter, not necessarily discernible by the patient; or enhancement or improvement of condition, disorder, or disease. Treatment includes eliciting a clinically significant response without excessive levels of side effects. Treatment also includes prolonging survival as compared to expected survival if not receiving treatment. Compounds of the invention may also be used to "prophylactically treat" or "prevent" a disorder, for example, in a subject at increased risk of developing the disorder.

The details of one or more embodiments of the invention are set forth in the description below. Other features, objects, and advantages of the invention will be apparent from the description and from the claims.

### Detailed Description

The present disclosure features compounds useful for the inhibition of BRG1 and optionally BRM. These compounds may be used to modulate the activity of a BAF complex, for example, for the treatment of a BAF-related disorder, such as cancer (e.g., BRG1-loss of function disorders). Exemplary compounds described herein include compounds having a structure according to **Formula I or II:** 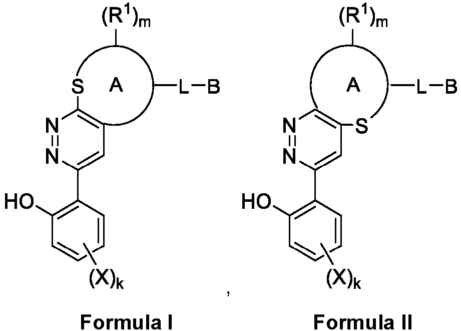 wherein
ring system A is a 5 to 9-membered heterocyclyl or heteroaryl;
m is 0, 1, 2, or 3;
k is 0, 1, or 2;
each R¹ is, independently, halo, optionally substituted C₁-C₆ alkyl, optionally substituted C₁-C₆ heteroalkyl, optionally substituted C₂-C₉ heterocyclyl, optionally substituted C₃-C₈ cycloalkyl or optionally substituted CH₂-C₃-C₈ cycloalkyl;
each X is, independently, halo;
L is a linker of Formula III:

   A¹-(B¹)_{f}-(C¹)_{g}-(B²)ₕ-(B³)ᵢ-(C²)ⱼ-(B⁴)ₖ-A², **Formula III**
wherein
   A¹ is a bond between the linker and ring system A;
   A² is a bond between the degradation moiety and the linker;
   each of B¹, B², B³, and B⁴ is, independently, optionally substituted ethynyl, optionally substituted C₆-C₁₀ aryl, optionally substituted C₃-C₁₀ cycloalkyl, optionally substituted C₂-C₉ heterocyclyl, optionally substituted C₂-C₉ heteroaryl, O, S, S(O)₂, or NR^{N};
   each R^{N} is, independently, H, optionally substituted C₁₋₄ alkyl, optionally substituted C₂₋₄ alkenyl, optionally substituted C₂₋₄ alkynyl, optionally substituted C₂₋₆ heterocyclyl, optionally substituted C₆₋₁₂ aryl, or optionally substituted C₁₋₇ heteroalkyl;
   each of C¹ and C² is, independently, carbonyl, thiocarbonyl, sulphonyl, or phosphoryl; and
   each of f, g, h, i, j, and k is, independently, 0 or 1; and
B is a degradation moiety, wherein the degradation moiety has the structure of Formula C: 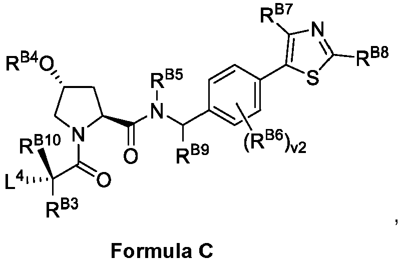
wherein
L⁴ is -N(R^{B1})(R^{B2}), 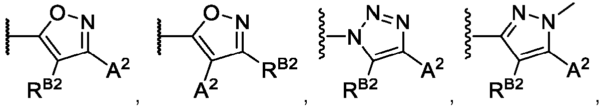 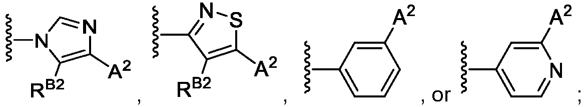
R^{B1} is H, A², optionally substituted C₁-C₆ alkyl, or optionally substituted C₁-C₆ heteroalkyl;
R^{B2} is H, optionally substituted C₁-C₆ alkyl, or optionally substituted C₁-C₆ heteroalkyl;
R^{B3} is A², optionally substituted C₁-C₆ alkyl, optionally substituted C₁-C₆ heteroalkyl, optionally substituted C₃-C₁₀ carbocyclyl, optionally substituted C₆-C₁₀ aryl, optionally substituted C₁-C₆ alkyl C₃-C₁₀ carbocyclyl, or optionally substituted C₁-C₆ alkyl C₆-C₁₀ aryl;
R^{B4} is H, optionally substituted C₁-C₆ alkyl, optionally substituted C₃-C₁₀ carbocyclyl, optionally substituted C₆-C₁₀ aryl, optionally substituted C₁-C₆ alkyl C₃-C₁₀ carbocyclyl, or optionally substituted C₁-C₆ alkyl C₆-C₁₀ aryl;
R^{B5} is H, optionally substituted C₁-C₆ alkyl, or optionally substituted C₁-C₆ heteroalkyl;
v2 is 0, 1, 2, 3, or 4;
each R^{B6} is, independently, A², halogen, optionally substituted C₁-C₆ alkyl, optionally substituted C₂-C₆ alkynyl, optionally substituted C₁-C₆ heteroalkyl, optionally substituted C₃-C₁₀ carbocyclyl, optionally substituted C₂-C₉ heterocyclyl, optionally substituted C₆-C₁₀ aryl, optionally substituted C₂-C₉ heteroaryl, optionally substituted C₂-C₆ alkenyl, optionally substituted C₂-C₆ heteroalkenyl, hydroxy, thiol, cyano, or optionally substituted amino;
each of R^{B7} and R^{B8} is, independently, H, halogen, optionally substituted C₁-C₆ alkyl, or optionally substituted C₆-C₁₀ aryl;
R^{B9} is H or optionally substituted C₁-C₆ alkyl;
R^{B10} is H or F; and
A² is a bond between the degradation moiety and the linker;
wherein one and only one of R^{B1}, R^{B3}, and R^{B6} is A²,
or a pharmaceutically acceptable salt thereof.

Exemplary compounds described herein include compounds wherein each R¹ is independently halo, optionally substituted C₁-C₆ alkyl, optionally substituted C₁-C₆ heteroalkyl, optionally substituted C₃-C₈ cycloalkyl, or optionally substituted C₂-C₉ heterocyclyl, or a pharmaceutically acceptable salt thereof.

In some embodiments, the compound has the structure of any one of compounds 1-103 in Table 1, or pharmaceutically acceptable salt thereof.

Other embodiments, as well as exemplary methods for the synthesis of production of these compounds, are described herein.

### Pharmaceutical Uses

As already indicated, in the present disclosure all references to methods of medical treatment comprising administration of compounds or compositions are to be interpreted as being directed to the compounds or compositions for use in such methods of medical treatment.

The compounds described herein are useful in the methods of the disclosure and, while not bound by theory, are believed to exert their ability to modulate the level, status, and/or activity of a BAF complex, i.e., by inhibiting the activity of the BRG1 and/or BRM proteins within the BAF complex in a mammal. BAF complex-related disorders include, but are not limited to, BRG1 loss of function mutation-related disorders.

An aspect of the present invention relates to methods of treating disorders related to BRG1 loss of function mutations such as cancer (e.g., non-small cell lung cancer, colorectal cancer, bladder cancer, cancer of unknown primary, glioma, breast cancer, melanoma, non-melanoma skin cancer, endometrial cancer, or penile cancer) in a subject in need thereof. In some embodiments, the compound is administered in an amount and for a time effective to result in one or more (e.g., two or more, three or more, four or more) of: (a) reduced tumor size, (b) reduced rate of tumor growth, (c) increased tumor cell death (d) reduced tumor progression, (e) reduced number of metastases, (f) reduced rate of metastasis, (g) decreased tumor recurrence (h) increased survival of subject, (i) increased progression free survival of subject.

Treating cancer can result in a reduction in size or volume of a tumor. For example, after treatment, tumor size is reduced by 5% or greater (e.g., 10%, 20%, 30%, 40%, 50%, 60%, 70%, 80%, 90%, or greater) relative to its size prior to treatment. Size of a tumor may be measured by any reproducible means of measurement. For example, the size of a tumor may be measured as a diameter of the tumor.

Treating cancer may further result in a decrease in number of tumors. For example, after treatment, tumor number is reduced by 5% or greater (e.g., 10%, 20%, 30%, 40%, 50%, 60%, 70%, 80%, 90%, or greater) relative to number prior to treatment. Number of tumors may be measured by any reproducible means of measurement, e.g., the number of tumors may be measured by counting tumors visible to the naked eye or at a specified magnification (e.g., 2x, 3x, 4x, 5x, 10x, or 50x).

Treating cancer can result in a decrease in number of metastatic nodules in other tissues or organs distant from the primary tumor site. For example, after treatment, the number of metastatic nodules is reduced by 5% or greater (e.g., 10%, 20%, 30%, 40%, 50%, 60%, 70%, 80%, 90% or greater) relative to number prior to treatment. The number of metastatic nodules may be measured by any reproducible means of measurement. For example, the number of metastatic nodules may be measured by counting metastatic nodules visible to the naked eye or at a specified magnification (e.g., 2x, 10x, or 50x).

Treating cancer can result in an increase in average survival time of a population of subjects treated according to the present invention in comparison to a population of untreated subjects. For example, the average survival time is increased by more than 30 days (more than 60 days, 90 days, or 120 days). An increase in average survival time of a population may be measured by any reproducible means. An increase in average survival time of a population may be measured, for example, by calculating for a population the average length of survival following initiation of treatment with the compound of the invention. An increase in average survival time of a population may also be measured, for example, by calculating for a population the average length of survival following completion of a first round of treatment with a pharmaceutically acceptable salt of the invention.

Treating cancer can also result in a decrease in the mortality rate of a population of treated subjects in comparison to an untreated population. For example, the mortality rate is decreased by more than 2% (e.g., more than 5%, 10%, or 25%). A decrease in the mortality rate of a population of treated subjects may be measured by any reproducible means, for example, by calculating for a population the average number of disease-related deaths per unit time following initiation of treatment with a pharmaceutically acceptable salt of the invention. A decrease in the mortality rate of a population may also be measured, for example, by calculating for a population the average number of disease-related deaths per unit time following completion of a first round of treatment with a pharmaceutically acceptable salt of the invention.

Exemplary cancers that may be treated by the invention include, but are not limited to, non-small cell lung cancer, small-cell lung cancer, colorectal cancer, bladder cancer, glioma, breast cancer, melanoma, non-melanoma skin cancer, endometrial cancer, esophagogastric cancer, pancreatic cancer, hepatobiliary cancer, soft tissue sarcoma, ovarian cancer, head and neck cancer, renal cell carcinoma, bone cancer, non-Hodgkin lymphoma, prostate cancer, embryonal tumor, germ cell tumor, cervical cancer, thyroid cancer, salivary gland cancer, gastrointestinal neuroendocrine tumor, uterine sarcoma, gastrointestinal stromal tumor, CNS cancer, thymic tumor, Adrenocortical carcinoma, appendiceal cancer, small bowel cancer and penile cancer.

### Combination Formulations and Uses Thereof

The compounds of the invention can be combined with one or more therapeutic agents. In particular, the therapeutic agent can be one that treats or prophylactically treats any cancer described herein.

### Combination Therapies

A compound of the invention can be used alone or in combination with an additional therapeutic agent, e.g., other agents that treat cancer or symptoms associated therewith, or in combination with other types of treatment to treat cancer. In combination treatments, the dosages of one or more of the therapeutic compounds may be reduced from standard dosages when administered alone. For example, doses may be determined empirically from drug combinations and permutations or may be deduced by isobolographic analysis (e.g., Black et al., Neurology 65:S3-S6, 2005). In this case, dosages of the compounds when combined should provide a therapeutic effect.

In some embodiments, the second therapeutic agent is a chemotherapeutic agent (e.g., a cytotoxic agent or other chemical compound useful in the treatment of cancer). These include alkylating agents, antimetabolites, folic acid analogs, pyrimidine analogs, purine analogs and related inhibitors, vinca alkaloids, epipodopyyllotoxins, antibiotics, L-Asparaginase, topoisomerase inhibitors, interferons, platinum coordination complexes, anthracenedione substituted urea, methyl hydrazine derivatives, adrenocortical suppressant, adrenocorticosteroides, progestins, estrogens, antiestrogen, androgens, antiandrogen, and gonadotropin-releasing hormone analog. Also included is 5-fluorouracil (5-FU), leucovorin (LV), irenotecan, oxaliplatin, capecitabine, paclitaxel and doxetaxel. Non-limiting examples of chemotherapeutic agents include alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e.g., calicheamicin, especially calicheamicin gammall and calicheamicin omegall (see, e.g., Agnew, Chem. Intl. Ed Engl. 33:183-186 (1994)); dynemicin, including dynemicin A; bisphosphonates, such as clodronate; an esperamicin; as well as neocarzinostatin chromophore and related chromoprotein enediyne antiobiotic chromophores), aclacinomysins, actinomycin, authramycin, azaserine, bleomycins, cactinomycin, carabicin, caminomycin, carzinophilin, chromomycinis, dactinomycin, daunorubicin, detorubicin, 6-diazo- 5-oxo-L-norleucine, Adriamycin^{®} (doxorubicin, including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5- FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfomithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK^{®} polysaccharide complex (JHS Natural Products, Eugene, Oreg.); razoxane; rhizoxin; sizofuran; spirogermanium; tenuazonic acid; triaziquone; 2,2',2"-trichlorotriethylamine; trichothecenes (especially T- 2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., Taxol^{®} paclitaxel (Bristol-Myers Squibb Oncology, Princeton, N.J.), ABraxane^{®}, cremophor-free, albumin-engineered nanoparticle formulation of paclitaxel (American Pharmaceutical Partners, Schaumberg, III.), and Taxotere^{®} doxetaxel (Rhone-Poulenc Rorer, Antony, France); chloranbucil; Gemzar^{®} gemcitabine; 6-thioguanine; mercaptopurine; methotrexate; platinum coordination complexes such as cisplatin, oxaliplatin and carboplatin; vinblastine; platinum; etoposide (VP-16); ifosfamide; mitoxantrone; vincristine; Navelbine^{®} vinorelbine; novantrone; teniposide; edatrexate; daunomycin; aminopterin; xeloda; ibandronate; irinotecan (e.g., CPT-11); topoisomerase inhibitor RFS 2000; difluoromethylornithine (DMFO); retinoids such as retinoic acid; capecitabine; and pharmaceutically acceptable salts, acids or derivatives of any of the above. Two or more chemotherapeutic agents can be used in a cocktail to be administered in combination with the first therapeutic agent described herein. Suitable dosing regimens of combination chemotherapies are known in the art and described in, for example, Saltz et al. (1999) Proc ASCO 18:233a and Douillard et al. (2000) Lancet 355:1041-7.

In some embodiments, the second therapeutic agent is a therapeutic agent which is a biologic such a cytokine (e.g., interferon or an interleukin (e.g., IL-2)) used in cancer treatment. In some embodiments the biologic is an anti-angiogenic agent, such as an anti-VEGF agent, e.g., bevacizumab (Avastin^{®}). In some embodiments the biologic is an immunoglobulin-based biologic, e.g., a monoclonal antibody (e.g., a humanized antibody, a fully human antibody, an Fc fusion protein or a functional fragment thereof) that agonizes a target to stimulate an anti-cancer response or antagonizes an antigen important for cancer. Such agents include Rituxan (Rituximab); Zenapax (Daclizumab); Simulect (Basiliximab); Synagis (Palivizumab); Remicade (Infliximab); Herceptin (Trastuzumab); Mylotarg (Gemtuzumab ozogamicin); Campath (Alemtuzumab); Zevalin (Ibritumomab tiuxetan); Humira (Adalimumab); Xolair (Omalizumab); Bexxar (Tositumomab-I-131); Raptiva (Efalizumab); Erbitux (Cetuximab); Avastin (Bevacizumab); Tysabri (Natalizumab); Actemra (Tocilizumab); Vectibix (Panitumumab); Lucentis (Ranibizumab); Soliris (Eculizumab); Cimzia (Certolizumab pegol); Simponi (Golimumab); Ilaris (Canakinumab); Stelara (Ustekinumab); Arzerra (Ofatumumab); Prolia (Denosumab); Numax (Motavizumab); ABThrax (Raxibacumab); Benlysta (Belimumab); Yervoy (Ipilimumab); Adcetris (Brentuximab Vedotin); Perjeta (Pertuzumab); Kadcyla (Ado-trastuzumab emtansine); and Gazyva (Obinutuzumab). Also included are antibody-drug conjugates.

The second agent may be a therapeutic agent which is a non-drug treatment. For example, the second therapeutic agent is radiation therapy, cryotherapy, hyperthermia and/or surgical excision of tumor tissue.

The second agent may be a checkpoint inhibitor. In one embodiment, the inhibitor of checkpoint is an inhibitory antibody (e.g., a monospecific antibody such as a monoclonal antibody). The antibody may be, e.g., humanized or fully human. In some embodiments, the inhibitor of checkpoint is a fusion protein, e.g., an Fc-receptor fusion protein. In some embodiments, the inhibitor of checkpoint is an agent, such as an antibody, that interacts with a checkpoint protein. In some embodiments, the inhibitor of checkpoint is an agent, such as an antibody, that interacts with the ligand of a checkpoint protein. In some embodiments, the inhibitor of checkpoint is an inhibitor (e.g., an inhibitory antibody or small molecule inhibitor) of CTLA-4 (e.g., an anti-CTLA4 antibody such as ipilimumab/Yervoy or tremelimumab). In some embodiments, the inhibitor of checkpoint is an inhibitor (e.g., an inhibitory antibody or small molecule inhibitor) of PD-1 (e.g., nivolumab/Opdivo^{®}; pembrolizumab/Keytruda^{®}; pidilizumab/CT-011). In some embodiments, the inhibitor of checkpoint is an inhibitor (e.g., an inhibitory antibody or small molecule inhibitor) of PDL1 (e.g., MPDL3280A/RG7446; MEDI4736; MSB0010718C; BMS 936559). In some embodiments, the inhibitor of checkpoint is an inhibitor (e.g., an inhibitory antibody or Fc fusion or small molecule inhibitor) of PDL2 (e.g., a PDL2/Ig fusion protein such as AMP 224). In some embodiments, the inhibitor of checkpoint is an inhibitor (e.g., an inhibitory antibody or small molecule inhibitor) of B7-H3 (e.g., MGA271), B7-H4, BTLA, HVEM, TIM3, GAL9, LAG3, VISTA, KIR, 2B4, CD160, CGEN-15049, CHK 1, CHK2, A2aR, B-7 family ligands, or a combination thereof.

In any of the combination embodiments described herein, the first and second therapeutic agents are administered simultaneously or sequentially, in either order. The first therapeutic agent may be administered immediately, up to 1 hour, up to 2 hours, up to 3 hours, up to 4 hours, up to 5 hours, up to 6 hours, up to 7 hours, up to, 8 hours, up to 9 hours, up to 10 hours, up to 11 hours, up to 12 hours, up to 13 hours, 14 hours, up to hours 16, up to 17 hours, up 18 hours, up to 19 hours up to 20 hours, up to 21 hours, up to 22 hours, up to 23 hours up to 24 hours or up to 1-7, 1-14, 1-21 or 1-30 days before or after the second therapeutic agent.

### Pharmaceutical Compositions

The compounds of the invention are preferably formulated into pharmaceutical compositions for administration to a mammal, preferably, a human, in a biologically compatible form suitable for administration in vivo. Accordingly, in an aspect, the present invention provides a pharmaceutical composition comprising a compound of the invention in admixture with a suitable diluent, carrier, or excipient.

The compounds of the invention may be used in the form of the free base, in the form of salts, solvates, and as prodrugs. All forms are within the scope of the invention. In accordance with the methods of the invention, the described compounds or salts, solvates, or prodrugs thereof may be administered to a patient in a variety of forms depending on the selected route of administration, as will be understood by those skilled in the art. The compounds of the invention may be administered, for example, by oral, parenteral, buccal, sublingual, nasal, rectal, patch, pump, or transdermal administration and the pharmaceutical compositions formulated accordingly. Parenteral administration includes intravenous, intraperitoneal, subcutaneous, intramuscular, transepithelial, nasal, intrapulmonary, intrathecal, rectal, and topical modes of administration. Parenteral administration may be by continuous infusion over a selected period of time.

A compound of the invention may be orally administered, for example, with an inert diluent or with an assimilable edible carrier, or it may be enclosed in hard- or soft-shell gelatin capsules, or it may be compressed into tablets, or it may be incorporated directly with the food of the diet. For oral therapeutic administration, a compound of the invention may be incorporated with an excipient and used in the form of ingestible tablets, buccal tablets, troches, capsules, elixirs, suspensions, syrups, and wafers. A compound of the invention may also be administered parenterally. Solutions of a compound of the invention can be prepared in water suitably mixed with a surfactant, such as hydroxypropylcellulose. Dispersions can also be prepared in glycerol, liquid polyethylene glycols, DMSO, and mixtures thereof with or without alcohol, and in oils. Under ordinary conditions of storage and use, these preparations may contain a preservative to prevent the growth of microorganisms. Conventional procedures and ingredients for the selection and preparation of suitable formulations are described, for example, in Remington's Pharmaceutical Sciences (2003, 20th ed.) and in The United States Pharmacopeia: The National Formulary (USP 24 NF19), published in 1999. The pharmaceutical forms suitable for injectable use include sterile aqueous solutions or dispersions and sterile powders for the extemporaneous preparation of sterile injectable solutions or dispersions. In all cases the form must be sterile and must be fluid to the extent that may be easily administered via syringe. Compositions for nasal administration may conveniently be formulated as aerosols, drops, gels, and powders. Aerosol formulations typically include a solution or fine suspension of the active substance in a physiologically acceptable aqueous or non-aqueous solvent and are usually presented in single or multidose quantities in sterile form in a sealed container, which can take the form of a cartridge or refill for use with an atomizing device. Alternatively, the sealed container may be a unitary dispensing device, such as a single dose nasal inhaler or an aerosol dispenser fitted with a metering valve which is intended for disposal after use. Where the dosage form comprises an aerosol dispenser, it will contain a propellant, which can be a compressed gas, such as compressed air or an organic propellant, such as fluorochlorohydrocarbon. The aerosol dosage forms can also take the form of a pump-atomizer. Compositions suitable for buccal or sublingual administration include tablets, lozenges, and pastilles, where the active ingredient is formulated with a carrier, such as sugar, acacia, tragacanth, gelatin, and glycerine. Compositions for rectal administration are conveniently in the form of suppositories containing a conventional suppository base, such as cocoa butter. A compound described herein may be administered intratumorally, for example, as an intratumoral injection. Intratumoral injection is injection directly into the tumor vasculature and is specifically contemplated for discrete, solid, accessible tumors. Local, regional, or systemic administration also may be appropriate. A compound described herein may advantageously be contacted by administering an injection or multiple injections to the tumor, spaced for example, at approximately, 1 cm intervals. In the case of surgical intervention, the present invention may be used preoperatively, such as to render an inoperable tumor subject to resection. Continuous administration also may be applied where appropriate, for example, by implanting a catheter into a tumor or into tumor vasculature.

The compounds of the invention may be administered to an animal, e.g., a human, alone or in combination with pharmaceutically acceptable carriers, as noted herein, the proportion of which is determined by the solubility and chemical nature of the compound, chosen route of administration, and standard pharmaceutical practice.

### Dosages

The dosage of the compounds of the invention, and/or compositions comprising a compound of the invention, can vary depending on many factors, such as the pharmacodynamic properties of the compound; the mode of administration; the age, health, and weight of the recipient; the nature and extent of the symptoms; the frequency of the treatment, and the type of concurrent treatment, if any; and the clearance rate of the compound in the animal to be treated. One of skill in the art can determine the appropriate dosage based on the above factors. The compounds of the invention may be administered initially in a suitable dosage that may be adjusted as required, depending on the clinical response. In general, satisfactory results may be obtained when the compounds of the invention are administered to a human at a daily dosage of, for example, between 0.05 mg and 3000 mg (measured as the solid form). Dose ranges include, for example, between 10-1000 mg (e.g., 50-800 mg). In some embodiments, 50, 100, 150, 200, 250, 300, 350, 400, 450, 500, 550, 600, 650, 700, 750, 800, 850, 900, 950, or 1000 mg of the compound is administered.

Alternatively, the dosage amount can be calculated using the body weight of the patient. For example, the dose of a compound, or pharmaceutical composition thereof, administered to a patient may range from 0.1-100 mg/kg (e.g., 0.25-25 mg/kg). In exemplary, non-limiting embodiments, the dose may range from 0.5-5.0 mg/kg (e.g., 0.5, 1.0, 1.5, 2.0, 2.5, 3.0, 3.5, 4.0, 4.5, or 5.0 mg/kg) or from 5.0-20 mg/kg (e.g., 5.5, 6.0, 6.5, 7.0, 7.5, 8.0, 8.5, 9.0, 9.5, 10, 11, 12, 13, 14, 15, 16, 17, 18, 19, or 20 mg/kg).

### EXAMPLES

The following abbreviations are used throughout the Examples below.
Ac acetyl
ACN or MeCN acetonitrile
AcOH acetic acid
Ac₂O acetic anhydride
aq. aqueous
Boc tert-butoxycarbonyl
Bu or n-Bu butyl
CDI 1,1'-carbonyldiimidazole
DCE or 1,2-DCE 1,2-dichloroethane
DCM dichloromethane
DIAD diisopropyl azodicarboxylate
DIPEA or DIEA N.N-diisopropylethylamine
DMAP 4-(dimethylamino)pyridine
DME 1,2-dimethoxyethane
DMF N.N-dimethylformamide
DMSO dimethyl sulfoxide
EA or EtOAc ethyl acetate
EDCl N-(3-dimethylaminopropyl)-N'-ethylcarbodiimide hydrochloride
equiv equivalents
Et₃N or TEA triethylamine
EtOH ethyl alcohol
FA formic acid
h or hr hour
HATU 1-[bis(dimethylamino)methylene]-1H-1,2,3-triazolo[4,5-b]pyridinium 3-oxid hexafluorophosphate
HOAt 1-hydroxy-7-azabenzotriazole
HOBt or HOBT 1-hydroxybenzotriazole hydrate
iPr Isopropyl
MeOH methyl alcohol
Me₄t-BuXphos ditert-butyl-[2,3,4,5-tetramethyl-6-(2,4,6-triisopropylphenyl)phenyl]phosphane
min minute
MTBE tert-butyl methyl ether
n-BuLi n-butylithium
NMP 1-methyl-2-pyrrolidinone
OAc acetate
Pd/C palladium on carbon
PDC pyridinium dichromate
PdCl₂(dtbpf) or Pd(dtbpf)Cl₂ dichloro[1,1'-bis(di-t-butylphosphino)ferrocene]palladium(II)
PdCl₂(dppf) or Pd(dppf)Cl₂ [1,1'-bis(diphenylphosphino)ferrocene]dichloropalladium(II)
Pd₂(dba)₃ tris(dibenzylideneacetone)dipalladium(0)
Pd(PPh₃)₄ tetrakis(triphenylphosphine)palladium(0
Pd(PPh₃)₂Cl₂ dichlorobis(triphenylphosphine)palladium(II)
PE petroleum ether
PPh₃ triphenylphosphine
Pr n-propyl
Py pyridine
rac racemic
Rf retention factor
r.t. or rt room temperature
sat. saturated
SFC supercritical fluid chromatography
t-Bu tert-butyl
tBuXphos-Pd-G3 or tBuXphos Pd G₃ or t-BuXphos-Pd (gen 3)[2-(2-aminophenyl)phenyl]-methylsulfonyloxypalladium;ditert-butyl-[2-(2,4,6-triisopropylphenyl)phenyl]phosphane
TFA trifluoroacetic acid
Tf₂O trifluoromethanesulfonic anhydride
THF tetrahydrofuran
TLC thin layer chromatography
Xantphos-Pd-G3 [2-(2- aminophenyl)phenyl]-methylsulfonyloxy-palladium;(5-diphenylphosphanyl-9,9-dimethyl-xanthen-4-yl)-diphenyl-phosphane

### Example 1. Preparation of Compounds

### Preparation of methyl 2-(3-hydroxy-1,2-oxazol-5-yl)-3-methylbutanoate (1-1)

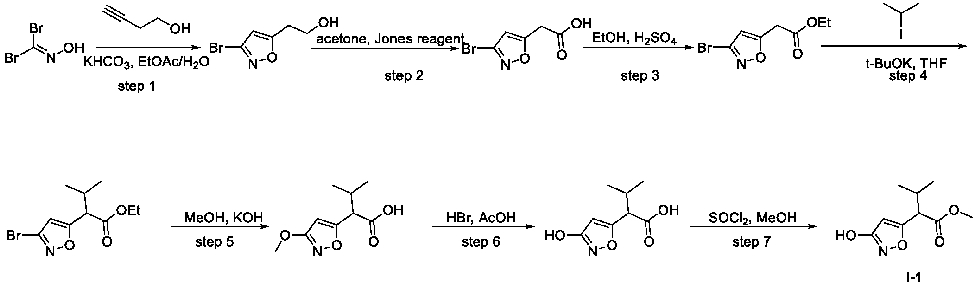

### Step 1: Preparation of 2-(3-bromoisoxazol-5-yl)ethan-1-ol

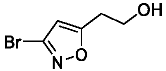

A solution of 3-butyn-1-ol (552.89 g, 7888.26 mmol, 4 equiv) and KHCO₃ (592.30 g, 5916.197 mmol, 3 equiv) in EtOAc (2600 mL) and H₂O (260 mL) was stirred at room temperature. To the above mixture was added 1-bromo-N-hydroxymethanecarbonimidoyl bromide (400.00 g in EA (840 mL), 1972.066 mmol, 1.00 equiv) dropwise over 60 min at room temperature. The resulting mixture was stirred for overnight at room temperature. The reaction mixture was washed with water (500 mL x 2) and the combined organic layers dried over anhydrous Na₂SO₄. After filtration, the filtrate was concentrated under reduced pressure. The residue was purified by silica gel column chromatography, eluted with PE/EtOAc (30:1) to afford intermediate the title compound (338.2 g, 88.98%) as off-white solid. LCMS (ESI) m/z [M+H]⁺ =192.

### Step 2: Preparation of 2-(3-bromoisoxazol-5-yl)acetic acid

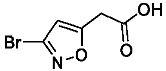

A solution of 2-(3-bromoisoxazol-5-yl)ethan-1-ol (360.00 g) in acetone (3600 mL) was stirred at 0 degrees C under a nitrogen atmosphere. To the above mixture was added Jones' reagent (1760 mL) dropwise over 1 h at 0 degrees C. The resulting mixture was stirred overnight at room temperature. The reaction was quenched with water/Ice at 0 degrees C. The resulting mixture was extracted with EtOAc (1000 mL x 3). The combined organic layers were washed with water (500 mL x 2), and dried over anhydrous Na₂SO₄. After filtration, the filtrate was concentrated under reduced pressure to afford the title compound (348.6 g, crude) as a green solid that was used directly without further purification. (LCMS (ESI) m/z [M+H]⁺ =206.

### Step 3: Preparation of ethyl 2-(3-bromoisoxazol-5-yl)acetate

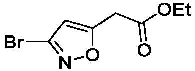

A solution of 2-(3-bromoisoxazol-5-yl)acetic acid (397.6 g, 1930.144 mmol, 1.00 equiv) and H₂SO₄ (18.92 g, 193.014 mmol, 0.1 equiv) in EtOH (2000 mL) was stirred for 2 h at 70 degrees C. The reaction mixture was concentrated under reduced pressure. The residue was diluted with EtOAc (3000 mL), washed with water (500 mL x 2), and dried over anhydrous Na₂SO₄. After filtration, the filtrate was concentrated under reduced pressure. The residue was purified by silica gel column chromatography, eluted with PE/EtOAc (35:1) to afford the title compound (355 g, 78.61%) as a colorless oil. (LCMS (ESI) m/z [M+H]⁺ =234.

### Step 4: Preparation of ethyl 2-(3-bromoisoxazol-5-yl)-3-methylbutanoate

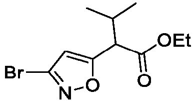

To a stirred solution of t-BuOK (244.51 g, 2179.031 mmol, 1.5 equiv) and ethyl 2-(3-bromoisoxazol-5-yl)acetate (340.00 g, 1452.687 mmol, 1.00 equiv) in THF (2000 mL) was added 2-iodopropane (321.03 g, 1888.493 mmol, 1.3 equiv) dropwise at 0 degrees C under a nitrogen atmosphere. The resulting mixture was stirred for overnight at room temperature, then diluted with Water/Ice at 0 degrees C. The resulting mixture was extracted with EtOAc (1000 mL x 2). The combined organic layers were washed with water (500 mL x 1), dried over anhydrous Na₂SO₄. After filtration, the filtrate was concentrated under reduced pressure. The residue was purified by silica gel column chromatography, eluted with PE/THF (10:1) to afford the title compound (284.1 g, 70.82%) as a colorless oil. (LCMS (ESI) m/z [M+H]⁺ =276.

### Step 5: Preparation of 2-(3-methoxy-1,2-oxazol-5-yl)-3-methylbutanoic acid

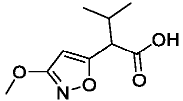

To a stirred solution of Preparation of ethyl 2-(3-bromoisoxazol-5-yl)-3-methylbutanoate (90.00 g, 325.933 mmol, 1.00 equiv) in MeOH (270 mL) was added a solution of KOH (274.30 g, 4888.995 mmol, 15.00 equiv) in MeOH (210 mL) at 0 degrees C. The reaction mixture was stirred overnight at 80 degrees C. The resulting solution was acidified to pH 4 with 1M solution of HCl (aq.) and concentrated under reduced pressure. The resulting mixture was diluted with EtOAc (1800 mL) and filtered. The filter cake was washed with EtOAc (100 mL x 3). The filtrate was concentrated under reduced pressure to afford the title compound (62.9 g, 96.88%) as a yellow oil that was used directly without further purification. LCMS (ESI) m/z: [M+H]⁺ =200.

### Step 6: Preparation of 2-(3-hydroxy-1,2-oxazol-5-yl)-3-methylbutanoic acid

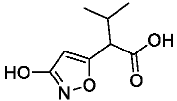

To a stirred solution of 2-(3-methoxy-1,2-oxazol-5-yl)-3-methylbutanoic acid (62.90 g, 315.754 mmol, 1.00 equiv) in HOAc (450.00 mL) was added 48% HBr (450.00 mL) at room temperature. The resulting mixture was stirred for 16 h at 60 degrees C. The resulting mixture was concentrated under reduced pressure, and the residue purified by flash C18-flash chromatography, elution gradient 0 to 100% MeCN in water (containing 0.05% FA). Pure fractions were evaporated to dryness to afford the title compound (43.3 g, 74.05%) as a white solid. LCMS (ESI) m/z: [M+H]⁺ = 186.

### Step 7: Preparation of methyl 2-(3-hydroxy-1,2-oxazol-5-yl)-3-methylbutanoate (I-1)

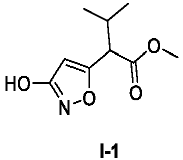

To a stirred solution of 2-(3-hydroxy-1,2-oxazol-5-yl)-3-methylbutanoic acid (20 g, 108.004 mmol, 1.00 equiv) in MeOH (72 mL) was added SOCl₂ (35.26 mL, 486.059 mmol, 4.50 equiv) at 0 degrees C. The resulting mixture was stirred for 16 h at room temperature. The resulting mixture was concentrated under reduced pressure and the residue was diluted with water (30 mL) and extracted with EtOAc (50 mL x 3). The combined organic layers were washed with saturated aqueous NaCl (30 mL), dried over anhydrous Na₂SO₄ and filtered. The filtrate was concentrated under reduced pressure. The crude product was purified by silica gel column chromatography, elution gradient 0 to 100% THF in petroleum ether. Pure fractions were evaporated to dryness to afford compound *I**-1*** (15.1 g, 70.18%) as an off-white solid. ¹H NMR (400 MHz, DMSO-*d*₆) δ 11.24 (s, 1H), 5.95 (s, 1H), 3.71 - 3.58 (m, 4H), 2.32 - 2.20 (m, 1H), 0.88 (dd, J = 34.2, 6.7 Hz, 6H). LCMS (ESI) m/z: [M+H]⁺ = 200.

### Preparation of methyl 2-(3-((2-chloropyrimidin-4-yl)oxy)isoxazol-5-yl)-3-methylbutanoate (Intermediate 2)

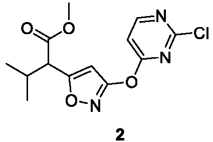

A solution of methyl 2-(3-hydroxy-1,2-oxazol-5-yl)-3-methylbutanoate (200 mg, 1.004 mmol, 1 equiv) and 4-bromo-2-chloropyrimidine (233.04 mg, 1.205 mmol, 1.2 equiv), Cs₂CO₃ (981.9 mg, 3.012 mmol, 3.0 equiv) in DMF (5 mL) was stirred for 1h at 100° C under nitrogen atmosphere. The residue was purified by reverse flash chromatography with the following conditions: column, C18 silica gel; mobile phase, MeCN in Water (0.1% FA), 10% to 100% gradient in 20 min; detector, UV 254 nm. The mixture was concentrated to afford intermediate 2 (258 mg, crude). LCMS (ESI) m/z: [M+H]⁺ = 312

### Preparation of methyl 2-[3-(2-chloropyrimidin-5-yl)-1,2-oxazol-5-yl]-3-methylbutanoate. (I-2)

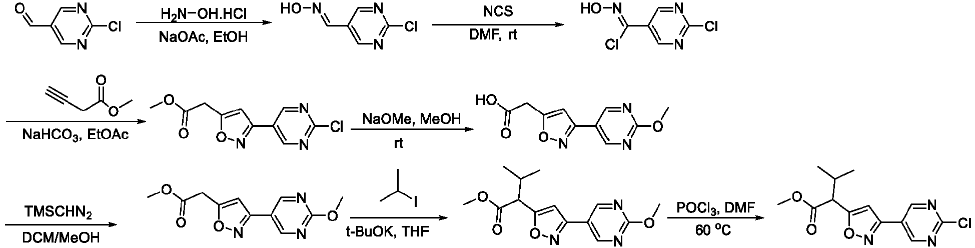

### Step 1: Preparation of (E)-N-[(2-chloropyrimidin-5-yl)methylidene]hydroxylamine.

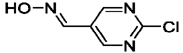

To a stirred solution of 2-chloropyrimidine-5-carbaldehyde (5 g, 35.078 mmol, 1 equiv) and NH₂OH·HCl (4.93 g, 70.945 mmol, 2.02 equiv) in EtOH (250 mL) was added NaOAc (14.48 g, 176.512 mmol, 5.03 equiv) at room temperature. The resulting mixture was stirred for 2 h at room temperature. The solvent was removed under reduced pressure. The residue was dissolved in EtOAc (500 mL), washed with brine (500 mL), and dried over anhydrous Na₂SO₄. After filtration, the filtrate was concentrated under reduced pressure to afford (E)-N-[(2-chloropyrimidin-5-yl)methylidene]hydroxylamine (4.6 g, crude product) as a light yellow solid which was used in the next step directly without further purification. LCMS (ESI) m/z: [M+H]⁺ = 158.

### Step 2: Preparation of (Z)-2-chloro-N-hydroxypyrimidine-5-carbonimidoyl chloride.

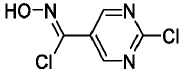

A solution of (E)-N-[(2-chloropyrimidin-5-yl)methylidene]hydroxylamine (4.6 g, 29.195 mmol, 1 equiv) and NCS (4.4 g, 32.951 mmol, 1.13 equiv) in DMF (150 mL) was stirred for 2 h at room temperature. The mixture was diluted with EtOAc (500 mL). The resulting mixture was washed with water (3 x 300 mL), brine (1 x 300 mL) and the organic phase was dried over anhydrous Na₂SO₄. After filtration, the filtrate was concentrated under reduced pressure to afford (Z)-2-chloro-N-hydroxypyrimidine-5-carbonimidoyl chloride (4.8 g, crude product) as a yellow solid. LCMS (ESI) m/z: [M+H]⁺ = 192.

### Step 3: Preparation of methyl 2-[3-(2-chloropyrimidin-5-yl)-1,2-oxazol-5-yljacetate.

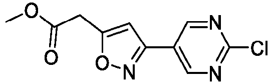

A solution of (Z)-2-chloro-N-hydroxypyrimidine-5-carbonimidoyl chloride (4.8 g, 25.00 mmol, 1 equiv) in EtOAc (80 mL) was treated with NaHCO₃ (3 g, 35.712 mmol, 1.43 equiv) for 30 min at 0 °C under an atmosphere of dry nitrogen followed by the addition of methyl but-3-ynoate (2.02 g, 20.591 mmol, 0.82 equiv) in portions at 0 °C. The resulting mixture was stirred for 12 h at room temperature. The resulting mixture was diluted with water (150 mL) and extracted with EtOAc (2 x 400 mL). The combined organic layers were washed with brine (1 x 400 mL) and dried over anhydrous Na₂SO₄. After filtration, the filtrate was concentrated under reduced pressure. The residue was purified by silica gel column chromatography, eluted with PE/EA (3:1) to afford methyl 2-[3-(2-chloropyrimidin-5-yl)-1,2-oxazol-5-yl]acetate (2.5 g, 38.64%) as a light yellow solid. LCMS (ESI) m/z: [M+H]⁺ = 254.

### Step 4: Preparation of [3-(2-methoxypyrimidin-5-yl)-1,2-oxazol-5-yl]acetic acid.

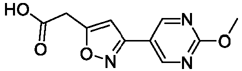

A solution of methyl 2-[3-(2-chloropyrimidin-5-yl)-1,2-oxazol-5-yl]acetate (3 g, 11.828 mmol, 1 equiv) and NaOMe (1.92 g, 35.484 mmol, 3.00 equiv) in MeOH (50 mL) was stirred for 1 h at room temperature under an atmosphere of dry nitrogen. The mixture was acidified to pH 6 with HCl (aq.). The residue was dissolved in EtOAc (300 mL). The resulting mixture was washed with water (2 x 300 mL). The combined organic layers were washed with brine (1 x 300 mL) and dried over anhydrous Na₂SO₄. After filtration, the filtrate was concentrated under reduced pressure to afford [3-(2-methoxypyrimidin-5-yl)-1,2-oxazol-5-yl]acetic acid (2.5 g, crude product) as a light yellow solid which was used in the next step directly without further purification. LCMS (ESI) m/z: [M+H]⁺ = 236.

### Step 5: Preparation of methyl 2-[3-(2-methoxypyrimidin-5-yl)-1,2-oxazol-5-yl]acetate

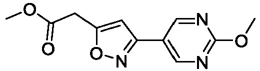

A solution of [3-(2-methoxypyrimidin-5-yl)-1,2-oxazol-5-yl]acetic acid (2.4 g, 10.204 mmol, 1 equiv) and (trimethylsilyl)diazomethane (2.33 g, 20.408 mmol, 2 equiv) in DCM (20 mL) and MeOH (5 mL) was stirred for 30 min at room temperature. The resulting mixture was concentrated under reduced pressure. The residue was purified by silica gel column chromatography, eluted with PE/EA (3:1) to afford methyl 2-[3-(2-methoxypyrimidin-5-yl)-1,2-oxazol-5-yl]acetate (1.2 g, 45.77%) as a white solid. LCMS (ESI) m/z: [M+H]⁺ = 250.

### Step 6: Preparation of methyl 2-[3-(2-methoxypyrimidin-5-yl)-1,2-oxazol-5-yl]-3-methylbutanoate.

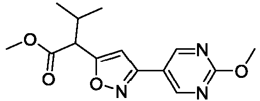

A solution of methyl 2-[3-(2-methoxypyrimidin-5-yl)-1,2-oxazol-5-yl]acetate (2.5 g, 10.031 mmol, 1 equiv) in THF (20 mL) was treated with t-BuOK (1.2 g, 10.694 mmol, 1.07 equiv) for 30 min at 0 °C under an atmosphere of dry nitrogen followed by the addition of 2-iodopropane (1.5 g, 8.824 mmol, 0.88 equiv) dropwise at 0 °C. The resulting mixture was stirred for 12 h at room temperature. The mixture was acidified to pH 6 with HCl (aq.). The resulting mixture was extracted with EtOAc (2 x 200 mL). The combined organic layers were washed with brine (2 x 200 mL) and dried over anhydrous Na₂SO₄. After filtration, the filtrate was concentrated under reduced pressure. The residue was purified by silica gel column chromatography, eluted with PE/EA (3:1) to afford methyl 2-[3-(2-methoxypyrimidin-5-yl)-1,2-oxazol-5-yl]-3-methylbutanoate (310 mg, 10.08%) as a yellow oil. LCMS (ESI) m/z: [M+H]⁺ = 292.

### Step 7: Preparation of methyl 2-[3-(2-chloropyrimidin-5-yl)-1,2-oxazol-5-yl]-3-methylbutanoate.

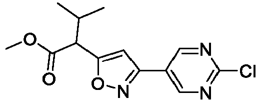

A solution of methyl 2-[3-(2-methoxypyrimidin-5-yl)-1,2-oxazol-5-yl]-3-methylbutanoate (200 mg, 0.687 mmol, 1 equiv) and POCl₃ (1.9 mL, 20.61 mmol, 30 equiv) in DMF (1.5 mL) was stirred for 3 h at 60 °C under an atmosphere of dry nitrogen. The residue was dissolved in EtOAc (100 mL). The resulting mixture was washed with brine (2 x 100 mL) and dried over anhydrous Na₂SO₄. After filtration, the filtrate was concentrated under reduced pressure to afford methyl 2-[3-(2-chloropyrimidin-5-yl)-1,2-oxazol-5-yl]-3-methylbutanoate (160 mg, crude product) as a brown oil which was used in the next step directly without further purification. LCMS (ESI) m/z: [M+H]⁺ = 296.

### Preparation of tert-butyl (2S,4R)-4-hydroxy-N-[(1S)-1-[4-(4-methyl-1,3-thiazol-5-yl)phenyl]ethyl]-1-(2R)-3-methyl-2-[3-(piperidin-4-yl)-1,2-oxazol-5-yl]butanoyl]pyrrolidine-2-carboxamide (1-3)

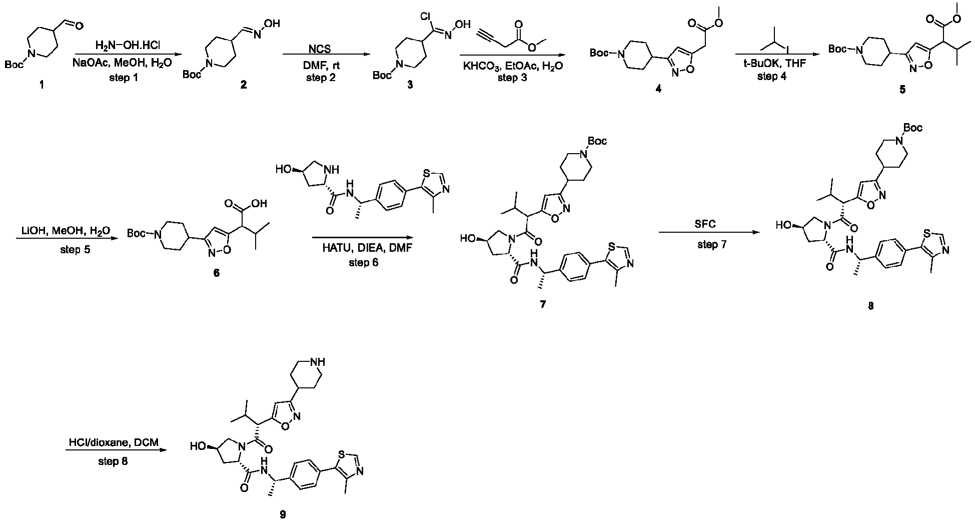

### Step 1: Preparation of tert-butyl 4-[(1E)-(hydroxyimino)methyl]piperidine-1-carboxylate (Intermediate 2)

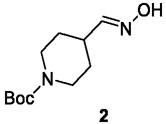

To a stirred solution of tert-butyl 4-formylpiperidine-1-carboxylate (5 g, 23.444 mmol, 1.00 equiv) in MeOH (10 mL) and H₂O (10 mL) was added hydroxylamine hydrochloride (1.95 g, 28.133 mmol, 1.2 equiv) and Na₂CO₃ (1.24 g, 11.722 mmol, 0.5 equiv) at 0 degrees C. The resulting mixture was stirred for overnight at room temperature. Desired product could be detected by LCMS. The resulting mixture was concentrated under reduced pressure. The resulting mixture was extracted with EtOAc (3 x 20 mL). The combined organic layers were washed with brine (50 mL), dried over anhydrous Na₂SO₄. After filtration, the filtrate was concentrated under reduced pressure to afford intermediate **2** (6 g, crude) as a colorless oil. LCMS (ESI) m/z: [M+H]⁺ = 229.

### Step 2: Preparation of tert-butyl 4-[(1Z)-chloro(hydroxyimino)methyl]piperidine-1-carboxylate (Intermediate 3)

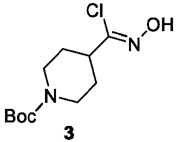

A mixture of intermediate **2** and NCS (3.5 g, 26.282 mmol, 1.0 equiv) in DMF (20 mL) was stirred for 2 h at room temperature. Desired product could be detected by LCMS. The resulting mixture was diluted with water (50.00 mL) and extracted with EtOAc (3 x 50 mL). The combined organic layers were washed with brine (50 mL), dried over anhydrous Na₂SO₄. After filtration, the filtrate was concentrated under reduced pressure to afford intermediate **3** (7.8 g, crude) as a colorless oil. LCMS (ESI) m/z [M+H]⁺ =263.

### Step 3: Preparation of tert-butyl 4-[5-(2-methoxy-2-oxoethyl)-1,2-oxazol-3-yl]piperidine-1-carboxylate (Intermediate 4 )

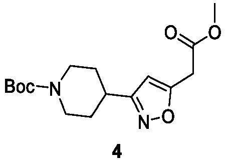

A mixture of intermediate **3** (7.8 g, crude) and NaHCO₃ (3.8 g, 45.675 mmol, 1.5 equiv) in EtOAc (100 mL) was stirred for 30 min at room temperature. To the above mixture was added methyl but-3-ynoate (2.99 g, 30.450 mmol, 1 equiv) at 0 degrees C. The resulting mixture was stirred for overnight at room temperature. Desired product could be detected by LCMS. The resulting mixture was concentrated under reduced pressure. The residue was purified by reverse flash chromatography with the following conditions: column, C18 silica gel; mobile phase, MeCN in water (0.05% FA), 0% to 100% gradient in 30 min; detector, UV 254 nm. The resulting mixture was concentrated under reduced pressure to afford intermediate **4** (4.1 g, 41.51%) as a light yellow oil. LCMS (ESI) m/z: [M+H]⁺ = 325.

### Step 4: Preparation of tert-butyl 4-[5-(1-methoxy-3-methyl-1-oxobutan-2-yl)-1,2-oxazol-3-yl]piperidine-1-carboxylate (Intermediate 5)

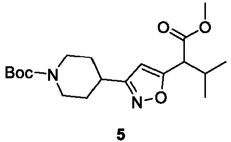

A mixture of intermediate **4** (1.0 g, 3.083 mmol, 1.5 equiv) and Na₂SO₄ (1.0 g) in THF (10 mL) was added t-BuOK (518.90 mg, 4.625 mmol, 1.5 equiv) and 2-iodopropane (628.87 mg, 3.700 mmol, 1.2 equiv) at 0 degrees C under nitrogen atmosphere. The resulting mixture was stirred for 3 h at 0 degrees C under nitrogen atmosphere. Desired product could be detected by LCMS. After filtration, the filtrate was concentrated under reduced pressure. The residue was purified by reverse flash chromatography with the following conditions: column, C18 silica gel; mobile phase, MeCN in water (0.05% FA), 0% to 100% gradient in 30 min; detector, UV 254 nm. The resulting mixture was concentrated under reduced pressure to afford intermediate **5** (330 mg, 29.21%) as a light yellow oil. LCMS (ESI) m/z: [M+H]⁺ = 367.

### Step 5: Preparation of 2-{3-[1-(tert-butoxycarbonyl)piperidin-4-yl]-1,2-oxazol-5-yl}-3-methylbutanoic acid (Intermediate 6 )

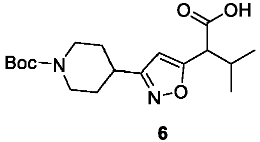

To a stirred solution of intermediate **5** (320 mg, 0.873 mmol, 1.00 equiv) in MeOH (5 mL) was added LiOH (62.74 mg, 2.619 mmol, 3 equiv) in H₂O (5 mL) dropwise at room temperature. The resulting mixture was stirred for 3 h at room temperature. Desired product could be detected by LCMS. The resulting mixture was concentrated under reduced pressure. To the above mixture was added aq. HCl (6M) adjusting PH to ~5. The resulting mixture was extracted with EtOAc (3 x 50 mL). The combined organic layers were washed with brine (50 mL), dried over anhydrous Na₂SO₄. After filtration, the filtrate was concentrated under reduced pressure to afford intermediate **6** (316 mg crude) as an off-white solid. LCMS (ESI) m/z: [M+H]⁺ = 353.

### Step 6: Preparation of tert-butyl 4-(5-{1-[(2S,4R)-4-hydroxy-2-{[(1S)-1-[4-(4-methyl-1,3-thiazol-5-yl)phenyl]ethyl]carbamoyl}pyrrolidin-1-yl]-3-methyl-1-oxobutan-2-yl}-1,2-oxazol-3-yl)piperidine-1-carboxylate (Intermediate 7)

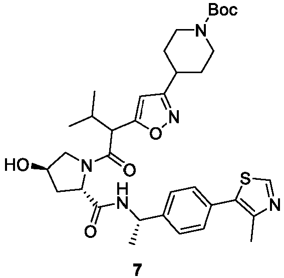

A mixture of intermediate **6** (310 mg, 0.880 mmol, 1.00 equiv) and HATU (668.90 mg, 1.760 mmol, 2 equiv) in DMF (5 mL) was stirred for 30 min at room temperature. To the above mixture was added (2S,4R)-4-hydroxy-N-[(1S)-1-[4-(4-methyl-1,3-thiazol-5-yl) phenyl]ethyl]pyrrolidine-2-carboxamide (291.53 mg, 0.880 mmol, 1 equiv) at room temperature. The resulting mixture was stirred for additional 2 h at room temperature. Desired product could be detected by LCMS. The residue was purified by reverse flash chromatography with the following conditions: column, C18 silica gel; mobile phase, MeCN in water (0.05% FA), 0% to 100% gradient in 30 min; detector, UV 254 nm. The resulting mixture was concentrated under reduced pressure to afford intermediate **7** (242 mg, 37.31%) as a light brown solid. LCMS (ESI) m/z: [M+H]⁺ = 666.

### Step 7: Preparation of tert-butyl 4-{5-[(2R)-1-[(2S,4R)-4-hydroxy-2-{[(1S)-1-[4-(4-methyl-1,3-thiazol-5-yl)phenyl]ethyl]carbamoyl}pyrrolidin-1-yl]-3-methyl-1-oxobutan-2-yl]-1,2-oxazol-3-yl}piperidine-1-carboxylate (Intermediate 8)

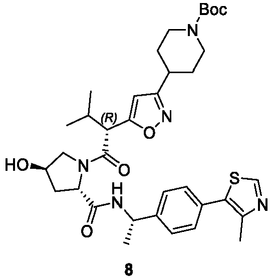

The product was purified by Prep-SFC with the following conditions (Column: CHIRAL ART Amylose-SA, 3*25 cm, 5 µm; Mobile Phase A: CO2, Mobile Phase B: MeOH--HPLC; Flow rate: 50 mL/min; Gradient: isocratic 45% B; Column Temperature(°C): 35; Back Pressure(bar): 100; Wave Length: 205 nm; RT1(min): 3.65; RT2(min): 4.88; Sample Solvent: MeOH--HPLC; Injection Volume: 1 mL) to afford intermediate **8** (the second peak) (208.1 mg, 43.52%) as a light brown solid. LCMS (ESI) m/z: [M+H]⁺ = 666.

### Step 8: Preparation of tert-butyl (2S,4R)-4-hydroxy-N-[(1S)-1-[4-(4-methyl-1,3-thiazol-5-yl)phenyl]ethyl]-1-[(2R)-3-methyl-2-[3-(piperidin-4-yl)-1,2-oxazol-5-yl]butanoyl]pyrrolidine-2-carboxamide (Intermediate 9)

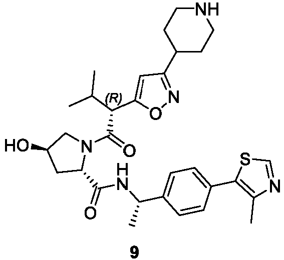

To a stirred solution of intermediate **8** (200 mg, 0.300 mmol, 1.00 equiv) in DCM (2 mL) was added 1M HCl in 1,4-dioxane (2 mL) dropwise at room temperature. The resulting mixture was stirred for 1 h at room temperature. Desired product could be detected by LCMS. The resulting mixture was concentrated under reduced pressure to afford intermediate **9** (247.5 mg) as a light-yellow solid. LCMS (ESI) m/z: [M+H]⁺ = 566.

### Preparation of (2S,4R)-4-hydroxy-N-[(1S)-1-[4-(4-methyl-1,3-thiazol-5-yl)phenyl]ethyl]-1-[(2S)-3-methyl-2-[3-(piperazin-1-yl)-1,2-oxazol-5-yl]butanoyl]pyrrolidine-2-carboxamide (1-4) and (2S,4R)-4-hydroxy-N-[(1S)-1-[4-(4-methyl-1,3-thiazol-5-yl)phenyl]ethyl]-1-[(2R)-3-methyl-2-[3-(piperazin-1-yl)-1,2-oxazol-5-yl]butanoyl]pyrrolidine-2-carboxamide (1-5)

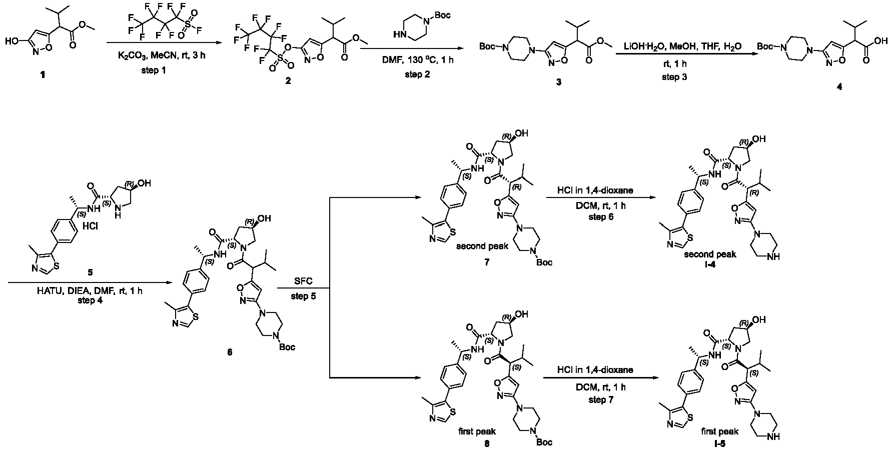

### Step 1: Preparation of methyl 3-methyl-2-[3-[(1,1,2,2,3,3,4,4,4-nonafluorobutanesulfonyl)oxy]-1,2-oxazol-5-yl]butanoate (Intermediate 2).

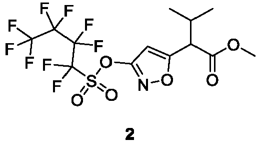

To a stirred solution methyl 2-(3-hydroxy-1,2-oxazol-5-yl)-3-methylbutanoate (100.00 mg, 0.502 mmol, 1.00 equiv) in MeCN (0.50 mL) was added perfluorobutanesulfonyl fluoride (303.29 mg, 1.004 mmol, 2.00 equiv) and K₂CO₃ (208.13 mg, 1.506 mmol, 3.00 equiv) at room temperature. The resulting mixture was stirred for 3 h, then carefully quenched with water at 0 degrees C. The resulting mixture was extracted with EA (2 x 50 mL), and the combined organic layers were washed with brine (50 mL), and dried over anhydrous Na₂SO₄. After filtration, the filtrate was concentrated under reduced pressure, and the residue purified by silica gel column chromatography, eluted with PE/EA (2/1) to afford Intermediate **2** (217 mg, crude) as a white solid. LCMS (ESI) m/z: [M+H]⁺ = 482.

### Step 2: Preparation of tert-butyl 4-[5-(1-methoxy-3-methyl-1-oxobutan-2-yl)-1,2-oxazol-3-yl]piperazine-1-carboxylate (Intermediate 3).

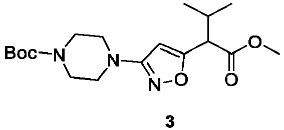

To a stirred solution of Intermediate **2** (217.00 mg, 0.451 mmol, 1.00 equiv) in DMF (3.00 mL) was added tert-butyl piperazine-1-carboxylate (83.98 mg, 0.451 mmol, 1.00 equiv) at room temperature. The resulting mixture was stirred for 1 h at 130 degrees C. The mixture was allowed to cool down to room temperature. The residue was purified by reverse flash chromatography with the following conditions: column, C18 silica gel; mobile phase, MeCN in water (0.1% FA), 0 to 100% gradient in 30 min. This provided intermediate **3** (54 mg, 32.59%) as a yellow oil. LCMS (ESI) m/z: [M+H]⁺ = 368.

### Step 3: Preparation of 2-[3-[4-(tert-butoxycarbonyl)piperazin-1-yl]-1,2-oxazol-5-yl]-3-methylbutanoic acid (Intermediate 4).

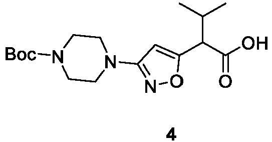

To a stirred solution of Intermediate **3** (54.00 mg, 0.147 mmol, 1.00 equiv) in MeOH (0.80 mL) was added THF (0.80 mL) and H₂O (0.80 mL) at room temperature, follew by addition of LiOH·H₂O (18.50 mg, 0.441 mmol, 3.00 equiv). The resulting mixture was stirred for an additional 1 h at room temperature. The mixture was acidified to pH 6 with HCl (1M, aq.), then extracted with EA (2 x 50 mL). The combined organic layers were washed with saturated brine (50 mL), dried over anhydrous Na₂SO₄, and filtered. The filtrate was concentrated under reduced pressure. This provided Intermediate **4** (52 mg, crude) as a yellow solid. LCMS (ESI) m/z: [M+H]⁺ = 354.

### Step 4: Preparation of tert-butyl 4-(5-[1-[(2S,4R)-4-hydroxy-2-[[(1S)-1-[4-(4-methyl-1,3-thiazol-5-yl)phenyl]ethyl]carbamoyl]pyrrolidin-1-yl]-3-methyl-1-oxobutan-2-yl]-1,2-oxazol-3-yl)piperazine-1-carboxylate (Intermediate 6).

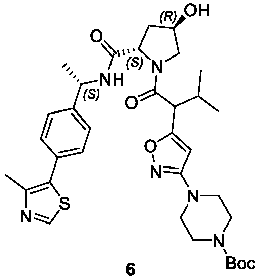

To a stirred solution of Intermediate **4** (52.00 mg, 0.119 mmol, 1.00 equiv) in DMF (2.00 mL) was added HATU (135.56 mg, 0.357 mmol, 3.00 equiv) and DIEA (76.80 mg, 0.595 mmol, 5.00 equiv) at room temperature. To the above mixture was added (2S,4R)-4-hydroxy-N-[(1S)-1-[4-(4-methyl-1,3-thiazol-5-yl)phenyl]ethyl]pyrrolidine-2-carboxamide (70.90 mg, 0.214 mmol, 1.80 equiv) at room temperature. The resulting mixture was stirred for an additional 1 h. The mixture was purified directly by reverse flash chromatography with the following conditions: column, C18 silica gel; mobile phase, MeCN in water (0.1% FA), 0 to 100% gradient in 30 min. This provided in Intermediate **6** (73 mg, 92.12%) as a white solid. LCMS (ESI) m/z: [M+H]⁺ = 667.

### Step 5: Preparation of (2S,4R)-4-hydroxy-N-[(1S)-1-[4-(4-methyl-1,3-thiazol-5-yl)phenyl]ethyl]-1-[(2R)-3-methyl-2-[3-(piperazin-1-yl)-1,2-oxazol-5-yl]butanoyl]pyrrolidine-2-carboxamide (Intermediate 7); (2S,4R)-4-hydroxy-N-[(1S)-1-[4-(4-methyl-1,3-thiazol-5-yl)phenyl]ethyl]-1-[(2S)-3-methyl-2-[3-(piperazin-1-yl)-1,2-oxazol-5-yl]butanoyl]pyrrolidine-2-carboxamide (Intermediate 8).

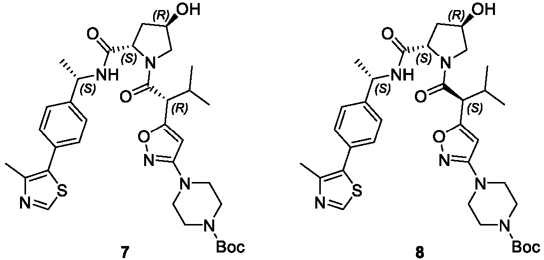

The Intermediate **6** (73 mg) was purified by SFC with the following conditions: Column, CHIRAL ART Amylose-C NEO, 3*25 cm, 5 um; mobile phase, MeOH. This provided Intermediate **7** (37 mg, second peak). LCMS (ESI) m/z: [M+H]⁺ = 667, and Intermediate 8 (34 mg, first peak). LCMS (ESI) m/z: [M+H]⁺ = 667.

### Step 6: Preparation of (2S,4R)-4-hydroxy-N-[(1S)-1-[4-(4-methyl-1,3-thiazol-5-yl)phenyl]ethyl]-1-[(2R)-3-methyl-2-[3-(piperazin-1-yl)-1,2-oxazol-5-yl]butanoyl]pyrrolidine-2-carboxamide (I-4).

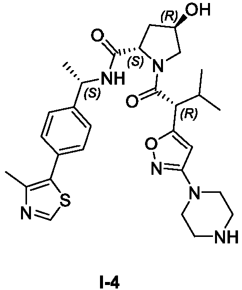

To a stirred solution of Intermediate **7** (37.00 mg, 0.055 mmol, 1.00 equiv) in DCM (1.50 mL) was added HCl in 1,4-dioxane (1.50 mL, 26.276 mmol, 473.57 equiv) at 0 degrees C. The resulting mixture was stirred for 1 h at room temperature, then concentrated under reduced pressure. This provided **I-4** (45 mg, crude) as a yellow oil. LCMS (ESI) m/z: [M+H]⁺ = 567.

### Step 7: Preparation of (2S,4R)-4-hydroxy-N-[(1S)-1-[4-(4-methyl-1,3-thiazol-5-yl)phenyl]ethyl]-1-[(2S)-3-methyl-2-[3-(piperazin-1-yl)-1,2-oxazol-5-yl]butanoyl]pyrrolidine-2-carboxamide (I-5).

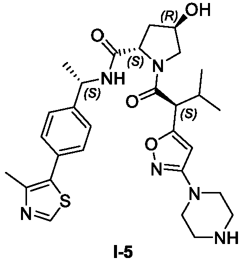

To a stirred solution of Intermediate 8 (34.00 mg, 0.051 mmol, 1.00 equiv) in DCM (1.50 mL) was added HC in 1,4-dioxane (1.50 mL, 26.276 mmol, 515.35 equiv) at 0 degrees C. The resulting mixture was stirred for 1 h at room temperature, then concentrated under reduced pressure. This provided **I-5** (45 mg, crude) as a yellow oil. LCMS (ESI) m/z: [M+H]⁺ = 567.

### Preparation of 2-[6-(azetidin-3-yl)thieno[2,3-c]pyridazin-3-yl]phenol (I-6)

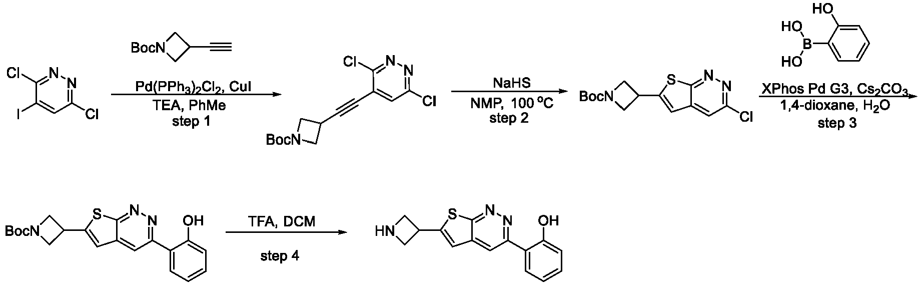

### Step 1: Preparation of tert-butyl 3-[2-(3,6-dichloropyridazin-4-yl)ethynyl]azetidine-1-carboxylate

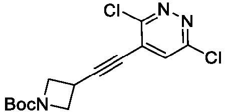

To a stirred mixture of 3,6-dichloro-4-iodopyridazine (200 mg, 0.728 mmol, 1.00 equiv) and tert-butyl 3-ethynylazetidine-1-carboxylate (145.06 mg, 0.801 mmol, 1.1 equiv) in toluene (5.00 mL) was added Pd(PPh₃)₂Cl₂ (76.61 mg, 0.109 mmol, 0.15 equiv), Cul (27.71 mg, 0.146 mmol, 0.2 equiv) and TEA (220.88 mg, 2.184 mmol, 3 equiv) at room temperature under a nitrogen atmosphere. The resulting mixture was stirred for 2 h at room temperature then filtered. The filtrate was concentrated under reduced pressure and the residue purified by reverse phase flash chromatography with the following conditions: column, C18; mobile phase, MeCN in water (0.05% FA), 40% to 60% gradient. This provided the title compound (170 mg, 64.07%) as a yellow solid. LCMS (ESI) m/z: [M+H]⁺ = 328.

### Step 2: Preparation of tert-butyl 3-{3-chlorothieno[2,3-c]pyridazin-6-yl}azetidine-1-carboxylate

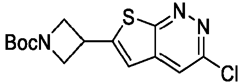

To a stirred mixture of tert-butyl 3-[2-(3,6-dichloropyridazin-4-yl)ethynyl]azetidine-1-carboxylate (160 mg, 0.488 mmol, 1.00 equiv) in NMP (5 mL) was added sodium hydrosulfide (32.80 mg, 0.586 mmol, 1.2 equiv) at room temperature under a nitrogen atmosphere. The resulting mixture was stirred for 2 h at 100 degrees C, cooled, and filtered. The filtrate was purified by reverse phase flash chromatography with the following conditions: column, C18; mobile phase, MeCN in water (0.05% FA), 30% to 50% gradient. This provided the title compound (122 mg, 71.43%) as a white solid. LCMS (ESI) m/z [M+H]⁺ =326.

### Step 3: Preparation of tert-butyl 3-[3-(2-hydroxyphenyl)thieno[2,3-c]pyridazin-6-yl]azetidine-1-carboxylate

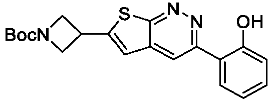

To a stirred mixture of tert-butyl 3-{3-chlorothieno[2,3-c]pyridazin-6-yl}azetidine-1-carboxylate (122 mg, 0.374 mmol, 1.00 equiv) and 2-hydroxyphenylboronic acid (154.94 mg, 1.122 mmol, 3 equiv) in dioxane (4 mL) and H₂O (1 mL) was added Cs₂CO₃ (244.01 mg, 0.748 mmol, 2 equiv) and XPhos Pd G3 (63.39 mg, 0.075 mmol, 0.2 equiv) at room temperature under a nitrogen atmosphere. The resulting mixture was stirred for 2 h at 80 degrees C, then filtered. The filtrated was concentrated under reduced pressure. The residue was purified by reverse flash chromatography with the following conditions: column, C18; mobile phase, MeCN in water (0.05% FA), 40% to 60% gradient. This provided the title compound (85 mg, 53.28%) as a white solid. LCMS (ESI) m/z: [M+H]⁺ = 384.

### Step 4: Preparation of 2-[6-(azetidin-3-yl)thieno[2,3-c]pyridazin-3-yl]phenol

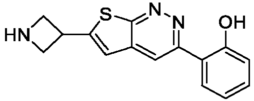

To a stirred mixture of tert-butyl 3-[3-(2-hydroxyphenyl)thieno[2,3-c]pyridazin-6-yl]azetidine-1-carboxylate (85 mg, 0.222 mmol, 1.00 equiv) in DCM (4 mL) was added TFA (1 mL) dropwise at room temperature under a nitrogen atmosphere. The resulting mixture was stirred for 2 h then concentrated under reduced pressure. The crude product was used in the next step directly without further purification. LCMS (ESI) m/z: [M+H]⁺ = 284.

### Preparation of 2-(6-(piperidin-4-yl)thieno[2,3-c]pyridazin-3-yl)phenol (I-7).

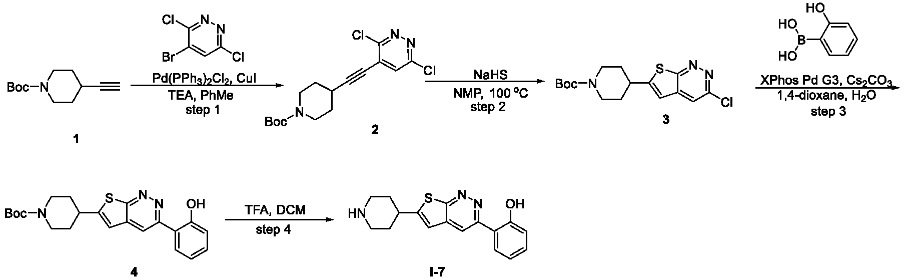

### Step 1: Preparation of tert-butyl 4-((3,6-dichloropyridazin-4-yl)ethynyl)piperidine-1-carboxylate (intermediate 2).

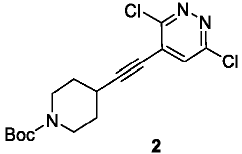

To a stirred mixture of tert-butyl 4-ethynylpiperidine-1-carboxylate (1.00 g, 4.778 mmol, 1.00 equiv) and 4-bromo-3,6-dichloropyridazine (1.20 g, 5.256 mmol, 1.1 equiv) in toluene (10.00 mL) were added Pd(PPh3)2Cl2 (0.50 g, 0.717 mmol, 0.15 equiv), Cul (0.18 g, 0.956 mmol, 0.2 equiv), and TEA (1.45 g, 14.334 mmol, 3 equiv) at room temperature under a nitrogen atmosphere. The resulting mixture was stirred overnight at room temperature then concentrated under reduced pressure. The residue was dissolved in DMF (15.00 mL) and purified by reverse flash chromatography with the following conditions: column, C18; mobile phase, MeCN in water (0.05% FA), 0% to 100% gradient in 25 min to afford intermediate 2 (596 mg, 33.61%) as a brown solid. LCMS (ESI) m/z: [M+H]+ = 356.25

### Step 2: Preparation of tert-butyl 4-(3-chlorothieno[2,3-c]pyridazin-6-yl)piperidine-1-carboxylate (intermediate 3).

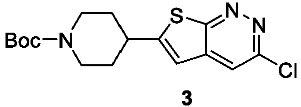

To a stirred solution of intermediate 2 (596.00 mg, 1.673 mmol, 1.00 equiv) in NMP (10.00 mL) was added NaSH (93.79 mg, 1.673 mmol, 1.0 equiv) at room temperature. The resulting mixture was stirred for 1 h at 100 degrees C, then allowed to cool down to room temperature. The resulting mixture was extracted with EtOAc (3 x 200 mL). The combined organic layers were washed with brine (100 mL), dried over anhydrous Na2SO4, and filtered. The filtrate was concentrated under reduced pressure. The residue was dissolved in DMF (10.00 mL) and was purified by reverse flash chromatography with the following conditions: column, C18; mobile phase, MeCN in water (0.05% FA), 0% to 100% gradient in 30 min; to afford intermediate 3 (356 mg, 49.91%) as a brown solid. LCMS (ESI) m/z: [M+H]+ = 353.87.

### Step 3: Preparation of tert-butyl 4-(3-(2-hydroxyphenyl)thieno[2,3-c]pyridazin-6-yl)piperidine-1-carboxylate (intermediate 4).

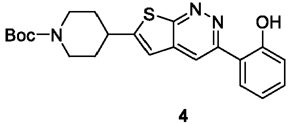

To a solution of intermediate 3 (350.00 mg, 0.989 mmol, 1.00 equiv) and 2-hydroxyphenylboronic acid (204.63 mg, 1.484 mmol, 1.5 equiv) in dioxane (5.00 mL) and H2O (1.00 mL) were added Cs2CO3 (644.51 mg, 1.978 mmol, 2.0 equiv) and XPhos Pd G3 (83.72 mg, 0.099 mmol, 0.1 equiv). After stirring overnight at 90 degrees C under a nitrogen atmosphere the mixture was concentrated under reduced pressure. The residue was dissolved in DMF (10.00 mL) and purified by reverse flash chromatography with the following conditions: column, C18; mobile phase, MeCN in water (0.05% FA), 0% to 100% gradient in 20 min; to afford intermediate 4 (188 mg, 44.85%) as a brown solid. LCMS (ESI) m/z: [M+H]+ = 411.52.

### Step 4: Preparation of 2-(6-(piperidin-4-yl)thieno[2,3-c]pyridazin-3-yl)phenol (I-7).

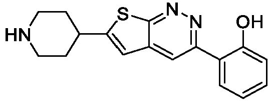

To a stirred solution of intermediate 4 (188.00 mg, 0.457 mmol, 1.00 equiv) in DCM (9.00 mL) was added TFA (3.00 mL) at room temperature. The resulting mixture was stirred for 4 h, then concentrated under reduced pressure. The residue was purified by reverse flash chromatography with the following conditions: column, C18; mobile phase, MeCN in water (0.05 FA), 0% to 100% gradient in 20 min; to afford title compound (130 mg, 91.38%) as a light brown solid. LCMS (ESI) m/z: [M+H]+ = 311.40.

### Preparation of 2-(5-methyl-6-(piperidin-4-yl)thieno[2,3-c]pyridazin-3-yl)phenol (I-8).

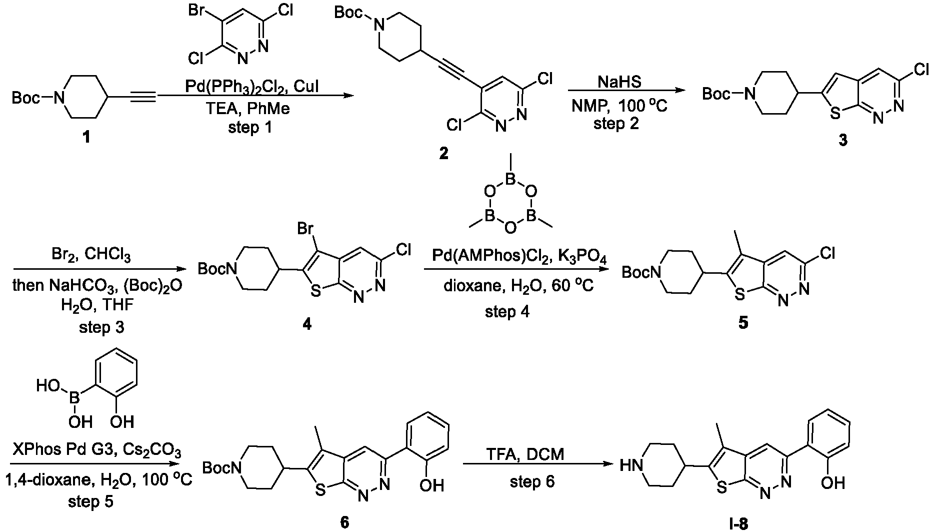

### Step 1: Preparation of tert-butyl 4-((3,6-dichloropyridazin-4-yl)ethynyl)piperidine-1-carboxylate (intermediate 2)

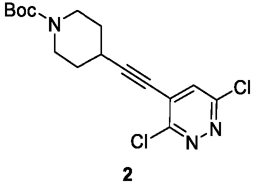

To a mixture of tert-butyl 4-ethynylpiperidine-1-carboxylate (8.00 g, 38.225 mmol, 1.00 equiv) and 4-bromo-3,6-dichloropyridazine (10.45 g, 45.870 mmol, 1.20 equiv) in toluene (80 mL) was added Pd(PPh3)2Cl2 (4.02 g, 5.734 mmol, 0.15 equiv), Cul (14.56 g, 76.450 mmol, 2.00 equiv) and TEA (11.60 g, 114.675 mmol, 3.00 equiv) under a nitrogen atmosphere. The resulting mixture was stirred overnight at room temperature and concentrated under reduced pressure. The residue was purified by silica gel column chromatography, eluted with EtOAc in petroleum ether from 0% to 50% to afford intermediate 2 (5.00 g, 36.7%) as a yellow solid. LCMS (ESI) m/z: [M+H]+ =356.

### Step 2: Preparation of tert-butyl 4-(3-chlorothieno[2,3-c]pyridazin-6-yl)piperidine-1-carboxylate (intermediate 3).

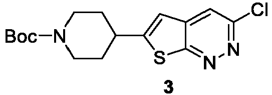

To a mixture of intermediate 2 (5.00 g, 14.035 mmol, 1.00 equiv) in NMP (50 mL) was added NaSH (0.79 g, 14.035 mmol, 1.0 equiv). The resulting mixture was stirred for an hour at 100 degrees C, cooled, and filtered. The filtrate was purified by reverse flash chromatography with the following conditions: column, C18; mobile phase, MeOH in water, 10% to 50% gradient in 10 min; to afford intermediate 3 (1.80 g, 36.2%) as a yellow solid. LCMS (ESI) m/z: [M+H]+ = 354.

### Step 3: Preparation of tert-butyl 4-(5-bromo-3-chlorothieno[2,3-c]pyridazin-6-yl)piperidine-1-carboxylate (intermediate 4).

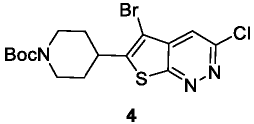

To a mixture of intermediate 3 (1.80 g, 5.087 mmol, 1.00 equiv) in CHCl3 (20 mL) was added Br₂ (8.13 g, 50.870 mmol, 10.00 equiv). The resulting mixture was stirred overnight at room temperature, then basified with aqueous NaHCO₃. Boc₂O (2.21 g, 10.174 mmol, 2.00 equiv) was then added and the mixture was stirred for 2 h. The mixture was extracted with EtOAc (3 x 100 mL) and the combined organic layers were washed with brine (50 mL), dried over anhydrous sodium sulfate, filtered, and concentrated under vacuum. The residue was purified by reverse phase flash chromatography with the following conditions: column, C18; mobile phase, MeOH in water, 10% to 50% gradient in 10 min; to afford intermediate 4 (710.0 mg, 32.4%) as a yellow solid. LCMS (ESI) m/z: [M+H]+ = 432.

### Step 4: Preparation of tert-butyl 4-(3-chloro-5-methylthieno[2,3-c]pyridazin-6-yl)piperidine-1-carboxylate (intermediate 5).

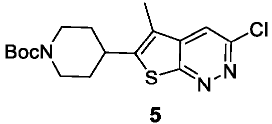

To a mixture of intermediate 4 (710.0 mg, 1.641 mmol, 1.00 equiv), K3PO4 (696.50 mg, 3.282 mmol, 2.00 equiv) and Pd(AMPhos)Cl₂ (174.25 mg, 0.246 mmol, 0.15 equiv) in dioxane (10 mL) and H₂O (2 mL) was added trimethyl-1,3,5,2,4,6-trioxatriborinane (411.90 mg, 3.282 mmol, 2.00 equiv), and the resulting mixture was stirred for an hour at 60 degrees C under a nitrogen atmosphere. The reaction mixture was filtered through a short pad of Celite and eluted with EtOAc. The filtrate was concentrated under vacuum, and the residue purified by reverse phase flash chromatography with the following conditions: column, C18; mobile phase, MeOH in water, 10% to 50% gradient in 10 min; to afford intermediate 5 (450.0 mg, 74.5%) as a yellow solid. LCMS (ESI) m/z: [M+H]+ = 368.

### Step 5: Preparation of tert-butyl 4-(3-(2-hydroxyphenyl)-5-methylthieno[2,3-c]pyridazin-6-yl)piperidine-1-carboxylate (intermediate 6)

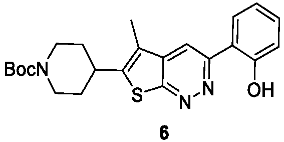

To a mixture of intermediate 5 (450.0 mg, 1.223 mmol, 1.00 equiv) and 2-hydroxyphenylboronic acid (337.43 mg, 2.446 mmol, 2.00 equiv) in dioxane (10 mL) and H₂O (2 mL) were added XPhos Pd G3 (155.31 mg, 0.183 mmol, 0.15 equiv) and Cs₂CO₃ (1.2 g, 3.669 mmol, 3.00 equiv), and the resulting mixture was stirred for an hour at 100 degrees C under a nitrogen atmosphere. The reaction mixture was filtered through a short pad of Celite and eluted with EtOAc. The filtrate was concentrated under vacuum, and the residue was purified by reverse flash chromatography with the following conditions: column, C18; mobile phase, MeOH in water, 10% to 50% gradient in 10 min; to afford intermediate 6 (380.0 mg, 73.0 %) as a yellow solid. LCMS (ESI) m/z: [M+H]+ = 426.

### Step 6: Preparation of 2-(5-methyl-6-(piperidin-4-yl)thieno[2,3-c]pyridazin-3-yl)phenol (I-8).

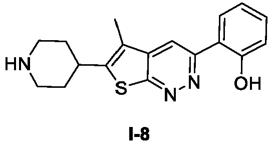

To a mixture of intermediate 6 (380.0 mg, 0.893 mmol, 1.00 equiv) in DCM (6 mL) was added TFA (3 mL, 40.389 mmol, 45.23 equiv), and the resulting mixture was stirred for 2 h at room temperature. The reaction mixture was concentrated under reduced pressure, and the residue purified by reverse flash chromatography with the following conditions: column, C18; mobile phase, MeOH in water, 10% to 50% gradient in 10 min; detector to afford **I-8** (246.4 mg, 84.7%) as a yellow solid. 1H NMR (400 MHz, DMSO-d6) δ 8.69 (s, 1H), 8.22-8.11 (m, 1H), 7.43-7.34 (m, 1H), 7.07-6.99 (m, 2H), 3.67-3.53 (m, 1H), 3.43-3.35 (m, 2H), 3.10-3.00 (m, 2H), 2.46 (s, 3H), 2.13-2.04 (m, 2H), 1.91-1.78 (m, 2H). LCMS (ESI) m/z: [M+H]+ =326.10.

### Preparation of methyl 2-[3-(6-fluoro-5-methylpyridin-3-yl)-1,2-oxazol-5-yl]-3-methylbutanoate (I-9)

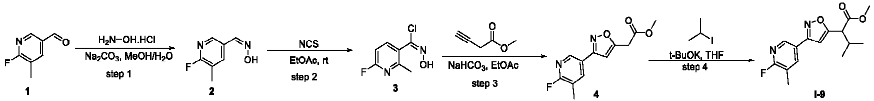

### Step 1: Preparation of (Z)-N-[(6-fluoro-5-methylpyridin-3-yl)methylidene] hydroxylamine (intermediate 2)

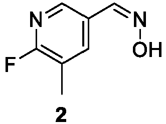

To a solution of 6-fluoro-5-methylpyridine-3-carbaldehyde (10 mg, 0.072 mmol, 1 equiv) ,hydroxylamine hydrochloride (9.99 mg, 0.144 mmol, 2 equiv) and Na₂CO₃ (22.85 mg, 0.216 mmol, 3 equiv) in MeOH (0.5 mL) and H₂O (0.5 mL), the resulting solution was stirred at 25 degrees C for overnight. The mixture was diluted with EtOAc (200 mL) and washed with water (200 mL x 3). The organic layer was dried over anhydrous sodium sulfate, filtered and concentrated to give intermediate **2** (2.2 mg, 99.29%) as a yellow solid. LCMS (ESI) m/z: [M+H]⁺ =155.

### Step 2: Preparation of (E)-6-fluoro-N-hydroxy-2-methylpyridine-3-carbonimidoyl chloride (intermediate 3)

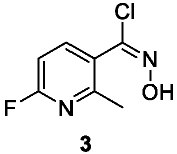

To a solution of intermediate **2** (2.2 g, 14.272 mmol, 1 equiv), NCS (2.86 g, 21.408 mmol, 1.5 equiv) in EtOAc (20 mL), the resulting solution was stirred at 25 degrees C for overnight. The mixture was diluted with EtOAc (100 mL) and washed with water (100 mL x 2). The organic layer was dried over anhydrous sodium sulfate, filtered and concentrated to give a intermediate **3** (3.6 g, crude) as a white solid. LCMS (ESI) m/z: [M+H]⁺ =189.

### Step 3: Preparation of methyl 2-[3-(6-fluoro-5-methylpyridin-3-yl)-1,2-oxazol-5-yl] acetate (intermediate 4)

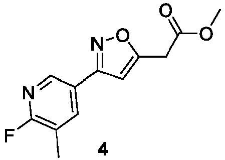

To a solution of intermediate **3** (3.6 g, 19.089 mmol, 1 equiv) in EtOAc (14 mL), were added methyl but-3-ynoate (3.75 g, 38.178 mmol, 2 equiv) and NaHCO₃ (4.81 g, 57.267 mmol, 3 equiv) at 0 degrees . the resulting solution was stirred at 25 degrees C for overnight. The mixture was diluted with EtOAc (100 mL) and washed with water (100 mL x 3). The organic layer was dried over anhydrous sodium sulfate, filtered and concentrated to give crude products. The residue was purified by reverse flash chromatography with the following conditions: column, C18 silica gel; mobile phase, ACN in H₂O, 0% to 100% gradient in 30 min to give intermediate **4** (2.5 g, 52.34%) as a white solid. LCMS (ESI) m/z: [M+H]⁺ =251.

### Step 4: Preparation of methyl 2-[3-(6-fluoro-5-methylpyridin-3-yl)-1,2-oxazol-5-yl]-3-methylbutanoate (I-9)

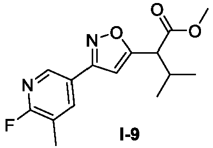

To a solution of intermediate **4** (500 mg, 1.998 mmol, 1 equiv), 2-iodopropane (679.35 mg, 3.996 mmol, 2 equiv), Cs₂CO₃ (1.3 g, 3.996 mmol, 2 equiv) in THF (5 mL), the resulting solution was stirred at 60 degrees C for overnight. The mixture was diluted with EtOAc (100 mL) and washed with water (100 mL x 2). The organic layer was dried over anhydrous sodium sulfate, filtered and concentrated to give a crude product. The crude product was purified by flash C18 chromatography, elution gradient 0 to 60% ACN in H₂O to give title compound (288 mg, 49.31%) as a white solid. LCMS (ESI) m/z: [M+H]⁺ =293.

### Preparation of 2-(5-cyclopropyl-6-(2,6-diazaspiro[3.3]heptan-2-yl)thieno[2,3-c]pyridazin-3-yl)phenol (I-11)

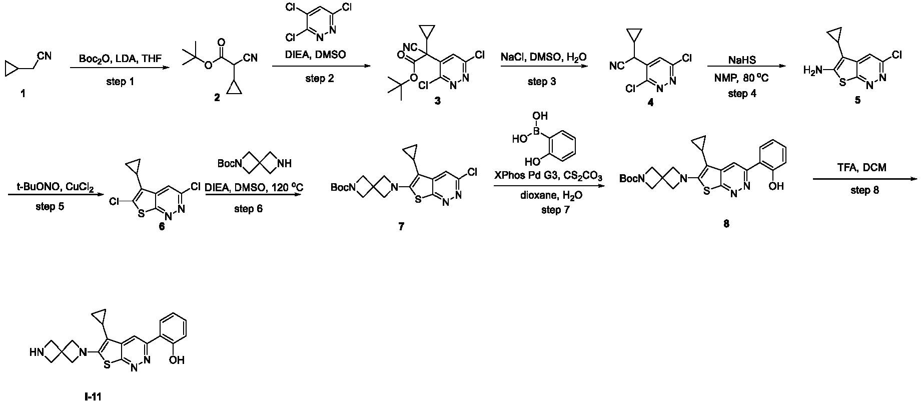

### Step 1: Preparation of tert-butyl 2-cyano-2-cyclopropylacetate (Intermediate 2)

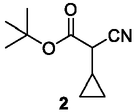

To a solution of 2-cyclopropylacetonitrile (10.00 g, 123.277 mmol, 1 equiv) and Boc₂O (53.81 g, 246.554 mmol, 2 equiv) in THF (100 mL) was added LDA (26.41 g, 246.554 mmol, 2 equiv)at - 78 degress C. After stirring for 2 hrs at -78 degress C, the reaction was quenched with water (100 mL) at 0 degress C. The resulting mixture was extracted with EtOAc (100 mL x 3). The combined organic layers were washed with brine (100 mL), dried over anhydrous sodium sulfate. After filtration, the filtrate was concentrated under reduced pressure. The residue was purified by silica gel column chromatography, eluted with EtOAc in PE from 0% to 20% to afford intermediate **2** (20.00 g, 80.57%) as yellow oil. No Ms signal, confirmed by H-NMR.

### Step 2: tert-butyl 2-cyano-2-cyclopropyl-2-(3,6-dichloropyridazin-4-yl)acetate (Intermediate 3).

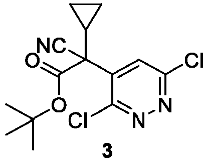

To a solution of intermediate **2** (15.00 g, 82.765 mmol, 1 equiv) and 3,4,6-trichloropyridazine (15.18 g, 82.765 mmol, 1 equiv) in DMSO (100 mL) was added DIEA (32.09 g, 248.295 mmol, 3 equiv). After stirring overnight at room temperature under a nitrogen atmosphere, the resulting mixture was diluted with water (500 mL). The resulting mixture was extracted with EtOAc (500 mL x 3). The combined organic layers were washed with brine (500 mL), dried over anhydrous sodium sulfate. After filtration, the filtrate was concentrated under reduced pressure. The residue was purified by silica gel column chromatography, eluted with EtOAc in PE from 0% to 50% to afford intermediate **3** (18.00 g, 59.64%) as a pink solid. LCMS (ESI) m/z: [M+H]⁺ =328.

### Step 3: Preparation of 2-cyclopropyl-2-(3,6-dichloropyridazin-4-yl)acetonitrile (Intermediate 4).

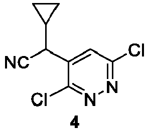

To a solution of intermediate **3** (10.00 g, 30.470 mmol, 1 equiv) in DMSO (50 mL) and H₂O (5 mL) was added NaCl (3.56 g, 60.940 mmol, 2 equiv). After stirring for 3 hrs at 100 degress C, the resulting mixture was diluted with water (500 mL). The resulting mixture was extracted with EtOAc (500 mL x 3). The combined organic layers were washed with brine (500 mL), dried over anhydrous sodium sulfate. After filtration, the filtrate was concentrated under reduced pressure. The residue was purified by silica gel column chromatography, eluted with EtOAc in PE from 0% to 50% to afford intermediate **4** (5.00 g, 64.7%) as a yellow solid. LCMS(ESI) m/z: [M+H]⁺ = 228.

### Step 4: Preparation of 3-chloro-5-cyclopropylthieno[2,3-c]pyridazin-6-amine (Intermediate 5).

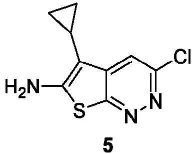

To a solution of intermediate **4** (3.00 g, 13.153 mmol, 1 equiv) in NMP (50 mL) was added NaHS.2H₂O (1.21 g, 13.153 mmol, 1 equiv). After stirring for 20 mins at 100 degress C, the resulting mixture was diluted with water (500 mL). The resulting mixture was extracted with EtOAc (200 mL x 3). The combined organic layers were washed with brine (200 mL), dried over anhydrous sodium sulfate. After filtration, the filtrate was concentrated under reduced pressure. The residue was purified by silica gel column chromatography, eluted with EtOAc in PE from 0% to 50% to afford intermediate 5 (2.00 g, 60.6%) as a yellow solid. LCMS (ESI) m/z: [M+H]⁺ = 226.

### Step 5: Preparation of 3,6-dichloro-5-cyclopropylthieno[2,3-c]pyridazine (Intermediate 6).

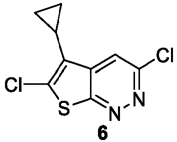

To a solution of intermediate **5** (2.00 g, 8.862 mmol, 1 equiv) and CuCl₂ (2.38 g, 17.724 mmol, 2 equiv) in ACN (50 mL) was added t-BuNO₂ (1.83 g, 17.724 mmol, 2 equiv). After stirring for 3 hrs at 50 degress C, the resulting mixture was concentrated under reduced pressure. The residue was purified by silica gel column chromatography, eluted with EtOAc in PE from 0% to 50% to afford intermediate **6** (1.00 g, 41.4%) as a yellow solid. LCMS (ESI) m/z: [M+H]⁺ = 245.

### Step 6: Preparation of tert-butyl 6-(3-chloro-5-cyclopropylthieno[2,3-c]pyridazin-6-yl)-2,6-diazaspiro[3.3]heptane-2-carboxylate (Intermediate 7).

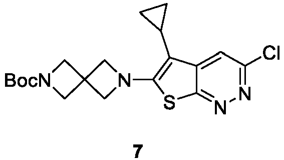

To a solution of intermediate **6** (1.00 g, 4.080 mmol, 1 equiv) and tert-butyl 2,6-diazaspiro[3.3]heptane-2-carboxylate (1.62 g, 8.160 mmol, 2 equiv) in DMSO (20 mL) was added DIEA (1.58 g, 12.240 mmol, 3 equiv). After stirring for 3 hrs at 100 degress C under a nitrogen atmosphere, the resulting mixture was diluted with water (100 mL). The resulting mixture was extracted with EtOAc (100 mL x 3). The combined organic layers were washed with brine (200 mL), dried over anhydrous sodium sulfate. After filtration, the filtrate was concentrated under reduced pressure. The residue was purified by silica gel column chromatography, eluted with EtOAc in PE from 0% to 50% to afford intermediate **7** (900.0 mg, 48.7%) as a yellow solid. LCMS (ESI) m/z: [M+H]⁺ = 407.

### Step 7: Preparation of tert-butyl 6-(5-cyclopropyl-3-(2-hydroxyphenyl)thieno[2,3-c]pyridazin-6-yl)-2,6-diazaspiro[3.3]heptane-2-carboxylate (Intermediate 7).

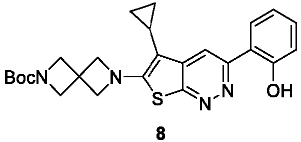

To a solution of intermediate **7** (900.0 mg, 2.212 mmol, 1 equiv) and 2-hydroxyphenylboronic acid (610.11 mg, 4.424 mmol, 2 equiv) in dioxane (20 mL) and H₂O (4 mL) were added Cs₂CO₃ (2161.8 mg, 6.636 mmol, 3 equiv) and XPhos Pd G3 (187.2 mg, 0.221 mmol, 0.1 equiv). After stirring for an hour at 80 degress C under a nitrogen atmosphere, the resulting mixture was diluted with water (50 mL). The resulting mixture was extracted with EtOAc (50 mL x 3). The combined organic layers were washed with brine (100 mL), dried over anhydrous sodium sulfate. After filtration, the filtrate was concentrated under reduced pressure. The residue was purified by reversed-phase flash chromatography with the following conditions: column, C₁₈ silica gel; mobile phase, ACN in water, 10% to 50% gradient in 10 min; detector, UV 254 nm to afford intermediate **8** (800.0 mg, 70.0%) as a yellow solid. LCMS (ESI) m/z: [M+H]⁺ = 465.

### Step 8: Preparation of 2-(5-cyclopropyl-6-(2,6-diazaspiro[3.3]heptan-2-yl)thieno[2,3-c]pyridazin-3-yl)phenol (I-11).

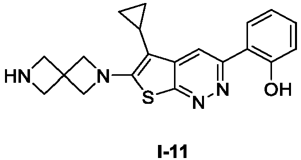

To a solution of intermediate **8** (500.0 mg, 1.076 mmol, 1 equiv) in DCM (9 mL) was added TFA (3 mL, 40.389 mmol, 37.53 equiv). After stirring for an hour at room temperature, the resulting mixture was concentrated under reduced pressure to afford **I-11** (700.0 mg, crude) as semi-solid. The crude product was used in next step directly without further purification. LCMS (ESI) m/z: [M+H]⁺ = 365.

### Preparation of 2-(6-{2,6-diazaspiro[3.3]heptan-2-yl}-5-methylthieno[2,3-c]pyridazin-3-yl)phenol (I-12)

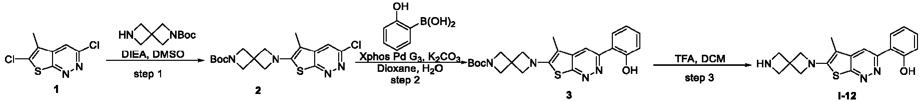

### Step 1: Preparation of tert-butyl 6-{3-chloro-5-methylthieno[2,3-c]pyridazin-6-yl}-2,6-diazaspiro[3.3]heptane-2-carboxylate (Intermediate 2)

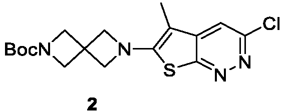

A solution of 3,6-dichloro-5-methylthieno[2,3-c]pyridazine (639.12 mg, 2.916 mmol, 2.41 equiv) in DMSO (8 mL) was added tert-butyl 2,6-diazaspiro[3.3]heptane-2-carboxylate (240 mg, 1.210 mmol, 1.00 equiv) and DIEA (469.36 mg, 3.630 mmol, 3 equiv) .The resulting mixture was stirred for 6 h at 60 °C under nitrogen atmosphere.Desired product could be detected by LCMS.The residue was purified by reversed-phase flash chromatography with the following conditions: column, C18 silica gel; mobile phase, MeCN in Water (0.1% FA), 10% to 80% gradient in 30 min; detector, UV 254 nm.This resulted in intermediate **2** (280 mg, 42.51%) as a yellow oil. LCMS (ESI) m/z: [M+H]⁺ = 381.

### Step 2: Preparation of tert-butyl 6-[3-(2-hydroxyphenyl)-5-methylthieno[2,3-c]pyridazin-6-yl]-2,6-diazaspiro[3.3]heptane-2-carboxylate (Intermediate 3)

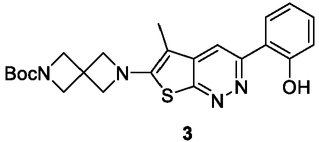

A solution of tert-butyl 6-{3-chloro-5-methylthieno[2,3-c]pyridazin-6-yl}-2,6-diazaspiro[3.3]heptane-2-carboxylate (300 mg, 0.788 mmol, 1 equiv) and 2-hydroxyphenylboronic acid (217.28 mg, 1.576 mmol, 2 equiv) in dioxane (6 mL) and H₂O (1 mL) was added Cs2CO3 (769.87 mg, 2.364 mmol, 3 equiv) and XPhos Pd G3 (66.67 mg, 0.079 mmol, 0.1 equiv) .The resulting mixture was stirred for 3 h at 80 °C under nitrogen atmosphere.The resulting mixture was concentrated under vacuum. The residue was purified by silica gel column chromatography, eluted with PE / EA (1:1) to intermediate 3 (160 mg, 46.32%) as a yellow solid. LCMS (ESI) m/z [M+H]⁺ = 439.

### Step 3: Preparation of 2-(6-{2,6-diazaspiro[3.3]heptan-2-yl}-5-methylthieno[2,3-c]pyridazin-3-yl)phenol (I-12)

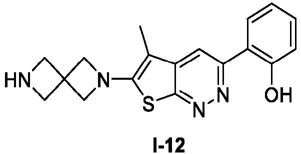

A solution of intermediate 3 (150 mg, 0.342 mmol, 1 equiv) in TFA (1 mL) and DCM (3 mL) was stirred for 1 h at room temperature. Desired product could be detected by LCMS.The resulting mixture was concentrated under vacuum. This resulted in **1-12** (100 mg, 86.39%) as a yellow oil. LCMS (ESI) m/z: [M+H]⁺ = 339.

### Preparation of 2-(6-{2,6-diazaspiro[3.3]heptan-2-yl}thieno[3,2-c]pyridazin-3-yl)phenol) (I-13)

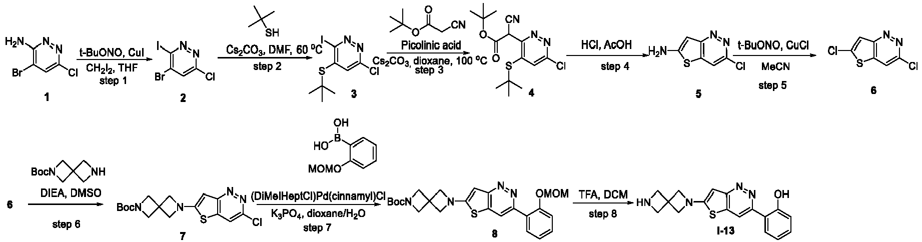

### Step 1: Preparation of 4-bromo-6-chloro-3-iodopyridazine (Intermediate 2)

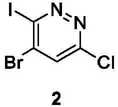

To a stirred solution of 4-bromo-6-chloropyridazin-3-amine (43.00 g, 206.294 mmol, 1 equiv) and CH₂I₂ (66.30 g, 247.553 mmol, 1.2 equiv) in THF (300 mL) were added Cul (47.15 g, 247.553 mmol, 1.2 equiv) and t-BuNO₂ (25.53 g, 247.553 mmol, 1.2 equiv).The resulting mixture was stirred overnight at 60 ° C. The resulting mixture was concentrated under reduced pressure. The residue was purified by silica gel column chromatography, eluted with PE / EtOAc (10:1) to afford intermediate **2** (25.00 g, 58.1%) as a light yellow solid. LCMS (ESI) m/z: [M+H]⁺ = 319.

### Step 2: Preparation of 4-(tert-butylsulfanyl)-6-chloro-3-iodopyridazine (Intermediate 3)

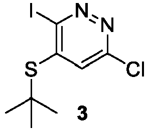

To a stirred solution of interrmediate 2 (25.00 g, 78.291 mmol, 1 equiv) and 2-methyl-2-propanethiol (7.77 g, 86.120 mmol, 1.1 equiv) in DMF (300 mL) was added Cs₂CO₃ (76.3 g, 234.873 mmol, 3 equiv). The resulting mixture was stirred for 1h at 100 °C. The resulting mixture was concentrated under reduced pressure. The resulting mixture was diluted with water (300 mL). The resulting mixture was extracted with EtOAc (3 x 500mL). The combined organic layers were washed with brine (1 x 200 mL), dried over anhydrous sodium sulfate. After filtration, the filtrate was concentrated under reduced pressure. The residue was purified by silica gel column chromatography, eluted with PE / EtOAc (10:1) to afford intermediate 3 (16.00 g, 62.2%) as a yellow solid. LCMS (ESI) m/z: [M+H]⁺ = 329.

### Step 3: Preparation of tert-butyl 2-[4-(tert-butylsulfanyl)-6-chloropyridazin-3-yl]-2-cyanoacetate) (Intermediate 4)

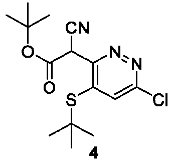

A solution of intermediate 3 (16.00 g, 48.691 mmol, 1 equiv) and tert-butyl 2-cyanoacetate (13.75 g, 97.382 mmol, 2 equiv) and Cs₂CO₃ (47.4 g, 146.073 mmol, 3 equiv) in 1,4-dioxane was stirred for 30 mins at room temperature. To the above mixture was added picolinic acid (3.00 g, 24.346 mmol, 0.5 equiv). The resulting mixture was stirred for additional 2 h at 80 ° C. The resulting mixture was concentrated under reduced pressure. The residue was purified by silica gel column chromatography, eluted with DCM/MeOH (10:1) to afford in intermediate **4** (5.30 g, 33.1%) as a brown yellow solid. LCMS (ESI) m/z: [M+H]⁺ = 342.

### Step 4: Preparation of 3-chlorothieno[3,2-c]pyridazin-6-amine (Intermediate 5)

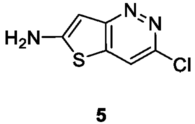

A solution of intermediate **4** (5.3 g, 15.504 mmol, 1 equiv) and 6 N HCl (50 mL) in AcOH (50 mL) was stirred overnight at 80 °C. The resulting mixture was concentrated under reduced pressure. The residue was neutralized to pH 7 with Saturated sodium bicarbonate solution. The resulting mixture was extracted with EtOAc (3 x 300mL). The combined organic layers were washed with brine (2 x 200 mL), dried over anhydrous sodium sulfate. After filtration, the filtrate was concentrated under reduced pressure. This resulted in intermediate **5** (3.60 g, crude) as a yellow solid. The crude was used in the next step directly without further purification. LCMS (ESI) m/z: [M+H]⁺ = 186.

### Step 5: Preparation of 3,6-dichlorothieno[3,2-c]pyridazine) (Intermediate 6)

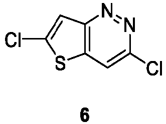

To a stirred solution of intermediate **5** (3.60 g, 19.393 mmol, 1 equiv) and CuCl (3.84 g, 38.786 mmol, 2 equiv) in MeCN (50 mL) was added tert-butyl nitrite (4.00 g, 38.789 mmol, 2 equiv) in portions at 0° C. The resulting mixture was stirred for 2 h 50 °C. The resulting mixture was concentrated under reduced pressure. The residue was purified by silica gel column chromatography, eluted with PE / EtOAc (1:1) to afford intermediate **6** (543.0 mg, 15.0%)as a light brown solid. LCMS (ESI) m/z: [M+H]⁺ = 205.

### Step 6: Preparation of tert-butyl 6-(3-chlorothieno[3,2-c]pyridazin-6-yl)-2,6-diazaspiro[3.3]heptane-2-carboxylate (Intermediate 7)

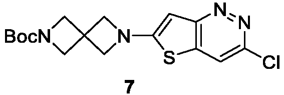

To a stirred solution of intermediate **6** (543.0 mg, 2.648 mmol, 1 equiv) and tert-butyl 2,6-diazaspiro[3.3]heptane-2-carboxylate (525.01 mg, 2.648 mmol, 1 equiv) in DMSO (8 mL) was added DIEA (1.03 g, 7.944 mmol, 3 equiv). The resulting mixture was stirred for 1h at 100° C. The residue was purified by reversed-phase flash chromatography with the following conditions: column, C₁₈ silica gel; mobile phase, MeCN in Water (0.1% FA), 10% to 50% gradient in 10 min; detector, UV 254 nm. This resulted in intermediate 7 (253.0 mg, 46.5%) as a brown yellow solid. LCMS (ESI) m/z: [M+H]⁺ = 367.

### Step 7: Preparation of tert-butyl 6-[3-(2-hydroxyphenyl)thieno[3,2-c]pyridazin-6-yl]-2,6-diazaspiro[3.3]heptane-2-carboxylate (Intermediate 8)

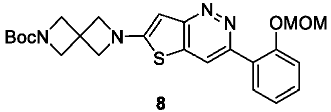

To a stirred solution of intermediate **7** (253.0 mg, 0.690 mmol, 1 equiv) and 2-(methoxymethoxy)phenylboronic acid (125.5 mg, 0.690 mmol, 1 equiv) in 1,4-dioxane (4 mL) and H₂O (1 mL) were added K₃PO₄ (292.7 mg, 1.380 mmol, 2 equiv) and (DiMelHeptCl)Pd(cinnamyl)Cl (80.5 mg, 0.069 mmol, 0.1 equiv) .The resulting mixture was stirred for 2h, the mixture was concentrated under reduced pressure. The resulting mixture was diluted with water (50 mL). The resulting mixture was extracted with EtOAc (3 x 50 mL). The combined organic layers were washed with brine (1 x 50 mL), dried over anhydrous sodium sulfate. After filtration, the filtrate was concentrated under reduced pressure. The residue was purified by reversed-phase flash chromatography with the following conditions: column, C₁₈ silica gel; mobile phase, MeCN in Water (0.1% FA), 10% to 50% gradient in 10 min; detector, UV 254 nm. This resulted in intermediate **8** (98.0 mg,41.8% ) as a brown yellow solid. LCMS (ESI) m/z: [M+H]⁺ = 469.

### Step 8: Preparation of 2-(6-{2,6-diazaspiro[3.3]heptan-2-yl}thieno[3,2-c]pyridazin-3-yl)phenol) (I-13)

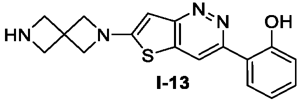

A solution of intermediate 8 (98.0 mg, 0.213 mmol, 1 equiv) and TFA (0.30 mL, 4.039 mmol, 19.31 equiv) in DCM (0.9 mL) was stirred for 30 mins at room temperature. The resulting mixture was concentrated under reduced pressure. This resulted in 1-13 (106.0 mg, crude) as reddish oil. The crude was used in the next step directly without further purification. LCMS (ESI) m/z: [M+H]⁺ = 325

### Preparation of (2S,4R)-4-hydroxy-1-[(2R)-2-(3-{4-[2-(4-({-[(3Z)-2-hydroxypenta-1,3-dien-3-yl]-5-methylthieno[2,3-c]pyridazin-6-yl}piperidin-1-yl)pyrimidin-5-yl]piperazin-1-yl}-1,2-oxazol-5-yl)-3-methylbutanoyl]-N-[(1S)-1-[4-(4-methyl-1,3-thiazol-5-yl)phenyl]ethyl]pyrrolidine-2-carboxamide (Compound 14)

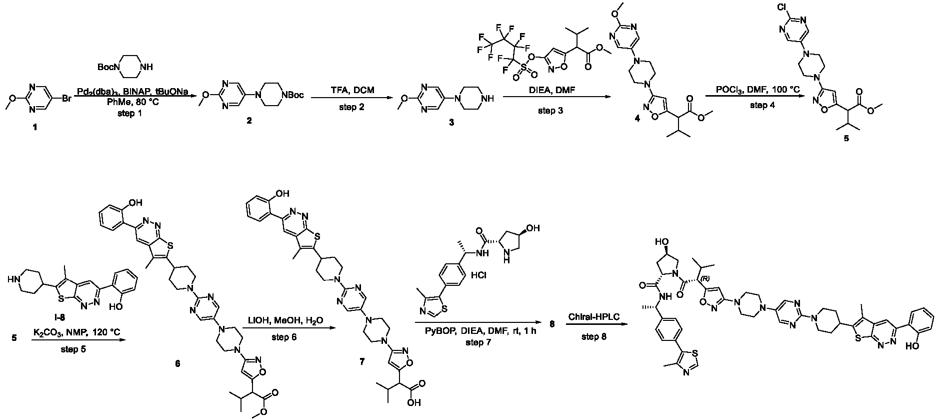

### Step 1: Preparation of tert-butyl 4-(2-methoxypyrimidin-5-yl )piperazine-1-carboxylate (intermediate 2)

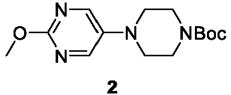

To a solution of 5-bromo-2-methoxypyrimidine (9 g, 47.616 mmol, 1 equiv), tert-butyl piperazine-1-carboxylate (13.30 g, 71.424 mmol, 1.5 equiv), Pd₂(dba)₃ (2.18 g, 2.381 mmol, 0.05 equiv), BINAP (2.96 g, 4.762 mmol, 0.1 equiv) in toluene (75 mL), the resulting solution was stirred at 80 degrees C for 6 h. Desired product could be detected by LCMS. The residue was purified by reverse flash chromatography with the following conditions: column, C18 silica gel; mobile phase, ACN in H₂O, 0% to 25% gradient in 30 min to give intermediate **2** (4.2 g, 29.97%) as a white solid. LCMS (ESI) m/z: [M+H]⁺ =295.

### Step 2: Preparation of 2-methoxy-5-(piperazin-1-yl) pyrimidine (intermediate 3)

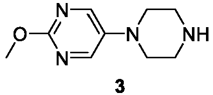

To a solution of intermediate **2** (4.2 g, 14.268 mmol, 1 equiv) in TFA (10 mL) and DCM (30 mL), the resulting solution was stirred at 25 degrees C for 5 h. The DCM in the reaction solution is rotated out in vacuum to give a crude intermediate **3** (10.0 g, crude) as a yellow oil. LCMS (ESI) m/z: [M+H]⁺ =195.

### Step 3: Preparation of methyl 2-{3-[4-(2-methoxypyrimidin-5-yl)piperazin-1-yl]-1,2-oxazol-5-yl}-3-methylbutanoate (intermediate 4)

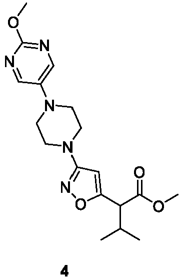

To a solution of intermediate 3 (10 g, 51.483 mmol, 1 equiv), methyl 3-methyl-2-{3-[(1,1,2,2,3,3,4,4,4-nonafluorobutanesulfonyl)oxy]-1,2-oxazol-5-yl}butanoate (24.78 g, 51.483 mmol, 1 equiv) and DIEA (19.96 g, 154.449 mmol, 3 equiv) in DMSO (40 mL), the resulting solution was stirred at 100 degrees C for overnight. Desired product could be detected by LCMS. The residue was purified by reverse flash chromatography with the following conditions: column, C18 silica gel; mobile phase, MeCN in water, 0 to 30% gradient in 30 min; detector, UV 220/200 nm to give intermediate **4** (3.7 g, 19.14%). LCMS (ESI) m/z: [M+H]⁺ =376.

### Step 4: Preparation of methyl 2-{3-[4-(2-chloropyrimidin-5-yl)piperazin-1-yl]-1,2-oxazol-5-yl}-3-methylbutanoate (intermediate 5)

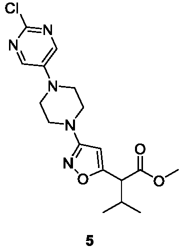

To a solution of intermediate **4** (900 mg, 2.397 mmol, 1 equiv) in POCl₃ (6 mL), the resulting solution was stirred at 110 degrees C for 18h. Desired product could be detected by LCMS. The residue was purified by reverse flash chromatography with the following conditions: column, C18 silica gel; mobile phase, MeCN in water (0.1% FA), 0 to 58% gradient in 30 min; detector, UV 220/200 nm. This resulted in give intermediate **5** (360 mg, 39.53%) as a yellow solid.

### Step 5: Preparation ofmethyl 2-{3-[4-(2-{4-[3-(2-hydroxyphenyl)-5-methylthieno[2,3-c]pyridazin-6-yl]piperidin-1-yl}pyrimidin-5-yl)piperazin-1-yl]-1,2-oxazol-5-yl}-3-methylbutanoate (intermediate 6)

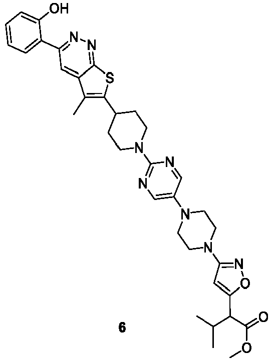

To a solution of intermediate **5** (200 mg, 0.527 mmol, 1 equiv) and 2-[5-methyl-6-(piperidin-4-yl)thieno[2,3-c]pyridazin-3-yl]phenol (342.69 mg, 1.054 mmol, 2 equiv) in dioxane (4 mL) was added Cs₂CO₃ (343.10 mg, 1.054 mmol, 2 equiv) and Pd-PEPPSI-IPentCl 2-methylpyridine (o-picoline (88.58 mg, 0.105 mmol, 0.2 equiv), the resulting solution was stirred at 90 degrees C for overnight. Desired product could be detected by LCMS. The residue was purified by reverse flash chromatography with the following conditions: column, silica gel; mobile phase, PE in EA, 0% to 56% to give intermediate **6** (100 mg, 28.4%) as a yellow solid. LCMS (ESI) m/z: [M+H]⁺ =668.

### Step 6: Preparation of 2-{3-[4-(2-{4-[3-(2-hydroxyphenyl)-5-methylthieno[2,3-c]pyridazin-6-yl]piperidin-1-yl}pyrimidin-5-yl)piperazin-1-yl]-1,2-oxazol-5-yl}-3-methylbutanoic acid (intermediate 7)

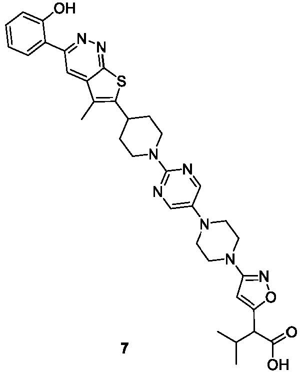

To a solution of methyl intermediate **6** (95 mg, 0.142 mmol, 1 equiv) in MeOH (4 mL) and H₂O (1 mL) was added LiOH (27.22 mg, 1.136 mmol, 8 equiv), the resulting solution was stirred at room temperature for 16 hours. The mixture was acidified to pH 5 with 1 M HCl (aq.). The mixture was diluted with EtOAc (50 mL) and washed with water (50 mL x 3). The organic layer was dried over anhydrous sodium sulfate, filtered and concentrated to give intermediate **7** (98 mg, crude) as a yellow solid that was used in the next step directly without further purification. LCMS (ESI) m/z: [M+H]⁺ =655.

### Step 7: Preparation of (2S,4R)-4-hydroxy-1-(2-{3-[4-(2-{4-[3-(2-hydroxyphenyl)-5-methylthieno[2, 3-c]pyridazin-6-yl]piperidin-1-yl}pyrimidin-5-yl)piperazin-1-yl]-1,2-oxazol-5-yl}-3-methylbutanoyl)-N-[(1S)-1-[4-(4-methyl-1, 3-thiazol-5-yl)phenyl]ethyl]pyrrolidine-2-carboxamide (intermediate 8)

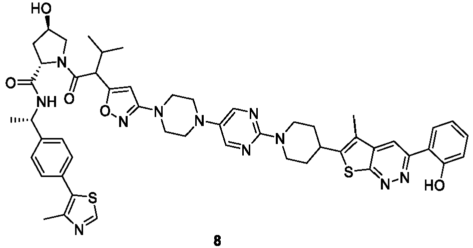

To a solution of intermediate **7** (98 mg, 0.150 mmol, 1 equiv), (2S,4R)-4-hydroxy-N-[(1S)-1-[4-(4-methyl-1,3-thiazol-5-yl)phenyl]ethyl]pyrrolidine-2-carboxamide (99.21 mg, 0.300 mmol, 2 equiv) and PyBOP (155.77 mg, 0.300 mmol, 2 equiv) in DMF (2 mL), the resulting solution was stirred at 25 degrees C for 10 minutes, then DIEA (96.72 mg, 0.750 mmol, 5 equiv) was added to the mixture. The resulting solution was stirred at 25 degrees C for 2 hours. The reaction was purified by flash C18 chromatography, elution gradient 0 to 83% ACN in H₂O to give intermediate 8 (87 mg, 59.28%) as a yellow solid. LCMS (ESI) m/z: [M+H]⁺ =968.

### Step 8: Preparation of (2S,4R)-4-hydroxy-1-[(2R)-2-(3-{4-[2-(4-{3-[(3Z)-2-hydroxypenta-1,3-dien-3-yl]-5-methylthieno[2,3-c]pyridazin-6-yl}piperidin-1-yl)pyrimidin-5-yl]piperazin-1-yl}-1,2-oxazol-5-yl)-3-methylbutanoyl]-N-[(1S)-1-[4-(4-methyl-1,3-thiazol-5-yl)phenyl]ethyl]pyrrolidine-2-carboxamide

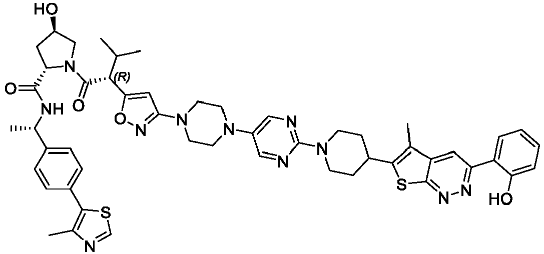

The Intermediate **8** (94 mg) was purified by CHIRAL_HPLC with the following conditions (Column: CHIRAL ART Cellulose-SB, 2*25 cm, 5 µm; Mobile Phase A: MtBE (10mM NH₃-MeOH), Mobile Phase B: MeOH--HPLC; Flow rate: 20 mL/min; Gradient: 20% B to 20% B in 20 min; Wave Length: 270/212 nm; RT1(min): 10.4; RT2(min): 14.3; Sample Solvent: MeOH--HPLC; Injection Volume: 0.5 mL; Number Of Runs: 6 to afford title compound (second peak) (37.6 mg, 25.62%) as an off-white solid. The resulting solid was dried by lyophilization. ¹H NMR (300 MHz, DMSO-d6) *δ* 12.79 (s, 1H), 8.99 (s, 1H), 8.71 (s, 1H), 8.42 (d, J = 7.6 Hz, 1H), 8.28 (s, 2H), 8.17 (d, J = 7.4 Hz, 1H), 7.52 - 7.41 (m, 2H), 7.40 - 7.33 (m, 3H), 7.08 - 6.97 (m, 2H), 6.25 (s, 1H), 5.12 (d, J = 3.7 Hz, 1H), 5.01 - 4.86 (m, 1H), 4.75 (d, J = 13.1 Hz, 2H), 4.38 (t, J = 7.8 Hz, 1H), 4.30 (s, 1H), 3.73 (dd, J = 10.4, 4.3 Hz, 1H), 3.60 (d, J = 10.0 Hz, 2H), 3.45 (d, J = 10.1 Hz, 1H),3.41 - 3.36 (m, 4H), 3.16 - 2.97 (m, 6H), 2.49 (s, 3H), 2.46 (s, 3H), 2.24 - 2.19 (m, 1H), 2.18 - 1.92 (m, 3H), 1.87 - 1.78 (m, 1H), 1.71 - 1.55 (m, 2H), 1.47 (d, J = 7.0 Hz, 1H), 1.39 (d, J = 7.0 Hz, 2H), 0.97 (d, J = 6.5 Hz, 3H), 0.87 - 0.77 (m, 3H). LCMS (ESI) m/z: [M+H]⁺ = 968.40.

### Preparation of (2S,4R)-4-hydroxy-1-[(2R)-2-(3-{2-[3-(2-hydroxyphenyl)-5-methylthieno[2,3-c]pyridazin-6-yl]ethynyl}-1,2-oxazol-5-yl)-3-methylbutanoyl]-N-[(1S)-1-[4-(4-methyl-1,3-thiazol-5-yl)phenyl]ethyl]pyrrolidine-2-carboxamide (Compound 37)

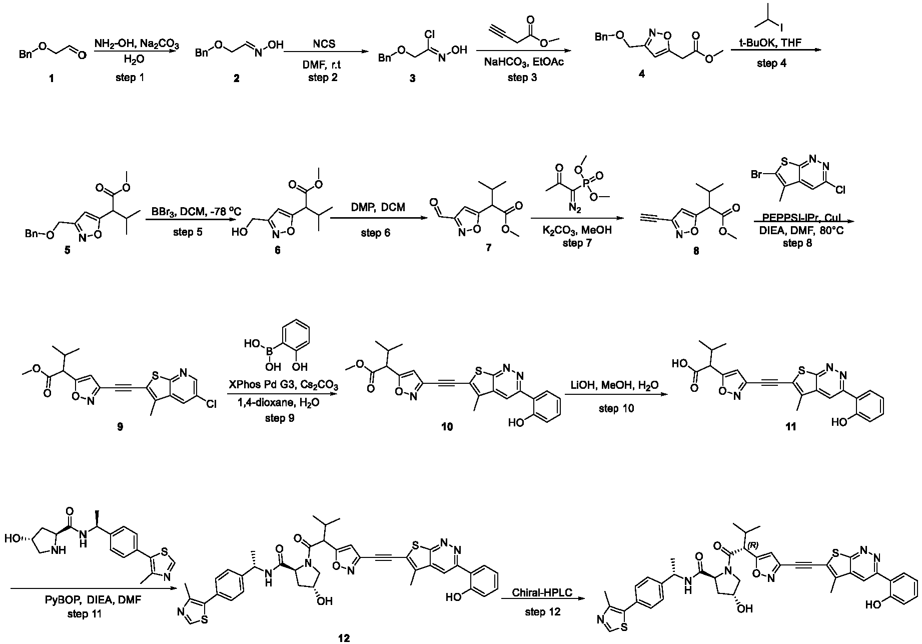

### Step 1: Preparation of (E)-N-[2-(benzyloxy)ethylidene]hydroxylamine (Intermediate 2)

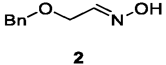

To a stirred mixture of 2-(benzyloxy)acetaldehyde (10 g, 66.588 mmol, 1 equiv) and Na₂CO₃ (3.53 g, 33.294 mmol, 0.5 equiv) in EtOH (100 mL) was added NH₂OH.HCl (5.09 g, 73.247 mmol, 1.1 equiv) at room temperature. The resulting mixture was stirred for 2 h at room temperature. Desired product could be detected by LCMS. The resulting mixture was diluted with water (200 mL). The resulting mixture was extracted with EtOAc (3 x 200 mL). The combined organic layers were washed with brine (3x50 mL), dried over anhydrous Na₂SO₄. After filtration, the filtrate was concentrated under reduced pressure to afford Intermediate 2 (13.196 g, crude) as a colorless liquid. LCMS (ESI) m/z: [M+H]⁺ = 166.

### Step 2: Preparation of (Z)-2-(benzyloxy)-N-hydroxyethanecarbonimidoyl chloride (Intermediate 3)

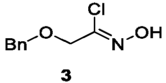

A mixture of Intermediate **2** (12 g, 72.643 mmol, 1 equiv) and NCS (10.67 g, 79.907 mmol, 1.1 equiv) in DMF (100 mL) was stirred for 2 h at room temperature. Desired product could be detected by LCMS. The resulting mixture was diluted with brine (200 mL). The resulting mixture was extracted with EtOAc (3 x 200 mL). The combined organic layers were washed with brine (3x50 mL), dried over anhydrous Na₂SO₄. After filtration, the filtrate was concentrated under reduced pressure to afford Intermediate 3 (18.1 g, crude) as a light blue liquid. LCMS (ESI) m/z [M+H]⁺ = 200.

### Step 3: Preparation of methyl 2-{3-[(benzyloxy)methyl]-1,2-oxazol-5-yl}acetate (Intermediate 4)

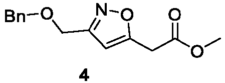

A mixture of Intermediate **3** (12 g, 60.111 mmol, 1 equiv) and NaHCO₃ (7.57 g, 90.166 mmol, 1.5 equiv) in EA (100 mL) was stirred for 30 min at room temperature. To the above mixture was added methyl but-3-ynoate (5.90 g, 60.111 mmol, 1 equiv) dropwise at 0 °C. The resulting mixture was stirred for additional overnight at room temperature. Desired product could be detected by LCMS. The resulting mixture was concentrated under reduced pressure. The residue was purified by reverse flash chromatography with the following conditions: column, C18 silica gel; mobile phase, MeCN in Water (0.1% FA), 0% to 100% gradient in 30 min; detector, UV 220 nm to afford Intermediate **4** (8.3 g, crude) as a yellow liquid. LCMS (ESI) m/z: [M+H]⁺ = 262.

### Step 4: Preparation of methyl 2-{3-[(benzyloxy)methyl]-1,2-oxazol-5-yl}-3-methylbutanoate (Intermediate 5)

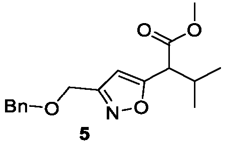

To a stirred mixture of Intermediate **4** (8 g, 30.619 mmol, 1 equiv) and MgSO4 (7.37 g, 61.238 mmol, 2 equiv) in THF (80 mL) were added t-BuOK (15.31 mL, 15.309 mmol, 0.5 equiv) at 0 °C under nitrogen atmosphere. The resulting mixture was stirred for 30 min at 0 °C under nitrogen atmosphere. To the above mixture was added 2-iodopropane (6.25 g, 36.743 mmol, 1.2 equiv) at 0 °C. The resulting mixture was stirred for additional overnight at room temperature. Desired product could be detected by LCMS. The resulting mixture was filtered, the filter cake was washed with DMF (3x5 mL). The filtrate was concentrated under reduced pressure. The residue was purified by reverse flash chromatography with the following conditions: column, C18 silica gel; mobile phase, MeCN in Water (0.1% FA), 0% to 100% gradient in 30 min; detector, UV 220 nm to afford Intermediate **5** (4.18 g, 45.00%) as a brown liquid. LCMS (ESI) m/z: [M+H]⁺ = 304.

### Step 5: Preparation of methyl 2-[3-(hydroxymethyl)-1,2-oxazol-5-yl]-3-methylbutanoate (Intermediate 6)

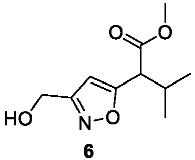

A mixture of Intermediate 5 and BBr₃ (9.91 g, 39.558 mmol, 3 equiv) in DCM (40 mL) was stirred for 2 h 0 °C under nitrogen atmosphere. Desired product could be detected by LCMS. The reaction was quenched with Water/Ice at 0 °C. The resulting mixture was extracted with CH₂Cl₂ (3 x 200 mL). The combined organic layers were washed with water (3x50 mL), dried over anhydrous Na₂SO₄. After filtration, the filtrate was concentrated under reduced pressure to afford Intermediate 6 (1.5 g, pure) as a yellow oil. LCMS (ESI) m/z: [M+H]⁺ = 214.

### Step 6: Preparation of methyl 2-(3-formyl-1,2-oxazol-5-yl)-3-methylbutanoate (Intermediate 7)

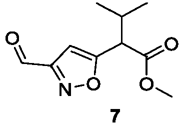

A mixture of methyl Intermediate 6 and Dess-Martin (1193.46 mg, 2.814 mmol, 1.2 equiv) in DCM (5 mL) was stirred for 2 h at room temperature. Desired product could be detected by LCMS. The reaction was quenched by the addition of NaHCO₃ (aq.) and Na₂S₂O₃ (aq.) (200 mL) at room temperature. The resulting mixture was extracted with EtOAc (3 x 200 mL). The combined organic layers were washed with brine (3x30 mL), dried over anhydrous Na₂SO₄. After filtration, the filtrate was concentrated under reduced pressure to afford Intermediate 7 (530 mg, crude) as a light yellow liquid. LCMS (ESI) m/z: [M+H]⁺ = 212.

### Step 7: Preparation of methyl 2-(3-ethynyl-1,2-oxazol-5-yl)-3-methylbutanoate (Intermediate 8)

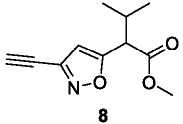

To a stirred mixture of Intermediate **7** (500 mg, 2.367 mmol, 1 equiv) and K₂CO₃ (981.49 mg, 7.101 mmol, 3 equiv) in MeOH (5 mL) were added seyferth-gilbert homologation (682.16 mg, 3.550 mmol, 1.5 equiv) dropwise at 0 ° C. The resulting mixture was stirred for overnight at room temperature. Desired product could be detected by LCMS. The reaction was quenched with Water at room temperature. The resulting mixture was extracted with CH₂Cl₂ (3 x 100 mL). The combined organic layers were washed with brine (3x10 mL), dried over anhydrous Na₂SO₄. After filtration, the filtrate was concentrated under reduced pressure. The residue was purified by reverse flash chromatography with the following conditions: column, C18 silica gel; mobile phase, MeCN in Water (0.1% FA), 0% to 100% gradient in 30 min; detector, UV 254 nm to afford Intermediate 8 (320 mg, 65.23%) as a light yellow oil. LCMS (ESI) m/z: [M+H]⁺ = 208.

### Step 8: Preparation of methyl 2-[3-(2-{3-chloro-5-methylthieno[2,3-c]pyridazin-6-yl}ethynyl)-1,2-oxazol-5-yl]-3-methylbutanoate (Intermediate 9)

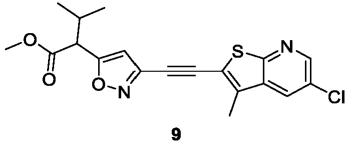

A mixture of Intermediate 8 (165.33 mg, 0.627 mmol, 1 equiv), [1,3-bis[2,6-bis(propan-2-yl)phenyl]-2,3-dihydro-1H-imidazol-2-yl]dichloro(3-chloropyridin-1-ium-1-yl)palladium (42.62 mg, 0.063 mmol, 0.1 equiv), Cul (11.95 mg, 0.063 mmol, 0.1 equiv) and DIEA (405.40 mg, 3.135 mmol, 5 equiv) in DMF (4 mL) was stirred for 3 h at 80 °C under nitrogen atmosphere. The residue was purified by reverse flash chromatography with the following conditions: column, C18 silica gel; mobile phase, MeCN in Water (0.1% FA), 0% to 100% gradient in 30 min; detector, UV 254 nm to afford Intermediate 9 (150 mg, 61.33%) as a brown solid. LCMS (ESI) m/z: [M+H]⁺ = 389.

### Step 9: Preparation of methyl 2-(3-{2-[3-(2-hydroxyphenyl)-5-methylthieno[2,3-c]pyridazin-6-yl]ethynyl}-1,2-oxazol-5-yl)-3-methylbutanoate (Intermediate 10)

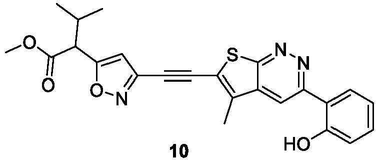

A mixture of Intermediate **9** (159.21 mg, 1.155 mmol, 3 equiv), XPhos Pd G3 (65.14 mg, 0.077 mmol, 0.2 equiv) and Cs₂CO₃ (376.09 mg, 1.155 mmol, 3 equiv) in dioxane (3 mL) and H2O (0.6 mL) was stirred for 2 h at 80 °C under nitrogen atmosphere. Desired product could be detected by LCMS. The resulting mixture was concentrated under reduced pressure. The resulting mixture was diluted with water (200 mL). The resulting mixture was extracted with EtOAc (3 x 200 mL). The combined organic layers were washed with brine (3x10 mL), dried over anhydrous Na₂SO₄. After filtration, the filtrate was concentrated under reduced pressure. The residue was purified by reverse flash chromatography with the following conditions: column, C18 silica gel; mobile phase, MeCN in Water (0.1% FA), 0% to 100% gradient in 30 min; detector, UV 254 nm to afford Intermediate **10** (109 mg, 63.30%) as a reddish brown solid. LCMS (ESI) m/z: [M+H]⁺ = 448.

### Step 10: Preparation of 2-(3-{2-[3-(2-hydroxyphenyl)-5-methylthieno[2,3-c]pyridazin-6-yl]ethynyl}-1,2-oxazol-5-yl)-3-methylbutanoic acid (Intermediate 11)

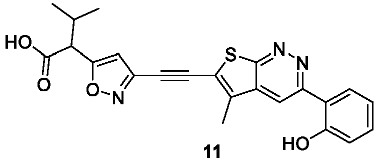

A mixture of Intermediate **10** (100 mg, 0.223 mmol, 1 equiv) and LiOH.H₂O (26.76 mg, 1.115 mmol, 5 equiv) in THF (3 mL) and H₂O (3 mL) was stirred for 2 h at room temperature. Desired product could be detected by LCMS. The mixture was acidified to pH 6 with conc. HCl. The resulting mixture was concentrated under reduced pressure to afford Intermediate **11** (400 mg, crude) as a brown solid. LCMS (ESI) m/z: [M+H]⁺ = 434.

### Step 11: Preparation of (2S,4R)-4-hydroxy-1-[2-(3-{2-[3-(2-hydroxyphenyl)-5-methylthieno[2,3-c]pyridazin-6-yl]ethynyl}-1,2-oxazol-5-yl)-3-methylbutanoyl]-N-[(1S)-1-[4-(4-methyl-1,3-thiazol-5-yl)phenyljethyljpyrrolidine-2-carboxamide (Intermediate 12)

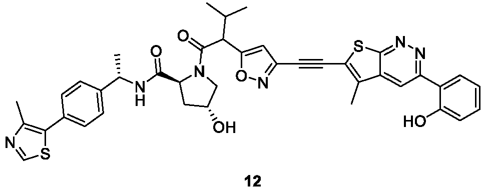

A mixture of Intermediate **11** (100 mg, 0.231 mmol, 1 equiv), (2S,4R)-4-hydroxy-N-[(1S)-1-[4-(4-methyl-1,3-thiazol-5-yl)phenyl]ethyl]pyrrolidine-2-carboxamide (76.46 mg, 0.231 mmol, 1 equiv), PyBOP (180.08 mg, 0.347 mmol, 1.5 equiv) and DIEA (119.26 mg, 0.924 mmol, 4 equiv) in DMF (2 mL) was stirred for 2 h at room temperature. Desired product could be detected by LCMS. The residue was purified by reverse flash chromatography with the following conditions: column, C18 silica gel; mobile phase, MeCN in Water (10mmol/L NH₄HCO₃), 0% to 100% gradient in 30 min; detector, UV 254 nm to afford Intermediate 12 (150 mg, 87.06%) as a yellow solid. LCMS (ESI) m/z: [M+H]⁺ = 747.

### Step 12: Preparation of (2S,4R)-4-hydroxy-1-[(2R)-2-(3-{2-[3-(2-hydroxyphenyl)-5-methylthieno[2,3-c]pyridazin-6-yl]ethynyl)-1,2-oxazol-5-yl)-3-methylbutanoyl]-N-[(1S)-1-[4-(4-methyl-1,3-thiazol-5-yl)phenyl]ethyl]pyrrolidine-2-carboxamide

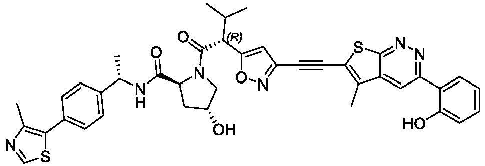

The crude product Intermediate **12** was purified by SFC-HPLC with the following conditions (Column: CHIRALPAK IA, 3*25 cm, 5 µm; Mobile Phase A: CO2, Mobile Phase B: MeOH: DCM=1: 1--HPLC; Flow rate: 60 mL/min; Gradient: isocratic 55% B; Column Temperature(°C): 35; Back Pressure(bar): 100; Wave Length: 274 nm; RT1(min): 4.27; RT2(min): 7.20; Sample Solvent: MeOH: DCM=1: 1--HPLC; Injection Volume: 1.6 mL) to afford title compound (second peak) (38.1 mg, 38.73%) as a light yellow solid. ¹H NMR (400 MHz, DMSO-d6) δ 12.09 (s, 1H), 9.01 - 8.94 (m, 1H), 8.88 - 8.81 (m, 1H), 8.45 (d, J = 7.6 Hz, 1H), 8.16 - 8.09 (m, 1H), 7.50 - 7.34 (m, 5H), 7.09 - 7.00 (m, 2H), 6.92 (s, 1H), 5.14 - 5.09 (m, 1H), 4.97 - 4.89 (m, 1H), 4.40 (t, J = 8.0 Hz, 1H), 4.30 (s, 1H), 3.97 (d, J = 9.6 Hz, 1H), 3.77 - 3.47 (m, 2H), 2.68 - 2.62 (m, 3H), 2.47 - 2.45 (m, 3H), 2.40 - 2.25 (m, 1H), 2.10 - 2.01 (m, 1H), 1.85 - 1.74 (m, 1H), 1.44 (dd, J = 37.1, 7.0 Hz, 3H), 1.01 (d, J = 6.4 Hz, 3H), 0.88 - 0.79 (m, 3H). LCMS (ESI) m/z: [M+H]⁺ = 747.20.

### Preparation of (2S,4R)-4-hydroxy-1-[(2R)-2-{3-[4-(2-{4-[3-(2-hydroxyphenyl)-5-methylthieno[2,3-c]pyridazin-6-yl]piperidin-1-yl}pyrimidin-5-yl)piperidin-1-yl]-1,2-oxazol-5-yl}-3-methylbutanoyl]-N-[(1S)-1-[4-(4-methyl-1,3-thiazol-5-yl])phenyl]ethyl]pyrrolidine-2-carboxamide (Compound 60)

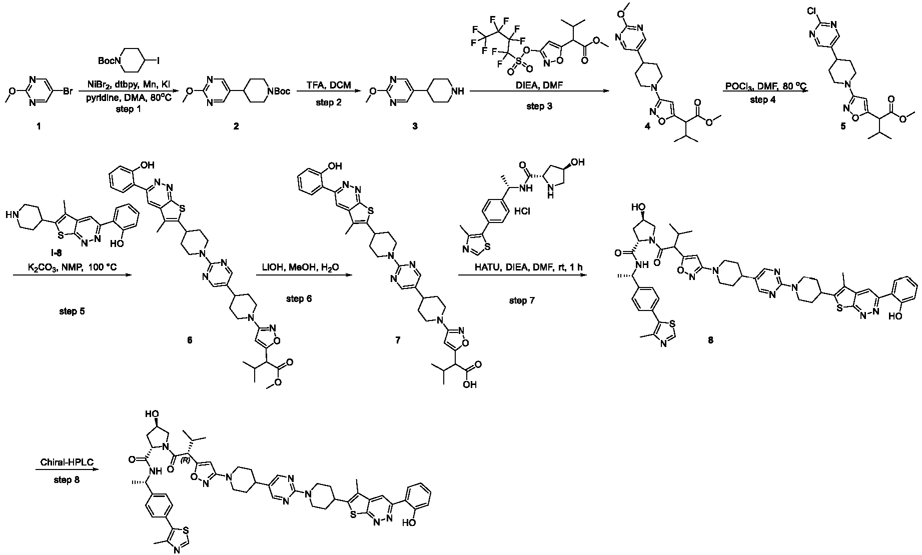

### Step 1: Preparation of tert-butyl 4-(2-methoxypyrimidin-5-yl)piperidine-1-carboxylate. (Intermediate 2).

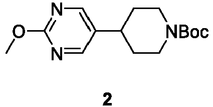

To a solution of 5-bromo-2-methoxypyrimidine (10.0 g, 52.907 mmol, 1.00 equiv) and tert-butyl 4-iodopiperidine-1-carboxylate (19.8 g, 63.488 mmol, 1.20 equiv), dtbpy (1.4 g, 5.291 mmol, 0.10 equiv), Mn (5.8 g, 105.814 mmol, 2.00 equiv), KI (8.8 g, 52.907 mmol, 1 equiv), NiBr2.DME (1.9 g, 5.291 mmol, 0.10 equiv) in DMA (100.0 mL) were added pyridine (4.6 g, 58.198 mmol, 1.10 equiv). After stirring for overnight at 80° C under a nitrogen atmosphere. Desired product could be detected by LCMS. The crude product was purified by reverse flash chromatography with the following conditions (column, C18 silica gel, mobile phase, MeCN in water (0.05% FA), 0% to 100% gradient in 30 min; detector, UV 254 nm) to afford Intermediate 2 (6.4 g, 75.16%) as a red oil. LCMS (ESI) m/z: [M+H]⁺ = 294.

### Step 2: Preparation of 2-methoxy-5-(piperidin-4-yl)pyrimidine. (Intermediate 3).

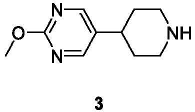

To a solution of Intermediate 2 (6.4 g, 21.816 mmol, 1.00 equiv) in DCM (60.0 mL) and TFA (20.0 mL). After stirring for 1h at room temperature. Desired product could be detected by LCMS. The resulting mixture was concentrated under vacuum. This resulted in Intermediate 3 (5.3 g, crude) as a green oil. LCMS (ESI) m/z: [M+H]⁺ = 194.

### Step 3: Preparation of methyl 2-{3-[4-(2-methoxypyrimidin-5-yl)piperidin-1-yl]-1,2-oxazol-5-yl}-3-methylbutanoate. (Intermediate 4).

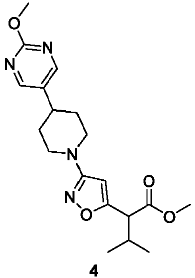

To a solution of Intermediate 3 (5.3 g, crude) and methyl 3-methyl-2-{3-[(1,1,2,2,3,3,4,4,4-nonafluorobutanesulfonyl)oxy]-1,2-oxazol-5-yl}butanoate (21.3 g, 44.150 mmol, 2.00 equiv) in DMF (60 mL) was added DIEA (14.3 g, 110.375 mmol, 5.00 equiv). After stirring for 3h at 130 °C. Desired product could be detected by LCMS. The crude product was purified by reverse flash chromatography with the following conditions (column, C18 silica gel, mobile phase, MeCN in water (0.05% FA), 0% to 100% gradient in 30 min; detector, UV 254 nm) to afford Intermediate **4** (736 mg, 8.90%) as a yellow oil. LCMS (ESI) m/z: [M+H]⁺ = 375.

### Step 4: Preparation of methyl 2-{3-[4-(2-chloropyrimidin-5-yl)piperidin-1-yl]-1,2-oxazol-5-yl}-3-methylbutanoate. (Intermediate 5).

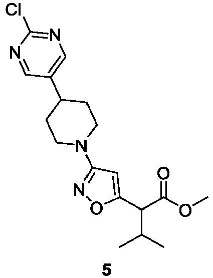

To a solution of POCl₃ (904.1 mg, 5.898 mmol, 3.00 equiv) in DMF (8.0 mL) were added Intermediate **4** (736.0 mg, 1.966 mmol, 1.00 equiv). After stirring for 2 h at 100 ° C. Desired product could be detected by LCMS. The crude product was purified by reverse flash chromatography with the following conditions (column, C18 silica gel, mobile phase, MeCN in water (0.05% FA), 0% to 100% gradient in 30 min; detector, UV 254 nm) to afford Intermediate 5 (75.0 mg, 10.07%) as a yellow solid. LCMS (ESI) m/z: [M+H]⁺ = 379.

### Step 5: Preparation of methyl 2-{3-[4-(2-{4-[3-(2-hydroxyphenyl)-5-methylthieno[2,3-c]pyridazin-6-yl]piperidin-1-yl}pyrimidin-5-yl)piperidin-1-yl]-1,2-oxazol-5-yl}-3-methylbutanoate. (Intermediate 6).

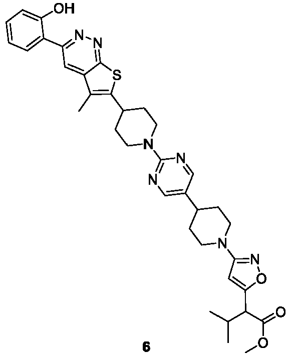

To a solution of Intermediate **5** (75.0 mg, 0.198 mmol, 1.00 equiv) and 2-[5-methyl-6-(piperidin-4-yl)thieno[2,3-c]pyridazin-3-yl]phenol (77.3 mg, 0.238 mmol, 1.20 equiv) in NMP (2 mL) were added K₂CO₃ (54.7 mg, 0.396 mmol, 2.00 equiv). After stirring for 3h at 100° C, the desired product could be detected by LCMS. The crude product was purified by reverse flash chromatography with the following conditions (column, C18 silica gel, mobile phase, MeCN in water (0.05% FA), 0% to 100% gradient in 30 min; detector, UV 254 nm) to afford Intermediate **6** (58.0 mg, 43.87%) as a light yellow solid. LCMS (ESI) m/z: [M+H]⁺ = 668.

### Step 6: Preparation of 2-{3-[4-(2-{4-[3-(2-hydroxyphenyl)-5-methylthieno[2,3-c]pyridazin-6-yl]piperidin-1-yl}pyrimidin-5-yl)piperidin-1-yl]-1,2-oxazol-5-yl}-3-methylbutanoic acid. (Intermediate 7).

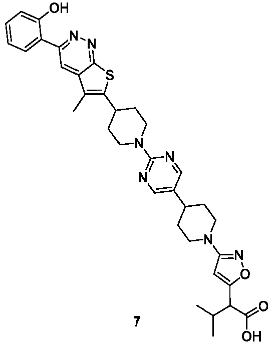

To a solution of Intermediate 6 (58.0 mg, 0.087 mmol, 1.00 equiv) and LiOH (6.3 mg, 0.261 mmol, 3.00 equiv) in MeOH (1.0 mL) and H₂O (1.0 mL). After stirring for 1h at room temperature, the desired product could be detected by LCMS. The resulting mixture was extracted with EA (3 x 3.0 mL). The combined organic layers were washed with water (1 x 3.0 mL), dried over anhydrous Na₂SO₄. After filtration, the filtrate was concentrated under reduced pressure to afford Intermediate 7 (48.0 mg, crude) as a white solid. LCMS (ESI) m/z: [M+H]⁺ = 654.

### Step 7: Preparation of (2S,4R)-4-hydroxy-1-[(2R)-2-{3-[4-(2-{4-[(4Z)-4-[2-(2-hydroxyphenyl)-2-iminoethylidene]-3-methyl-5H-thiophen-2-yl]piperidin-1-yl}pyrimidin-5-yl)piperidin-1-yl]-1,2-oxazol-5-yl}-3-methylbutanoyl]-N-[(1S)-1-[4-(4-methyl-1,3-thiazol-5-yl)phenyl]ethyl]pyrrolidine-2-carboxamide. (Intermediate 8).

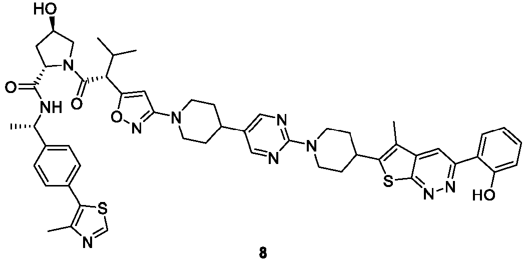

To a solution of Intermediate **7** (45 mg, 0.069 mmol, 1 equiv) and (2S,4R)-4-hydroxy-N-[(1S)-1-[4-(4-methyl-1,3-thiazol-5-yl)phenyl]ethyl]pyrrolidine-2-carboxamide (22.8 mg, 0.069 mmol, 1.00 equiv) in DMF (2.0 mg) were added DIEA (26.7 mg, 0.207 mmol, 3.00 equiv) and HATU (31.4 mg, 0.083 mmol, 1.20 equiv). After stirring for 1h at room temperature, the desired product could be detected by LCMS. The crude product was purified by reverse flash chromatography with the following conditions (column, C18 silica gel, mobile phase, MeCN in water (0.05% FA), 0% to 100% gradient in 30 min; detector, UV 254 nm) to afford Intermediate 8 (48.0 mg, 72.93%) as a white solid. LCMS (ESI) m/z: [M+H]⁺ = 966.

### Step 8: Preparation of (2S,4R)-4-hydroxy-1-[(2R)-2-{3-[4-(2-{4-[3-(2-hydroxyphenyl)-5-methylthieno[2,3-c]pyridazin-6-yl]piperidin-1-yl}pyrimidin-5-yl)piperidin-1-yl]-1,2-oxazol-5-yl}-3-methylbutanoyl]-N-[(1S)-1-[4-(4-methyl-1,3-thiazol-5-yl)phenyl]ethyl]pyrrolidine-2-carboxamide.

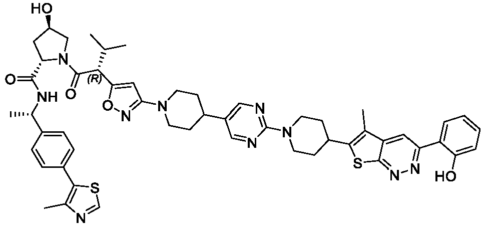

The Intermediate **8** (48.0 mg) was purified by Chiral-HPLC with the following conditions: Column, CHIRALPAK ID, 2*25 cm, 5 µm; Mobile phase A: MtBE(10Mm NH₃-MeOH), Mobile Phase B: EtOH-HPLC; Flow rate: 18 mL/min; Gradient: 30% B to 30% B in 24 min; Wave Length: 253/210 nm; RT1(min): 11.24; RT2(min): 18.89; Sample Solvent: MeOH: DCM=1:1-HPLC; Injection Volume:1.5 mL; Number Of Runs:4 to afford title compound (second peak) (4.6 mg, 29.11%) as an off-white solid.

¹H NMR (400 MHz, DMSO-d6) δ 12.78 (s, 1H), 8.99 (s, 1H), 8.71 (s, 1H), 8.41 (d, J = 7.7 Hz, 1H), 8.35 - 8.29 (m, 2H), 8.17 (d, 1H), 7.49 - 7.41 (m, 2H), 7.40 - 7.33 (m, 3H), 7.07 - 6.97 (m, 2H), 6.12 (s, 1H), 5.12 (d, J = 3.6 Hz, 1H), 5.02 - 4.63 (m, 3H), 4.38 (t, J = 7.8 Hz, 1H), 4.33 - 4.24 (m, 1H), 3.83 - 3.66 (m, 3H), 3.66 - 3.53 (m, 2H), 3.51 (s, 1H), 3.47 - 3.39 (m, 1H), 3.06 (t, J = 12.4 Hz, 2H), 2.86 (t, J = 12.0 Hz, 2H), 2.70 - 2.56 (m, 1H), 2.46 (s, 3H), 2.37 - 2.12 (m, 2H), 2.09 - 1.97 (m, 3H), 1.88 - 1.74 (m, 3H), 1.76 - 1.53 (m, 5H), 1.42 (d, J = 32.3, 7.0 Hz, 3H), 0.97 (d, J = 7.0 Hz, 3H), 0.83 (d, J = 15.7, 6.6 Hz, 3H). LCMS (ESI) m/z: [M+H]⁺ = 967.25.

### Preparation of 2S,4R)-4-hydroxy-1-((R)-2-(3-((1s,4S)-4-(2-(4-(3-(2-hydroxyphenyl)-5-methylthieno[2,3-c]pyridazin-6-yl)piperidin-1-yl)pyrimidin-5-yl)cyclohexyl)isoxazol-5-yl)-3-methylbutanoyl)-N-((S)-1-(4-(4-methylthiazol-5-yl)phenyl)ethyl)pyrrolidine-2-carboxamide and (2S,4R)-4-hydroxy-1-((R)-2-(3-((1r,4R)-4-(2-(4-(3-(2-hydroxyphenyl)-5-methylthieno[2,3-c]pyridazin-6-yl)piperidin-1-yl)pyrimidin-5-yl)cyclohexyl)isoxazol-5-yl)-3-methylbutanoyl)-N-((S)-1-(4-(4-methylthiazol-5-yl)phenyl)ethyl)pyrrolidine-2-carboxamide (Compound 64 and Compound 65)

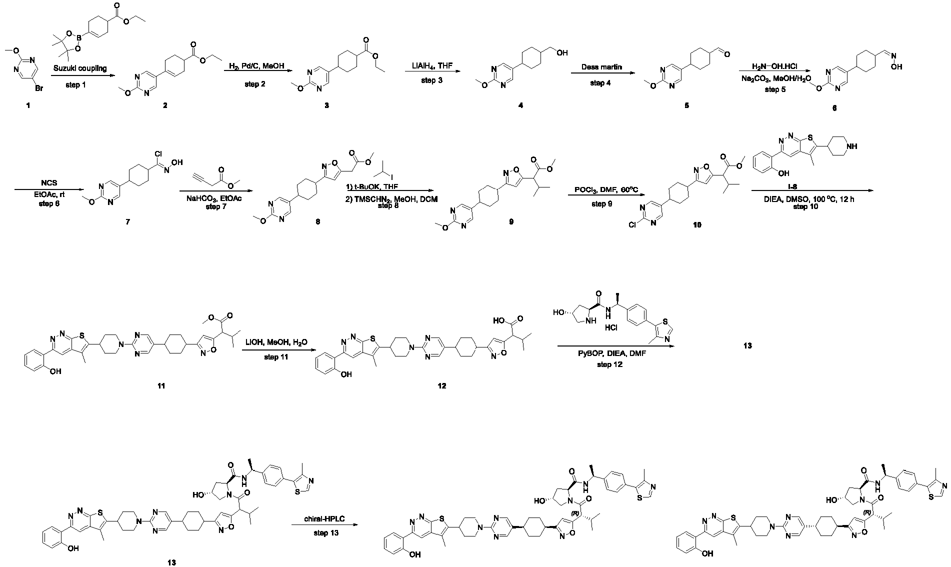

### Step 1: Preparation of ethyl 4-(2-methoxypyrimidin-5-yl)cyclohex-3-ene-1-carboxylate (Intermediate 2).

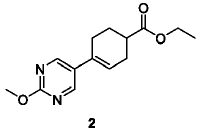

To a stirred solution of 5-bromo-2-methoxypyrimidine (3.37 g, 17.846 mmol, 1 equiv) and ethyl 4-(4,4,5,5-tetramethyl-1,3,2-dioxaborolan-2-yl)cyclohex-3-ene-1-carboxylate (5 g, 17.846 mmol, 1.00 equiv) in 1,4-dioxane (40 mL) and H₂O (10 mL) were added Pd(dppf)Cl₂.CH₂Cl₂ (1.45 g, 1.785 mmol, 0.1 equiv) and K₂CO₃ (7.40 g, 53.538 mmol, 3 equiv) at room temperature. The resulting mixture was stirred for 2 h at 80 °C under nitrogen atmosphere. The mixture was allowed to cool down to room temperature. The resulting mixture was concentrated under reduced pressure. The residue was purified by silica gel column chromatography, eluted with PE/EA (1/2) to afford Intermediate **2** (3.8, 81.4%) as a yellow oil. LCMS (ESI) m/z: [M+H]⁺ = 263.

### Step 2: Preparation of ethyl 4-(2-methoxypyrimidin-5-yl)cyclohexane-1-carboxylate (Intermediate 3).

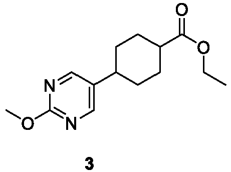

To a stirred solution of Intermediate **2** (3.8 g, 14.50 mmol, 1 equiv) in THF (50 mL) was added Pd(OH)₂/C (2 g) at room temperature. The resulting mixture was stirred for 16 h at room temperature under hydrogen atmosphere. The resulting mixture was filtered, the filter cake was washed with THF. The filtrate was concentrated under reduced pressure. This resulted in Intermediate **3** (3.1 g, 81.0%) as a grey oil. LCMS (ESI) m/z: [M+H]⁺ = 265.

### Step 3: Preparation of [4-(2-methoxypyrimidin-5-yl)cyclohexyl]methanol (Intermediate 4).

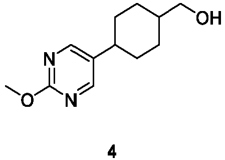

To a stirred solution of Intermediate **3** (4 g, 15.133 mmol, 1 equiv) in THF (50 mL) was added LiAlH₄ (0.57 g, 15.133 mmol, 1 equiv) at 0 °C under nitrogen atmosphere. The resulting mixture was stirred for 1 h at 0 °C under nitrogen atmosphere. The reaction was quenched with water at 0 °C. The resulting mixture was extracted with DCM (2 x 100 mL). The combined organic layers were washed with saturated brine (100 mL), dried over anhydrous Na₂SO₄. After filtration, the filtrate was concentrated under reduced pressure. This resulted in Intermediate **4** (566 mg, 16.83%) as a yellow oil. LCMS (ESI) m/z: [M+H]⁺ = 223.

### Step 4: Preparation of 4-(2-methoxypyrimidin-5-yl)cyclohexane-1-carbaldehyde (Intermediate 5).

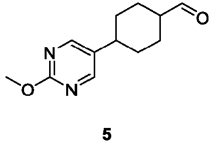

To a stirred solution of (COCl)₂ (969.51 mg, 7.638 mmol, 3 equiv) in DCM (10 mL) was added DMSO (795.75 mg, 10.184 mmol, 4 equiv) at -78 °C under nitrogen atmosphere. The resulting mixture was stirred for 30 min at -78 °C under nitrogen atmosphere. To the above mixture was added Intermediate **4** (566 mg, 2.546 mmol, 1 equiv) at -78 °C under nitrogen atmosphere. The resulting mixture was stirred for additional 30 min at -78 °C under nitrogen atmosphere. To the above mixture was added Et₃N (1.29 g, 12.730 mmol, 5 equiv) at -78 °C under nitrogen atmosphere. The resulting mixture was stirred for additional 1 h from -78 °C to room temperature under nitrogen atmosphere. The reaction was quenched with water at room temperature, and extracted with DCM (2 x 100 mL). The combined organic layers were washed with saturated brine (100 mL), dried over anhydrous Na₂SO₄. After filtration, the filtrate was concentrated under reduced pressure. This resulted in Intermediate **5** (450 mg, 79.5%) as a yellow oil. LCMS (ESI) m/z: [M+H]⁺ = 221.

### Step 5: Preparation of (Z)-N-{[4-(2-methoxypyrimidin-5-yl)cyclohexyl]methylidene}hydroxylamine (Intermediate 6).

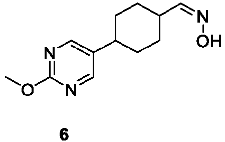

To a stirred solution of hydroxylamine hydrochloride (2.46 g, 35.412 mmol, 3 equiv) in MeOH (8 mL) and H₂O (8 mL) was added Na₂CO₃ (3.75 g, 35.412 mmol, 3 equiv) at 0 °C. To the above mixture was added Intermediate **5** (2.6 g, 11.804 mmol, 1 equiv) at 0 °C. The resulting mixture was stirred for additional 1 h at room temperature. The reaction was quenched with water at 0 °C. The resulting mixture was extracted with EA (2 x 200 mL). The combined organic layers were washed with saturated brine (200 mL), dried over anhydrous Na₂SO₄. After filtration, the filtrate was concentrated under reduced pressure. This resulted in Intermediate **6** (2.8 g, crude) as a yellow oil. LCMS (ESI) m/z: [M+H]⁺ = 236.

### Step 6: Preparation of (Z)-N-hydroxy-4-(2-methoxypyrimidin-5-yl)cyclohexane-1-carbimidoyl chloride (Intermediate 7).

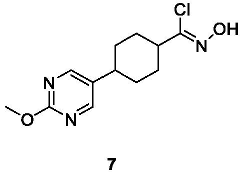

To a stirred solution of Intermediate **6** (2.8 g, 11.900 mmol, 1 equiv) in EA (30 mL) was added NCS (1.91 g, 14.280 mmol, 1.2 equiv) at 0 °C. The resulting mixture was stirred for 1 h at room temperature. Desired product could be detected by LCMS. The mixture was diluted with EtOAc (80 mL) and washed with water (80 mL x 2). The organic layer was dried over anhydrous sodium sulfate, filtered and concentrated to give a crude product Intermediate 7 (3.2 g, crude) as a yellow solid. LCMS (ESI) m/z: [M+H]⁺ = 270.

### Step 7: Preparation of methyl 2-{3-[4-(2-methoxypyrimidin-5-yl)cyclohexyl]-1,2-oxazol-5-yl}acetate (Intermediate 8).

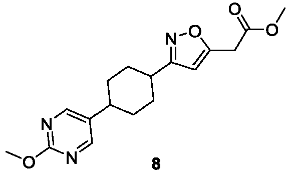

To a stirred solution of Intermediate **7** (3.2 g, 11.864 mmol, 1 equiv) in EA (30 mL) was added NaHCO₃ (2.99 g, 35.592 mmol, 3 equiv) at 0 °C. To the above mixture was added methyl but-3-ynoate (4.66 g, 47.456 mmol, **4** equiv) at 0 °C. The resulting mixture was stirred for additional 16 h at room temperature. The reaction was quenched with water. The resulting mixture was extracted with EA (2 x 200 mL). The combined organic layers were concentrated under reduced pressure. The residue was purified by reverse flash chromatography with the following conditions: column, C18 silica gel; mobile phase, MeCN in water (0.1% FA), 0 to 100% gradient in 30 min; detector, UV 254/220 nm. This resulted in Intermediate **8** (2.7 g, 68.68%) as a brown oil. LCMS (ESI) m/z: [M+H]⁺ = 332.

### Step 8: Preparation of methyl 2-(3-(4-(2-methoxypyrimidin-5-yl)cyclohexyl)isoxazol-5-yl)-3-methylbutanoate (Intermediate 9).

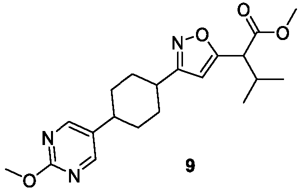

To a stirred solution of Intermediate **8** (2.7 g, 8.148 mmol, 1 equiv) in THF (15 mL) was added t-BuOK (2.74 g, 24.444 mmol, 3 equiv) at 0 °C under nitrogen atmosphere. The resulting mixture was stirred for 30 min at 0 °C under nitrogen atmosphere. To the above mixture was added 2-iodopropane (2.77 g, 16.296 mmol, 2 equiv) at 0 °C under nitrogen atmosphere. The resulting mixture was stirred for additional 2 h at room temperature. The reaction was quenched with water at 0 °C. The resulting mixture was extracted with DCM (2 x 200 mL). The combined organic layers were concentrated under reduced pressure. This resulted in 2-{3-[4-(2-methoxypyrimidin-5-yl)cyclohexyl]-1,2-oxazol-5-yl}-3-methylbutanoic acid (2 g, crude) as a brown oil. The product was dissolved in DCM (16 mL) and MeOH (4 mL), was added TMSCHN₂ (1.91 g, 16.692 mmol, 3 equiv) at 0 °C. The resulting mixture was stirred for 1 h at room temperature. The reaction was quenched with water at 0 °C. The resulting mixture was extracted with DCM (2 x 100 mL). The combined organic layers were concentrated under reduced pressure. The residue was purified by reverse flash chromatography with the following conditions: column, C18 silica gel; mobile phase, MeCN in water (0.1% FA), 0 to 100% gradient in 30 min; detector, UV 254/220 nm. This resulted in Intermediate **9** (795 mg, 38.26%) as a brown oil. LCMS (ESI) m/z: [M+H]⁺ = 374.

### Step 9: Preparation of methyl 2-(3-(4-(2-chloropyrimidin-5-yl)cyclohexyl)isoxazol-5-yl)-3-methylbutanoate (Intermediate 10).

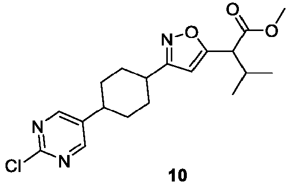

To a stirred solution of Intermediate **9** (795 mg, 2.129 mmol, 1 equiv) in DMF (5 mL) was added POCl₃ (979.15 mg, 6.387 mmol, 3 equiv) at room temperature. The resulting mixture was stirred for 16 h at 80 °C. The mixture was allowed to cool down to room temperature. The residue was purified by reverse flash chromatography with the following conditions: column, C18 silica gel; mobile phase, MeCN in water (0.1% FA), 0 to 100% gradient in 30 min; detector, UV 254/220 nm. This resulted in Intermediate **10** (154 mg, 19.14%) as a brown oil. LCMS (ESI) m/z: [M+H]⁺ = 378.

### Step 10: Preparation of methyl 2-[3-[4-(2-{4-[3-(2-hydroxyphenyl)-5-methylthieno[2,3-c]pyridazin-6-yl]piperidin-1-yl}pyrimidin-5-yl)cyclohexyl]-1,2-oxazol-5-yl}-3-methylbutanoate (Intermediate 11).

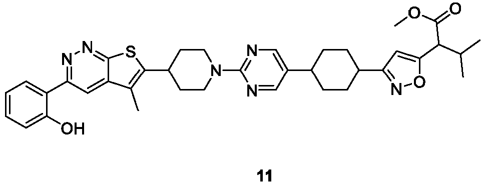

To a stirred solution of Intermediate **10** (100 mg, 0.265 mmol, 1 equiv) and 2-[5-methyl-6-(piperidin-4-yl)thieno[2,3-c]pyridazin-3-yl]phenol (103.35 mg, 0.318 mmol, 1.2 equiv) in DMSO (5 mL) was added DIEA (102.61 mg, 0.795 mmol, 3.0 equiv) at room temperature under nitrogen atmosphere. The resulting mixture was stirred for 2 h at 100 °C under nitrogen atmosphere. Desired product could be detected by LCMS. The residue was purified by reverse flash chromatography with the following conditions: column, C18 silica gel; mobile phase, MeOH in Water (0.1% FA), 0% to 100% gradient in 25 min; detector, UV 254 nm. This resulted in Intermediate **11** (105 mg, 59.50%) as a light yellow solid. LCMS (ESI) m/z: [M+H]⁺ = 667.

### Step 11: Preparation of methyl 2-(3-(4-(2-chloropyrimidin-5-yl)cyclohexyl)isoxazol-5-yl)-3-methylbutanoate (Intermediate 12).

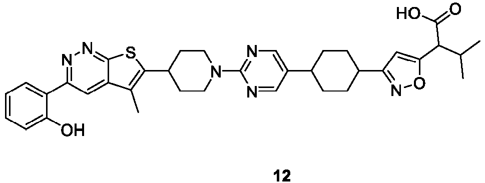

To a stirred solution of Intermediate **11** (100 mg, 0.150 mmol, 1 equiv) in MeOH (5 mL) was added LiOH (17.96 mg, 0.750 mmol, 5.0 equiv) in H₂O (2 mL). The resulting mixture was stirred for 2 h at room temperature. The mixture was acidified to pH 5 with HCl (aq.). The resulting mixture was extracted with EtOAc (3 x100 mL). The combined organic layers were washed with brine (50 mL), dried over anhydrous Na₂SO₄. After filtration, the filtrate was concentrated under reduced pressure. This resulted in Intermediate **12** (85 mg, 86.83%) as a light yellow solid. LCMS (ESI) m/z: [M+H]⁺ = 653.

### Step 12: Preparation of (2S,4R)-4-hydroxy-1-(2-{3-[4-(2-{4-[3-(2-hydroxyphenyl)-5-methylthieno[2,3-c]pyridazin-6-yl]piperidin-1-yl}pyrimidin-5-yl)cyclohexyl]-1,2-oxazol-5-yl}-3-methylbutanoyl)-N-[(1S)-1-[4-(4-methyl-1,3-thiazol-5-yl)phenyl]ethyl]pyrrolidine-2-carboxamide (Intermediate 13).

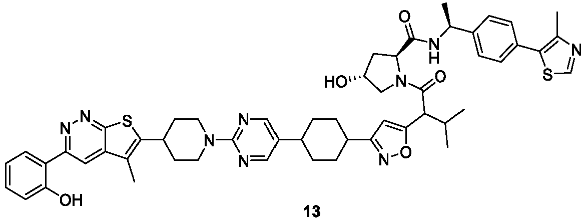

To a stirred solution of Intermediate **12** (85 mg, 0.130 mmol, 1 equiv) and (2S,4R)-4-hydroxy-N-[(1S)-1-[4-(1,3-thiazol-5-yl)phenyl]ethyl]pyrrolidine-2-carboxamide (61.99 mg, 0.195 mmol, 1.5 equiv) in DMF (5 mL) were added PyBOP (101.64 mg, 0.195 mmol, 1.5 equiv) and DIEA (50.49 mg, 0.390 mmol, 3.0 equiv) at room temperature. The resulting mixture was stirred for 4h at room temperature. The residue was purified by reverse flash chromatography with the following conditions: column, C18 silica gel; mobile phase, MeOH in water (0.1% TFA), 0% to 100% gradient in 30 min; detector, UV 254 nm. This resulted in Intermediate **13** (106 mg, 84.25%) as an off-white solid. LCMS (ESI) m/z: [M+H]⁺ = 966.

### Step 13: Preparation of (2S,4R)-4-hydroxy-1-((R)-2-(3-((1s,4S)-4-(2-(4-(3-(2-hydroxyphenyl)-5-methylthieno[2,3-c]pyridazin-6-yl)piperidin-1-yl)pyrimidin-5-yl)cyclohexyl)isoxazol-5-yl)-3-methylbutanoyl)-N-((S)-1-(4-(4-methylthiazol-5-yl)phenyl)ethyl)pyrrolidine-2-carboxamide and (2S,4R)-4-hydroxy-1-((R)-2-(3-((1r,4R)-4-(2-(4-(3-(2-hydroxyphenyl)-5-methylthieno[2,3-c]pyridazin-6-yl)piperidin-1-yl)pyrimidin-5-yl)cyclohexyl)isoxazol-5-yl)-3-methylbutanoyl)-N-((S)-1-(4-(4-methylthiazol-5-yl)phenyl)ethyl)pyrrolidine-2-carboxamide

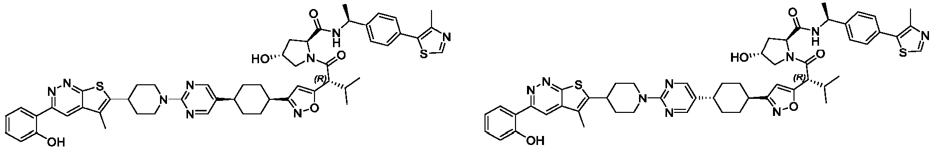

The Intermediate **13** was purified by Chiral-Prep-HPLC with the following conditions: Column, CHIRAL ART Cellulose-SB, 2*25 cm, 5 um; mobile phase, MtBE (10mM NH3-MeOH) and MeOH-(hold 20% MeOH- in 12 min); Detector, UV 254 nm to afford compound (2S,4R)-4-hydroxy-1-((R)-2-(3-((1s,4S)-4-(2-(4-(3-(2-hydroxyphenyl)-5-methylthieno[2,3-c]pyridazin-6-yl)piperidin-1-yl) pyrimidin-5-yl)cyclohexyl)isoxazol-5-yl)-3-methylbutanoyl)-N-((S)-1-(4-(4-methylthiazol-5-yl)phenyl)ethyl)pyrrolidine-2-carboxamide (28.1 mg) as an off white solid. ¹H NMR (400 MHz, DMSO-d6) *δ* 12.79 (s, 1H), 8.96 (d, J = 15.7 Hz, 1H), 8.70 (d, J = 2.5 Hz, 1H), 8.43 (d, J = 7.7 Hz, 1H), 8.24 (d, J = 5.2 Hz, 2H), 8.20 - 8.13 (m, 1H), 7.47 - 7.30 (m, 5H), 7.06 - 6.98 (m, 2H), 6.35 (s, 1H), 5.10 (d, J = 3.6 Hz, 1H), 4.91 (h, J = 6.7 Hz, 1H), 4.80 (t, J = 14.7 Hz, 2H), 4.38 (t, J = 7.9 Hz, 1H), 4.32 - 4.27 (m, 1H), 3.80 - 3.68 (m, 2H), 3.65 - 3.54 (m, 1H), 3.47 (d, J = 10.6 Hz, 1H), 3.12 - 2.93 (m, 3H), 2.59 (d, J = 12.9 Hz, 1H), 2.48 (d, J = 8.0 Hz, 3H), 2.44 (d, J = 11.0 Hz, 3H), 2.33 - 2.21 (m, 1H), 2.11 - 1.96 (m, 5H), 1.87 - 1.75 (m, 3H), 1.73 - 1.69 (m, 2H), 1.66 - 1.52 (m, 4H), 1.41 (d, J = 16.1 Hz, 3H), 0.98 (d, J = 6.7 Hz, 3H), 0.79 (d, J = 6.7 Hz, 3H). LCMS (ESI) m/z: [M+H]⁺ = 966.55.
and compound (2S,4R)-4-hydroxy-1-((R)-2-(3-((1r,4R)-4-(2-(4-(3-(2-hydroxyphenyl)-5-methylthieno[2,3-c]pyridazin-6-yl)piperidin-1-yl)pyrimidin-5-yl)cyclohexyl)isoxazol-5-yl)-3-methylbutanoyl)-N-((S)-1-(4-(4-methylthiazol-5-yl)phenyl)ethyl)pyrrolidine-2-carboxamide (9.1 mg) as an off white solid. ¹H NMR (400 MHz, DMSO-d6) δ 12.79 (s, 1H), 9.00 (d, J = 4.8 Hz, 1H), 8.71 (s, 1H), 8.44 (d, J = 7.7 Hz, 1H), 8.33 (s, 2H), 8.17 (d, J = 7.8 Hz, 1H), 7.51 - 7.42 (m, 2H), 7.38 (t, J = 6.5 Hz, 3H), 7.03 (d, J = 7.8 Hz, 2H), 6.33 (s, 1H), 5.12 (d, J = 3.6 Hz, 1H), 4.93 (q, J = 6.8 Hz, 1H), 4.84 (d, J = 13.2 Hz, 2H), 4.38 (t, J = 7.9 Hz, 1H), 4.30 (s, 1H), 3.79 - 3.68 (m, 2H), 3.66 - 3.56 (m, 1H), 3.53 - 3.45 (m, 1H), 3.05 (t, J = 12.5 Hz, 2H), 2.74 (s, 1H), 2.46 (d, J = 2.6 Hz, 3H), 2.32 - 2.14 (m, 2H), 2.04 (d, J = 12.3 Hz, 5H), 1.89 - 1.85 (m, 2H), 1.84 - 1.74 (m, 1H), 1.67 - 1.51 (m, 7H), 1.39 (d, J = 7.0 Hz, 3H), 1.26 - 1.09 (m, 2H), 0.98 (d, J = 6.3 Hz, 3H), 0.79 (d, J = 6.7 Hz, 3H). LCMS (ESI) m/z: [M+H]⁺ = 966.60.

### Preparation of (2S,4R)-4-hydroxy-1-[(2R)-2-(3-{6-[3-(2-hydroxyphenyl)thieno[2,3-c]pyridazin-6-yl]-2-azaspiro[3.3]heptan-2-yl}-1,2-oxazol-5-yl)-3-methylbutanoyl]-N-[(1S)-1-[4-(4-methyl-1,3-thiazol-5-yl)phenyl]ethyl]pyrrolidine-2-carboxamide (Compound 1)

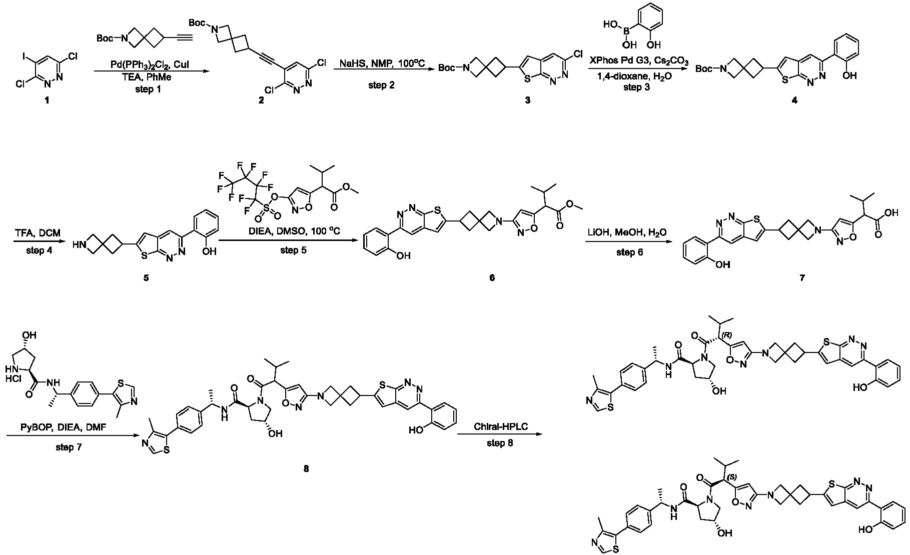

### Step 1: Preparation of tert-butyl 6-[2-(3,6-dichloropyridazin-4-yl)ethynyl]-2-azaspiro[3.3]heptane-2-carboxylate (intermediate 2)

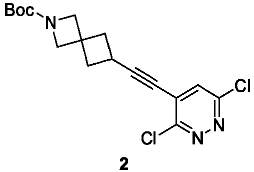

To a solution of 3,6-dichloro-4-iodopyridazine (1 g, 3.638 mmol, 1 equiv) and tert-butyl 6-ethynyl-2-azaspiro[3.3]heptane-2-carboxylate (0.81 g, 3.638 mmol, 1 equiv) in toluene (10 mL) were added Pd(PPh₃)₂Cl₂ (0.26 g, 0.364 mmol, 0.1 equiv) and TEA (1.10 g, 10.914 mmol, 3 equiv), the resulting solution was stirred at 60 degrees C for 6 hours. The mixture was diluted with EtOAc (400 mL) and washed with water (400 mL x 3). The organic layer was dried over anhydrous sodium sulfate, filtered and concentrated to give a crude product. The crude product was purified by silica gel column chromatography, elution gradient 0 to 43% PE in EA to give intermediate **2** (1.11 g, 82.85%) as a brown solid. LCMS (ESI) m/z: [M+H]⁺ =368.

### Step 2: Preparation of tert-butyl 6-{3-chlorothieno[2,3-c]pyridazin-6-yl}-2-azaspiro[3.3]heptane-2-carboxylate (intermediate 3)

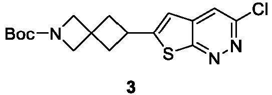

To a solution of intermediate 2 (1.1 g, 2.987 mmol, 1 equiv) in NMP (10 mL) was added NaSH (0.17 g, 2.987 mmol, 1 equiv), the resulting solution was stirred at 100 degrees C for 6 hours. Without additional work-up, the crude reaction solution was purified by flash C18 chromatography, elution gradient 0 to 46% ACN in H₂O to give intermediate 3 (422 mg, 38.61%) as a brown solid. LCMS (ESI) m/z: [M+H]⁺ =366.

### Step 3: Preparation of tert-butyl 6-[3-(2-hydroxyphenyl)thieno[2,3-c]pyridazin-6-yl]-2-azaspiro[3.3]heptane -2-carboxylate (intermediate 4)

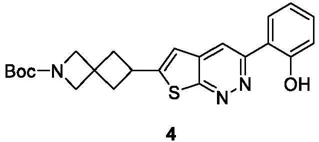

To a solution of tert-butyl intermediate **3** (400 mg, 1.093 mmol, 1 equiv) and 2-hydroxyphenylboronic acid (226.19 mg, 1.639 mmol, 1.5 equiv) in 1,4-dioxane (4 mL) and H₂O (1 mL) were added XPhos Pd G3 (92.54 mg, 0.109 mmol, 0.1 equiv) and Cs₂CO₃ (712.41 mg, 2.186 mmol, 2 equiv), the resulting solution was stirred at 80 degrees C for 3 hours. The mixture was diluted with EtOAc (300 mL) and washed with water (300 mL x 2). The organic layer was dried over anhydrous sodium sulfate, filtered and concentrated to give a crude product. The crude product was purified by flash C18 chromatography, elution gradient 0 to 41% ACN in H₂O to give intermediate **4** (347 mg, 74.94%) as a brown solid. LCMS (ESI) m/z: [M+H]⁺ =424.

### Step 4: Preparation of 2-(6-{2-azaspiro[3. 3]heptan-6-yl}thieno[2, 3-c]pyridazin-3-yl)phenol (intermediate 5)

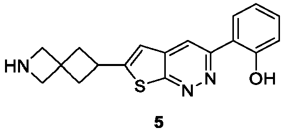

To a solution of intermediate **4** (347 mg, 0.819 mmol, 1 equiv) in DCM (3 mL) was added TFA (1 mL), the resulting solution was stirred at 25 degrees C for 2 hours. The resulting mixture was concentrated under reduced pressure to give intermediate **5** (308 mg, crude) as a yellow solid. LCMS (ESI) m/z: [M+H]⁺ =324.

### Step 5: Preparation of methyl 2-(3-{6-[3-(2-hydroxyphenyl)thieno[2,3-c]pyridazin-6-yl]-2-azaspiro[3.3]heptan-2-yl}-1,2-oxazol-5-yl)-3-methylbutanoate (intermediate 6)

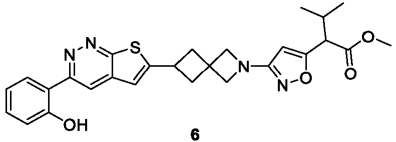

To a solution of intermediate **5** (250 mg, 0.773 mmol, 1 equiv) and methyl 3-methyl-2-{3-[(1,1,2,2,3,3,4,4,4-nonafluorobutanesulfonyl)oxy]-1,2-oxazol-5-yl}butanoate (372.04 mg, 0.773 mmol, 1 equiv) in DMSO (3 mL) was added DIEA (299.73 mg, 2.319 mmol, 3 equiv), the resulting solution was stirred at 100 degrees C for 5 hours. The crude reaction solution was purified by flash C18 chromatography, elution gradient 0 to 45% ACN in H₂O to give intermediate **6** (203 mg, 52.04%) as a yellow solid. LCMS (ESI) m/z: [M+H]⁺ =505.

### Step 6: Preparation of 2-(3-{6-[3-(2-hydroxyphenyl)thieno[2,3-c]pyridazin-6-yl]-2-azaspiro[3.3]heptan-2-yl}-1,2-oxazol-5-yl)-3-methylbutanoic acid (intermediate 7)

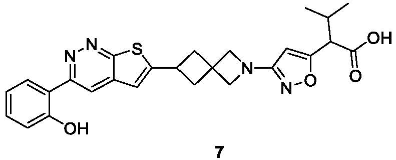

To a solution of intermediate 6 (200 mg, 0.396 mmol, 1 equiv) in MeOH (2 mL) and H₂O (0.5 mL) was added LiOH (47.46 mg, 1.980 mmol, 5 equiv), the resulting solution was stirred at 25 degrees C for 2 hours. The mixture was acidified to pH 5~6 with HCl (1 M in H₂O). The resulting mixture was diluted with water (100 mL). The resulting mixture was extracted with EA (100 mL x 3). The combined organic layers were dried over anhydrous sodium sulfate. After filtration, the filtrate was concentrated under reduced pressure to give intermediate **7** (297 mg, crude) as a brown solid. LCMS (ESI) m/z: [M+H]⁺ =491.

### Step 7: Preparation of (2S,4R)-4-hydroxy-1-[2-(3-{6-[3-(2-hydroxyphenyl)thieno[2,3-c]pyridazin-6-yl]-2-azaspiro[3.3]heptan-2-yl}-1,2-oxazol-5-yl)-3-methylbutanoyl]-N-[(1S)-1-[4-(4-methyl-1,3-thiazol-5-yl)phenyl]ethyl]pyrrolidine-2-carboxamide (intermediate 8)

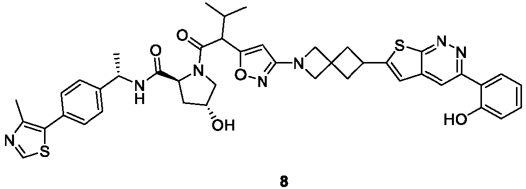

To a solution of intermediate **7** (200 mg, 0.408 mmol, 1 equiv) and (2S,4R)-4-hydroxy-N-[(1S)-1-[4-(4-methyl-1,3-thiazol-5-yl) phenyl] ethyl]pyrrolidine-2-carboxamide (270.24 mg, 0.816 mmol, 2 equiv) in DMF (3 mL) were added PyBOP (424.31 mg, 0.816 mmol, 2 equiv) and DIEA (263.46 mg, 2.040 mmol, 5 equiv), the resulting solution was stirred at 25 degrees C for 2 hours. Without additional work-up, the crude reaction solution was purified by flash C18 chromatography, elution gradient 0 to 52% ACN in H₂O to give intermediate **8** (184 mg, 56.2%). LCMS (ESI) m/z: [M+H]⁺ =804.

### Step 7: Preparation of (2S,4R)-4-hydroxy-1-[(2R)-2-(3-{6-[3-(2-hydroxyphenyl)thieno[2,3-c]pyridazin-6-yl]-2-azaspiro[3.3]heptan-2-yl}-1,2-oxazol-5-yl)-3-methylbutanoyl]-N-[(1S)-1-[4-(4-methyl-1,3-thiazol-5-yl)phenyl]ethyl]pyrrolidine-2-carboxamide

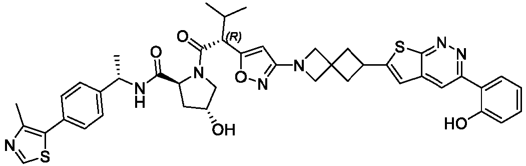

The intermediate **8** (184 mg) was purified by Chiral-HPLC (Column: CHIRALPAK ID, 2*25 cm, 5 µm; Mobile Phase A: MtBE(10mM NH₃-MeOH), Mobile Phase B: MeOH--HPLC; Flow rate: 20 mL/min; Gradient: 30% B to 30% B in 17 min; Wave Length: 208/272 nm; RT1(min): 6.345; RT2(min): 8.7275; Sample Solvent: MeOH--HPLC; Injection Volume: 0.7 mL; Number Of Runs: 7) to give title compound (30.5 mg, 16.57%) (second peak) as an off-white solid. ¹H NMR (400 MHz, Methanol-d4) *δ* 8.87 (s, 1H), 8.58 (s, 1H), 7.95 (d, J = 8.1 Hz, 1H), 7.61 - 7.31 (m, 5H), 7.27 (s, 1H), 7.01 (d, J = 8.0 Hz, 2H), 5.88 (s, 1H), 5.03 (q, J = 7.3 Hz, 1H), 4.51 (t, J = 8.2 Hz, 1H), 4.44 (s, 1H), 4.11 (s, 2H), 3.93 (s, 2H), 4.03 - 3.89 (m, 2H), 3.62 (t, J = 12.1 Hz, 2H), 2.83 (s, 2H), 2.57 (t, J = 10.2 Hz, 2H), 2.48 (s, 3H), 2.41 - 2.29 (m, 1H), 2.23 - 2.08 (m, 1H), 2.03 - 1.81 (m, 1H), 1.52 (d, J = 7.1 Hz, 3H), 1.05 (d, J = 6.5 Hz, 3H), 0.89 (d, J = 7.2 Hz, 3H). LCMS (ESI) m/z: [M+H]⁺ =804.20.

The compounds in Table 2 were prepared using procedures similar to the one used above for the preparation of (2S,4R)-4-hydroxy-1-[(2R)-2-(3-{6-[3-(2-hydroxyphenyl)thieno[2,3-c]pyridazin-6-yl]-2-azaspiro[3.3]heptan-2-yl}-1,2-oxazol-5-yl)-3-methylbutanoyl]-N-[(1S)-1-[4-(4-methyl-1,3-thiazol-5-yl)phenyl]ethyl]pyrrolidine-2-carboxamide (Compound 1).

**Table 2.**

| **No.** | **Name** | **LCMS (ESI) m/z** | **¹H NMR** |
|---|---|---|---|
| 16 | (2S,4R)-4-hydroxy-1-((R)-2-(3-((1R,5S,6R)-6-(3-(2-hydroxyphenyl)-5-methylthieno[2,3-c]pyridazin-6-yl)-3-azabicyclo[3.1.0]hexan-3-yl)isoxazol-5-yl)-3-methylbutanoyl)-N-((S)-1-(4-(4-methylthiazol-5-yl)phenyl)ethyl)pyrrolidine-2-carboxamide | 804.1 | 1H NMR (300 MHz, DMSO-d6) δ 12.87 (s, 1H), 8.99 - 8.65 (m, 2H), 8.41 - 8.18 (m, 2H), 7.52 - 7.33 (m, 5H), 7.08 - 6.96 (m, 2H), 6.02 (s, 1H), 5.12 (d, J = 3.5 Hz, 1H), 5.01 - 4.87 (m, 1H), 4.38 - 4.30 (m, 2H), 3.80 - 3.69 (m, 3H), 3.60 (d, J = 9.9 Hz, 1H), 3.48 - 3.38 (m, 3H), 2.50 (s, 3H), 2.46 - 2.42 (m, 4H), 2.31 - 2.18 (m, 3H), 2.01 - 1.98 (m, 1H), 1.88 - 1.74 (m, 1H), 1.51 - 1.32 (m, 3H), 0.98 (t, J = 6.0 Hz, 3H), 0.84 (dd, J = 10.9, 6.6 Hz, 3H). |
| 19 | (2S,4R)-4-hydroxy-1-((R)-2-(3-(3-(3-(2-hydroxyphenyl)-5-methylthieno[2,3-c]pyridazin-6-yl)azetidin-1-yl)isoxazol-5-yl)-3-methylbutanoyl)-N-((S)-1-(4-(4-methylthiazol-5-yl)phenyl)ethyl)pyrrolidine-2-carboxamide | 778.1 | 1H NMR (400 MHz, Methanol-d4) δ 8.86 (d, J = 6.9 Hz, 1H), 8.55 (s, 1H), 8.05 (dd, J = 8.3, 1.6 Hz, 1H), 7.47 - 7.31 (m, 5H), 7.06 - 6.97 (m, 2H), 5.96 (d, J = 26.9 Hz, 1H), 5.09 - 4.97 (m, 1H), 4.68 - 4.57 (m, 1H), 4.56 - 4.38 (m, 4H), 4.16 - 4.06 (m, 2H), 3.90 - 3.73 (m, 1H), 3.71 - 3.45 (m, 2H), 2.51 - 2.44 (m, 5H), 2.43 - 2.27 (m, 2H), 2.25 - 2.15 (m, 1H), 2.09 - 1.91 (m, 1H), 1.63 - 1.44 (m, 3H), 1.07 (d, J = 6.5 Hz, 3H), 0.97 - 0.88 (m, 3H). |
| 45 | (2S,4R)-4-hydroxy-1-((R)-2-(3-(2-(3-(2-hydroxyphenyl)-5-methylthieno[2,3-c]pyridazin-6-yl)-7-azaspiro[3.5]nonan-7-yl)isoxazol-5-yl)-3-methylbutanoyl)-N-((S)-1-(4-(4-methylthiazol-5-yl)phenyl)ethyl)pyrrolidine-2-carboxamide | 846.5 | 1H NMR (400 MHz, DMSO-d6) δ 12.87 (s, 1H), 8.99 (d, J = 1.8 Hz, 1H), 8.66 (s, 1H), 8.42 (d, J = 7.7 Hz, 1H), 8.30 - 8.09 (m, 1H), 7.58 - 7.29 (m, 5H), 7.02 (dd, J = 8.2, 6.9 Hz, 2H), 6.10 (d, J = 53.0 Hz, 1H), 5.07 (dd, J = 41.7, 3.3 Hz, 1H), 4.91 (q, J = 7.2 Hz, 1H), 4.37 (t, J = 7.8 Hz, 1H), 4.29 (s, 1H), 4.13 (p, J = 9.0 Hz, 1H), 3.72 (dd, J = 10.6, 4.5 Hz, 1H), 3.57 (d, J = 9.9 Hz, 1H), 3.51 (s, 1H), 3.44 (s, 1H), 3.24 (s, 2H), 3.12 (s, 2H), 3.50 (brs, 1H), 2.46 (s, 2H), 2.38 (s, 3H), 2.34 - 2.16 (m, 2H), 2.08 - 1.96 (m, 3H), 1.88 - 1.72 (m, 3H), 1.64 (s, 2H), 1.42 (dd, J = 31.9, 7.0 Hz, 3H), 0.95 (d, J = 6.5 Hz, 3H), 0.82 (dd, J = 15.9, 6.6 Hz, 3H). |
| 23 | (2S,4R)-4-hydroxy-1-((R)-2-(3-(6-(3-(2-hydroxyphenyl)-5-methylthieno[2,3-c]pyridazin-6-yl)-2-azaspiro[3.3]heptan-2-yl)isoxazol-5-yl)-3-methylbutanoyl)-N-((S)-1-(4-(4-methylthiazol-5-yl)phenyl)ethyl)pyrrolidine-2-carboxamide | 818.15 | 1H NMR (400 MHz, DMSO-d6) δ 12.82 (s, 1H), 9.02 - 8.97 (m, 1H), 8.67 (s, 1H), 8.42 (d, J = 7.7 Hz, 1H), 8.16 (d, 1H), 7.51 - 7.41 (m, 2H), 7.42 - 7.33 (m, 3H), 7.06 - 6.98 (m, 2H), 5.88 (s, 1H), 5.11 (d, J = 3.6 Hz, 1H), 4.96 - 4.86 (m, 1H), 4.37 (t, J = 7.9 Hz, 1H), 4.29 (s, 1H), 4.06 (s, 2H), 4.05 - 3.94 (m, 1H), 3.89 - 3.85 (m, 2H), 3.75 - 3.67 (m, 1H), 3.59 (d, J = 9.8 Hz, 1H), 3.53 - 3.39 (m, 1H), 2.88 - 2.78 (m, 2H), 2.48 - 2.39 (m, 5H), 2.37 (s, 3H), 2.27 - 2.13 (m, 1H), 2.07 - 1.98 (m, 1H), 1.84 - 1.73 (m, 1H), 1.50 - 1.34 (m, 3H), 1.00 - 0.92 (m, 3H), 0.88 - 0.76 (m, 3H). |
| 56 | (2S,4R)-4-hydroxy-1-((R)-2-(3-(4-(3-(2-hydroxyphenyl)-5-methylthieno[2,3-c]pyridazin-6-yl)piperidin-1-yl)isoxazol-5-yl)-3-methylbutanoyl)-N-((S)-1-(4-(4-methylthiazol-5-yl)phenyl)ethyl)pyrrolidine-2-carboxamide | 806.1 | 1H NMR (300 MHz, DMSO-d6) δ 12.79 (s, 1H), 8.99 (s, 1H), 8.71 (s, 1H), 8.42 (d, J = 7.6 Hz, 1H), 8.25 - 7.98 (m, 1H), 7.65 - 7.29 (m, 5H), 7.07 - 6.99 (m, 2H), 6.22 (s, 1H), 5.13 (d, J = 3.6 Hz, 1H), 5.04 - 4.80 (m, 1H), 4.60 - 4.15 (m, 2H), 3.82 - 3.65 (m, 3H), 3.65 - 3.55 (m, 1H), 3.52 - 3.41 (m, 2H), 3.01 (t, J = 12.2 Hz, 2H), 2.48 - 2.45 (m, 6H), 2.32 - 2.15 (m, 1H), 2.09 - 1.94 (m, 3H), 1.90 - 1.65 (m, 3H), 1.39 (d, J = 7.0 Hz, 3H), 0.97 (d, J = 6.5 Hz, 3H), 0.82 (d, J = 6.6 Hz, 3H). |
| 74 | (2S,4R)-4-hydroxy-1-((R)-2-(3-((R)-3-(3-(2-hydroxyphenyl)-5-methylthieno[2,3-c]pyridazin-6-yl)pyrrolidin-1-yl)isoxazol-5-yl)-3-methylbutanoyl)-N-((S)-1-(4-(4-methylthiazol-5-yl)phenyl)ethyl)pyrrolidine-2-carboxamide | 792.15 | 1H NMR (400 MHz, DMSO-d6) δ 12.76 (d, J = 10.7 Hz, 1H), 8.98 (d, 1H), 8.72 (s, 1H), 8.42 (d, J = 7.7 Hz, 1H), 8.21 - 8.14 (m, 1H), 7.49 - 7.42 (m, 2H), 7.42 - 7.34 (m, 3H), 7.07 - 6.99 (m, 2H), 6.05 (s, 1H), 5.12 (d, J = 3.6 Hz, 1H), 4.96 - 4.88 (m, 1H), 4.38 (t, J = 7.9 Hz, 1H), 4.30 (s, 1H), 4.24 - 4.14 (m, 1H), 3.82 - 3.69 (m, 2H), 3.64 - 3.51 (m, 2H), 3.54 - 3.14 (m, 3H), 2.48 - 2.43 (m, 4H), 2.20 - 2.08 (m, 3H), 2.08 - 1.98 (m, 2H), 1.98- 1.85 (m, 1H), 1.85 - 1.74 (m, 1H), 1.38 (d, J = 7.0 Hz, 3H), 1.23 (s, 3H), 1.01 - 0.94 (m, 3H). |
| 77 | (2S,4R)-4-hydroxy-1-((R)-2-(3-((R)-2-(3-(2-hydroxyphenyl)-5-methylthieno[2,3-c]pyridazin-6-yl)-8-azaspiro[4.5]decan-8-yl)isoxazol-5-yl)-3-methylbutanoyl)-N-((S)-1-(4-(4-methylthiazol-5-yl)phenyl)ethyl)pyrrolidine-2-carboxamide | 860.15 | 1H NMR (400 MHz, DMSO-d6) δ 12.87 (s, 1H), 8.99 (s, 1H), 8.66 (s, 1H), 8.40 (d, J = 7.7 Hz, 1H), 8.20 - 8.13 (m, 1H), 7.48 - 7.32 (m, 5H), 7.06 - 6.97 (m, 2H), 6.09 (d, J = 52.8 Hz, 1H), 5.16 - 4.84 (m, 2H), 4.42 - 4.21 (m, 2H), 3.89 - 3.78 (m, 1H), 3.76 - 3.68 (m, 1H), 3.57 (d, J = 9.8 Hz, 1H), 3.43 (d, J = 10.2 Hz, 1H), 3.24 - 3.14 (m, 4H), 2.45 (d, J = 6.1 Hz, 6H), 2.30 - 2.11 (m, 3H), 2.10 - 1.98 (m, 1H), 1.79 (s, 3H), 1.74 - 1.55 (m, 5H), 1.55 - 1.43 (m, 1H), 1.38 (d, J = 7.0 Hz, 3H), 0.95 (d, J = 6.6 Hz, 3H), 0.82 (dd, J = 16.0, 6.7 Hz, 3H). |
| 78 | (2S,4R)-4-hydroxy-1-((R)-2-(3-((S)-3-(3-(2-hydroxyphenyl)-5-methylthieno[2,3-c]pyridazin-6-yl)pyrrolidin-1-yl)isoxazol-5-yl)-3-methylbutanoyl)-N-((S)-1-(4-(4-methylthiazol-5-yl)phenyl)ethyl)pyrrolidine-2-carboxamide | 792.25 | 1H NMR (400 MHz, DMSO-d6) δ 12.78 (s, 1H), 8.99 (s, 1H), 8.72 (d, 1H), 8.42 (d, J = 7.7 Hz, 1H), 8.17 (d, J = 8.0, 1.7 Hz, 1H), 7.50 - 7.41 (m, 2H), 7.41 - 7.33 (m, 3H), 7.02 (t, J = 7.8 Hz, 2H), 6.00 (d, J = 41.3 Hz, 1H), 5.15 - 4.99 (m, 1H), 4.96 - 4.65 (m, 1H), 4.38 (t, J = 7.9 Hz, 1H), 4.30 (s, 1H), 4.23 - 4.13 (m, 1H), 3.84 - 3.68 (m, 2H), 3.64 - 3.53 (m, 2H), 3.53 - 3.34 (m, 3H), 2.59 - 2.51 (m, 1H), 2.49 (s, 3H), 2.46 (s, 3H), 2.33 (s, 1H), 2.32 - 2.07 (m, 1H), 2.05 - 1.88 (m, 1H), 1.85 - 1.74 (m, 1H), 1.43 (dd, J = 37.8, 7.0 Hz, 3H), 0.98 (t, J = 7.1 Hz, 3H), 0.85 (dd, J = 15.4, 6.6 Hz, 3H). |
| 81 | (2S,4R)-4-hydroxy-1-((R)-2-(3-((S)-2-(3-(2-hydroxyphenyl)-5-methylthieno[2,3-c]pyridazin-6-yl)-8-azaspiro[4.5]decan-8-yl)isoxazol-5-yl)-3-methylbutanoyl)-N-((S)-1-(4-(4-methylthiazol-5-yl)phenyl)ethyl)pyrrolidine-2-carboxamide | 860.15 | 1H NMR (400 MHz, Methanol-d4) δ 8.89 - 8.81 (m, 1H), 8.46 (s, 1H), 8.07 - 8.00 (m, 1H), 7.55 - 7.38 (m, 4H), 7.40 - 7.30 (m, 1H), 7.05 - 6.97 (m, 2H), 6.14 - 6.03 (m, 1H), 5.08 - 4.99 (m, 1H), 4.60 - 4.40 (m, 2H), 3.89 - 3.78 (m, 2H), 3.78 - 3.70 (m, 1H), 3.66 - 3.51 (m, 2H), 3.36 - 3.24 (m, 3H), 2.50 - 2.42 (m, 6H), 2.40 - 2.13 (m, 4H), 2.04 - 1.82 (m, 3H), 1.79 - 1.57 (m, 6H), 1.52 (d, J = 7.0 Hz, 3H), 1.05 (d, J = 6.5 Hz, 3H), 0.95 - 0.86 (m, 3H). |
| 85 | (2S,4R)-4-hydroxy-1-((R)-2-(3-(9-(3-(2-hydroxyphenyl)-5-methylthieno[2,3-c]pyridazin-6-yl)-3-azaspiro[5.5]undecan-3-yl)isoxazol-5-yl)-3-methylbutanoyl)-N-((S)-1-(4-(4-methylthiazol-5-yl)phenyl)ethyl)pyrrolidine-2-carboxamide | 874.35 | 1H NMR (300 MHz, DMSO-d6) δ 12.85 (s, 1H), 8.99 (s, 1H), 8.69 (s, 1H), 8.42 (d, J = 7.7 Hz, 1H), 8.17 (d, J = 7.8 Hz, 1H), 7.51 - 7.41 (m, 2H), 7.41 - 7.33 (m, 3H), 7.07 - 6.96 (m, 2H), 6.14 (s, 1H), 5.12 (d, J = 3.6 Hz, 1H), 4.92 (t, J = 7.1 Hz, 1H), 4.37 (t, J = 7.8 Hz, 1H), 4.29 (s, 1H), 3.76 - 3.68 (m, 1H), 3.57 (d, J = 9.8 Hz, 1H), 3.47 - 3.38 (m, 1H), 3.25 - 3.16 (m, 5H), 2.46 (s, 3H), 2.44 (s, 3H), 2.28 - 2.19 (m, 1H), 2.05 - 1.99 (m, 1H), 1.91 - 1.77 (m, 5H), 1.73 - 1.57 (m, 4H), 1.50 - 1.32 (m, 7H), 1.00 - 0.92 (m, 3H), 0.83 - 0.74 (m, 3H). |
| 88 | (2S,4R)-4-hydroxy-1-((R)-2-(3-(7-(3-(2-hydroxyphenyl)-5-methylthieno[2,3-c]pyridazin-6-yl)-2-azaspiro[3.5]nonan-2-yl)isoxazol-5-yl)-3-methylbutanoyl)-N-((S)-1-(4-(4-methylthiazol-5-yl)phenyl)ethyl)pyrrolidine-2-carboxamide | 846.2 | 1H NMR (300 MHz, DMSO-d6) δ 12.83 (s, 1H), 8.99 (s, 1H), 8.69 (s, 1H), 8.42 (d, J = 7.6 Hz, 1H), 8.17 (d, J = 7.7 Hz, 1H), 7.52 - 7.38 (m, 2H), 7.38 (d, J = 8.2 Hz, 3H), 7.08 - 6.97 (m, 2H), 5.88 (s, 1H), 5.12 (d, J = 3.6 Hz, 1H), 4.92 (t, J = 7.1 Hz, 1H), 4.38 (t, J = 7.8 Hz, 1H), 4.30 (s, 1H), 3.78 - 3.67 (m, 3H), 3.65 - 3.55 (m, 3H), 3.48 - 3.39 (m, 1H), 3.27 - 3.21 (m, 1H), 2.46 (d, J = 3.8 Hz, 6H), 2.31 - 2.02 (m, 4H), 2.00 - 1.90 (m, 2H), 1.87 - 1.64 (m, 3H), 1.51 (d, J = 16.2 Hz, 2H), 1.39 (d, J = 7.0 Hz, 3H), 0.96 (d, J = 6.4 Hz, 3H), 0.81 (d, J = 6.7 Hz, 3H). |
| 89 | (2S,4R)-4-hydroxy-1-((R)-2-(3-(7-(3-(2-hydroxyphenyl)thieno[2,3-c]pyridazin-6-yl)-2-azaspiro[3.5]nonan-2-yl)isoxazol-5-yl)-3-methylbutanoyl)-N-((S)-1-(4-(4-methylthiazol-5-yl)phenyl)ethyl)pyrrolidine-2-carboxamide | 832.15 | 1H NMR (400 MHz, DMSO-d6) δ 12.39 (s, 1H), 8.99 (s, 1H), 8.71 (s, 1H), 8.39 (d, J = 7.7 Hz, 1H), 8.01 (dd, J = 7.9, 1.7 Hz, 1H), 7.50 - 7.41 (m, 2H), 7.41 - 7.32 (m, 4H),7.06 - 6.97 (m, 2H), 5.86 (s, 1H), 5.10 (d, J = 3.6 Hz, 1H), 5.02 - 4.86 (m, 1H), 4.41 - 4.33 (m, 1H), 4.31 - 4.27 (m, 1H), 3.75 - 3.68 (m, 3H), 3.61 - 3.57 (m, 3H), 3.42 (d, J = 11.0 Hz, 1H), 3.11 - 3.04 (m, 1H), 2.46 (d, J = 1.9 Hz, 3H), 2.27 - 2.16 (m, 1H), 2.07 (d, J = 11.2 Hz, 5H), 1.85 - 1.70 (m, 1H), 1.70 - 1.53 (m, 4H), 1.41 - 1.35 (m, 3H),1.00 - 0.92 (m, 3H), 0.88 - 0.77 (m, 3H). |
| 98 | (2S,4R)-4-hydroxy-1-((R)-2-(3-(2-(3-(2-hydroxyphenyl)thieno[2,3-c]pyridazin-6-yl)-7-azaspiro[3.5]nonan-7-yl)isoxazol-5-yl)-3-methylbutanoyl)-N-((S)-1-(4-(4-methylthiazol-5-yl)phenyl)ethyl)pyrrolidine-2-carboxamide | 832.3 | 1H NMR (400 MHz, DMSO-d6) δ 12.38 (s, 1H), 9.00 (d, J = 3.0 Hz, 1H), 8.70 (s, 1H), 8.43 (d, J = 7.6 Hz, 1H), 8.01 (d, J = 7.9 Hz, 1H), 7.50 - 7.41 (m, 2H), 7.41 - 7.34 (m, 4H), 7.02 (dd, J = 11.1, 8.2 Hz, 2H), 6.17 (s, 1H), 5.12 (d, J = 3.6 Hz, 1H), 4.91 (t, J = 7.2 Hz, 1H), 4.36 (t, J = 8.0 Hz, 2H), 4.00 (q, J = 8.3 Hz, 1H), 3.71 (d, J = 7.0 Hz, 1H), 3.57 (d, J = 9.8 Hz, 1H), 3.22 (s, 2H), 3.11 (s, 3H), 2.46 (d, J = 1.3 Hz, 3H), 2.22 (s, 1H), 2.12 - 1.99 (m, 4H), 1.79 (s, 3H), 1.63 (s, 2H), 1.46 (d, J = 7.0 Hz, 1H), 1.41 - 1.33 (m, 6H), 1.01 (s, 2H), 0.95 (d, J = 6.6 Hz, 3H), 0.82 (dd, J = 21.1, 6.8 Hz, 3H). |
| 69 | (2S,4R)-4-hydroxy-1-((R)-2-(3-(6-(3-(2-hydroxyphenyl)thieno[3,2-c]pyridazin-6-yl)-2-azaspiro[3.3]heptan-2-yl)isoxazol-5-yl)-3-methylbutanoyl)-N-((S)-1-(4-(4-methylthiazol-5-yl)phenyl)ethyl)pyrrolidine-2-carboxamide | 804.3 | 1H NMR (400 MHz, Methanol-d4) δ 8.93 (s, 1H), 8.88 (s, 1H), 8.00 - 7.92 (m, 1H), 7.54 (s, 1H), 7.47 - 7.31 (m, 5H), 7.01 (t, J = 7.7 Hz, 2H), 5.86 (d, J = 19.7 Hz, 1H), 5.03 (q, J = 7.1 Hz, 1H), 4.51 (t, J = 8.2 Hz, 1H), 4.44 (s, 1H), 4.12 (d, J = 4.3 Hz, 2H), 3.94 (d, J = 4.4 Hz, 2H), 3.90 - 3.77 (m, 2H), 3.68 - 3.61 (m, 1H), 3.61 - 3.42 (m, 1H), 2.88 - 2.77 (m, 2H), 2.59 (dd, J = 12.4, 9.1 Hz, 2H), 2.48 (s, 3H), 2.42 - 2.29 (m, 1H), 2.22 - 2.09 (m, 1H), 1.95 (ddd, J = 13.3, 8.8, 4.6 Hz, 1H), 1.60 - 1.49 (m, 3H), 1.05 (d, J = 6.5 Hz, 3H), 0.90 (dd, J = 10.0, 6.7 Hz, 3H). |
| 70 | (2S,4R)-4-hydroxy-1-((2R)-2-(3-((1R,5S)-6-(3-(2-hydroxyphenyl)thieno[3,2-c]pyridazin-6-yl)-3-azabicyclo[3.1.0]hexan-3-yl)isoxazol-5-yl)-3-methylbutanoyl)-N-((S)-1-(4-(4-methylthiazol-5-yl)phenyl)ethyl)pyrrolidine-2-carboxamide | 790.3 | 1H NMR (400 MHz, DMSO-d6) δ 13.03 (s, 1H), 9.05 (d, J = 48.2 Hz, 2H), 8.42 (d, J = 7.7 Hz, 1H), 8.01 (dd, J = 8.1, 1.7 Hz, 1H), 7.64 (d, J = 3.3 Hz, 1H), 7.50 - 7.41 (m, 2H), 7.41 - 7.32 (m, 3H), 7.01 (dd, J = 11.9, 7.9 Hz, 2H), 6.02 (s, 1H), 5.12 (d, J = 3.5 Hz, 1H), 4.92 (p, J = 6.9 Hz, 1H), 4.37 (t, J = 7.8 Hz, 1H), 4.30 (s, 1H), 3.70 (ddd, J = 17.9, 10.3, 6.2 Hz, 3H), 3.59 (d, J = 10.0 Hz, 1H), 3.42 (t, J = 10.3 Hz, 3H), 2.49 - 2.41 (m, 4H), 2.35 (d, J = 3.3 Hz, 2H), 2.29 - 2.17 (m, 1H), 2.10 - 1.98 (m, 1H), 1.48 (d, J = 7.0 Hz, 1H), 1.39 (d, J = 7.0 Hz, 2H), 1.32 (d, J = 14.9 Hz, 1H), 0.97 (t, J = 7.1 Hz, 3H), 0.83 (dd, J = 14.6, 6.7 Hz, 3H). |
| 72 | (2S,4R)-4-hydroxy-1-((R)-2-(3-((R)-3-(3-(2-hydroxyphenyl)thieno[3,2-c]pyridazin-6-yl)pyrrolidin-1-yl)isoxazol-5-yl)-3-methylbutanoyl)-N-((S)-1-(4-(4-methylthiazol-5-yl)phenyl)ethyl)pyrrolidine-2-carboxamide | 863.4 | 1H NMR (400 MHz, Methanol-d4) δ 8.85 (d, J = 17.8 Hz, 1H), 7.78 (d, J = 8.0 Hz, 1H), 7.47 - 7.34 (m, 4H), 7.25 - 7.14 (m, 2H), 6.88 (d, J = 8.1 Hz, 2H), 6.26 (d, J = 18.8 Hz, 1H), 5.03 (t, J = 7.0 Hz, 1H), 4.50 (t, J = 8.2 Hz, 1H), 4.43 (s, 1H), 3.97 - 3.71 (m, 3H), 3.66 - 3.47 (m, 4H), 3.19 - 3.06 (m, 2H), 3.05 - 2.94 (m, 1H), 2.74 - 2.66 (m, 4H), 2.62 - 2.50 (m, 4H), 2.46 (d, J = 10.6 Hz, 3H), 2.39 - 2.27 (m, 5H), 2.22 - 2.12 (m, 1H), 2.01 - 1.82 (m, 4H), 1.61 - 1.46 (m, 3H), 1.09 - 1.01 (m, 3H), 0.92 - 0.80 (m, 3H). |
| 73 | (2S,4R)-4-hydroxy-1-((R)-2-(3-((S)-3-(3-(2-hydroxyphenyl)thieno[3,2-c]pyridazin-6-yl)pyrrolidin-1-yl)isoxazol-5-yl)-3-methylbutanoyl)-N-((S)-1-(4-(4-methylthiazol-5-yl)phenyl)ethyl)pyrrolidine-2-carboxamide | 778.1 | 1H NMR (400 MHz, DMSO-d6) δ 12.91 (s, 1H), 9.15 (d, J = 3.4 Hz, 1H), 8.99 (s, 1H), 8.41 (d, J = 7.7 Hz, 1H), 8.01 (dd, J = 8.0, 1.7 Hz, 1H), 7.80 (s, 1H), 7.49 - 7.42 (m, 2H), 7.40 - 7.32 (m, 3H), 7.02 (dd, J = 13.0, 7.8 Hz, 2H), 6.04 (s, 1H), 5.13 (s, 1H), 4.93 (q, J = 7.2 Hz, 1H), 4.37 (t, J = 7.9 Hz, 1H), 4.29 (s, 1H), 4.03 (q, J = 7.1 Hz, 1H), 3.76 (dd, J = 10.1, 5.7 Hz, 2H), 3.60 (d, J = 9.8 Hz, 1H), 3.56 - 3.41 (m, 2H), 3.22 - 3.04 (m, 2H), 2.46 (s, 3H), 2.23 (s, 2H), 2.21 - 2.16 (m, 1H), 2.02 (t, J = 10.2 Hz, 2H), 1.38 (d, J = 7.0 Hz, 3H), 0.97 (t, J = 7.0 Hz, 3H), 0.84 (dd, J = 14.3, 6.4 Hz, 3H). |

### Preparation of (2S,4R)-4-hydroxy-1-((R)-2-(3-(2-(3-(2-hydroxyphenyl)thieno[3,2-c]pyridazin-6-yl)-7-azaspiro[3.5]nonan-7-yl)isoxazol-5-yl)-3-methylbutanoyl)-N-((S)-1-(4-(4-methylthiazol-5-yl)phenyl)ethyl)pyrrolidine-2-carboxamide (Compound 12)

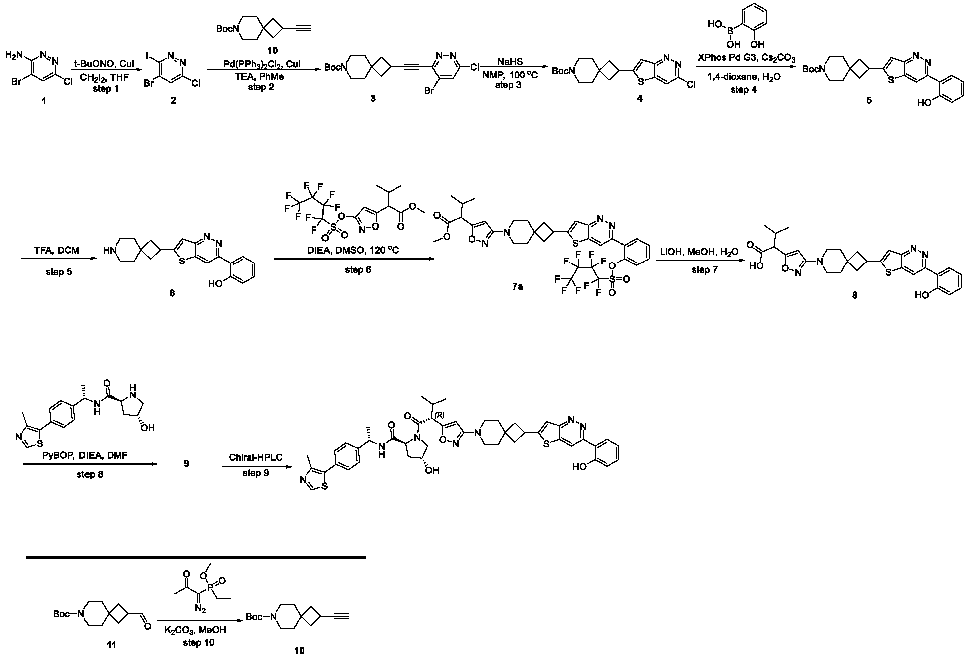

### Step 1: Preparation of 4-bromo-6-chloro-3-iodopyridazine (intermediate 2)

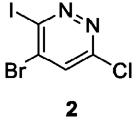

To a stirred solution of 4-bromo-6-chloropyridazin-3-amine (5 g, 23.988 mmol, 1 equiv) and Cul (5.48 g, 28.786 mmol, 1.2 equiv) in THF (60 mL) and CH₂I₂ (7.71 g, 28.786 mmol, 1.2 equiv), t-BuNO₂ (2.47 g, 23.988 mmol, 1 equiv) was added after the resulting solution was stirred at 25 degrees C for 0.5 hours. The crude product was purified by silica column chromatography, elution gradient 0 to 80% EA in PE to give intermediate 2 (2.9 g, 37.86%) as a yellow solid. LCMS (ESI) m/z: [M+H]⁺ =319.

### Step 2: Preparation of tert-butyl 2-((4-bromo-6-chloropyridazin-3-yl)ethynyl)-7-azaspiro[3.5]nonane-7-carboxylate (intermediate 3)

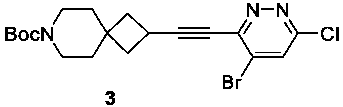

To a solution of intermediate 2 (2 g, 6.263 mmol, 1 equiv) and tert-butyl2-ethynyl-7-azaspiro[3.5]nonane-7-carboxylate (1.56g,6.263mmol, 1 equiv) and Pd(PPh₃)₂Cl₂ (0.88 g, 1.253 mmol, 0.2 equiv) and Cul (0.24 g, 1.253 mmol, 0.2 equiv) in methylbenzene (15 mL, 0.011 mmol) and TEA (1.90 g, 18.789 mmol, 3 equiv) , the resulting solution was stirred at 25 degrees C for 2h. The crude product was purified by silica column chromatography, elution gradient 0 to 17% EA in PE to give intermediate 3 (1.6 g, 57.96%) as a yellow oil. LCMS (ESI) m/z: [M+H]⁺ =441.

### Step 3: Preparation of tert-butyl 2-(3-chlorothieno[3,2-c]pyridazin-6-yl)-7-azaspiro[3.5]nonane-7-carboxylate (intermediate 4)

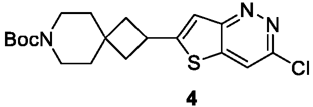

To a solution of intermediate 3 (1.6 g, 3.630 mmol, 1 equiv) and NaSH (203.50 mg, 3.630 mmol, 1 equiv) in NMP (10 mL, 51.850 mmol), the resulting solution was stirred at 100 degrees C for 2h. The resulting mixture was diluted with EA (400 mL) and washed with water (3x 400 mL). The organic layer was dried over anhydrous Na₂SO₄. After filtration, the filtrate was concentrated under reduced pressure. After filtration, the filtrate was concentrated under reduced pressure. The residue was purified by silica gel column chromatography, elution gradient 0 to 34% EA in PE to give intermediate 4 (890 mg, 62.24%) as an orange solid. LCMS (ESI) m/z: [M+H]+ =394.

### Step 4: Preparation of tert-butyl 2-(3-(2-hydroxyphenyl)thieno[3,2-c]pyridazin-6-yl)-7-azaspiro[3.5]nonane-7-carboxylate (intermediate 5)

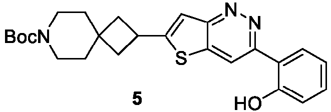

To a solution of intermediate 4 (890 mg, 2.259 mmol, 1 equiv) and 2-hydroxyphenylboronic acid (467.43 mg, 3.388 mmol, 1.5 equiv) ,Cs₂CO₃ (1.47 g, 4.518 mmol, 2 equiv), XPhos Pd G3 (191.24 mg, 0.226 mmol, 0.1 equiv) in 1,4-dioxane (4 mL) and H₂O (1 mL) , the resulting solution was stirred at 80 degrees C for 2 hours. The resulting mixture was dilutd with EA (400 mL) and washed with water (3x 400 mL). The organic layer was dried over anhydrous Na₂SO₄. After filtration, the filtrate was concentrated under reduced pressure. The residue was purified by silica gel column chromatography, elution gradient 0 to 15% EA in PE to give intermediate 5 (571 mg, 55.97%) as a brown oil. LCMS (ESI) m/z: [M+H]⁺ =452.

### Step 5: Preparation of 2-(6-(7-azaspiro[3.5]nonan-2-yl)thieno[3,2-c]pyridazin-3-yl)phenol (intermediate 6)

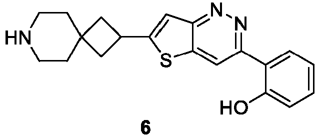

To a solution of intermediate 5 (571 mg, 1.264 mmol, 1 equiv) in TFA (1 mL) and DCM (3 mL), the resulting solution was stirred at 25 degrees C for 2 hours. The reaction was concentrated under reduced pressure to give intermediate 6 (667 mg, crude) as a brown oil that was used in the next step directly without further purification. LCMS (ESI) m/z: [M+H]⁺ =352

### Step 6: Preparation of methyl 3-methyl-2-{3-[2-(3-{2-[(1,1,2,2,3,3,4,4,4-nonafluorobutanesulfonyl)oxy]phenyl}thieno[3,2-c]pyridazin-6-yl)-7-azaspiro[3.5]nonan-7-yl]-1,2-oxazol-5-yl}butanoate (intermediate 7a)

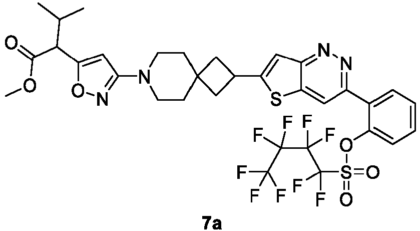

To a stirred solution of intermediate 6 (300 mg, 0.854 mmol, 1 equiv) and methyl 3-methyl-2-{3-[(1,1,2,2,3,3,4,4,4-nonafluorobutanesulfonyl)oxy]-1,2-oxazol-5-yl}butanoate (410.81 mg, 0.854 mmol, 1.0 equiv) in DMSO (5 mL) were added DIEA (330.96 mg, 2.562 mmol, 3.0 equiv) at room temperature under nitrogen atmosphere. The resulting mixture was stirred for 2 h at 100 °C under nitrogen atmosphere. The residue was purified by reverse flash chromatography with the following conditions: column, C18 silica gel; mobile phase, MeCN in Water (0.1% TFA), 0% to 100% gradient in 10 min; detector, UV 254 nm. This resulted in intermediate 7a (181 mg, 26.03%) as a Brown yellow solid. LCMS (ESI) m/z: [M+H]⁺ =815.

### Step 7: Preparation of 2-(3-(2-(3-(2-hydroxyphenyl)thieno[3,2-c]pyridazin-6-yl)-7-azaspiro[3.5]nonan-7-yl)isoxazol-5-yl)-3-methylbutanoic acid (intermediate 8)

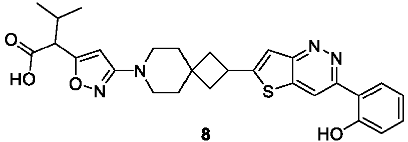

To a solution of intermediate **7** (170 mg, 0.209 mmol, 1 equiv) and NaOH (83.46 mg, 2.090 mmol, 10 equiv) in MeOH (4 mL) and H₂O (1 mL), the resulting solution was stirred at 25 degrees C for 4 hours. The mixture was acidified to pH 6 with conc. HCl. The resulting mixture was extracted with EtOAc (3 x 200 mL). The combined organic layers were washed with brine (3x10 mL), dried over anhydrous Na₂SO₄. After filtration, the filtrate was concentrated under reduced pressure to give intermediate **8** (155 mg, crude) as a brown solid. LCMS (ESI) m/z: [M+H]⁺ =519.

### Step8: Preparation of (2S,4R)-4-hydroxy-1-(2-(3-(2-(3-(2-hydroxyphenyl)thieno[3,2-c]pyridazin-6-yl)-7-azaspiro[3.5]nonan-7-yl)isoxazol-5-yl)-3-methylbutanoyl)-N-((S)-1-(4-(4-methylthiazol-5-yl)phenyl)ethyl)pyrrolidine-2-carboxamide (intermediate 9)

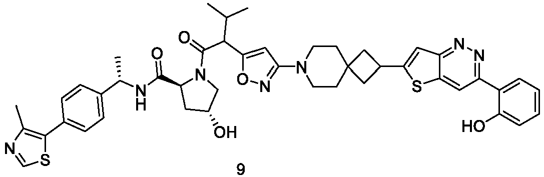

To a solution of intermediate **8** (155 mg, 0.299 mmol, 1 equiv) and (2S,4R)-4-hydroxy-N-[(1S)-1-[4-(4-methyl-1,3-thiazol-5-yl) phenyl] ethyl] pyrrolidine-2-carboxamide (99.05 mg, 0.299 mmol, 1 equiv) and PyBOP (311.06 mg, 0.598 mmol, 2 equiv) in DMF (2 mL) and DIEA (193.14 mg, 1.495 mmol, 5 equiv), the resulting solution was stirred at 25 degrees C for 2 hours. Without additional work-up, the crude reaction solution was purified by Prep-HPLC (Column: XBridge Prep OBD C18 Column, 30*150 mm, 5µm; Mobile Phase A: water (10 mmol/L NH₄HCO₃+0.1%NH₃.H₂O), Mobile Phase B: ACN; Flow rate: 60 mL/min; Gradient: 45% B to 70% B in 7 min, 70% B; Wave Length: 254/220 nm; RT1(min): 7.55; to give intermediate **9** (30 mg,34,72%) as a yellow solid. LCMS (ESI) m/z: [M+H]⁺ =832.

### Step9: Preparation of (2S,4R)-4-hydroxy-1-((R)-2-(3-(2-(3-(2-hydroxyphenyl)thieno[3,2-c]pyridazin-6-yl)-7-azaspiro[3.5]nonan-7-yl)isoxazol-5-yl)-3-methylbutanoyl)-N-((S)-1-(4-(4-methylthiazol-5-yl)phenyl)ethyl)pyrrolidine-2-carboxamide

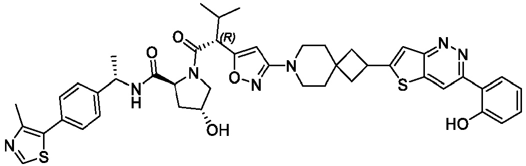

The intermediate 9 was purified by Chiral-Prep-HPLC with the following conditions:Column: CHIRAL ART Cellulose-SB, 2*25 cm, 5 µm; Mobile Phase A: MtBE (10mM NH3-MeOH), Mobile Phase B: MeOH--HPLC; Flow rate: 20 mL/min; Gradient: 20% B to 20% B in 13 min; Wave Length: 270/212 nm; RT1(min): 7.8; RT2(min): 10.4; Sample Solvent: MeOH: DCM=1: 2; Injection Volume: 0.5 mL; Number Of Runs: 4.This resulted in title compound (second peak) (11.8 mg, 4.51%) as a white solid. (¹H NMR (300 MHz, Methanol-d4) *δ* 8.92 (d, J = 15.4 Hz, 2H), 7.99 (d, J = 8.0 Hz, 1H), 7.56 (s, 1H), 7.52 - 7.37 (m, 4H), 7.04 (d, J = 7.8 Hz, 2H), 6.11 (d, J = 16.1 Hz, 1H), 5.12 - 5.02 (m, 1H), 4.54 (t, J = 8.1 Hz, 1H), 4.46 (s, 1H), 4.07 - 4.42 (m, 1H), 4.05 (dd, J = 10.8, 4.1 Hz, 1H), 3.92- 3.83 (m, 1H), 3.64 (dd, J = 10.4, 6.1 Hz, 2H), 3.45 (s, 2H), 3.35 (m, 2H), 3.18 (d, J = 6.3 Hz, 2H), 2.56 (t, J = 10.3 Hz, 2H), 2.50 (s, 3H), 2.39 (s, 1H), 2.20 (t, J = 10.3 Hz, 3H), 2.06 - 1.93 (m, 1H), 1.91 (s, 2H), 1.75 (s, 2H), 1.58 (dd, J = 20.1, 7.0 Hz, 3H), 1.08 (d, J = 6.5 Hz, 3H), 0.92 (d, J = 6.7 Hz, 3H). LCMS (ESI) m/z: [M+H]⁺= 832.35

### Preparation of (2S,4R)-4-hydroxy-1-((R)-2-(3-(6-(4-(3-(2-hydroxyphenyl)thieno[2,3-c]pyridazin-6-yl)piperidin-1-yl)-5-methylpyridin-3-yl)isoxazol-5-yl)-3-methylbutanoyl)-N-((S)-1-(4-(4-methylthiazol-5-yl)phenyl)ethyl)pyrrolidine-2-carboxamide (Compound 2)

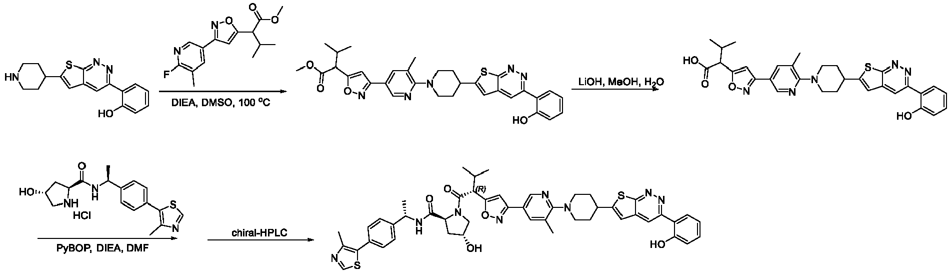

### Step 1: Preparation of methyl 2-(3-(6-(4-(3-(2-hydroxyphenyl)thieno[2,3-c]pyridazin-6-yl)piperidin-1-yl)-5-methylpyridin-3-yl)isoxazol-5-yl)-3-methylbutanoate

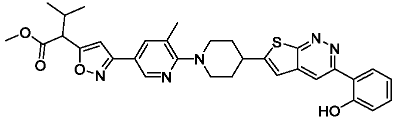

To a stirred solution of Intermediate 3 (200 mg, 0.642 mmol, 1 equiv) and Intermediate 7 (187.74 mg, 0.642 mmol, 1 equiv) in DMSO (4 mL) was added DIEA (249.04 mg, 1.926 mmol, 3 equiv) dropwise at room temperature under nitrogen atmosphere. The resulting mixture was stirred for 36 h at 110°C under nitrogen atmosphere. Desired product could be detected by LCMS. The residue was purified by reverse flash chromatography with the following conditions: column, C18 silica gel; mobile phase, MeCN in Water (10mmol/L NH₄HCO₃), 0% to 100% gradient in 30 min; detector, UV 254 nm. The resulting mixture was concentrated under reduced pressure. This resulted in methyl 2-(3-(6-(4-(3-(2-hydroxyphenyl)thieno[2,3-c]pyridazin-6-yl)piperidin-1-yl)-5-methylpyridin-3-yl)isoxazol-5-yl)-3-methylbutanoate (50 mg) as a yellow solid. LCMS (ESI) m/z: [M+H]⁺ = 384.

### Step 2: Preparation of 2-(3-(6-(4-(3-(2-hydroxyphenyl)thieno[2,3-c]pyridazin-6-yl)piperidin-1-yl)-5-methylpyridin-3-yl)isoxazol-5-yl)-3-methylbutanoic acid

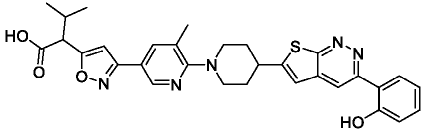

A solution of methyl 2-(3-(6-(4-(3-(2-hydroxyphenyl)thieno[2,3-c]pyridazin-6-yl)piperidin-1-yl)-5-methylpyridin-3-yl)isoxazol-5-yl)-3-methylbutanoate (50 mg, 0.086 mmol, 1 equiv) in MeOH (2 mL) was treated with LiOH.H₂O (20.52 mg, 0.860 mmol, 10 equiv) at room temperature followed by the addition of H₂O (1 mL) dropwise at room temperature. The resulting mixture was stirred for 1 h at room temperature. The mixture was acidified to pH 5 with HCl (aq.). The precipitated solids were collected by filtration and washed with H₂O (3 x 10 mL). This resulted in 2-(3-(6-(4-(3-(2-hydroxyphenyl)thieno[2,3-c]pyridazin-6-yl)piperidin-1-yl)-5-methylpyridin-3-yl)isoxazol-5-yl)-3-methylbutanoic acid (47 mg) as a yellow solid. LCMS (ESI) m/z: [M+H]⁺ = 570.

### Step 3: Preparation of (2S,4R)-4-hydroxy-1-(2-(3-(6-(4-(3-(2-hydroxyphenyl)thieno[2,3-c]pyridazin-6-yl)piperidin-1-yl)-5-methylpyridin-3-yl)isoxazol-5-yl)-3-methylbutanoyl)-N-((S)-1-(4-(4-methylthiazol-5-yl)phenyl)ethyl)pyrrolidine-2-carboxamide

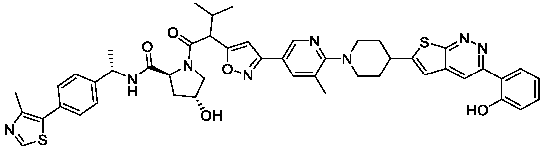

To a stirred solution of 2-(3-(6-(4-(3-(2-hydroxyphenyl)thieno[2,3-c]pyridazin-6-yl)piperidin-1-yl)-5-methylpyridin-3-yl)isoxazol-5-yl)-3-methylbutanoic acid (40 mg, 0.070 mmol, 1 equiv) and Intermediate 8 (27.93 mg, 0.084 mmol, 1.2 equiv) in DMF (0.5 mL) were added PyBOP (73.08 mg, 0.140 mmol, 2 equiv) and DIEA (27.23 mg, 0.210 mmol, 3 equiv) in portions at room temperature. The resulting mixture was stirred for 1.5 h at room temperature. Desired product could be detected by LCMS. The residue was purified by reverse flash chromatography with the following conditions: column, C18 silica gel; mobile phase, MeCN in Water (10mmol/L NH₄HCO₃), 0% to 100% gradient in 30 min; detector, UV 254 nm. The resulting mixture was concentrated under reduced pressure. This resulted in (2S,4R)-4-hydroxy-1-(2-(3-(6-(4-(3-(2-hydroxyphenyl)thieno[2,3-c]pyridazin-6-yl)piperidin-1-yl)-5-methylpyridin-3-yl)isoxazol-5-yl)-3-methylbutanoyl)-N-((S)-1-(4-(4-methylthiazol-5-yl)phenyl)ethyl)pyrrolidine-2-carboxamide (35 mg) as a light yellow solid. LCMS (ESI) m/z: [M+H]⁺ = 883.

### Step 6: Preparation of (2S,4R)-4-hydroxy-1-((R)-2-(3-(6-(4-(3-(2-hydroxyphenyl)thieno[2,3-c]pyridazin-6-yl)piperidin-1-yl)-5-methylpyridin-3-yl)isoxazol-5-yl)-3-methylbutanoyl)-N-((S)-1-(4-(4-methylthiazol-5-yl)phenyl)ethyl)pyrrolidine-2-carboxamide

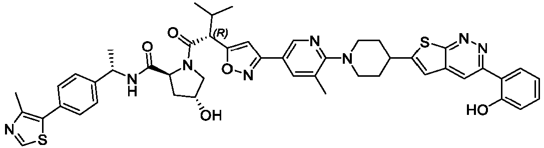

The above product was purified by Chiral-Prep-HPLC with the following conditions: Column, CHIRALPAK ID, 2*25 cm, 5 um; mobile phase, MtBE:DCM=2:1(10mM/L) and MeOH- (hold 50% MeOH- in 12 min); Detector, UV 254. This resulted in title compound (second peak) (14.3 mg) as an off-white solid. ¹H NMR (300 MHz, DMSO-d₆) *δ* 12.38 (s, 1H), 8.99 (s, 1H), 8.74 (s, 1H), 8.60 (d, J = 2.3 Hz, 1H), 8.46 (t, J = 8.1 Hz, 1H), 8.07 - 7.96 (m, 2H), 7.48 - 7.34 (m, 6H), 7.09 - 6.97 (m, 2H), 6.87 (d, J = 39.5 Hz, 1H), 5.13 (s, 1H), 5.02 - 4.88 (m, 1H), 4.40 (t, J = 7.9 Hz, 1H), 4.31 (s, 1H), 3.86 (d, J = 9.6 Hz, 1H), 3.78 - 3.68 (m, 3H), 3.53 (d, J = 7.6 Hz, 2H), 3.00 (t, J = 12.1 Hz, 2H), 2.47 (s, 3H), 2.37 - 2.33 (m, 3H), 2.22 (d, J = 12.5 Hz, 3H), 2.11 - 1.89 (m, 3H), 1.82 (d, J = 8.7 Hz, 1H), 1.54 - 1.36 (m, 3H), 1.02 (d, J = 6.4 Hz, 3H), 0.84 (d, J = 6.8 Hz, 3H). LCMS (ESI) m/z: [M+H]⁺ = 883.25.

The compound in Table 3 was prepared using a procedure similar to the one used above for the preparation of (2S,4R)-4-hydroxy-1-((R)-2-(3-(6-(4-(3-(2-hydroxyphenyl)thieno[2,3-c]pyridazin-6-yl)piperidin-1-yl)-5-methylpyridin-3-yl)isoxazol-5-yl)-3-methylbutanoyl)-N-((S)-1-(4-(4-methylthiazol-5-yl)phenyl)ethyl)pyrrolidine-2-carboxamide (Compound 2) using the appropriate fluoropyridine and 2-(5-methyl-6-(piperidin-4-yl)thieno[2,3-c]pyridazin-3-yl)phenol.

**Table 3.**

| **No.** | **Name** | **LCMS (ESI) m/z** | **¹H NMR** |
|---|---|---|---|
| 10 | (2S,4R)-4-hydroxy-1-((R)-2-(3-(2-(4-(3-(2-hydroxyphenyl)-5-methylthieno[2,3-c]pyridazin-6-yl)piperidin-1-yl)pyridin-4-yl)isoxazol-5-yl)-3-methylbutanoyl)-N-((S)-1-(4-(4-methylthiazol-5-yl)phenyl)ethyl)pyrrolidine-2-carboxamide | 883.25 | 1H NMR (300 MHz, DMSO-d6) δ 12.79 (d, J = 3.9 Hz, 1H), 8.99 (d, J = 1.7 Hz, 1H), 8.72 (s, 1H), 8.43 (d, J = 7.7 Hz, 1H), 8.24 (dd, J = 5.4, 2.4 Hz, 1H), 8.18 (dd, J = 8.3, 1.7 Hz, 1H), 7.49 - 7.31 (m, 6H), 7.17 (s, 1H), 7.13 - 6.97 (m, 3H), 5.12 (d, J = 3.7 Hz, 1H), 4.94 (t, J = 7.2 Hz, 1H), 4.65 (d, J = 12.9 Hz, 2H), 4.40 (t, J = 7.9 Hz, 1H), 4.31 (s, 1H), 3.89 (d, J = 9.6 Hz, 1H), 3.77 (dd, J = 10.6, 4.3 Hz, 1H), 3.65 (d, J = 16.1 Hz, 1H), 3.53 (d, J = 7.2 Hz, 1H), 3.08 (t, J = 12.5 Hz, 2H), 2.45 (d, J = 5.6 Hz, 4H), 2.39 - 2.24 (m, 1H), 2.07 (d, J = 12.5 Hz, 3H), 1.85 - 1.65 (m, 3H), 1.44 (dd, J = 33.2, 6.9 Hz, 3H), 1.24 (s, 1H), 1.03 (d, J = 6.5 Hz, 3H), 0.86 (t, J = 6.3 Hz, 3H). |
| 20 | (2S,4R)-4-hydroxy-1-((R)-2-(3-(6-(4-(3-(2-hydroxyphenyl)-5-methylthieno[2,3-c]pyridazin-6-yl)piperidin-1-yl)-2-methoxypyridin-3-yl)isoxazol-5-yl)-3-methylbutanoyl)-N-((S)-1-(4-(4-methylthiazol-5-yl)phenyl)ethyl)pyrrolidine-2-carboxamide | 913.3 | 1H NMR (400 MHz, DMSO-d6) δ 12.77 (d, J = 4.2 Hz, 1H), 8.99 (s, 1H), 8.71 (s, 1H), 8.43 (d, J = 7.6 Hz, 1H), 8.20 - 8.13 (m, 1H), 8.00 (dd, J = 8.5, 2.0 Hz, 1H), 7.45 (d, J = 8.2 Hz, 2H), 7.38 (dt, J = 8.5, 3.6 Hz, 3H), 7.06 - 6.98 (m, 2H), 6.71 (s, 1H), 6.58 (d, J = 8.7 Hz, 1H), 5.10 (d, J = 3.7 Hz, 1H), 4.95 (dt, J = 14.2, 6.9 Hz, 1H), 4.58 (d, J = 13.1 Hz, 2H), 4.40 (t, J = 7.9 Hz, 1H), 4.30 (s, 1H), 3.95 (d, J = 1.5 Hz, 3H), 3.85 - 3.71 (m, 3H), 3.63 (t, J = 12.7 Hz, 1H), 3.23 - 3.12 (t, J = 12.5 Hz, 2H), 2.45 (d, J = 7.2 Hz, 3H), 2.10 - 2.06 (m, 3H), 2.02 (d, J = 9.7 Hz, 1H), 1.70 (d, J = 11.3 Hz, 3H), 1.50 (d, J = 6.9 Hz, 3H), 1.40 (d, J = 7.0 Hz, 3H), 1.00 (d, J = 6.3 Hz, 3H), 0.83 (dd, J = 11.6, 6.6 Hz, 3H). |
| 21 | (2S,4R)-1-((S)-2-(4-chloro-3-(6-(4-(3-(2-hydroxyphenyl)-5-methylthieno[2,3-c]pyridazin-6-yl)piperidin-1-yl)-2-methylpyridin-3-yl)isoxazol-5-yl)-3-methylbutanoyl)-4-hydroxy-N-((S)-1-(4-(4-methylthiazol-5-yl)phenyl)ethyl)pyrrolidine-2-carboxamide | 931.25 | 1H NMR (400 MHz, DMSO-d6) δ 12.78 (s, 1H), 8.99 (s, 1H), 8.72 (s, 1H), 8.45 (d, J = 7.6 Hz, 1H), 8.17 (d, J = 7.9 Hz, 1H), 7.57 (d, J = 8.8 Hz, 1H), 7.45 (d, J = 8.0 Hz, 2H), 7.38 (d, J = 7.6 Hz, 3H), 7.03 (d, J = 7.7 Hz, 2H), 6.88 (d, J = 8.9 Hz, 1H), 5.14 (d, J = 3.8 Hz, 1H), 4.94 (t, J = 7.3 Hz, 1H), 4.63 (d, J = 13.1 Hz, 2H), 4.41 (t, J = 7.8 Hz, 1H), 4.31 (s, 1H), 3.89 (d, J = 10.1 Hz, 1H), 3.80 (d, J = 9.8 Hz, 1H), 3.62 (s, 1H), 3.53 - 3.40 (m, 1H), 3.08 (t, J = 12.6 Hz, 2H), 2.46 (s, 3H), 2.31 (s, 3H), 2.08 (d, J = 13.4 Hz, 4H), 1.82 (d, J = 6.2 Hz, 1H), 2.12 - 2.04 (m, 3H), 1.43 - 1.32 (m, 3H), 1.06 (d, J = 6.6 Hz, 3H), 0.88 - 0.80 (m, 3H). |
| 38 | (2S,4R)-4-hydroxy-1-((R)-2-(3-(6-(4-(3-(2-hydroxyphenyl)-5-methylthieno[2,3-c]pyridazin-6-yl)piperidin-1-yl)-4-methylpyridin-3-yl)isoxazol-5-yl)-3-methylbutanoyl)-N-((S)-1-(4-(4-methylthiazol-5-yl)phenyl)ethyl)pyrrolidine-2-carboxamide | 897.4 | 1H NMR (400 MHz, DMSO-d6) δ 12.77 (d, J = 4.2 Hz, 1H), 8.98 (d, J = 1.2 Hz, 1H), 8.71 (s, 1H), 8.43 (d, J = 7.7 Hz, 1H), 8.32 (d, J = 13.7 Hz, 1H), 8.22 - 8.03 (m, 1H), 7.48 - 7.42 (m, 2H), 7.42 - 7.33 (m, 3H), 7.06 - 6.98 (m, 2H), 6.90 (d, J = 4.5 Hz, 1H), 6.76 (s, 1H), 5.21 - 4.80(m, 2H), 4.60 (d, J = 13.1 Hz, 2H), 4.40 (t, J = 7.9 Hz, 1H), 4.31 (s, 1H), 3.84 (d, J = 9.7 Hz, 1H), 3.76 (dd, J = 10.6, 4.4 Hz, 1H), 3.70 - 3.57 (m, 1H), 3.55 - 3.45 (m, 1H), 3.08 (t, J = 12.6 Hz, 2H), 2.51 - 2.49 (m, 1H), 2.47 - 2.44 (m, 3H), 2.43 - 2.39 (m, 4H), 2.38 - 2.35 (m, 1H), 2.34 - 2.20 (m, 1H), 2.06 (d, J = 12.6 Hz, 3H), 1.93 - 1.75 (m, 1H), 1.74 - 1.60 (m, 2H), 1.39 (d, J = 7.0 Hz, 3H), 1.02 (d, J = 6.4 Hz, 3H), 0.87 (dd, J = 11.9, 6.7 Hz, 3H). |
| 42 | (2S,4R)-4-hydroxy-1-((R)-2-(3-(6-(4-(3-(2-hydroxyphenyl)-5-methylthieno[2,3-c]pyridazin-6-yl)piperidin-1-yl)-2-methylpyridin-3-yl)isoxazol-5-yl)-3-methylbutanoyl)-N-((S)-1-(4-(4-methylthiazol-5-yl)phenyl)ethyl)pyrrolidine-2-carboxamide | 897.4 | 1H NMR (300 MHz, DMSO-d6) δ 12.79 (s, 1H), 9.01 (s, 1H), 8.76 (s, 1H), 8.45 (d, J = 7.6 Hz, 1H), 8.18 (dd, J = 8.4, 1.7 Hz, 1H), 7.78 (t, J = 9.1 Hz, 1H), 7.50 - 7.31 (m, 5H), 7.15 - 6.94 (m, 2H), 6.85 (d, J = 8.9 Hz, 1H), 6.72 (s, 1H), 5.14 - 5.01 (m, 1H), 5.00 - 4.81 (m, 1H), 4.66 (d, J = 13.1 Hz, 2H), 4.40 (t, J = 7.9 Hz, 1H), 4.32 (s, 1H), 3.85 (d, J = 11.1 Hz, 1H), 3.80 - 3.70 (m, 1H), 3.70 - 3.49 (m, 2H), 3.05 (t, J = 12.4 Hz, 2H), 2.56 (s, 3H), 2.49 - 2.40 (m, 6H), 2.39 - 2.22 (m, 1H), 2.08 (d, J = 12.6 Hz, 3H), 1.97 - 1.78 (m, 1H), 1.77 - 1.59 (m, 2H), 1.50 - 1.39 (m, 3H), 1.03 (d, J = 6.5 Hz, 3H), 0.93 - 0.81 (m, 3H). |
| 43 | (2S,4R)-4-hydroxy-1-((R)-2-(3-(6-(4-(3-(2-hydroxyphenyl)-5-methylthieno[2,3-c]pyridazin-6-yl)piperidin-1-yl)-5-methylpyridin-3-yl)isoxazol-5-yl)-3-methylbutanoyl)-N-((S)-1-(4-(4-methylthiazol-5-yl)phenyl)ethyl)pyrrolidine-2-carboxamide | 897.4 | 1H NMR (400 MHz, DMSO-d6) δ 12.81 (d, J = 3.6 Hz, 1H), 8.99 (s, 1H), 8.72 (s, 1H), 8.60 (d, J = 2.3 Hz, 1H), 8.45 (d, J = 7.7 Hz, 1H), 8.18 (d, J = 7.9 Hz, 1H), 7.99 (d, J = 2.4 Hz, 1H), 7.45 (d, J = 8.1 Hz, 2H), 7.42 - 7.34 (m, 3H), 7.04 (d, J = 7.8 Hz, 2H), 6.94 (s, 1H), 5.12 (d, J = 3.6 Hz, 1H), 4.97 - 4.89 (m, 1H), 4.40 (t, J = 7.9 Hz, 1H), 4.31 (s, 1H), 3.86 (d, J = 9.7 Hz, 1H), 3.77 - 3.63 (m, 3H), 3.52 (d, J = 10.7 Hz, 2H), 3.02 (t, J = 12.2 Hz, 2H), 2.55 - 2.48 (m, 6H), 2.46 - 2.36 (m, 4H), 2.21 - 2.11 (m, 3H), 1.98 - 1.91 (m, 3H), 1.55 - 1.44 (m, 3H), 1.02 (d, J = 6.5 Hz, 3H), 0.85 (dd, J = 8.9, 6.7 Hz, 3H). |
| 92 | (2S,4R)-4-hydroxy-1-((R)-2-(3-(6-(4-(3-(2-hydroxyphenyl)-5-methylthieno[2,3-c]pyridazin-6-yl)piperidin-1-yl)-2-methylpyridin-3-yl)-4-methylisoxazol-5-yl)-3-methylbutanoyl)-N-((S)-1-(4-(4-methylthiazol-5-yl)phenyl)ethyl)pyrrolidine-2-carboxamide | 911.45 | 1H NMR (400 MHz, DMSO-d6) δ 12.78 (s, 1H), 8.99 (s, 1H), 8.72 (s, 1H), 8.42 (d, J = 7.7 Hz, 1H), 8.20 - 8.14 (m, 1H), 7.49 - 7.42 (m, 3H), 7.41 - 7.33 (m, 3H), 7.06 - 6.98 (m, 2H), 6.83 (d, J = 8.8 Hz, 1H), 5.12 - 4.87 (m, 2H), 4.60 (d, J = 12.8 Hz, 2H), 4.40 (t, J = 7.8 Hz, 1H), 4.30 (s, 1H), 3.83 - 3.76 (m, 2H), 3.66 - 3.55 (m, 2H), 3.05 (t, J = 12.3 Hz, 2H), 2.48 - 2.44 (m, 6H), 2.24 (s, 3H), 2.12 - 1.97 (m, 4H), 1.91 (s, 3H), 1.87 - 1.75 (m, 1H), 1.72 - 1.65 (m, 2H), 1.40 (d, J = 7.1 Hz, 3H), 1.03 (d, J = 6.5 Hz, 3H), 0.78 (d, J = 6.7 Hz, 3H). |
| 94 | (2S,4R)-4-hydroxy-1-((R)-2-(3-(6-((R)-3-(3-(2-hydroxyphenyl)-5-methylthieno[2,3-c]pyridazin-6-yl)pyrrolidin-1-yl)-2-methylpyridin-3-yl)isoxazol-5-yl)-3-methylbutanoyl)-N-((S)-1-(4-(4-methylthiazol-5-yl)phenyl)ethyl)pyrrolidine-2-carboxamide | 883.3 | 1H NMR (300 MHz, DMSO-d6) δ 12.74 (d, J = 2.8 Hz, 1H), 8.99 (s, 1H), 8.86 - 8.70 (m, 1H), 8.43 (d, J = 7.7 Hz, 1H), 8.22 - 8.08 (m, 1H), 7.75 (t, J = 9.6 Hz, 1H), 7.45 (d, J = 8.2 Hz, 2H), 7.44 - 7.33 (m, 3H), 7.08 - 6.97 (m, 2H), 6.71 (s, 1H), 6.57 - 6.47 (m, 1H), 5.07 - 4.88 (m, 2H), 4.40 (t, J = 7.9 Hz, 1H), 4.33 - 4.18 (m, 2H), 4.06 (t, J = 9.0 Hz, 1H), 3.88 - 3.70 (m, 3H), 3.70 - 3.48 (m, 3H), 2.54 (d, J = 1.7 Hz, 6H), 2.49 - 2.43 (m, 3H), 2.39 - 2.13 (m, 3H), 2.10 - 1.98 (m, 1H), 1.87 - 1.73 (m, 1H), 1.52 - 1.35 (m, 3H), 1.01 (d, J = 6.5 Hz, 3H), 0.85 (d, J = 6.9 Hz, 3H). |
| 95 | (2S,4R)-4-hydroxy-1-((R)-2-(3-(6-((S)-3-(3-(2-hydroxyphenyl)-5-methylthieno[2,3-c]pyridazin-6-yl)pyrrolidin-1-yl)-2-methylpyridin-3-yl)isoxazol-5-yl)-3-methylbutanoyl)-N-((S)-1-(4-(4-methylthiazol-5-yl)phenyl)ethyl)pyrrolidine-2-carboxamide | 883.2 | 1H NMR (400 MHz, DMSO-d6) δ 12.73 (d, J = 6.5 Hz, 1H), 8.97 (d, J = 16.0 Hz, 1H), 8.72 (s, 1H), 8.25 (d, J = 7.8 Hz, 1H), 8.21 - 8.14 (m, 1H), 7.82 (s, 1H), 7.70 (d, J = 8.7 Hz, 1H), 7.52 - 7.37 (m, 4H), 7.04 (d, J = 7.9 Hz, 2H), 6.71 (d, J = 3.0 Hz, 1H), 6.52 (d, J = 9.2 Hz, 1H), 5.12 (d, J = 3.7 Hz, 2H), 4.88 (q, J = 7.2 Hz, 1H), 4.45 (t, J = 7.7 Hz, 1H), 4.32 - 4.18 (m, 2H), 4.05 (dd, J = 10.9, 7.1 Hz, 1H), 3.93 (d, J = 8.6 Hz, 1H), 3.66 - 3.49 (m, 4H), 2.57 - 2.50 (m, 5H), 2.47 (s, 1H), 2.40 (s, 4H), 2.36 (d, J = 8.0 Hz, 2H), 2.07 (td, J = 9.4, 4.5 Hz, 1H), 1.81 (dt, J = 12.5, 5.8 Hz, 1H), 1.34 (d, J = 7.0 Hz, 3H), 1.02 (d, J = 6.7 Hz, 2H), 0.88 (d, J = 6.7 Hz, 4H). |
| 96 | (2S,4R)-4-hydroxy-1-((R)-2-(3-(6-((R)-3-(3-(2-hydroxyphenyl)-5-methylthieno[2,3-c]pyridazin-6-yl)pyrrolidin-1-yl)-4-methylpyridin-3-yl)isoxazol-5-yl)-3-methylbutanoyl)-N-((S)-1-(4-(4-methylthiazol-5-yl)phenyl)ethyl)pyrrolidine-2-carboxamide | 883.25 | 1H NMR (400 MHz, DMSO-d6) δ 12.74 (s, 1H), 8.99 (s, 1H), 8.73 (s, 1H), 8.43 (d, J = 7.6 Hz, 1H), 8.36 - 8.28 (m, 1H), 8.18 (d, J = 8.1 Hz, 1H), 7.48 - 7.41 (m, 2H), 7.07 - 6.99 (m, 3H), 7.41 - 7.34 (m, 2H), 6.75 (s, 1H), 6.58 - 6.53 (m, 1H), 5.15 - 4.88 (m, 2H), 4.40 (t, J = 7.9 Hz, 1H), 4.33 - 4.19 (m, 2H), 4.05 (t, J = 8.9 Hz, 1H), 3.87 - 3.71 (m, 3H), 3.64 - 3.50 (m, 3H), 2.67 (s, 1H), 2.49 (s, 3H), 2.46 (s, 3H), 2.45 - 2.43 (m,3H), 2.40 - 2.32 (m, 1H), 2.22 - 2.17 (m, 1H), 2.06 - 2.01 (m, 1H), 1.84 - 1.75 (m, 1H), 1.43 (dd, J = 36.7, 6.9 Hz, 3H), 1.04 - 0.98 (m, 3H), 0.91 - 0.82 (m, 3H). |
| 97 | (2S,4R)-4-hydroxy-1-((R)-2-(3-(6-((S)-3-(3-(2-hydroxyphenyl)-5-methylthieno[2,3-c]pyridazin-6-yl)pyrrolidin-1-yl)-4-methylpyridin-3-yl)isoxazol-5-yl)-3-methylbutanoyl)-N-((S)-1-(4-(4-methylthiazol-5-yl)phenyl)ethyl)pyrrolidine-2-carboxamide | 883.45 | 1H NMR (400 MHz, DMSO-d6) δ 12.74 (s, 1H), 8.97 (d, J = 14.9 Hz, 1H), 8.72 (s, 1H), 8.39 - 8.23 (m, 2H), 8.17 (d, J = 8.0 Hz, 1H), 7.52 - 7.43 (m, 1H), 7.40 - 7.27 (m, 4H), 7.03 (d, J = 7.8 Hz, 2H), 6.76 (d, J = 1.7 Hz, 1H), 6.50 (s, 1H), 5.12 (d, J = 3.7 Hz, 1H), 4.89 (t, J = 7.3 Hz, 1H), 4.45 (t, J = 7.7 Hz, 1H), 4.25 (dd, J = 18.2, 10.6 Hz, 2H), 4.03 (dd, J = 10.6, 6.8 Hz, 1H), 3.93 (d, J = 8.6 Hz, 1H), 3.78 (s, 1H), 3.65 - 3.52 (m, 4H), 2.55 - 2.52 (m, 4H), 2.46 , 2.40 (s, 6H), 2.19 (q, J = 8.6 Hz, 1H), 2.06 (t, J = 9.6 Hz, 1H), 1.98 (s, 1H), 1.81 (dt, J = 12.6, 5.9 Hz, 1H), 1.34 (d, J = 6.9 Hz, 3H), 1.02 (d, J = 6.6 Hz, 3H), 0.89 (d, J = 6.7 Hz, 3H). |
| 101 | (2S,4R)-4-hydroxy-1-((R)-2-(3-(6-((R)-3-(3-(2-hydroxyphenyl)-5-methylthieno[2,3-c]pyridazin-6-yl)pyrrolidin-1-yl)-5-methylpyridin-3-yl)isoxazol-5-yl)-3-methylbutanoyl)-N-((S)-1-(4-(4-methylthiazol-5-yl)phenyl)ethyl)pyrrolidine-2-carboxamide | 883.4 | 1H NMR (400 MHz, DMSO-d6) δ 12.80 (d, J = 4.5 Hz, 1H), 8.99 (s, 1H), 8.72 (s, 1H), 8.50 - 8.42 (m, 2H), 8.18 (d, J = 7.9 Hz, 1H), 7.86 (d, J = 2.4 Hz, 1H), 7.48 - 7.38 (m, 2H), 7.41 - 7.34 (m, 3H), 7.04 (d, J = 7.8 Hz, 2H), 6.88 (s, 1H), 5.13 (d, J = 3.6 Hz, 1H), 4.93 (p, J = 6.9 Hz, 1H), 4.40 (t, J = 7.9 Hz, 1H), 4.31 (s, 1H), 4.14 (q, J = 7.3 Hz, 1H), 4.06 (dd, J = 10.4, 7.0 Hz, 1H), 3.90 - 3.72 (m, 5H), 3.52 (d, J = 10.5 Hz, 1H), 2.51 (d, J = 7.5 Hz, 4H), 2.45 (d, J = 9.7 Hz, 6H), 2.40 - 2.29 (m, 1H), 2.17 - 2.00 (m, 2H), 1.85 - 1.74 (m, 1H), 1.39 (d, J = 7.0 Hz, 3H), 1.01 (d, J = 6.3 Hz, 3H), 0.84 (d, J = 6.6 Hz, 3H). |
| 102 | (2S,4R)-4-hydroxy-1-((R)-2-(3-(6-((S)-3-(3-(2-hydroxyphenyl)-5-methylthieno[2,3-c]pyridazin-6-yl)pyrrolidin-1-yl)-5-methylpyridin-3-yl)isoxazol-5-yl)-3-methylbutanoyl)-N-((S)-1-(4-(4-methylthiazol-5-yl)phenyl)ethyl)pyrrolidine-2-carboxamide | 883.45 | 1H NMR (300 MHz, DMSO-d6) δ 12.79 (d, J = 3.4 Hz, 1H), 9.00 (s, 1H), 8.73 (s, 1H), 8.52 - 8.41 (m, 2H), 8.19 (dd, J = 8.3, 1.7 Hz, 1H), 7.89 (d, J = 5.7 Hz, 1H), 7.50 - 7.33 (m, 5H), 7.09 - 6.98 (m, 2H), 6.88 (s, 1H), 5.13 (d, J = 3.7 Hz, 1H), 4.94 (t, J = 7.2 Hz, 1H), 4.40 (t, J = 7.9 Hz, 1H), 4.31 (s, 1H), 4.21 - 4.01 (m, 2H), 3.91 - 3.68 (m, 5H), 3.52 (d, J = 10.4 Hz, 1H), 2.53 (s, 3H), 2.45 (d, J = 6.8 Hz, 6H), 2.39 - 2.18 (m, 2H), 2.19 - 1.98 (m, 2H), 1.87 - 1.73 (m, 1H), 1.45 (dd, J = 6.9, 7.0 Hz, 3H), 1.02 (d, J = 6.5 Hz, 3H), 0.84 (d, J = 6.8 Hz, 3H). |
| 90 | (2S,4R)-4-hydroxy-1-((R)-2-(3-(6-(4-(3-(2-hydroxyphenyl)-5-methylthieno[2,3-c]pyridazin-6-yl)piperidin-1-yl)-5-methoxypyridin-3-yl)isoxazol-5-yl)-3-methylbutanoyl)-N-((S)-1-(4-(4-methylthiazol-5-yl)phenyl)ethyl)pyrrolidine-2-carboxamide | 913.2 | 1H NMR (300 MHz, DMSO-d6) δ 12.79 (s, 1H), 8.99 (s, 1H), 8.71 (s, 1H), 8.44 (d, J = 7.7 Hz, 1H), 8.35 - 8.27 (m, 1H), 8.22 - 8.13 (m, 1H), 7.61 (d, J = 1.9 Hz, 1H), 7.45 (d, J = 8.2 Hz, 2H), 7.39 (s, 3H), 7.08 - 6.95 (m, 3H), 5.12 (d, J = 3.6 Hz, 1H), 5.08 - 4.88 (m, 1H), 4.40 (t, J = 7.8 Hz, 1H), 4.28 (d, J = 13.7 Hz, 3H), 3.92 (s, 3H), 3.85 (d, J = 9.7 Hz, 1H), 3.81 - 3.71 (m, 1H), 3.71 - 3.46 (m, 2H), 3.02 (t, J = 12.3 Hz, 2H), 2.48 - 2.44 (m, 3H), 2.42 - 2.24 (m, 3H), 2.08 (s, 1H), 2.03 (s, 3H), 1.91 - 1.73 (m, 3H), 1.45 (dd, J = 32.8, 7.0 Hz, 3H), 1.24 - 1.14 (m, 3H), 0.84 (d, J = 6.7 Hz, 3H). |

### Preparation of (2S,4R)-4-hydroxy-1-[(2R)-2-[3-(4-{4-[3-(2-hydroxyphenyl)-5-methylthieno[2,3-c]pyridazin-6-yl]piperidin-1-yl}-3-methylphenyl)-1,2-oxazol-5-yl]-3-methylbutanoyl]-N-[(1S)-1-[4-(4-methyl-1,3-thiazol-5-yl)phenyl]ethyl]pyrrolidine-2-carboxamide (Compound 3)

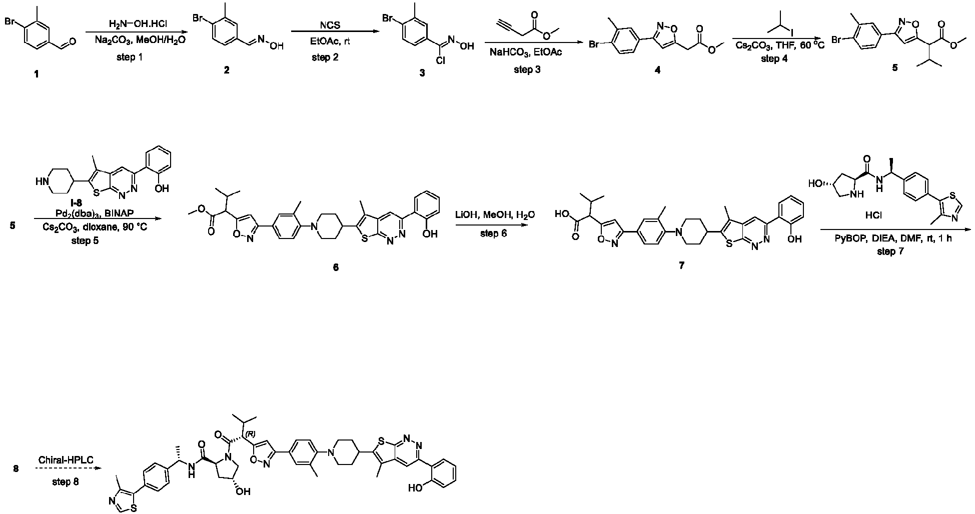

### Step 1: Preparation of ethyl (E)-N-[(4-bromo-3-methylphenyl)methylidene]hydroxylamine (Intermediate 2)

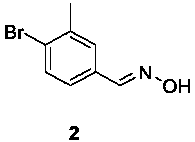

A solution of 4-bromo-3-methylbenzaldehyde (4 g, 20.096 mmol, 1 equiv) and hydroxylamine hydrochloride (2.79 g, 40.192 mmol, 2.0 equiv) in MeOH (20 mL) , H₂O (20 mL) was stirred for 4 h at room temperature. The resulting mixture was diluted with water (100 mL). The resulting mixture was extracted with CH₂Cl₂ (3 x 150 mL). The combined organic layers were washed with brine (2x100 mL), dried over anhydrous Na₂SO₄. After filtration, the filtrate was concentrated under reduced pressure. To afford Intermediate **2** (4.5 g, 104.61%) as a white solid. The crude product was used in the next step directly without further purification LCMS (ESI) m/z [M+H]⁺ =214.

### Step 2: Preparation of (Z)-4-bromo-N-hydroxy-3-methylbenzenecarbonimidoyl chloride (Intermediate 3)

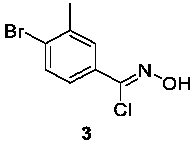

A solution of Intermediate **2** (4.4 g, 20.555 mmol, 1 equiv) and NCS (4.12 g, 30.832 mmol, 1.5 equiv) in EtOAc (40 mL) was stirred for 12 h at room temperature. The resulting mixture was diluted with water (300 mL). The resulting mixture was extracted with EtOAc (2 x 300 mL). The combined organic layers were washed with brine (1x100 mL), dried over anhydrous Na₂SO₄. After filtration, the filtrate was concentrated under reduced pressure. To afford Intermediate **3** (3.0 g, 58.73%) as a yellow solid. The crude product was used in the next step directly without further purification. LCMS (ESI) m/z [M+H]⁺ =248.

### Step 3: Preparation of methyl 2-[3-(4-bromo-3-methylphenyl)-1,2-oxazol-5-yl]acetate (Intermediate 4)

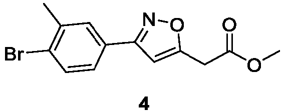

A solution of Intermediate 3 (3.0 g, 12.072 mmol, 1 equiv) and methyl but-3-ynoate (1.18 g, 12.072 mmol, 1.0 equiv) in EtOAc (2 mL) was stirred for 12 h at room temperature. The residue was purified by silica gel column chromatography, eluted with PE / EA (1:1) to afford Intermediate **4** (3.0 g, 80.12%) as a yellow solid. LCMS (ESI) m/z [M+H]⁺ =310.

### Step 4: Preparation of ethyl methyl 2-[3-(4-bromo-3-methylphenyl)-1,2-oxazol-5-yl]-3-methylbutanoate (Intermediate 5)

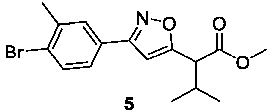

A solution of Intermediate **4** (3.0 g, 9.673 mmol, 1 equiv) and 2-iodopropane (3.29 g, 19.346 mmol, 2.0 equiv) in THF (20 mL) was stirred for 4 h at 60 °C. The resulting mixture was concentrated under reduced pressure. The residue was purified by silica gel column chromatography, eluted with PE / EA (7:3) to afford Intermediate **5** (1.7 g, 49.90%) as a yellow oil. LCMS (ESI) m/z [M+H]⁺ =352.

### Step 5: Preparation of methyl 2-[3-(4-{4-[3-(2-hydroxyphenyl)-5-methylthieno[2,3-c]pyridazin-6-yl]piperidin-1-yl}-3-methylphenyl)-1,2-oxazol-5-yl]-3-methylbutanoate (Intermediate 6)

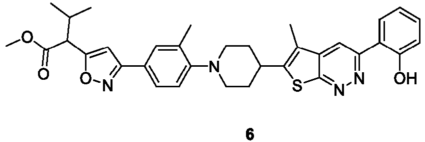

To a solution of Intermediate **5** (200 mg, 0.568 mmol, 1 equiv) and 2-[5-methyl-6-(piperidin-4-yl)thieno[2,3-c]pyridazin-3-yl]phenol (221.74 mg, 0.682 mmol, 1.2 equiv) in dioxane (1 mL) were added Cs₂CO₃ (555.01 mg, 1.704 mmol, 3.0 equiv), Pd₂(dba)₃ (311.98 mg, 0.341 mmol, 0.6 equiv) and BINAP (106.07 mg, 0.170 mmol, 0.3 equiv). After stirring for 6 h at 100 °C under a nitrogen atmosphere. the resulting mixture was concentrated under reduced pressure. The residue was purified by silica gel column chromatography, eluted with PE / EA (7:3) to afford Intermediate **6** (80 mg, 23.61%) as a yellow solid. LCMS (ESI) m/z [M+H]⁺ =597.

### Step 6: Preparation of 2-[3-(4-{4-[3-(2-hydroxyphenyl)-5-methylthieno[2,3-c]pyridazin-6-yl]piperidin-1-yl}-3-methylphenyl)-1,2-oxazol-5-yl]-3-methylbutanoic acid (Intermediate 7)

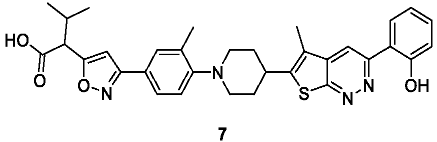

A solution of Intermediate **6** (80 mg, 0.134 mmol, 1 equiv) and LiOH (64.21 mg, 2.680 mmol, 20 equiv) in MeOH (4 mL) was stirred for 1 h at 60 °C. The residue was purified by reverse flash chromatography with the following conditions: column, C18 silica gel; mobile phase, MeCN in Water (0.1% FA), 0% to 60% gradient in 40 min; detector, UV 254 nm. This resulted in Intermediate 7 (45 mg, 57.60%) as a white solid. LCMS (ESI) m/z [M+H]⁺ =583.

### Step 7: Preparation of tert-butyl (2S,4R)-4-hydroxy-1-{2-[3-(4-{4-[3-(2-hydroxyphenyl)-5-methylthieno[2,3-c]pyridazin-6-yl]piperidin-1-yl}-3-methylphenyl)-1,2-oxazol-5-yl]-3-methylbutanoyl}-N-[(1S)-1-[4-(4-methyl-1,3-thiazol-5-yl)phenyl]ethyl]pyrrolidine-2-carboxamide (Intermediate 8)

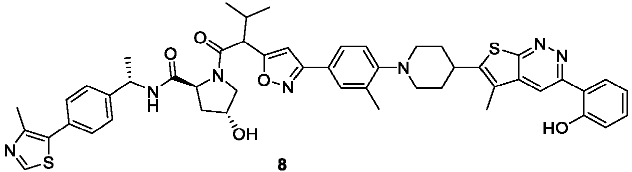

A solution of Intermediate **7** (45 mg, 0.077 mmol, 1 equiv) and (2S,4R)-4-hydroxy-N-[(1S)-1-[4-(4-methyl-1,3-thiazol-5-yl)phenyl]ethyl]pyrrolidine-2-carboxamide (30.71 mg, 0.092 mmol, 1.2 equiv), PyBOP (80.37 mg, 0.154 mmol, 2.0 equiv), DIEA (29.94 mg, 0.231 mmol, 3.0 equiv) in DMF (1 mL) was stirred for 1 h at room temperature. The residue was purified by reverse flash chromatography with the following conditions: column, C18 silica gel; mobile phase, MeCN in Water (0.1% FA), 0% to 80% gradient in 40 min; detector, UV 254 nm. This resulted in Intermediate **8** (34 mg, 49.13%) as a white solid. LCMS (ESI) m/z [M+H]⁺ =896.

### Step 8: Preparation of (2S,4R)-4-hydroxy-1-[(2R)-2-[3-(4-{4-[3-(2-hydroxyphenyl)-5-methylthieno[2,3-c]pyridazin-6-yl]piperidin-1-yl}-3-methylphenyl)-1,2-oxazol-5-yl]-3-methylbutanoyl]-N-[(1S)-1-[4-(4-methyl-1,3-thiazol-5-yl)phenyl]ethyl]pyrrolidine-2-carboxamide

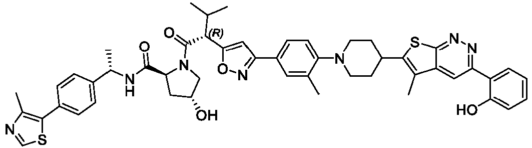

The Intermediate **8** (34 mg) was purified by Chiral-Prep-HPLC with the following conditions (Column: CHIRALPAK ID-3, 4.6*50mm, 3um; Mobile Phase A: MtBE(0.1%DEA): MeOH=70: 30; Flow rate: 1 mL/min; Gradient: 0% B to 0% B; Injection Volume: 5ul mL) to afford title compound (second peak) (11.3 mg, 33.24%) as a white solid. ¹H NMR (400 MHz, DMSO-d6) *δ* 12.81 (d, J = 3.3 Hz, 1H), 8.99 (s, 1H), 8.72 (s, 1H), 8.44 (d, J = 7.6 Hz, 1H), 8.22 - 8.15 (m, 1H), 7.73 - 7.64 (m, 2H), 7.48 - 7.42 (m, 2H), 7.39 (t, J = 6.8 Hz, 3H), 7.16 (dd, J = 8.5, 3.9 Hz, 1H), 7.07 - 6.99 (m, 2H), 6.86 (s, 1H), 5.11 (d, J = 3.7 Hz, 1H), 4.93 (p, J = 7.2 Hz, 1H), 4.40 (t, J = 7.9 Hz, 1H), 4.30 (brs, 1H), 3.84 (d, J = 9.7 Hz, 1H), 3.76 (dd, J = 10.7, 4.3 Hz, 1H), 3.68 - 3.38 (m, 3H), 3.33 - 3.32 (m, 3H), 2.89 (t, J = 11.6 Hz, 2H), 2.63 - 2.53 (m, 1H), 2.45 (s, 3H), 2.37 (s, 3H), 2.18 - 1.72 (m, 7H), 1.39 (d, J = 7.0 Hz, 3H), 1.05 - 0.98 (m, 3H), 0.85 (dd, J = 11.6, 6.6 Hz, 3H). LCMS (ESI) m/z [M+H]⁺ =896.25.

The compound in Table 4 was prepared using a procedure similar to the one used above for the preparation of (2S,4R)-4-hydroxy-1-[(2R)-2-[3-(4-{4-[3-(2-hydroxyphenyl)-5-methylthieno[2,3-c]pyridazin-6-yl]piperidin-1-yl}-3-methylphenyl)-1,2-oxazol-5-yl]-3-methylbutanoyl]-N-[(1S)-1-[4-(4-methyl-1,3-thiazol-5-yl)phenyl]ethyl]pyrrolidine-2-carboxamide (Compound 3) using the appropriate aryl bromide and 2-(5-methyl-6-(piperidin-4-yl)thieno[2,3-c]pyridazin-3-yl)phenol.

**Table 4.**

| **No.** | **Name** | **LCMS (ESI) m/z** | **¹H NMR** |
|---|---|---|---|
| 4 | (2S,4R)-1-((R)-2-(3-(2-fluoro-4-(4-(3-(2-hydroxyphenyl)-5-methylthieno[2,3-c]pyridazin-6-yl)piperidin-1-yl)phenyl)isoxazol-5-yl)-3-methylbutanoyl)-4-hydroxy-N-((S)-1-(4-(4-methylthiazol-5-yl)phenyl)ethyl)pyrrolidine-2-carboxamide | 900.45 | 1H NMR (400 MHz, DMSO-d6) δ 12.78 (s, 1H), 8.99 (s, 1H), 8.72 (s, 1H), 8.45 (d, J = 7.6 Hz, 1H), 8.21 - 8.14 (m, 1H), 7.74 (t, J = 8.8 Hz, 1H), 7.48 - 7.42 (m, 2H), 7.42 - 7.34 (m, 3H), 7.06 - 7.00 (m, 2H), 6.99 - 6.92 (m, 2H), 6.68 (d, J = 3.2 Hz, 1H), 5.11 (d, J = 3.7 Hz, 1H), 4.94 (q, J = 7.1 Hz, 1H), 4.40 (t, J = 7.9 Hz, 1H), 4.30 (s, 1H), 4.07 (d, J = 12.7 Hz, 2H), 3.87 (d, J = 9.7 Hz, 1H), 3.79 - 3.70 (m, 1H), 3.61 - 3.48 (m, 2H), 3.06 (t, J = 12.2 Hz, 2H), 2.49 (s, 3H), 2.48 - 2.42 (m, 3H), 2.39 - 2.27 (m, 1H), 2.10 - 2.02 (m, 3H), 1.85 - 1.70 (m, 3H), 1.44 (dd, J = 37.9, 7.0 Hz, 3H), 1.05 - 0.98 (m, 3H), 0.84 (dd, J = 12.6, 6.6 Hz, 3H). |
| 26 | (2S,4R)-4-hydroxy-1-((R)-2-(3-(4-(4-(3-(2-hydroxyphenyl)-5-methylthieno[2,3-c]pyridazin-6-yl)piperidin-1-yl)phenyl)isoxazol-5-yl)-3-methylbutanoyl)-N-((S)-1-(4-(4-methylthiazol-5-yl)phenyl)ethyl)pyrrolidine-2-carboxamide | 882.25 | 1H NMR (300 MHz, DMSO-d6) δ 12.78 (d, J = 3.1 Hz, 1H), 8.99 - 8.72 (m, 2H), 8.42 - 8.18 (m, 2H), 7.73 (d, J = 8.6 Hz, 2H), 7.45 - 7.38 (m, 5H), 7.11 - 6.98 (m, 4H), 6.81 (s, 1H), 5.10 (d, J = 3.7 Hz, 1H), 5.06 - 4.89 (m, 1H), 4.41 (t, J = 7.8 Hz, 1H), 4.31 (s, 1H), 4.03 (d, J = 12.6 Hz, 2H), 3.79 (dd, J = 15.0, 8.0 Hz, 2H), 3.70 - 3.46 (m, 2H), 3.01 (t, J = 12.2 Hz, 2H), 2.46 - 2.36 (m, 6H), 2.35 - 2.31 (m, 1H), 2.25 - 2.01 (m, 3H),1.81 (d, J = 13.2 Hz, 3H), 1.50 (d, J = 6.9 Hz, 3H), 1.02 (d, J = 6.5 Hz, 3H), 0.85 (d, J = 6.9 Hz, 3H). |
| 34 | (2S,4R)-4-hydroxy-1-((R)-2-(3-(5-(4-(3-(2-hydroxyphenyl)-5-methylthieno[2,3-c]pyridazin-6-yl)piperidin-1-yl)pyridin-2-yl)isoxazol-5-yl)-3-methylbutanoyl)-N-((S)-1-(4-(4-methylthiazol-5-yl)phenyl)ethyl)pyrrolidine-2-carboxamide | 883.1 | 1H NMR (400 MHz, DMSO-d6) δ 12.76 (s, 1H), 8.98 (d, J = 2.3 Hz, 1H), 8.71 (d, J = 2.4 Hz, 1H), 8.58 - 8.30 (m, 2H), 8.17 (d, J = 7.9 Hz, 1H), 7.83 (dd, J = 8.7, 2.5 Hz, 1H), 7.78 - 7.27 (m, 6H), 7.24 - 6.98 (m, 3H), 6.79 (d, J = 2.4 Hz, 1H), 5.09 (s, 1H), 4.97 - 4.89 (m, 1H), 4.39 (t, J = 8.1 Hz, 1H), 4.29 (s, 1H), 4.09 (d, J = 12.6 Hz, 2H), 3.87 (d, J = 9.9 Hz, 1H), 3.75 (d, J = 10.2 Hz, 1H), 3.52 -3.21(m, 3H), 3.06 (t, J = 12.3 Hz, 2H), 2.45 (dd, J = 7.6, 2.5 Hz, 4H), 2.38 - 1.97 (m, 3H), 1.95 - 1.69 (m, 3H), 1.65-1.45(m, 3H), 1.39 (d, J = 7.0, 2.4 Hz, 3H), 1.01 (d, J = 6.0 Hz, 3H), 0.85 (ddd, J = 15.0, 6.7, 2.5 Hz, 3H). |
| 36 | (2S,4R)-4-hydroxy-1-((R)-2-(3-(5-(4-(3-(2-hydroxyphenyl)-5-methylthieno[2,3-c]pyridazin-6-yl)piperidin-1-yl)pyrimidin-2-yl)isoxazol-5-yl)-3-methylbutanoyl)-N-((S)-1-(4-(4-methylthiazol-5-yl)phenyl)ethyl)pyrrolidine-2-carboxamide | 884.35 | 1H NMR (400 MHz, DMSO-d6) δ 12.77 (d, J = 4.1 Hz, 1H), 8.99 (s, 1H), 8.70 (d, J = 13.1 Hz, 3H), 8.44 (d, J = 7.6 Hz, 1H), 8.20 - 8.14 (m, 1H), 7.48 - 7.38 (m, 2H), 7.42 - 7.33 (m, 3H), 7.03 (d, J = 7.7 Hz, 2H), 6.82 (s, 1H), 5.10 (d, J = 3.7 Hz, 1H), 4.97 - 4.89 (m, 1H), 4.40 (t, J = 7.9 Hz, 1H), 4.30 (s, 1H), 4.18 (d, J = 12.7 Hz, 2H), 3.90 (d, J = 9.7 Hz, 1H), 3.79 - 3.65 (m, 1H), 3.64 - 3.57 (m, 1H), 3.52 (d, J = 10.8 Hz, 1H), 3.11 (t, J = 12.3 Hz, 2H), 2.50 - 2.42 (m, 6H), 2.36 (s, 1H), 2.14 - 1.99 (m, 3H), 1.86 - 1.76 (m, 3H), 1.44 (d, J = 7.0 Hz, 3H), 1.02 (d, J = 6.7 Hz, 3H), 0.85 (d, J = 6.7 Hz, 3H). |
| 103 | (2S,4R)-1-((R)-2-(3-(3-fluoro-4-(4-(3-(2-hydroxyphenyl)-5-methylthieno[2,3-c]pyridazin-6-yl)piperidin-1-yl)phenyl)isoxazol-5-yl)-3-methylbutanoyl)-4-hydroxy-N-((S)-1-(4-(4-methylthiazol-5-yl)phenyl)ethyl)pyrrolidine-2-carboxamide | 900.15 | 1H NMR (400 MHz, DMSO-d6) δ 12.78 (s, 1H), 8.99 (s, 1H), 8.72 (s, 1H), 8.42 (d, J = 7.7 Hz, 1H), 8.21 - 8.14 (m, 1H), 7.70 - 7.60 (m, 2H), 7.48 - 7.43 (m, 2H), 7.42 - 7.34 (m, 3H), 7.21 (t, J = 8.6 Hz, 1H), 7.07 - 6.99 (m, 2H), 6.93 (s, 1H), 5.10 (d, J = 3.7 Hz, 1H), 4.99 - 4.88 (m, 1H), 4.40 (t, J = 7.9 Hz, 1H), 4.31 (s, 1H), 3.84 (d, J = 9.7 Hz, 1H), 3.80 - 3.72 (m, 1H), 3.68 - 3.59 (m, 2H), 3.55 - 3.44 (m, 2H), 2.99 (t, J = 11.8 Hz, 2H), 2.55 - 2.51 (m, 3H), 2.48 - 2.43 (m, 3H), 2.41 - 2.25 (m, 1H), 2.15 - 1.99 (m, 3H), 1.96 - 1.86 (m, 2H), 1.86 - 1.75 (m, 1H), 1.52 - 1.36 (m, 3H), 1.05 - 0.98 (m, 3H), 0.86 - 0.81 (m, 3H). |

### Preparation of (2S,4R)-4-hydroxy- 1-[(2R)-2-(3-{2-[(3R)-3-[3-(2-hydroxyphenyl)thieno[2,3-c]pyridazin-6-yl]pyrrolidin-1-yl]pyrimidin-5-yl}-1,2-oxazol-5-yl)-3-methylbutanoyl]N-[(1S)-1-[4-(4-methyl-1,3-thiazol-5-yl)phenyl]ethyl]pyrrolidine-2-carboxamide (Compound 5)

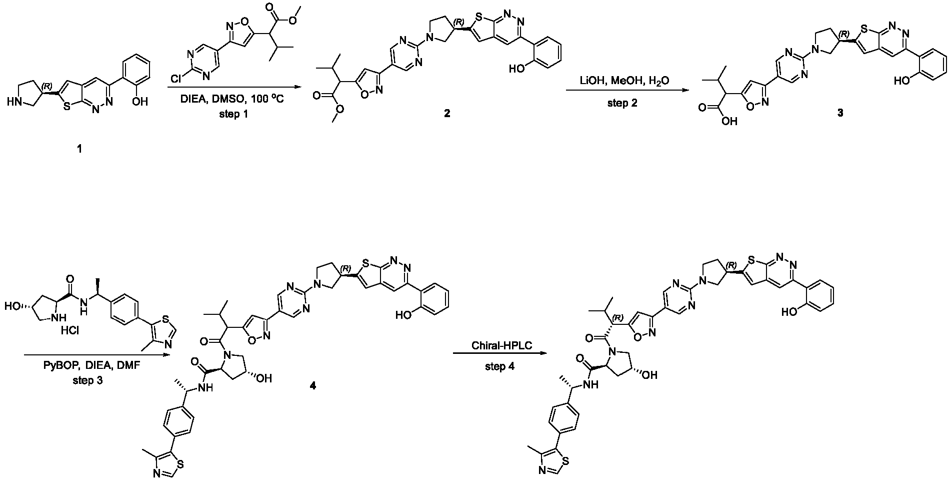

### Step 1: Preparation of methyl 2-(3-{2-[(3R)-3-[3-(2-hydroxyphenyl)thieno[2,3-c]pyridazin-6-yl]pyrrolidin-1-yl]pyrimidin-5-yl}-1,2-oxazol-5-yl)-3-methylbutanoate (Intermediate 2)

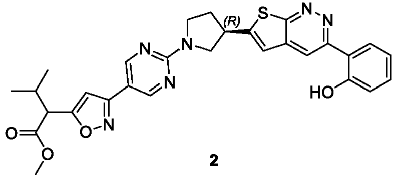

A mixture of 2-{6-[(3S)-pyrrolidin-3-yl]thieno[2,3-c]pyridazin-3-yl}phenol (400 mg, 1.345 mmol, 1 equiv), methyl 2-[3-(2-chloropyrimidin-5-yl)-1,2-oxazol-5-yl]-3-methylbutanoate (397.77 mg, 1.345 mmol, 1 equiv) and DIEA (869.24 mg, 6.725 mmol, 5 equiv) in DMSO (4 mL) was stirred for 3 h at 100 °C under nitrogen atmosphere. Desired product could be detected by LCMS. The residue was purified by reverse flash chromatography with the following conditions: column, C18 silica gel; mobile phase, MeOH in Water (0.1% FA), 0% to 100% gradient in 30 min; detector, UV 254 nm to afford Intermediate 2 (130 mg, 17.36%) as a yellow solid. LCMS (ESI) m/z: [M+H]⁺ = 557.

### Step 2: Preparation of 2-(3-{2-[(3R)-3-[3-(2-hydroxyphenyl)thieno[2,3-c]pyridazin-6-yl]pyrrolidin-1-yl]pyrimidin-5-yl}-1,2-oxazol-5-yl)-3-methylbutanoic acid (Intermediate 3)

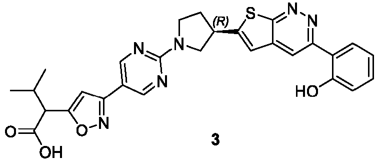

A mixture of Intermediate 2 (130 mg, 0.234 mmol, 1 equiv) and LiOH (16.78 mg, 0.702 mmol, 3 equiv) in MeOH (2 mL), THF (2 mL) and H₂O (1 mL) was stirred for 2 h at room temperature. Desired product could be detected by LCMS. The mixture was acidified to pH 6 with conc. HCl. The resulting mixture was concentrated under reduced pressure to afford Intermediate 3 (142 mg, crude) as a yellow solid. LCMS (ESI) m/z [M+H]⁺ = 543.

### Step 3: Preparation of (2S,4R)-4-hydroxy-1-[2-(3-{2-[(3R)-3-[3-(2-hydroxyphenyl)thieno[2,3-c]pyridazin-6-yl]pyrrolidin-1-yl]pyrimidin-5-yl}-1,2-oxazol-5-yl)-3-methylbutanoyl]-N-[(1S)-1-[4-(4-methyl-1,3-thiazol-5-yl)phenyl]ethyl]pyrrolidine-2-carboxamide (Intermediate 4)

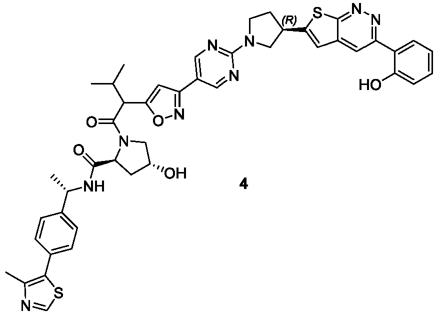

A mixture of Intermediate 3 (142 mg, 0.262 mmol, 1 equiv), (2S,4R)-4-hydroxy-N-[(1S)-1-[4-(4-methyl-1,3-thiazol-5-yl)phenyl]ethyl]pyrrolidine-2-carboxamide (86.73 mg, 0.262 mmol, 1 equiv), PyBOP (204.28 mg, 0.393 mmol, 1.5 equiv) and DIEA (169.12 mg, 1.310 mmol, 5 equiv) in DMF (3 mL) was stirred for 2 h at room temperature. Desired product could be detected by LCMS. The residue was purified by reverse flash chromatography with the following conditions: column, C18 silica gel; mobile phase, MeCN in Water (10mmol/L NH₄HCO₃), 0% to 100% gradient in 30 min; detector, UV 254 nm to afford Intermediate 4 (100 mg, 44.64%) as a yellow solid. LCMS (ESI) m/z: [M+H]⁺ = 856.

### Step 4: Preparation of (2S,4R)-4-hydroxy-1-[(2R)-2-(3-{2-[(3R)-3-[3-(2-hydroxyphenyl)thieno[2,3-c]pyridazin-6-yl]pyrrolidin-1-yl]pyrimidin-5-yl}-1,2-oxazol-5-yl)-3-methylbutanoyl]-N-[(1S)-1-[4-(4-methyl-1,3-thiazol-5-yl)phenyl]ethyl]pyrrolidine-2-carboxamide

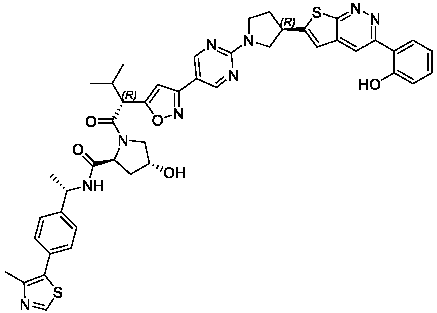

The product Intermediate 4 was purified by Prep-SFC with the following conditions (Column: CHIRAL ART Cellulose-SB, 3*25 cm, 5 µm; Mobile Phase A: CO2, Mobile Phase B: IPA: DCM=1: 1--HPLC; Flow rate: 90 mL/min; Gradient: isocratic 60% B; Column Temperature(°C): 35; Back Pressure(bar): 100; Wave Length: 277 nm; RT1(min): 3.33; RT2(min): 4.28; Sample Solvent: MeOH: DCM=1: 1--HPLC; Injection Volume: 1 mL) to afford title compound (second peak) (36.9 mg, 35.46%) as a white solid. ¹H NMR (400 MHz, DMSO-d6) δ 12.30 - 12.25 (m, 1H), 9.01 - 8.96 (m, 1H), 8.89 - 8.79 (m, 2H), 8.75 (s, 1H), 8.44 (d, J = 7.7 Hz, 1H), 8.01 (d, J = 7.9, 1.7 Hz, 1H), 7.50 (s, 1H), 7.47 - 7.42 (m, 2H), 7.40 - 7.34 (m, 3H), 7.07 - 6.92 (m, 3H), 5.11 (d, J = 3.6 Hz, 1H), 4.94 (q, J = 7.2, 6.5 Hz, 1H), 4.39 (t, J = 7.9 Hz, 1H), 4.30 (s, 1H), 4.24 - 4.07 (m, 2H), 3.93 - 3.59 (m, 5H), 3.51 (d, J = 10.7 Hz, 1H), 2.65 - 2.56 (m, 1H), 2.48 - 2.42 (m, 3H), 2.38 - 2.23 (m, 2H), 2.10 - 1.99 (m, 1H), 1.85 - 1.69 (m, 1H), 1.44 (dd, J = 40.6, 7.0 Hz, 3H), 1.05 - 0.98 (m, 3H), 0.89 - 0.81 (m, 3H). LCMS (ESI) m/z: [M+H]⁺ = 856.40.

The compound in Table 5 was prepared using a procedure similar to the one used above for the preparation of (2S,4R)-4-hydroxy-1-[(2R)-2-(3-{2-[(3R)-3-[3-(2-hydroxyphenyl)thieno[2,3-c]pyridazin-6-yl]pyrrolidin-1-yl]pyrimidin-5-yl}-1,2-oxazol-5-yl)-3-methylbutanoyl]-N-[(1S)-1-[4-(4-methyl-1,3-thiazol-5-yl)phenyl]ethyl]pyrrolidine-2-carboxamide (Compound 5) using the appropriate aryl chlorides and amines.

**Table 5.**

| **No.** | **Name** | **LCMS (ESI) m/z** | **¹H NMR** |
|---|---|---|---|
| 6 | (2S,4R)-4-hydroxy-1-((R)-2-(3-(2-((S)-3-(3-(2-hydroxyphenyl)thieno[2,3-c]pyridazin-6-yl)pyrrolidin-1-yl)pyrimidin-5-yl)isoxazol-5-yl)-3-methylbutanoyl)-N-((S)-1-(4-(4-methylthiazol-5-yl)phenyl)ethyl)pyrrolidine-2-carboxamide | 856.4 | 1H NMR (400 MHz, DMSO-d6) δ 12.30 - 12.25 (m, 1H), 9.01 - 8.96 (m, 1H), 8.88 - 8.78 (m, 2H), 8.74 (s, 1H), 8.42 (d, J = 7.7 Hz, 1H), 8.01 (dd, J = 7.9, 1.7 Hz, 1H), 7.50 (s, 1H), 7.48 - 7.41 (m, 2H), 7.41 - 7.32 (m, 3H), 7.07 - 6.89 (m, 3H), 5.10 (d, J = 3.7 Hz, 1H), 4.97 - 4.89 (m, 1H), 4.39 (t, J = 7.9 Hz, 1H), 4.30 (s, 1H), 4.24 - 4.16 (m, 1H), 4.11 (p, J = 7.1 Hz, 1H), 3.90 - 3.58 (m, 5H), 3.50 (d, J = 10.7 Hz, 1H), 2.65 - 2.56 (m, 1H), 2.48 - 2.42 (m, 3H), 2.32 - 2.23 (m, 2H), 2.08 - 1.99 (m, 1H), 1.86 - 1.69 (m, 1H), 1.44 (dd, J = 39.7, 7.0 Hz, 3H), 1.05 - 0.98 (m, 3H), 0.89 - 0.81 (m, 3H). |
| 7 | (2S,4R)-4-hydroxy-1-((R)-2-(3-(2-((R)-3-(3-(2-hydroxyphenyl)-5-methylthieno[2,3-c]pyridazin-6-yl)pyrrolidin-1-yl)pyrimidin-4-yl)isoxazol-5-yl)-3-methylbutanoyl)-N-((S)-1-(4-(4-methylthiazol-5-yl)phenyl)ethyl)pyrrolidine-2-carboxamide | 870.2 | 1H NMR (400 MHz, DMSO-d6) δ 12.35 - 12.28 (m, 1H), 9.01 - 8.96 (m, 1H), 8.67 - 8.49 (m, 2H), 8.40 (s, 1H), 7.98 (s, 1H), 7.48 - 7.26 (m, 5H), 7.24 - 7.16 (m, 1H), 7.04 - 6.86 (m, 3H), 5.07 (s, 1H), 4.89 (s, 1H), 4.43 - 3.79 (m, 7H), 3.68 (s, 2H), 3.58 - 3.42 (m, 1H), 2.76 - 2.71 (m, 3H), 2.47 - 2.41 (m, 3H), 2.35 - 2.31 (m, 3H), 2.11 - 1.90 (m, 1H), 1.77 (s, 1H), 1.35 (s, 3H), 1.23 (s, 3H), 0.98 (s, 3H), 0.89 - 0.70 (m, 3H). |
| 8 | (2S,4R)-4-hydroxy-1 -((R)-2-(3-(2-((S)-3-(3-(2-hydroxyphenyl)-5-methylthieno[2,3-c]pyridazin-6-yl)pyrrolidin-1-yl)pyrimidin-4-yl)isoxazol-5-yl)-3-methylbutanoyl)-N-((S)-1-(4-(4-methylthiazol-5-yl)phenyl)ethyl)pyrrolidine-2-carboxamide | 870.2 | 1H NMR (400 MHz, DMSO-d6) δ 12.74 (s, 1H), 9.02 - 8.81 (m, 1H), 8.76 - 8.69 (m, 1H), 8.59 - 8.53 (m, 1H), 8.42 (s, 1H), 8.21 - 8.14 (m, 1H), 7.46 - 7.33 (m, 5H), 7.21 (d, J = 5.0 Hz, 1H), 7.07 - 6.98 (m, 2H), 6.93 (s, 1H), 5.17 - 4.68 (m, 2H), 4.45 - 4.09 (m, 4H), 3.94 (s, 2H), 3.80 - 3.61 (m, 3H), 3.51 (s, 1H), 2.63 - 2.52 (m, 4H), 2.45 (s, 3H), 2.36 - 2.15 (m, 2H), 2.10 - 1.69 (m, 2H), 1.54 - 1.30 (m, 3H), 1.00 (s, 3H), 0.82 (s, 3H). |
| 11 | (2S,4R)-4-hydroxy-1-((R)-2-(3-(2-(4-(3-(2-hydroxyphenyl)-5-methylthieno[2,3-c]pyridazin-6-yl)piperidin-1-yl)-4,6-dimethylpyrimidin-5-yl)isoxazol-5-yl)-3-methylbutanoyl)-N-((S)-1-(4-(4-methylthiazol-5-yl)phenyl)ethyl)pyrrolidine-2-carboxamide | 913.45 | 1H NMR (300 MHz, Methanol-d4) δ 8.89 (d, J = 1.8 Hz, 1H), 8.57 (s, 1H), 8.09 (dd, J = 8.4, 1.6 Hz, 1H), 7.53 - 7.31 (m, 5H), 7.10 - 6.99 (m, 2H), 6.49 - 6.31 (m, 1H), 5.22 - 5.01 (m, 3H), 4.64 - 4.39 (m, 2H), 4.02 - 3.58 (m, 4H), 3.12 (t, J = 12.3 Hz, 2H), 2.57 (s, 3H), 2.48 (d, J = 11.8 Hz, 4H), 2.26 (d, J = 10.4 Hz, 6H), 2.21 - 1.92 (m, 4H), 1.91 - 1.70 (m, 2H), 1.64 (dd, J = 7.1 Hz, J = 7.0 Hz, 3H), 1.14 (d, J = 6.6 Hz, 3H), 0.99 - 0.90 (m, 3H). |
| 13 | (2S,4R)-4-hydroxy-1-((R)-2-(3-(2-((1R,4S,5R)-5-(3-(2-hydroxyphenyl)thieno[2,3-c]pyridazin-6-yl)-2-azabicyclo[2.2.1]heptan-2-yl)pyrimidin-5-yl)isoxazol-5-yl)-3-methylbutanoyl)-N-((S)-1-(4-(4-methylthiazol-5-yl)phenyl)ethyl)pyrrolidine-2-carboxamide | 882.4 | 1H NMR (400 MHz, DMSO-d6) δ 12.29 (s, 1H), 8.98 (d, J = 3.5 Hz, 1H), 8.82 (s, 1H), 8.72 (s, 1H), 8.66 (s, 1H), 8.42 (d, J = 7.7 Hz, 1H), 7.96 (dd, J = 8.0, 1.7 Hz, 1H), 7.44 (d, J = 8.1 Hz, 2H), 7.40 - 7.32 (m, 3H), 7.29 (d, J = 1.4 Hz, 1H), 7.05 - 6.95 (m, 2H), 6.83 (d, J = 43.2 Hz, 1H), 5.32 (t, J = 4.8 Hz, 1H), 5.10 (s, 1H), 4.93 (q, J = 7.0 Hz, 1H), 4.77 (d, J = 45.9 Hz, 1H), 4.38 (t, J = 7.8 Hz, 1H), 4.30 (s, 1H), 4.01 - 3.91 (m, 1H), 3.83 (d, J = 9.8 Hz, 1H), 3.79 - 3.56 (m, 1H), 3.52 - 3.46 (m, 1H), 3.40 (d, J = 10.3 Hz, 1H), 3.10 (s, 1H), 2.45 (d, J = 9.2 Hz, 3H), 2.39 (d, J = 11.6 Hz, 1H), 2.16 (t, J = 7.4 Hz, 1H), 2.06 - 1.94 (m, 4H), 1.84 - 1.74 (m, 1H), 1.43 (dd, J = 39.3, 7.0 Hz, 3H), 1.01 (t, J = 5.6 Hz, 3H), 0.83 (dd, J = 8.6, 6.8 Hz, 3H). |
| 15 | (2S,4R)-4-hydroxy-1-((R)-2-(3-(2-((1R,5S,6S)-6-(3-(2-hydroxyphenyl)thieno[2,3-c]pyridazin-6-yl)-3-azabicyclo[3.1.1]heptan-3-yl)pyrimidin-5-yl)isoxazol-5-yl)-3-methylbutanoyl)-N-((S)-1-(4-(4-methylthiazol-5-yl)phenyl)ethyl)pyrrolidine-2-carboxamide | 882.3 | 1H NMR (400 MHz, DMSO-d6) δ 12.25 (s, 1H), 9.00 - 8.94 (m, 1H), 8.82 - 8.74 (m, 2H), 8.66 (s, 1H), 8.40 (d, J = 7.7 Hz, 1H), 7.98 - 7.92 (m, 1H), 7.47 - 7.29 (m, 6H), 7.03 - 6.93 (m, 2H), 6.85 (s, 1H), 5.37 - 4.84 (m, 2H), 4.36 (t, J = 7.9 Hz, 1H), 4.28 (s, 1H), 4.11 - 4.03 (m, 2H), 3.99 (t, J = 5.9 Hz, 1H), 3.84 - 3.77 (m, 3H), 3.76 - 3.70 (m, 1H), 3.52 - 3.44 (m, 1H), 3.13 - 3.06 (m, 2H), 2.48 - 2.40 (m, 3H), 2.05 - 1.88 (m, 3H), 1.83 - 1.72 (m, 1H), 1.62 - 1.55 (m, 1H), 1.41 (dd, J = 31.2, 7.0 Hz, 3H), 1.02 - 0.95 (m, 3H), 0.89 - 0.76 (m, 3H). |
| 17 | (2S,4R)-4-hydroxy-1-((R)-2-(3-(2-(6-(3-(2-hydroxyphenyl)thieno[3,2-c]pyridazin-6-yl)-2-azaspiro[3.3]heptan-2-yl)pyrimidin-5-yl)isoxazol-5-yl)-3-methylbutanoyl)-N-((S)-1-(4-(4-methylthiazol-5-yl)phenyl)ethyl)pyrrolidine-2-carboxamide | 882.3 | 1H NMR (400 MHz, DMSO-d6) δ 9.14 (s, 1H), 8.99 (s, 1H), 8.80 (d, J = 3.0 Hz, 2H), 8.43 (d, J = 7.7 Hz, 1H), 8.03 (dd, J = 8.1, 1.7 Hz, 1H), 7.74 (s, 1H), 7.48 - 7.41 (m, 2H), 7.40 - 7.31 (m, 3H), 7.02 (dd, J = 11.6, 7.8 Hz, 2H), 6.86 (d, J = 45.6 Hz, 1H), 5.07 (dd, J = 28.6, 3.3 Hz, 1H), 4.93 (p, J = 7.3 Hz, 1H), 4.38 (t, J = 7.9 Hz, 1H), 4.30 (s, 3H), 4.13 (s, 2H), 3.98 - 3.81 (m, 2H), 3.75 (dd, J = 10.7, 4.4 Hz, 1H), 3.50 (d, J = 10.4 Hz, 1H), 2.89 - 2.78 (m, 2H), 2.63 - 2.54 (m, 2H), 2.45 (d, J = 6.5 Hz, 3H), 2.30 (d, J = 6.9 Hz, 1H), 2.03 (t, J = 10.5 Hz, 1H), 1.79 (ddd, J = 12.8, 8.1, 4.7 Hz, 1H), 1.44 (dd, J = 39.0, 6.9 Hz, 3H), 1.01 (d, J = 6.3 Hz, 3H), 0.84 (t, J = 7.3 Hz, 3H). |
| 18 | (2S,4R)-4-hydroxy-1-((R)-2-(3-(2-((1R,5S,6R)-6-(3-(2-hydroxyphenyl)thieno[2,3-c]pyridazin-6-yl)-3-azabicyclo[3.1.1]heptan-3-yl)pyrimidin-5-yl)isoxazol-5-yl)-3-methylbutanoyl)-N-((S)-1-(4-(4-methylthiazol-5-yl)phenyl)ethyl)pyrrolidine-2-carboxamide | 882.3 | 1H NMR (400 MHz, DMSO-d6) δ 12.32 (s, 1H), 8.99 (s, 1H), 8.93 - 8.88 (m, 2H), 8.75 (s, 1H), 8.43 (d, J = 7.7 Hz, 1H), 8.03 (d, J = 7.7 Hz, 1H), 7.59 (s, 1H), 7.48 - 7.41 (m, 2H), 7.41 - 7.33 (m, 3H), 7.08 - 6.98 (m, 2H), 6.95 (s, 1H), 5.34 - 4.89 (m, 2H), 4.40 (t, J = 7.9 Hz, 1H), 4.31 (s, 1H), 4.03 (q, J = 12.0 Hz, 5H), 3.86 (d, J = 9.7 Hz, 1H), 3.80 - 3.74 (m, 1H), 3.56 - 3.47 (m, 1H), 3.00 - 2.94 (m, 2H), 2.48 - 2.42 (m, 3H), 2.05 - 1.95 (m, 3H), 1.85 - 1.76 (m, 1H), 1.66 - 1.59 (m, 1H), 1.51 - 1.36 (m, 3H), 1.05 - 0.99 (m, 3H), 0.90 - 0.81 (m, 3H). |
| 22 | (2S,4R)-4-hydroxy-1-((R)-2-(3-(2-((1R,4S,5S)-5-(3-(2-hydroxyphenyl)thieno[2,3-c]pyridazin-6-yl)-2-azabicyclo[2.2.1]heptan-2-yl)pyrimidin-5-yl)isoxazol-5-yl)-3-methylbutanoyl)-N-((S)-1-(4-(4-methylthiazol-5-yl)phenyl)ethyl)pyrrolidine-2-carboxamide | 882.4 | 1H NMR (400 MHz, DMSO-d6) δ 12.30 (d, J = 4.7 Hz, 1H), 8.99 (s, 1H), 8.81 (s, 2H), 8.71 (s, 1H), 8.44 (d, J = 7.7 Hz, 1H), 8.01 (d, J = 7.7 Hz, 1H), 7.53 - 7.42 (m, 3H), 7.41 - 7.32 (m, 3H), 7.02 (dd, J = 12.1, 7.8 Hz, 2H), 6.85 (d, J = 45.2 Hz, 1H), 5.16 - 5.02 (m, 1H), 5.00 - 4.90 (m, 1H), 4.86 (s, 1H), 4.50 - 4.35 (m, 1H), 4.31 (s, 1H), 4.08 - 3.82 (m, 1H), 3.80 - 3.63 (m, 1H), 3.63 - 3.55 (m, 2H), 3.52 (d, J = 7.4 Hz, 2H), 2.99 (s, 1H), 2.46 (s, 3H), 2.36 - 2.27 (m, 2H), 2.13 - 2.00 (m, 2H), 1.87 (s, 2H), 1.84 - 1.74 (m, 1H), 1.44 (dd, J = 41.2, 7.0 Hz, 3H), 1.01 (d, J = 6.2 Hz, 3H), 0.85 (t, J = 7.2 Hz, 3H). |
| 24 | (2S,4R)-4-hydroxy-1-((R)-2-(3-(6-(3-(3-(2-hydroxyphenyl)thieno[2,3-c]pyridazin-6-yl)azetidin-1-yl)pyridazin-3-yl)isoxazol-5-yl)-3-methylbutanoyl)-N-((S)-1-(4-(4-methylthiazol-5-yl)phenyl)ethyl)pyrrolidine-2-carboxamide | 842.15 | 1H NMR (400 MHz, Methanol-d4) δ 8.86 (d, J = 2.9 Hz, 1H), 8.67 (d, J = 2.0 Hz, 1H), 8.04 - 7.93 (m, 2H), 7.57 - 7.51 (m, 1H), 7.48 - 7.31 (m, 5H), 7.08 - 6.97 (m, 4H),5.05 (q, J = 7.0 Hz, 1H), 4.76 (t, J = 8.5 Hz, 2H), 4.55 (q, J = 8.9, 8.2 Hz, 2H), 4.43 (dd, J = 8.5, 5.5 Hz, 3H), 3.95 - 3.85 (m, 1H), 3.82 - 3.53 (m, 2H), 2.47 (d, J = 11.0 Hz, 4H), 2.27 - 2.13 (m, 1H), 2.03 - 1.92 (m, 1H), 1.57 (dd, J = 32.8, 7.0 Hz, 3H), 1.11 (d, J = 6.6 Hz, 3H), 0.94 (t, J = 6.4 Hz, 3H). |
| 25 | (2S,4R)-4-hydroxy-1-((R)-2-(3-(2-((3S,4R)-4-(3-(2-hydroxyphenyl)-5-methylthieno[2,3-c]pyridazin-6-yl)-3-methylpiperidin-1-yl)pyrimidin-5-yl)isoxazol-5-yl)-3-methylbutanoyl)-N-((S)-1-(4-(4-methylthiazol-5-yl)phenyl)ethyl)pyrrolidine-2-carboxamide | 898.35 | 1H NMR (300 MHz, DMSO-d6) δ 12.70 (s, 1H), 8.99 (s, 1H), 8.86 - 8.83 (m, 2H), 8.70 (s, 1H), 8.42 (d, J = 7.7 Hz, 1H), 8.16 (d, J = 8.4, 1H), 7.45 (d, J = 8.3 Hz, 2H), 7.44 - 7.31 (m, 3H), 7.09 - 6.97 (m, 2H), 6.91 (s, 1H), 5.10 (d, J = 3.6 Hz, 1H), 5.05 - 4.87 (m, 3H), 4.41 (t, J = 7.9 Hz, 1H), 4.32 (s, 1H), 3.87 (d, J = 7.2, 1H), 3.84 - 3.65 (m, 1H), 3.56 - 3.48 (m, 1H), 3.44 - 3.35 (m, 1H), 3.16 (t, J = 12.5 Hz, 1H), 2.86 (t, J = 12.2 Hz, 1H), 2.53 (s, 3H), 2.46 (d, J = 4.3 Hz, 3H), 2.40 - 2.24 (m, 1H), 2.10 - 1.98 (m, 2H), 1.89 - 1.65 (m, 3H), 1.53 - 1.35 (m, 3H), 1.03 (d, J = 6.5, 3H), 0.94 - 0.81 (m, 6H). |
| 27 | (2S,4R)-4-hydroxy-1-((R)-2-(3-(2-((2S,4S)-4-(3-(2-hydroxyphenyl)-5-methylthieno[2,3-c]pyridazin-6-yl)-2-methylpiperidin-1-yl)pyrimidin-5-yl)isoxazol-5-yl)-3-methylbutanoyl)-N-((S)-1-(4-(4-methylthiazol-5-yl)phenyl)ethyl)pyrrolidine-2-carboxamide | 898.15 | 1H NMR (400 MHz, DMSO-d6) δ 12.80 (s, 1H), 8.99 (s, 1H), 8.88 - 8.83 (m, 2H), 8.72 (s, 1H), 8.43 (d, J = 7.6 Hz, 1H), 8.21 - 8.14 (m, 1H), 7.48 - 7.41 (m, 2H), 7.42 - 7.34 (m, 3H), 7.06 - 6.98 (m, 2H), 6.93 (s, 1H), 5.31 - 5.23 (m, 1H), 5.11 (d, J = 3.6 Hz, 1H), 5.01 - 4.88 (m, 1H), 4.88 - 4.80 (m, 1H), 4.40 (t, J = 7.9 Hz, 1H), 4.31 (s, 1H), 3.90 - 3.79 (m, 2H), 3.80 - 3.58 (m, 1H), 3.51 (d, J = 10.6 Hz, 1H), 3.32 - 3.23 (m, 1H), 2.55 - 2.50 (m, 3H), 2.48 - 2.42 (m, 3H), 2.40 - 2.18 (m, 1H), 2.16 - 2.08 (m, 1H), 2.09 - 1.84 (m, 2H), 1.86 - 1.74 (m, 1H), 1.72 - 1.59 (m, 1H), 1.49 (d, J = 7.0 Hz, 1H), 1.45 - 1.32 (m, 6H), 1.06 - 0.99 (m, 3H), 0.89 - 0.81 (m, 3H). |
| 28 | (2S,4R)-4-hydroxy-1-((R)-2-(3-(5-(4-(3-(2-hydroxyphenyl)-5-methylthieno[2,3-c]pyridazin-6-yl)piperidin-1-yl)pyrazin-2-yl)isoxazol-5-yl)-3-methylbutanoyl)-N-((S)-1-(4-(4-methylthiazol-5-yl)phenyl)ethyl)pyrrolidine-2-carboxamide | 884.35 | 1H NMR (400 MHz, DMSO-d6) δ 12.77 (s, 1H), 8.99 (s, 1H), 8.69 (d, J = 18.7 Hz, 2H), 8.47 (d, J = 28.5 Hz, 2H), 8.17 (d, J = 7.7 Hz, 1H), 7.55 - 7.25 (m, 5H), 7.03 (d, J = 7.7 Hz, 2H), 6.80 (s, 1H), 5.11 (s, 1H), 4.94 (d, J = 8.2 Hz, 1H), 4.67 (d, J = 13.1 Hz, 2H), 4.46 - 4.24 (m, 2H), 3.96 - 3.46 (m, 4H), 3.19 (s, 2H), 2.48 - 2.19 (m, 6H), 2.10 (d, J = 13.5 Hz, 3H), 1.72 (d, J = 13.4 Hz, 3H), 1.55 - 1.19 (m, 4H), 1.01 (d, J = 6.5 Hz, 3H), 0.84 (t, J = 7.4 Hz, 3H). |
| 29 | (2S,4R)-1-((R)-2-(3-(2-(4-(3-(3-chloro-2-hydroxyphenyl)-5-methylthieno[2,3-c]pyridazin-6-yl)piperidin-1-yl)pyrimidin-5-yl)isoxazol-5-yl)-3-methylbutanoyl)-4-hydroxy-N-((S)-1-(4-(4-methylthiazol-5-ylphenyl)ethyl)pyrrolidine-2-carboxamide | 918.25 | 1H NMR (400 MHz, DMSO-d6) δ 14.49 (s, 1H), 8.99 (s, 1H), 8.89 - 8.80 (m, 3H), 8.45 (d, J = 7.7 Hz, 1H), 8.30 - 8.23 (m, 1H), 7.61 - 7.54 (m, 1H), 7.41 (m, 4H), 7.13 - 6.81 (m, 2H), 5.15 - 5.03 (m, 1H), 4.99 - 4.88 (m, 3H), 4.40 (t, J = 7.9 Hz, 1H), 4.31 (s, 1H), 3.86 (d, J = 9.7 Hz, 1H), 3.80 - 3.59 (m, 2H), 3.55 - 3.48 (m, 1H), 3.18 (t, J = 12.4 Hz, 2H), 2.53 (s, 3H), 2.48 - 2.42 (m, 3H), 2.40 - 2.27 (m, 1H), 2.14 - 1.99 (m, 3H), 1.86 - 1.75 (m, 1H), 1.74 - 1.59 (m, 2H), 1.52 - 1.34 (m, 3H), 1.06 - 0.99 (m, 3H), 0.91 - 0.81 (m, 3H). |
| 30 | (2S,4R)-4-hydroxy-1-((R)-2-(3-(2-((2R,4R)-4-(3-(2-hydroxyphenyl)-5-methylthieno[2,3-c]pyridazin-6-yl)-2-methylpiperidin-1-yl)pyrimidin-5-yl)isoxazol-5-yl)-3-methylbutanoyl)-N-((S)-1-(4-(4-methylthiazol-5-yl)phenyl)ethyl)pyrrolidine-2-carboxamide | 898.3 | 1H NMR (300 MHz, DMSO-d6) δ 12.80 (s, 1H), 9.01 (s, 1H), 8.86 (s, 2H), 8.73 (s, 1H), 8.43 (d, J = 7.7 Hz, 1H), 8.21 - 8.15 (m, 1H), 7.50 - 7.35 (m, 5H), 7.08 - 6.98 (m, 2H), 6.94 (s, 1H), 5.35 - 5.22 (m, 1H), 5.10 (d, J = 3.7 Hz, 1H), 5.02 - 4.79 (m, 2H), 4.41 (t, J = 7.8 Hz, 1H), 4.29 (s, 1H), 3.96 - 3.57 (m, 3H), 3.51 (d, J = 10.2 Hz, 1H), 3.25 (s, 1H), 2.50 (s, 3H),2.46 (d, J = 4.6 Hz, 3H), 2.40 - 2.29 (m, 1H), 2.18 - 1.99 (m, 3H), 1.98 - 1.80 (m, 2H), 1.72 - 1.60 (m, 1H), 1.51 - 1.34 (m, 6H), 1.02 (d, J = 6.4 Hz, 3H), 0.86 (t, J = 6.3 Hz, 3H). |
| 33 | (2S,4R)-4-hydroxy-1-((R)-2-(3-(6-(4-(3-(2-hydroxyphenyl)thieno[2,3-c]pyridazin-6-yl)piperidin-1-yl)pyridazin-3-yl)isoxazol-5-yl)-3-methylbutanoyl)-N-((S)-1-(4-(4-methylthiazol-5-yl)phenyl)ethyl)pyrrolidine-2-carboxamide | 870.3 | 1H NMR (400 MHz, DMSO-d6) δ 8.98 (d, J = 2.9 Hz, 1H), 8.71 (s, 1H), 8.00 (dd, J = 7.9, 1.7 Hz, 1H), 7.92 (d, J = 9.6 Hz, 1H), 7.50 (dd, J = 9.7, 4.8 Hz, 1H), 7.47 - 7.41 (m, 3H), 7.41 - 7.35 (m, 3H), 7.07 - 6.99 (m, 2H), 6.88 (d, J = 44.6 Hz, 1H), 4.93 (q, J = 7.0, 6.5 Hz, 1H), 4.65 (d, J = 13.2 Hz, 2H), 4.40 (t, J = 7.9 Hz, 1H), 4.30 (s, 1H), 3.93 (d, J = 9.7 Hz, 1H), 3.79 - 3.70 (m, 1H), 3.53 (d, J = 12.6 Hz, 2H), 3.23 (t, J = 12.5 Hz, 2H), 2.45 (d, J = 9.9 Hz, 3H), 2.39 - 2.32 (m, 1H), 2.24 (d, J = 12.7 Hz, 2H), 1.99 - 1.74 (m, 4H), 1.44 (dd, J = 37.7, 7.0 Hz, 3H), 1.02 (t, J = 6.3 Hz, 3H), 0.86 (dd, J = 13.0, 6.6 Hz, 3H). |
| 35 | (2S,4R)-4-hydroxy-1-((R)-2-(3-(2-((3R,4R)-4-(3-(2-hydroxyphenyl)-5-methylthieno[2,3-c]pyridazin-6-yl)-3-methylpiperidin-1-yl)pyrimidin-5-yl)isoxazol-5-yl)-3-methylbutanoyl)-N-((S)-1-(4-(4-methylthiazol-5-yl)phenyl)ethyl)pyrrolidine-2-carboxamide | 898.45 | 1H NMR (300 MHz, DMSO-d6) δ 12.70 (s, 1H), 8.99 (s, 1H), 8.85 (d, J = 2.8 Hz, 2H), 8.70 (s, 1H), 8.42 (d, J = 7.7 Hz, 1H), 8.21 - 8.12 (m, 1H), 7.51 - 7.35 (m, 5H), 7.10 - 6.98 (m, 2H), 6.94 (s, 1H), 5.11 (d, J = 3.6 Hz, 1H), 5.02 - 4.85 (m, 3H), 4.40 (t, J = 7.9 Hz, 1H), 4.31 (s, 1H), 3.86 (d, J = 9.7 Hz, 1H), 3.80 - 3.59 (m, 1H), 3.51 (d, J = 10.6 Hz, 1H), 3.43 - 3.35 (m, 1H), 3.15 (t, J = 12.5 Hz, 1H), 2.86 (t, J = 12.2 Hz, 1H), 2.49 (s, 3H), 2.45 (s, 3H), 2.38 - 2.19 (m, 1H), 2.11 - 1.98 (m, 2H), 1.90 - 1.69 (m, 3H), 1.51 - 1.35 (m, 3H), 1.03 (d, J = 6.5 Hz, 3H), 0.97 - 0.80 (m, 6H). |
| 39 | (2S,4R)-4-hydroxy-1-((R)-2-(3-(6-(4-(3-(2-hydroxyphenyl)thieno[2,3-c]pyridazin-6-yl)piperidin-1-yl)pyridin-3-yl)isoxazol-5-yl)-3-methylbutanoyl)-N-((S)-1-(4-(4-methylthiazol-5-yl)phenyl)ethyl)pyrrolidine-2-carboxamide | 869.4 | 1H NMR (400 MHz, DMSO-d6) δ 12.33 (s, 1H), 8.98 (s, 1H), 8.72 (s, 1H), 8.60 (dd, J = 5.9, 2.4 Hz, 1H), 8.42 (d, J = 7.7 Hz, 1H), 8.05 - 7.95 (m, 2H), 7.50 - 7.29 (m, 6H), 7.07 - 6.96 (m, 3H), 6.87 (s, 1H), 5.11 (d, J = 3.7 Hz, 1H), 7.50 - 7.29 (m, 1H), 4.58 (d, J = 13.3 Hz, 2H), 4.39 (t, J = 7.9 Hz, 1H), 4.30 (s, 1H), 3.86 - 3.80 (m, 1H), 3.80 - 3.71 (m, 1H), 3.52 - 3.40 (m, 2H), 3.09 (t, J = 12.5 Hz, 2H), 2.45 - 2.40 (m, 3H), 2.38 - 2.29 (m, 1H), 2.19 (d, J = 12.6 Hz, 2H), 2.03 (t, J = 9.8 Hz, 1H), 1.81 - 1.66 (m, 3H), 1.52 - 1.36 (m, 3H), 1.02 - 0.98 (m, 3H), 0.89 - 0.78 (m, 3H). |
| 40 | (2S,4R)-4-hydroxy-1-((R)-2-(3-(2-((1R,5R)-6-(3-(2-hydroxyphenyl)-5-methylthieno[2,3-c]pyridazin-6-yl)-3-azabicyclo[3.1.0]hexan-3-yl)pyrimidin-5-yl)isoxazol-5-yl)-3-methylbutanoyl)-N-((S)-1-(4-(4-methylthiazol-5-yl)phenyl)ethyl)pyrrolidine-2-carboxamide | 882.4 | 1H NMR (400 MHz, DMSO-d6) δ 12.87 (s, 1H), 8.99 (s, 1H), 8.84 (d, J = 5.1 Hz, 2H), 8.65 (s, 1H), 8.43 (d, J = 7.7 Hz, 1H), 8.20 - 8.14 (m, 1H), 7.54 - 7.28 (m, 5H), 7.06 - 6.98 (m, 2H), 6.93 (s, 1H), 5.11 (d, J = 3.6 Hz, 1H), 4.95 (dt, J = 14.2, 7.2 Hz, 1H), 4.39 (t, J = 7.9 Hz, 1H), 4.31 (s, 1H), 4.12 (d, J = 11.7 Hz, 2H), 3.85 (d, J = 9.7 Hz, 1H), 3.75 (d, J = 10.7 Hz, 3H), 3.68 - 3.60 (m, 1H), 3.55 - 3.47 (m, 1H), 3.30 (s, 2H), 2.49 - 2.39 (m, 5H), 2.35 (d, J = 3.7 Hz, 3H), 2.04 (t, J = 10.7 Hz, 1H), 1.80 (ddd, J = 12.8, 8.2, 4.7 Hz, 1H), 1.44 (dd, J = 40.2, 7.0 Hz, 3H), 1.02 (dd, J = 6.5, 3.6 Hz, 3H), 0.85 (t, J = 6.8 Hz, 3H). |
| 41 | (2S,4R)-4-hydroxy-1-((R)-2-(3-(2-((S)-3-(3-(2-hydroxyphenyl)-5-methylthieno[2,3-c]pyridazin-6-yl)pyrrolidin-1-yl)pyrimidin-5-yl)isoxazol-5-yl)-3-methylbutanoyl)-N-((S)-1-(4-(4-methylthiazol-5-yl)phenyl)ethyl)pyrrolidine-2-carboxamide | 870.2 | 1H NMR (400 MHz, DMSO-d6) δ 12.72 (d, J = 4.5 Hz, 1H), 8.99 (s, 1H), 8.87 (s, 2H), 8.72 (s, 1H), 8.43 (d, J = 7.7 Hz, 1H), 8.21 - 8.14 (m, 1H), 7.48 - 7.42 (m, 2H), 7.37 (dd, J = 6.7, 1.8 Hz, 3H), 7.07 - 6.98 (m, 2H), 6.93 (s, 1H), 5.11 (d, J = 3.7 Hz, 1H), 4.94 (q, J = 7.3, 6.6 Hz, 1H), 4.39 (t, J = 7.9 Hz, 1H), 4.33 - 4.15 (m, 3H), 3.98 - 3.89 (m, 1H), 3.85 (d, J = 9.7 Hz, 1H), 3.80 - 3.58 (m, 3H), 3.51 (d, J = 10.9 Hz, 1H), 2.59 - 2.57 (m, 1H), 2.52 (s, 3H), 2.45 (s, 3H), 2.38 - 2.27 (m, 1H), 2.26 - 2.15 (m, 1H), 2.04 (t, J = 9.2 Hz, 1H), 1.86 - 1.75 (m, 1H), 1.39 (d, J = 7.0 Hz, 3H), 1.06 - 0.98 (m, 3H), 0.92 - 0.81 (m, 3H). |
| 44 | (2S,4R)-4-hydroxy-1-((R)-2-(3-(2-((3-(3-(2-hydroxyphenyl)-5-methylthieno[2,3-c]pyridazin-6-ylbicyclo[1.1.1]pentan-1-yl)amino)pyrimidin-5-yl)isoxazol-5-yl)-3-methylbutanoyl)-N-((S)-1-(4-(4-methylthiazol-5-yl)phenyl)ethyl)pyrrolidine-2-carboxamide | 882.3 | 1H NMR (400 MHz, Methanol-d4) δ 8.90 - 8.85 (m, 1H), 8.78 - 8.74 (m, 2H), 8.53 (s, 1H), 8.09 - 8.02 (m, 1H), 7.48 - 7.33 (m, 5H), 7.07 - 6.98 (m, 2H), 6.77 (s, 1H), 5.08 - 5.02 (m, 1H), 4.53 (t, J = 8.2 Hz, 1H), 4.46 (s, 1H), 3.93 - 3.71 (m, 2H), 3.70 - 3.62 (m, 1H), 2.77 - 2.72 (m, 6H), 2.56 - 2.51 (m, 3H), 2.51 - 2.43 (m, 4H), 2.24 - 2.14 (m, 1H), 2.02 - 1.91 (m, 1H), 1.58 (dd, J = 36.4, 7.0 Hz, 3H), 1.10 (d, J = 6.5 Hz, 3H), 0.98 - 0.88 (m, 3H). |
| 46 | (2S,4R)-4-hydroxy-1-((R)-2-(3-(2-(6-(3-(2-hydroxyphenyl)-5-methylthieno[2,3-c]pyridazin-6-yl)-2-azaspiro[3.3]heptan-2-yl)pyrimidin-5-yl)isoxazol-5-yl)-3-methylbutanoyl)-N-((S)-1-(4-(4-methylthiazol-5-yl)phenyl)ethyl)pyrrolidine-2-carboxamide | 896.3 | 1H NMR (400 MHz, DMSO-d6) δ 12.83 (s, 1H), 8.99 (s, 1H), 8.83 - 8.79 (m, 2H), 8.74 - 8.65 (m, 1H), 8.44 (d, J = 7.7 Hz, 1H), 8.20 - 8.13 (m, 1H), 7.48 - 7.41 (m, 2H), 7.42 - 7.34 (m, 3H), 7.08 - 6.78 (m, 3H), 5.15 - 5.09 (m, 1H), 5.00 - 4.67 (m, 1H), 4.42 - 4.23 (m, 4H), 4.18 - 4.00 (m, 3H), 3.85 (d, J = 9.7 Hz, 1H), 3.79 - 3.71 (m, 1H), 3.68 - 3.45 (m, 2H), 2.92 - 2.82 (m, 2H), 2.56 - 2.51 (m, 1H), 2.48 - 2.42 (m, 3H), 2.39 (s, 3H), 2.37 - 2.16 (m, 1H), 2.08 - 1.98 (m, 1H), 1.85 - 1.74 (m, 1H), 1.57 - 1.30 (m, 3H), 1.05 - 0.98 (m, 3H), 0.88 - 0.80 (m, 3H). |
| 47 | (2S,4R)-4-hydroxy-1-((R)-2-(3-(2-((R)-3-(3-(2-hydroxyphenyl)-5-methylthieno[2,3-c]pyridazin-6-yl)pyrrolidin-1-yl)pyrimidin-5-yl)isoxazol-5-yl)-3-methylbutanoyl)-N-((S)-1-(4-(4-methylthiazol-5-yl)phenyl)ethyl)pyrrolidine-2-carboxamide | 870.35 | 1H NMR (400 MHz, DMSO-d6) δ 12.71 (s, 1H), 8.99 (s, 1H), 8.87 (s, 2H), 8.73 (s, 1H), 8.44 (d, J = 7.7 Hz, 1H), 8.24 - 8.12 (m, 1H), 7.50 - 7.42 (m, 2H), 7.42 - 7.25 (m, 3H), 7.09 - 6.80 (m, 3H), 5.12 (d, J = 3.6 Hz, 1H), 5.06 - 4.88 (m, 1H), 4.39 (t, J = 7.9 Hz, 1H), 4.23 (ddd, J = 32.3, 13.1, 7.4 Hz, 3H), 3.93 (d, J = 8.1 Hz, 1H), 3.85 (d, J = 9.8 Hz, 1H), 3.80 - 3.61 (m, 3H), 3.55 - 3.46 (m, 1H), 2.59 (s, 1H), 2.53 (s, 3H), 2.45 (d, J = 6.1 Hz, 3H), 2.35 - 2.20 (m, 2H), 2.04 (t, J = 10.3 Hz, 1H), 1.79 (ddd, J = 12.7, 8.1, 4.7 Hz, 1H), 1.44 (dd, J = 41.4, 7.0 Hz, 3H), 1.02 (dd, J = 6.7, 4.1 Hz, 3H), 0.85 (t, J = 7.2 Hz, 3H). |
| 48 | (2S,4R)-4-hydroxy-1-((R)-2-(3-(2-(2-(3-(2-hydroxyphenyl)-5-methylthieno[2,3-c]pyridazin-6-yl)-7-azaspiro[3.5]nonan-7-yl)pyrimidin-5-yl)isoxazol-5-yl)-3-methylbutanoyl)-N-((S)-1-(4-(4-methylthiazol-5-yl)phenyl)ethyl)pyrrolidine-2-carboxamide | 924.3 | 1H NMR (300 MHz, DMSO-d6) δ 12.88 (s, 1H), 8.99 (s, 1H), 8.81 (s, 2H), 8.67 (s, 1H), 8.45 (d, J = 7.7 Hz, 1H), 8.22 - 8.13 (m, 1H), 7.45 (d, J = 8.2 Hz, 2H), 7.41 - 7.35 (m, 3H), 7.08 - 6.97 (m, 2H), 6.94 - 6.76 (m, 1H), 5.09 (dd, J = 21.9, 3.2 Hz, 1H), 5.01 - 4.88 (m, 1H), 4.39 (t, J = 7.9 Hz, 1H), 4.34 - 4.28 (m, 1H), 4.23 - 4.08 (m, 1H), 3.95 - 3.72 (m, 6H), 3.51 (d, J = 10.7 Hz, 1H), 2.62 - 2.54 (m, 1H), 2.49 - 2.43 (m, 3H), 2.39 (s, 3H), 2.37 - 2.24 (m, 1H), 2.13 - 2.00 (m, 3H), 1.86 - 1.80 (m, 3H), 1.68 - 1.62 (m, 2H), 1.44 (d, 3H), 1.24 (s, 1H), 1.06 - 0.97 (m, 3H), 0.85 - 0.80 (m, 3H). |
| 49 | (2S,4R)-4-hydroxy-1-((R)-2-(3-(6-(3-(3-(2-hydroxyphenyl)-5-methylthieno[2,3-c]pyridazin-6-yl)azetidin-1-yl)pyridin-3-yl)isoxazol-5-yl)-3-methylbutanoyl)-N-((S)-1-(4-(4-methylthiazol-5-yl)phenyl)ethyl)pyrrolidine-2-carboxamide | 855.25 | 1H NMR (300 MHz, DMSO-d6) δ 12.70 (s, 1H), 9.00 (s, 1H), 8.73 (s, 1H), 8.63 (d, J = 2.4 Hz, 1H), 8.44 (d, J = 7.7 Hz, 1H), 8.18 (d, J = 7.6 Hz, 1H), 8.04 (dd, J = 8.6, 2.3 Hz, 1H), 7.50 - 7.33 (m, 5H), 7.09 - 6.98 (m, 2H), 6.90 (s, 1H), 6.65 (d, J = 8.7 Hz, 1H), 5.12 (d, J = 3.7 Hz, 1H), 4.94 (t, J = 7.1 Hz, 1H), 4.63 (d, J = 7.3 Hz, 3H), 4.40 (t, J = 7.9 Hz, 1H), 4.31 (s, 1H), 4.15 (d, J = 5.3 Hz, 2H), 3.84 (d, J = 9.7 Hz, 1H), 3.81 - 3.72 (m, 1H), 3.70 - 3.56 (m, 1H),2.46 (d, J = 1.7 Hz, 6H), 2.46 (m , 1H)2.07 - 1.97 (m, 1H), 1.88 - 1.77 (m, 1H), 1.56 - 1.46 (m, 1H), 1.44 - 1.32 (m, 3H), 1.02 (d, J = 6.4 Hz, 3H), 0.86 (t, J = 7.1 Hz, 3H). |
| 50 | (2S,4R)-4-hydroxy-1-((R)-2-(3-(6-(3-(3-(2-hydroxyphenyl)-5-methylthieno[2,3-c]pyridazin-6-yl)azetidin-1-yl)pyridazin-3-yl)isoxazol-5-yl)-3-methylbutanoyl)-N-((S)-1-(4-(4-methylthiazol-5-yl)phenyl)ethyl)pyrrolidine-2-carboxamide | 856.3 | 1H NMR (400 MHz, DMSO-d6) δ 12.68 (s, 1H), 8.99 (s, 1H), 8.73 (s, 1H), 8.52 - 8.42 (m, 1H), 8.17 (d, 1H), 8.00 - 7.92 (m, 1H), 7.48 - 7.34 (m, 5H), 7.11 - 6.99 (m, 3H), 6.93 (s, 1H), 5.11 (d, J = 3.7 Hz, 1H), 5.01 - 4.89 (m, 1H), 4.77 - 4.71 (m, 3H), 4.40 (t, J = 8.0 Hz, 1H), 4.29 (d, J = 4.0 Hz, 3H), 3.94 (d, J = 9.7 Hz, 1H), 3.79 - 3.66 (m, 1H), 3.65 - 3.49 (m, 1H), 2.48 - 2.42 (m, 6H), 2.42 - 2.32 (m, 1H), 2.11 - 1.99 (m, 1H), 1.85 - 1.74 (m, 1H), 1.52 - 1.36 (m, 3H), 1.06 - 0.99 (m, 3H), 0.91 - 0.81 (m, 3H). |
| 52 | (2S,4R)-4-hydroxy-1-((R)-2-(3-(2-(4-hydroxy-4-(3-(2-hydroxyphenyl)-5-methylthieno[2,3-c]pyridazin-6-yl)piperidin-1-yl)pyrimidin-5-yl)isoxazol-5-yl)-3-methylbutanoyl)-N-((S)-1-(4-(4-methylthiazol-5-yl)phenyl)ethyl)pyrrolidine-2-carboxamide | 900.3 | 1H NMR (400 MHz, Methanol-d4) δ 8.87 - 8.77 (m, 3H), 8.51 (s, 1H), 8.03 (d, J = 8.0 Hz, 1H), 7.48 - 7.30 (m, 5H), 7.00 (dd, J = 8.3, 4.9 Hz, 2H), 6.77 (s, 1H), 5.05 (d, J = 7.4 Hz, 1H), 4.55 - 4.46 (m, 3H), 3.88 (dd, J = 10.4, 4.0 Hz, 2H), 3.71 - 3.61 (m, 1H), 3.47 (t, J = 12.4 Hz, 2H), 2.55 - 2.47 (m, 6H), 2.36 - 2.15 (m, 3H), 2.11 - 1.98 (m, 3H), 1.53 (d, J = 7.0 Hz, 3H), 1.27 (d, J = 9.7 Hz, 2H), 1.10 (d, J = 6.6 Hz, 3H), 0.94 (t, J = 7.0 Hz, 3H). |
| 54 | (2S,4R)-4-hydroxy-1-((R)-2-(3-(2-(4-(3-(2-hydroxyphenyl)thieno[2,3-c]pyridazin-6-yl)piperidin-1-yl)pyrimidin-5-yl)isoxazol-5-yl)-3-methylbutanoyl)-N-((S)-1-(4-(4-methylthiazol-5-yl)phenyl)ethyl)pyrrolidine-2-carboxamide | 870.7 | 1H NMR (300 MHz, DMSO-d6) δ 12.35 (d, J = 4.1 Hz, 1H), 8.99 (d, J = 1.8 Hz, 1H), 8.85 (d, J = 2.5 Hz, 2H), 8.73 (s, 1H), 8.44 (d, J = 7.7 Hz, 1H), 8.02 (dd, J = 7.9, 1.7 Hz, 1H), 7.49 - 7.31 (m, 6H), 7.08 - 6.96 (m, 2H), 6.93 (s, 1H), 5.12 (d, J = 3.6 Hz, 1H), 4.99 - 4.86 (m, 3H), 4.40 (t, J = 7.9 Hz, 1H), 4.31 (s, 1H), 3.86 (d, J = 9.7 Hz, 1H), 3.76 (dd, J = 10.5, 4.3 Hz, 1H), 3.51 (d, J = 11.1 Hz, 2H), 3.18 (t, J = 12.5 Hz, 2H), 2.46 (s, 3H), 2.40 - 2.27 (m, 1H), 2.22 (d, J = 12.5 Hz, 2H), 2.11 - 1.98 (m, 1H), 1.88 - 1.65 (m, 3H), 1.39 (d, J = 7.0 Hz, 3H), 1.02 (d, J = 6.5 Hz, 3H), 0.84 (d, J = 6.5 Hz, 3H). |
| 55 | (2S,4R)-4-hydroxy-1-((R)-2-(3-(2-(4-(3-(2-hydroxyphenyl)thieno[3,2-c]pyridazin-6-yl)piperidin-1-yl)pyrimidin-5-yl)isoxazol-5-yl)-3-methylbutanoyl)-N-((S)-1-(4-(4-methylthiazol-5-yl)phenyl)ethyl)pyrrolidine-2-carboxamide | 870.35 | 1H NMR (300 MHz, DMSO-d6) δ 12.96 (s, 1H), 9.16 (s, 1H), 8.99 (s, 1H), 8.85 (s, 2H), 8.44 (d, J = 7.7 Hz, 1H), 8.03 (dd, J = 8.1, 1.6 Hz, 1H), 7.76 (s, 1H), 7.45 (d, J = 8.2 Hz, 2H), 7.42 - 7.30 (m, 3H), 7.08 - 6.96 (m, 2H), 6.96 (d, J = 15.7 Hz, 1H), 5.12 (d, J = 3.6 Hz, 1H), 5.08 - 4.86 (m, 3H), 4.40 (t, J = 7.9 Hz, 1H), 4.31 (s, 1H), 3.90 - 3.71 (m, 2H), 3.56 - 3.38 (m, 2H), 3.18 (t, J = 12.5 Hz, 2H), 2.49 - 2.41 (m, 4H), 2.22 (d, J = 13.1 Hz, 2H), 2.11 - 1.98 (m, 1H), 1.87 - 1.67 (m, 3H), 1.39 (d, J = 7.0 Hz, 3H), 1.02 (d, J = 6.5 Hz, 3H), 0.90 - 0.80 (m, 3H). |
| 57 | (2S,4R)-4-hydroxy-1-((R)-2-(3-(6-(4-(3-(2-hydroxyphenyl)-5-methylthieno[2,3-c]pyridazin-6-yl)piperidin-1-yl)pyridazin-3-yl)isoxazol-5-yl)-3-methylbutanoyl)-N-((S)-1-(4-(4-methylthiazol-5-yl)phenyl)ethyl)pyrrolidine-2-carboxamide | 884.2 | 1H NMR (400 MHz, DMSO-d6) δ 12.76 (d, J = 4.4 Hz, 1H), 8.99 (s, 1H), 8.72 (s, 1H), 8.45 (d, J = 7.6 Hz, 1H), 8.17 (d, J = 7.9 Hz, 1H), 7.90 (d, J = 9.5 Hz, 1H), 7.52 - 7.31 (m, 6H), 7.03 (d, J = 7.6 Hz, 2H), 6.94 (s, 1H), 5.11 (d, J = 3.7 Hz, 1H), 4.99 - 4.89 (m, 1H), 4.70 (d, J = 13.4 Hz, 2H), 4.40 (t, J = 7.9 Hz, 1H), 4.30 (s, 1H), 3.93 (d, J = 9.7 Hz, 1H), 3.79 - 3.64 (m, 2H), 3.53 (d, J = 14.8 Hz, 2H), 3.24 - 3.17 (m, 3H), 2.45 (s, 3H), 2.39 - 2.34 (m, 1H), 2.15 - 1.98 (m, 4H), 1.86 - 1.65 (m, 3H), 1.40 (d, J = 7.0 Hz, 3H), 1.02 (d, J = 6.6 Hz, 3H), 0.84 (d, J = 6.7 Hz, 3H). |
| 58 | (2S,4R)-4-hydroxy-1-((R)-2-(3-(2-(4-(3-(2-hydroxyphenyl)-5-methylthieno[2,3-c]pyridazin-6-yl)piperidin-1-yl)pyrimidin-4-yl)isoxazol-5-yl)-3-methylbutanoyl)-N-((S)-1-(4-(4-methylthiazol-5-yl)phenyl)ethyl)pyrrolidine-2-carboxamide | 884.2 | 1H NMR (400 MHz, DMSO-d6) δ 12.79 (d, J = 5.6 Hz, 1H), 8.97 (d, J = 7.1 Hz, 1H), 8.85-8.71 (m, 1H), 8.54 (dd, J = 5.0, 3.4 Hz, 1H), 8.43 (d, J = 7.7 Hz, 1H), 8.17 (dd, J = 8.4, 1.7 Hz, 1H), 7.51 - 7.31 (m, 5H), 7.17 (d, J = 4.9 Hz, 1H), 7.06 - 6.96 (m, 2H), 6.81 (s, 1H), 5.10 (d, J = 3.7 Hz, 1H), 5.05 - 4.80 (m, 3H), 4.39 (t, J = 7.9 Hz, 1H), 4.29 (s, 1H), 3.94 (d, J = 9.7 Hz, 1H), 3.79 - 3.61 (m, 2H), 3.60 - 3.51 (m, 1H), 3.15 (t, J = 12.6 Hz, 2H), 2.41 (s, 3H), 2.39 - 2.22 (m, 2H), 2.14 - 1.94 (m, 3H), 1.90 - 1.61 (m, 4H), 1.39 (d, J = 7.0 Hz, 3H), 1.01 (dd, J = 6.6, 1.9 Hz, 3H), 0.82 (dd, J = 11.6, 6.7 Hz, 3H). |
| 59 | (2S,4R)-4-hydroxy-1-((R)-2-(3-(6-(4-(3-(2-hydroxyphenyl)-5-methylthieno[2,3-c]pyridazin-6-yl)piperidin-1-yl)pyridin-3-yl)isoxazol-5-yl)-3-methylbutanoyl)-N-((S)-1-(4-(4-methylthiazol-5-yl)phenyl)ethyl)pyrrolidine-2-carboxamide | 883.1 | 1H NMR (400 MHz, DMSO-d6) δ 12.77 (d, J = 3.9 Hz, 1H), 8.99 (s, 1H), 8.71 (s, 1H), 8.61 (d, J = 2.4 Hz, 1H), 8.44 (d, J = 7.7 Hz, 1H), 8.20 - 8.14 (m, 1H), 7.99 (dd, J = 8.9, 2.5 Hz, 1H), 7.45 (d, J = 8.2 Hz, 2H), 7.40 - 7.34 (m, 3H), 7.06 - 6.98 (m, 3H), 6.88 (s, 1H), 5.11 (d, J = 3.6 Hz, 1H), 4.97 - 4.87 (m, 1H), 4.61 (d, J = 13.0 Hz, 2H), 4.40 (t, J = 7.9 Hz, 1H), 4.31 (s, 1H), 3.83 (d, J = 9.7 Hz, 1H), 3.76 (dd, J = 10.6, 4.4 Hz, 1H), 3.65 (t, J = 11.0 Hz, 1H), 3.55 - 3.50 (m, 1H), 3.11 (t, J = 12.6 Hz, 2H), 2.49 (s, 3H), 2.45 (s, 3H), 2.34 (d, J = 8.6 Hz, 1H), 2.11 - 1.99 (m, 3H), 1.86 - 1.74 (m, 1H), 1.74 - 1.61 (m, 2H), 1.39 (d, J = 7.0 Hz, 3H), 1.06 - 0.98 (m, 3H), 0.88 - 0.81 (m, 3H). |
| 61 | (2S,4R)-4-hydroxy-1 -((R)-2-(3-((4-(4-(3-(2-hydroxyphenyl)-5-methylthieno[2,3-c]pyridazin-6-yl)piperidin-1-yl)pyrimidin-2-yl)oxy)isoxazol-5-yl)-3-methylbutanoyl)-N-((S)-1-(4-(4-methylthiazol-5-yl)phenyl)ethyl)pyrrolidine-2-carboxamide | 900.2 | 1H NMR (400 MHz, DMSO-d6) δ 12.78 - 12.73 (m, 1H), 9.00 - 8.94 (m, 1H), 8.73 - 8.67 (m, 1H), 8.40 (d, J = 7.7 Hz, 1H), 8.19 - 8.09 (m, 2H), 7.47 - 7.30 (m, 5H), 7.06 - 6.97 (m, 2H), 6.82 - 6.76 (m, 1H), 6.51 (s, 1H), 5.11 (d, J = 3.7 Hz, 1H), 4.88 (t, J = 7.2 Hz, 1H), 4.50 (s, 2H), 4.39 (t, J = 7.8 Hz, 1H), 4.29 (s, 1H), 3.79 (d, J = 9.6 Hz, 1H), 3.76 - 3.54 (m, 2H), 3.53 - 3.46 (m, 1H), 3.13 (s, 2H), 2.50 - 2.41 (m, 5H), 2.33 - 2.23 (m, 2H), 2.06 - 2.01 (m, 3H), 1.84 - 1.73 (m, 1H), 1.68 - 1.60 (m, 2H), 1.48 - 1.28 (m, 3H), 1.02 - 0.95 (m, 3H), 0.90 - 0.81 (m, 3H). |
| 62 | (2S,4R)-4-hydroxy-1-((R)-2-(3-((2-(4-(3-(2-hydroxyphenyl)-5-methylthieno[2,3-c]pyridazin-6-yl)piperidin-1-yl)pyrimidin-4-yl)oxy)isoxazol-5-yl)-3-methylbutanoyl)-N-((S)-1-(4-(4-methylthiazol-5-yl)phenyl)ethyl)pyrrolidine-2-carboxamide | 900.1 | 1H NMR (300 MHz, Methanol-d4) δ 8.86 (s, 1H), 8.52 (d, J = 17.0 Hz, 1H), 8.33 (dd, J = 5.5, 2.9 Hz, 1H), 8.06 (d, J = 7.9 Hz, 1H), 7.44 - 7.31 (m, 5H), 7.08 - 6.98 (m, 2H), 6.55 (s, 1H), 6.38 (dd, J = 5.6, 2.1 Hz, 1H), 5.00 (d, J = 6.9 Hz, 1H), 4.51 (dd, J = 18.1, 9.9 Hz, 2H), 3.88 - 3.82 (m, 1H), 3.73 - 3.58 (m, 3H), 3.09 (d, J = 25.4 Hz, 2H), 2.53 (s, 2H), 2.51 - 2.35 (m, 5H), 2.16 - 1.95 (m, 4H), 1.82 - 1.69 (m, 2H), 1.45 (d, J = 7.0 Hz, 3H), 1.09 (d, J = 6.6 Hz, 3H), 0.96 (d, J = 6.6 Hz, 3H). |
| 63 | (2S,4R)-4-hydroxy-1-((R)-2-(3-(2-(3-(3-(2-hydroxyphenyl)-5-methylthieno[2,3-c]pyridazin-6-yl)azetidin-1-yl)pyrimidin-5-yl)isoxazol-5-yl)-3-methylbutanoyl)-N-((S)-1-(4-(4-methylthiazol-5-yl)phenyl)ethyl)pyrrolidine-2-carboxamide | 856.4 | 1H NMR (400 MHz, Methanol-d4) δ 8.87 (d, J = 1.8 Hz, 1H), 8.80 (d, J = 7.3 Hz, 2H), 8.56 (s, 1H), 8.05 (dd, J = 8.3, 1.6 Hz, 1H), 7.57 - 7.28 (m, 5H), 7.07 - 6.98 (m, 2H), 6.75 (d, J = 40.1 Hz, 1H), 5.05 (d, J = 6.9 Hz, 1H), 4.76 (d, J = 8.8 Hz, 2H), 4.66 (s, 1H), 4.58 - 4.52 (m, 1H), 4.46 (s, 1H), 4.36 (dd, J = 9.2, 5.5 Hz, 2H), 3.88 (dd, J = 10.4, 3.8 Hz, 2H), 3.72 - 3.62 (m, 1H), 2.48 (d, J = 0.9 Hz, 7H), 2.25 - 2.15 (m, 1H), 1.97 (ddd, J = 13.3, 8.7, 4.6 Hz, 1H), 1.57 (dd, J = 34.3, 7.0 Hz, 3H), 1.10 (d, J = 6.6 Hz, 3H), 0.93 (t, J = 6.5 Hz, 3H). |
| 66 | (2S,4R)-4-hydroxy-1-((R)-2-(3-(2-(4-(3-(2-hydroxyphenyl)-5-methylthieno[2,3-c]pyridazin-6-yl)piperidin-1-yl)pyrimidin-5-yl)isoxazol-5-yl)-3-methylbutanoyl)-N-((S)-1-(4-(4-methylthiazol-5-yl)phenyl)ethyl)pyrrolidine-2-carboxamide | 884.2 | 1H NMR (400 MHz, DMSO-d6) δ 12.79 (d, J = 4.5 Hz, 1H), 9.03 - 8.68 (m, 4H), 8.43 (d, J = 7.7 Hz, 1H), 8.22 - 8.12 (m, 1H), 7.45 (d, J = 8.2 Hz, 2H), 7.42 - 7.33 (m, 3H), 7.02 (dd, J = 7.8, 6.5 Hz, 2H), 6.88 (d, J = 45.1 Hz, 1H), 5.11 (d, J = 3.7 Hz, 1H), 4.95 (dd, J = 13.2, 4.6 Hz, 3H), 4.41 (t, J = 7.9 Hz, 1H), 4.36 - 4.26 (m, 1H), 3.86 (d, J = 9.7 Hz, 1H), 3.77 (dd, J = 10.6, 4.4 Hz, 1H), 3.66 (td, J = 11.0, 3.8 Hz, 1H), 3.51 (d, J = 10.7 Hz, 1H), 3.17 (t, J = 12.4 Hz, 2H), 2.53 - 2.50 (m, 3H), 2.46 (d, J = 6.2 Hz, 3H), 2.40 - 2.28 (m, 1H), 2.14 - 2.00 (m, 3H), 1.81 (ddd, J = 12.7, 8.0, 4.7 Hz, 1H), 1.73 - 1.59 (m, 2H), 1.45 (dd, J = 40.5, 7.0 Hz, 3H), 1.03 (dd, J = 6.6, 4.2 Hz, 3H), 0.86 (t, J = 7.6 Hz, 3H). |
| 53 | (2S,4R)-4-hydroxy-1-((R)-2-(3-(2-(4-(3-(2-hydroxyphenyl)-5-methylthieno[2,3-c]pyridazin-6-yl)piperazin-1-yl)pyrimidin-5-yl)isoxazol-5-yl)-3-methylbutanoyl)-N-((S)-1-(4-(4-methylthiazol-5-yl)phenyl)ethyl)pyrrolidine-2-carboxamide | 885.3 | 1H NMR (300 MHz, Methanol-d4) δ 8.90 (s, 1H), 8.82 (d, J = 6.1 Hz, 2H), 8.34 (s, 1H), 8.05 (d, J = 8.2 Hz, 1H), 7.52 - 7.42 (m, 4H), 7.40 - 7.31 (m, 1H), 7.03 (t, J = 7.2 Hz, 2H), 6.81 (s, 1H), 5.12 - 5.03 (d, J = 6.9 Hz, 1H), 4.60 - 4.52 (m, 1H), 4.50 - 4.42 (m, 1H), 4.22 - 4.14 (m, 4H), 3.95 - 3.88 (m, 1H), 3.75 - 3.71 (m, 1H), 3.70 - 3.64 (m, 1H), 3.49 - 3.40 (m, 4H), 2.55 - 2.48 (m, 4H), 2.47 - 2.42 (m, 3H), 2.28 - 2.13 (m, 1H), 2.11 - 1.97 (m, 1H), 1.56 (d, J = 7.0 Hz, 3H), 1.13 (d, J = 6.6 Hz, 3H), 0.95 (d, J = 6.6 Hz, 3H). |
| 75 | (2S,4R)-1-((R)-2-(3-(2-((R)-3-(5-cyclopropyl-3-(2-hydroxyphenyl)thieno[2,3-c]pyridazin-6-yl)pyrrolidin-1-yl)pyrimidin-5-yl)isoxazol-5-yl)-3-methylbutanoyl)-4-hydroxy-N-((S)-1-(4-(4-methylthiazol-5-yl)phenyl)ethyl)pyrrolidine-2-carboxamide | 896.25 | 1H NMR (400 MHz, DMSO-d6) δ 12.06 (s, 1H), 8.99 (s, 1H), 8.87 (s, 2H), 8.63 (s, 1H), 8.43 (d, J = 7.7 Hz, 1H), 8.06 (dd, J = 7.8, 1.6 Hz, 1H), 7.50 - 7.42 (m, 2H), 7.41 - 7.33 (m, 3H), 7.08 - 6.90 (m, 3H), 5.07 - 4.88 (m, 2H), 4.43 (dt, J = 27.2, 7.8 Hz, 2H), 4.31 (s, 1H), 4.21 (dd, J = 11.4, 7.4 Hz, 1H), 3.96 (d, J = 6.0 Hz, 1H), 3.85 (d, J = 9.7 Hz, 1H), 3.80 - 3.66 (m, 3H), 3.50 (d, J = 6.4 Hz, 1H), 2.63 (d, J = 7.3 Hz, 1H), 2.45 (d, J = 6.2 Hz, 3H), 2.24 (t, J = 10.5 Hz, 2H), 2.05 - 1.94 (m, 2H), 1.79 (ddt, J = 12.9, 9.6, 4.6 Hz, 1H), 1.52 - 1.36 (m, 4H), 1.23 (s, 1H), 1.14 (d, J = 8.3 Hz, 2H), 1.01 (d, J = 6.6 Hz, 3H), 0.85 (t, J = 7.2 Hz, 3H), 0.79 (d, J = 3.8 Hz, 1H). |
| 76 | (2S,4R)-1-((R)-2-(3-(2-((S)-3-(5-cyclopropyl-3-(2-hydroxyphenyl)thieno[2,3-c]pyridazin-6-yl)pyrrolidin-1-yl)pyrimidin-5-yl)isoxazol-5-yl)-3-methylbutanoyl)-4-hydroxy-N-((S)-1-(4-(4-methylthiazol-5-yl)phenyl)ethyl)pyrrolidine-2-carboxamide | 896.25 | 1H NMR (300 MHz, DMSO-d6) δ 12.04 (s, 1H), 8.99 (s, 1H), 8.95 - 8.73 (m, 2H), 8.62 (s, 1H), 8.44 (d, J = 8.0 Hz, 1H), 8.07 (d, J= 8.6 Hz,1H), 7.56 - 7.47 (m, 2H), 7.45 - 7.27 (m, 3H), 7.13 - 7.02 (m, 2H), 6.97 - 6.78 (m, 1H), 5.12 (d, J = 1.5 Hz, 1H), 5.00 - 4.85 (m, 1H), 4.53 - 4.15 (m, 4H), 4.03 - 3.90 (m, 1H), 3.86 (d, J = 9.0 Hz, 1H), 3.80 - 3.62 (m, 3H), 3.58 - 3.45 (m, 1H), 2.70 - 2.59 (m, 1H), 2.54 (s, 3H), 2.48 - 2.41 (m, 2H), 2.11 - 1.90 (m, 2H), 1.86 - 1.74 (m, 1H), 1.55 - 1.35 (m, 3H), 1.26 - 1.20 (m, 1H), 1.19 - 1.10 (m, 2H), 1.01 (d, J = 6.4 Hz, 3H), 0.89 - 0.82 (m, 3H), 0.81 - 0.76 (m, 1H). |
| 84 | (2S,4R)-1-((R)-2-(3-(2-(4-(5-cyclopropyl-3-(2-hydroxyphenyl)thieno[2,3-c]pyridazin-6-yl)piperidin-1-yl)pyrimidin-5-yl)isoxazol-5-yl)-3-methylbutanoyl)-4-hydroxy-N-((S)-1-(4-(4-methylthiazol-5-yl)phenyl)ethyl)pyrrolidine-2-carboxamide | 910.35 | 1H NMR (400 MHz, Methanol-d4) δ 9.06 - 8.41 (m, 4H), 7.99 (d, J = 7.9 Hz, 1H), 7.86 - 7.10 (m, 5H), 7.10 - 6.90 (m, 2H), 6.72 (d, J = 39.0 Hz, 1H), 5.37 - 4.91 (m, 3H), 4.54 (t, J = 8.0 Hz, 1H), 4.46 (s, 1H), 3.98 (d, J = 12.0 Hz, 1H), 3.93 - 3.73 (m, 2H), 3.19 (d, J = 13.2 Hz, 2H), 2.57 (d, J = 68.6 Hz, 4H), 2.33 - 2.03 (m, 3H), 1.98 (ddd, J = 13.4, 8.8, 4.7 Hz, 2H), 1.89 - 1.71 (m, 2H), 1.58 (dd, J = 34.1, 7.0 Hz, 3H), 1.37 - 1.22 (m, 2H), 1.22 - 1.04 (m, 2H), 0.94 (t, J = 6.7 Hz, 3H), 0.80 (dd, J = 5.6, 1.7 Hz, 2H). |
| 93 | (2S,4R)-4-hydroxy-1-((R)-2-(3-(2-(4-(3-(2-hydroxyphenyl)-5-methylthieno[2,3-c]pyridazin-6-yl)piperidin-1-yl)-4-methylpyrimidin-5-yl)isoxazol-5-yl)-3-methylbutanoyl)-N-((S)-1-(4-(4-methylthiazol-5-yl)phenyl)ethyl)pyrrolidine-2-carboxamide | 898.45 | 1H NMR (300 MHz, DMSO-d6) δ 12.78 (d, J = 2.9 Hz, 1H), 8.99 (s, 1H), 8.72 (s, 1H), 8.57 (d, J = 10.3 Hz, 1H), 8.44 (d, J = 7.6 Hz, 1H), 8.22 - 8.13 (m, 1H), 7.50 - 7.33 (m, 5H), 7.11 - 6.68 (m, 3H), 5.12 (d, J = 3.6 Hz, 1H), 5.08 - 4.81 (m, 3H), 4.41 (t, J = 7.9 Hz, 1H), 4.32 (s, 1H), 3.86 (d, J = 9.7 Hz, 1H), 3.85 - 3.72 (m, 1H), 3.71 - 3.59 (m, 1H), 3.54 (d, J = 10.8 Hz, 1H), 3.15 (t, J = 12.5 Hz, 2H), 2.54 (d, J = 3.2 Hz, 3H), 2.49 (s, 3H), 2.46 (d, J = 4.2 Hz, 3H), 2.40 - 2.31 (m, 1H), 2.15 - 2.00 (m, 3H), 1.89 - 1.79 (m, 1H), 1.63 (dd, J = 9.7, 11.7 Hz, 2H), 1.49 (dd, J = 6.9 , 7.0 Hz, 3H), 1.03 (d, J = 6.5 Hz, 3H), 0.87 (d, J = 7.2 Hz, 3H). |
| 82 | (2S,4R)-4-hydroxy-1-((R)-2-(3-(2-((R)-3-(3-(2-hydroxyphenyl)thieno[3,2-c]pyridazin-6-yl)pyrrolidin-1-yl)pyrimidin-5-yl)isoxazol-5-yl)-3-methylbutanoyl)-N-((S)-1-(4-(4-methylthiazol-5-yl)phenyl)ethyl)pyrrolidine-2-carboxamide | 856.35 | 1H NMR (300 MHz, DMSO-d6) δ 12.87 (d, J = 4.4 Hz, 1H), 9.15 (s, 1H), 8.98 (s, 1H), 8.86 (s, 2H), 8.43 (d, J = 7.7 Hz, 1H), 8.01 (d, J = 7.4 Hz, 1H), 7.84 (s, 1H), 7.52 - 7.31 (m, 5H), 7.11 - 6.82 (m, 3H), 5.11 (d, J = 3.6 Hz, 1H), 4.99 - 4.88 (m, 1H), 4.39 (t, J = 7.9 Hz, 1H), 4.30 (s, 1H), 4.25 - 4.04 (m, 2H), 3.92 - 3.63 (m, 5H), 3.50 (d, J = 10.1 Hz, 1H), 2.70 - 2.58 (m, 1H), 2.45 (d, J = 5.7 Hz, 3H), 2.39 - 2.20 (m, 2H), 2.08 - 1.98 (m, 1H), 1.95 - 1.74 (m, 1H), 1.49 (d, J = 6.9 Hz, 1H), 1.37 (d, J = 7.0 Hz, 2H), 1.01 (d, J = 6.5 Hz, 3H), 0.84 (d, J = 6.6 Hz, 3H). |
| 91 | (2S,4R)-4-hydroxy-1-((R)-2-(3-(2-((S)-3-(3-(2-hydroxyphenyl)thieno[3,2-c]pyridazin-6-yl)pyrrolidin-1-yl)pyrimidin-5-yl)isoxazol-5-yl)-3-methylbutanoyl)-N-((S)-1-(4-(4-methylthiazol-5-yl)phenyl)ethyl)pyrrolidine-2-carboxamide | 856.4 | 1H NMR (300 MHz, DMSO-d6) δ 12.89 (s, 1H), 9.19 (s, 1H), 9.08 (s, 1H), 8.87 (s, 2H), 8.45 (d, J = 7.5 Hz, 1H), 8.02 (d, J = 7.9 Hz, 1H), 7.85 (s, 1H), 7.50 - 7.26 (m, 5H), 7.11 - 6.80 (m, 3H), 5.13 (d, J = 3.6 Hz, 1H), 5.01 - 4.88 (m, 1H), 4.39 (t, J = 7.6 Hz, 1H), 4.31 (s, 1H), 4.24 - 4.03 (m, 2H), 3.92 - 3.61 (m, 5H), 3.57 - 3.47 (m, 1H), 2.60 (s, 1H), 2.46 (d, J = 5.4 Hz, 3H), 2.39 - 2.20 (m, 2H), 2.10 - 1.90 (m, 1H), 1.89 - 1.75 (m, 1H), 1.40 (dd, J = 7.1, 6.9 Hz, 3H), 1.02 (d, J = 6.3 Hz, 3H), 0.84 (d, J = 6.7 Hz, 3H). |
| 83 | (2S,4R)-4-hydroxy-1-((R)-2-(3-(2-((R)-3-(3-(2-hydroxyphenyl)thieno[3,2-c]pyridazin-6-yl)pyrrolidin-1-yl)pyrimidin-4-yl)isoxazol-5-yl)-3-methylbutanoyl)-N-((S)-1-(4-(4-methylthiazol-5-yl)phenyl)ethyl)pyrrolidine-2-carboxamide | 856.2 | 1H NMR (400 MHz, DMSO-d6) δ 12.87 (s, 1H), 9.14 (d, J = 6.0 Hz, 1H), 8.98 (s, 1H), 8.58 - 8.39 (m, 2H), 8.00 (dd, J = 8.0, 1.6 Hz, 1H), 7.85 (s, 1H), 7.43 (d, J = 8.0 Hz, 2H), 7.41 - 7.31 (m, 3H), 7.19 (dd, J = 5.0, 2.1 Hz, 1H), 7.06 - 6.97 (m, 2H), 6.93 (s, 1H), 5.12 - 4.70 (m, 2H), 4.48 - 4.05 (m, 4H), 3.98 - 3.71 (m, 5H), 3.55 (dd, J = 28.9, 11.4 Hz, 1H), 2.60 (q, J = 5.8 Hz, 1H), 2.35 (t, J = 7.9 Hz, 3H), 2.28 - 2.20 (m, 1H), 2.16 - 1.88 (m, 2H), 1.79 (ddd, J = 12.8, 8.1, 4.8 Hz, 1H), 1.36 (dd, J = 14.0, 6.8 Hz, 3H), 1.01 (d, J = 6.5 Hz, 3H), 0.85 (d, J = 5.0 Hz, 3H). |
| 86 | (2S,4R)-4-hydroxy-1-((R)-2-(3-(2-((S)-3-(3-(2-hydroxyphenyl)thieno[3,2-c]pyridazin-6-yl)pyrrolidin-1-yl)pyrimidin-4-yl)isoxazol-5-yl)-3-methylbutanoyl)-N-((S)-1-(4-(4-methylthiazol-5-yl)phenyl)ethyl)pyrrolidine-2-carboxamide | 856.2 | 1H NMR (300 MHz, DMSO-d6) δ 12.90 (s, 1H), 9.15 (d, J = 4.8 Hz, 1H), 8.98 (d, J = 5.2 Hz, 1H), 8.55 (d, J = 5.0 Hz, 1H), 8.43 (d, J = 7.7 Hz, 1H), 8.01 (dd, J = 8.1, 1.7 Hz, 1H), 7.86 (s, 1H), 7.48 - 7.37 (m, 5H), 7.20 (d, J = 5.0 Hz, 1H), 7.08 - 6.96 (m, 3H), 5.06 (d, J = 17.5 Hz, 1H), 4.97 - 4.87 (m, 1H), 4.38 (d, J = 8.0 Hz, 1H), 4.16 - 4.05 (m, 3H), 3.87 - 3.78 (m, 5H), 3.52 (d, J = 10.6 Hz, 1H), 2.61 (d, J = 6.3 Hz, 1H), 2.44 (d, J = 11.6 Hz, 3H), 2.36 (t, J = 7.9 Hz, 2H), 2.03 (s, 1H), 1.47 (s, 1H), 1.38 (d, J = 6.9 Hz, 3H), 1.01 (d, J = 6.5 Hz, 3H), 0.83 (t, J = 7.3 Hz, 3H). |

### Preparation of (2S,4R)-4-hydroxy-1-{2-[3-(2-{4-[3-(2-hydroxyphenyl)thieno[3,2-c]pyridazin-6-yl]piperazin-1-yl}pyrimidin-5-yl)-1,2-oxazol-5-yl]3-methylbutanoyl}-N-[(1S)-1-[4-(4-methyl-1,3-thiazol-5-yl)phenyl]ethyl]pyrrolidine-2-carboxamide (Compound 9)

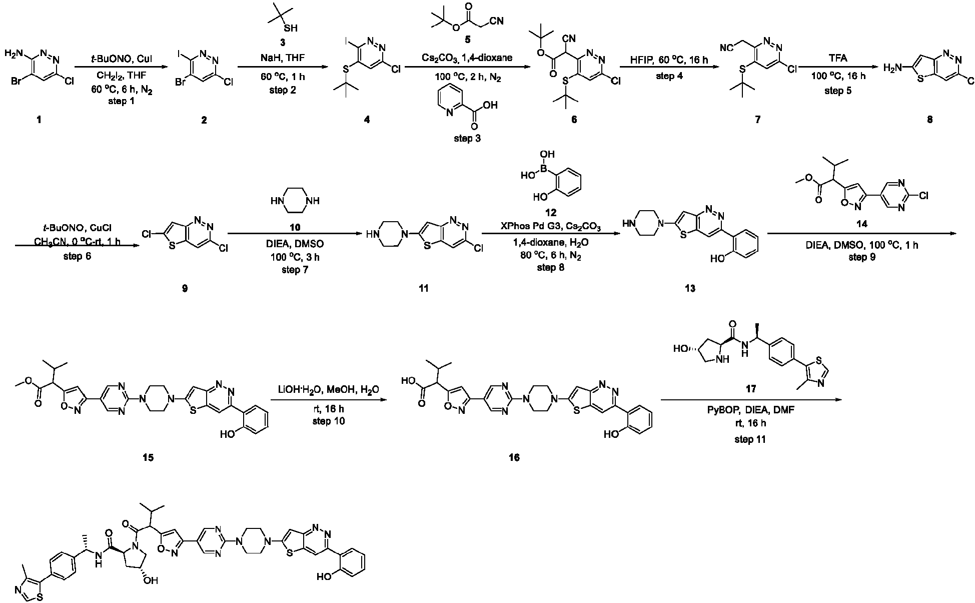

### Step 1: Preparation of 4-bromo-6-chloro-3-iodopyridazine (Intermediate 2).

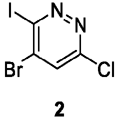

To a stirred solution of 4-bromo-6-chloropyridazin-3-amine (20.00 g, 95.951 mmol, 1.00 equiv) in THF (200.00 mL) was added Cul (21.93 g, 115.141 mmol, 1.20 equiv) and CH₂I₂ (30.84 g, 115.141 mmol, 1.20 equiv) at room temperature. To the above mixture was added *t*-BuNO₂ (11.87 g, 115.141 mmol, 1.20 equiv) at room temperature under nitrogen atmosphere. The resulting mixture was stirred for additional 6 h at 60 °C under nitrogen atmosphere. The mixture was allowed to cool down to room temperature. The reaction was quenched with water at 0 °C. The resulting mixture was extracted with EA (2 x 200 mL). The combined organic layers were washed with saturated brine (1x200 mL), dried over anhydrous Na₂SO₄. After filtration, the filtrate was concentrated under reduced pressure. The residue was purified by silica gel column chromatography, eluted with PE/EA (1/1) to afford Intermediate 2 (15 g, 48.96%) as a yellow solid. LCMS (ESI) m/z: [M+H]⁺ = 319.

### Step 2: Preparation of 4-(tert-butylsulfanyl)-6-chloro-3-iodopyridazine (Intermediate 4).

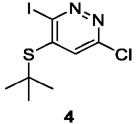

To a To a stirred solution of Intermediate 2 (15.00 g, 46.975 mmol, 1.00 equiv) in THF (100.00 mL) was added 2-methyl-2-propanethiol (5.08 g, 56.370 mmol, 1.20 equiv) and NaH (2.25 g, 93.950 mmol, 2.00 equiv, 60%) at 0 °C. The resulting mixture was stirred for 1 h at 60 °C. The reaction was quenched with water at 0 °C. The resulting mixture was extracted with EA (2 x 200 mL). The combined organic layers were washed with saturated brine (1x200 mL), dried over anhydrous Na₂SO₄. After filtration, the filtrate was concentrated under reduced pressure. The residue was purified by silica gel column chromatography, eluted with PE/EA (1/1) to afford Intermediate 4 (6.4 g, 41.46%) as a yellow solid. LCMS (ESI) m/z: [M+H]⁺ = 329.

### Step 3: Preparation of tert-butyl 2-[4-(tert-butylsulfanyl)-6-chloropyridazin-3-yl]-2-cyanoacetate (Intermediate 6).

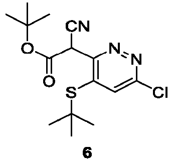

To a stirred solution of Intermediate 4 (6.40 g, 19.477 mmol, 1.00 equiv) and tert-butyl 2-cyanoacetate (5.50 g, 38.954 mmol, 2.00 equiv) in 1,4-dioxane (60.00 mL) was added Cul (0.74 g, 3.895 mmol, 0.20 equiv), picolinic acid (1.20 g, 9.739 mmol, 0.50 equiv) and Cs₂CO₃ (19.04 g, 58.431 mmol, 3.00 equiv) at room temperature. The resulting mixture was stirred for 2 h at 100 °C under nitrogen atmosphere. The mixture was allowed to cool down to room temperature. The residue was purified by reverse flash chromatography with the following conditions: column, C18 silica gel; mobile phase, H₂O (0.1% FA) in ACN, 0 to 100% gradient in 30 min; detector, UV 254/220 nm. This resulted in Intermediate 6 (5 g, 75.10%) as a brown oil. LCMS (ESI) m/z: [M+H]⁺ = 342.

### Step 4: Preparation of 2-[4-(tert-butylsulfanyl)-6-chloropyridazin-3-yl]acetonitrile (Intermediate 7).

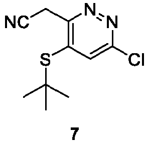

Into a HFIP (50.00 mL) was added Intermediate 6 (5.00 g, 14.626 mmol, 1.00 equiv) at room temperature. The resulting mixture was stirred for 16 h at 60 °C. The residue was allowed to cool down to room temperature. The resulting mixture was concentrated under vacuum. The residue was purified by reverse flash chromatography with the following conditions: column, C18 silica gel; mobile phase, H₂O (0.1% FA) in ACN, 0 to 100% gradient in 30 min; detector, UV 254/220 nm. This resulted in Intermediate 7 (1.2 g, 33.94%) as a brown oil. LCMS (ESI) m/z: [M+H]⁺ = 242.

### Step 5: Preparation of 3-chlorothieno[3,2-c]pyridazin-6-amine (Intermediate 8).

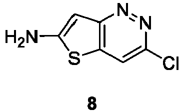

Into a trifluoroacetaldehyde (10.00 mL) was added Intermediate 7 (1.20 g, 4.964 mmol, 1.00 equiv) at room temperature. The resulting mixture was stirred for 16 h at 100 °C. The mixture was allowed to cool down to room temperature. The resulting mixture was concentrated under vacuum. The residue was purified by reverse flash chromatography with the following conditions: column, C18 silica gel; mobile phase, H₂O (10 mmol/L NH₄HCO₃) in ACN, 0 to 100% gradient in 30 min; detector, UV 254/220 nm. This resulted in Intermediate 8 (283 mg, 30.71%) as a yellow solid. LCMS (ESI) m/z: [M+H]⁺ = 186.

### Step 6: Preparation of 3,6-dichlorothieno[3,2-c]pyridazine (Intermediate 9).

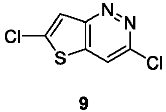

To a stirred solution of Intermediate **8** (283.00 mg, 1.525 mmol, 1.00 equiv) in ACN (10.00 mL) was added CuCl (301.86 mg, 3.050 mmol, 2.00 equiv) at 0 °C. To the above mixture was added t-BuNO₂ (314.42 mg, 3.050 mmol, 2.00 equiv) at 0 °C. The resulting mixture was stirred for additional 1 h at room temperature. The reaction was quenched with water at 0 °C. The resulting mixture was concentrated under reduced pressure. The residue was purified by reverse flash chromatography with the following conditions: column, C18 silica gel; mobile phase, H₂O (0.1% FA) in ACN, 0 to 100% gradient in 30 min; detector, UV 254/220 nm. This resulted in Intermediate 9 (153 mg, 48.94%) as a brown solid. LCMS (ESI) m/z: [M+H]⁺ = 205.

### Step 7: Preparation of 1-{3-chlorothieno[3,2-c]pyridazin-6-yl}piperazine (Intermediate 11).

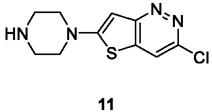

To a stirred solution of Intermediate 9 (153.00 mg, 0.746 mmol, 1.00 equiv) in DMSO (5.00 mL) was added piperazine (192.81 mg, 2.238 mmol, 3.00 equiv) and DIEA (482.17 mg, 3.730 mmol, 5.00 equiv) at room temperature. The resulting mixture was stirred for 1 h at 100 °C. The mixture was allowed to cool down to room temperature. The residue was purified by reverse flash chromatography with the following conditions: column, C18 silica gel; mobile phase, H₂O (0.1% FA) in ACN, 0 to 100% gradient in 30 min; detector, UV 254/220 nm. This resulted in Intermediate 11 (114 mg, 59.98%) as a brown solid. LCMS (ESI) m/z: [M+H]⁺ = 255.

### Step 8: Preparation of 2-[6-(piperazin-1-yl)thieno[3,2-c]pyridazin-3-yl]phenol (Intermediate 13).

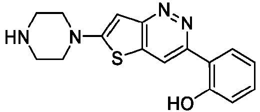

To a stirred solution of Intermediate 11 (114.00 mg, 0.448 mmol, 1.00 equiv) and 2-hydroxyphenylboronic acid (185.18 mg, 1.344 mmol, 3.00 equiv) in 1,4-dioxane (8.00 mL) and H₂O (2.00 mL) was added XPhos Pd G3 (37.88 mg, 0.045 mmol, 0.10 equiv) and Cs₂CO₃ (437.43 mg, 1.344 mmol, 3.00 equiv) at room temperature. The resulting mixture was stirred for 6 h at 80 °C under nitrogen atmosphere. The mixture was allowed to cool down to room temperature. The residue was purified by reverse flash chromatography with the following conditions: column, C18 silica gel; mobile phase, H₂O (0.1% FA) in ACN, 0 to 100% gradient in 30 min; detector, UV 254/220 nm. This resulted in Intermediate 13 (44 mg, 31.47%) as a yellow solid. LCMS (ESI) m/z: [M+H]⁺ = 313.

### Step 9: Preparation of methyl 2-[3-(2-{4-[3-(2-hydroxyphenyl)thieno[3,2-c]pyridazin-6-yljpiperazin-1-yl}pyrimidin-5-yl)-1,2-oxazol-5-yl]-3-methylbutanoate (Intermediate 15).

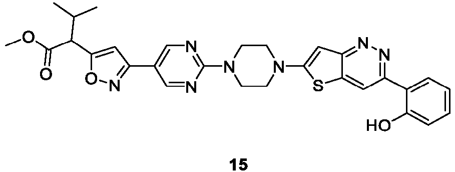

To a stirred solution of Intermediate 13 (44.00 mg, 0.141 mmol, 1.00 equiv) in DMSO (5.00 mL) was added methyl 2-[3-(2-chloropyrimidin-5-yl)-1,2-oxazol-5-yl]-3-methylbutanoate (41.74 mg, 0.141 mmol, 1.00 equiv) and DIEA (54.57 mg, 0.423 mmol, 3.00 equiv) at room temperature. The resulting mixture was stirred for 1 h at 100 °C. The residue was purified by reverse flash chromatography with the following conditions: column, C18 silica gel; mobile phase, H₂O (0.1% FA) in ACN, 0 to 100% gradient in 30 min; detector, UV 254/220 nm. This resulted in Intermediate 15 (19 mg, 23.75%) as a brown solid. LCMS (ESI) m/z: [M+H]⁺ = 572.

### Step 10: Preparation of 2-[3-(2-{4-[3-(2-hydroxyphenyl)thieno[3,2-c]pyridazin-6-yl]piperazin-1-yl}pyrimidin-5-yl)-1,2-oxazol-5-yl]-3-methylbutanoic acid (Intermediate 16).

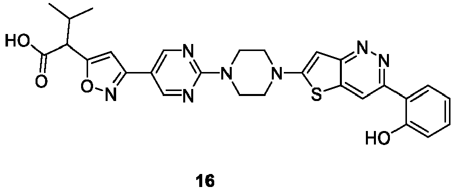

To a stirred solution of Intermediate 15 (19.00 mg, 0.033 mmol, 1.00 equiv) in MeOH (2.00 mL) and H₂O (2.00 mL) was added LiOH·H₂O (14.52 mg, 0.330 mmol, 10.00 equiv) at room temperature. The resulting mixture was stirred for 16 h at room temperature. The mixture was acidified to pH 3 with HCl (1 M). The resulting mixture was concentrated under vacuum. This resulted in Intermediate 16 (20 mg, crude) as a yellow solid. LCMS (ESI) m/z: [M+H]⁺ = 558.

### Step 11: Preparation of (2S,4R)-4-hydroxy-1-{2-[3-(2-{4-[3-(2-hydroxyphenyl)thieno[3,2-c]pyridazin-6-yl]piperazin-1-yl}pyrimidin-5-yl)-1,2-oxazol-5-yl]-3-methylbutanoyl}-N-[(1S)-1-[4-(4-methyl-1,3-thiazol-5-yl)phenyl]ethyl]pyrrolidine-2-carboxamide

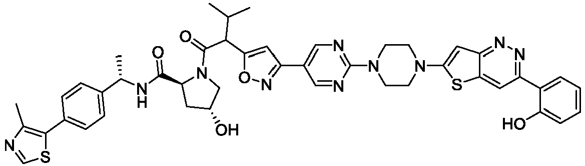

To a stirred solution of Intermediate 16 (20.00 mg, 0.036 mmol, 1.00 equiv) in DMF (1.00 mL) was added PyBOP (55.99 mg, 0.108 mmol, 3.00 equiv) and DIEA (23.18 mg, 0.180 mmol, 5.00 equiv) at room temperature. To the above mixture was added (2S,4R)-4-hydroxy-N-[(1S)-1-[4-(4-methyl-1,3-thiazol-5-yl)phenyl]ethyl]pyrrolidine-2-carboxamide (11.89 mg, 0.036 mmol, 1.00 equiv) at room temperature. The resulting mixture was stirred for additional 16 h at room temperature. The residue was purified by Prep-HPLC with the following conditions: Column: XBridge Prep OBD C18 Column, 30*150 mm, 5µm; Mobile Phase A: Water(10 mmol/L NH₄HCO₃), Mobile Phase B: ACN; Flow rate: 60 mL/min; Gradient: 42% B to 65% B in 9 min, 65% B; Wave Length: 254/220 nm; RT1(min): 8.47; Number Of Runs: 0. This resulted in title compound (0.7 mg, 2.16%) as a yellow solid. ¹H NMR (400 MHz, DMSO-*d*⁶) δ 13.81 - 13.76 (m, 1H), 9.01 - 8.87 (m, 3H), 8.84 (s, 1H), 8.45 - 8.23 (m, 1H), 7.98 - 7.93 (m, 1H), 7.51 - 7.42 (m, 1H), 7.40 - 7.26 (m, 4H), 7.01 - 6.94 (m, 3H), 6.71 (s, 1H), 6.15 - 4.98 (m, 1H), 4.99 - 4.84 (m, 1H), 4.60 - 4.36 (m, 1H), 4.34 - 4.22 (m, 1H), 4.10 - 3.99 (m, 4H), 3.99 - 3.82 (m, 1H), 3.68 - 3.57 (m, 5H), 3.40 - 3.34 (m, 1H), 2.48 - 2.43 (m, 2H), 2.40 (s, 2H), 2.11 - 1.99 (m, 1H), 1.85 - 1.73 (m, 1H), 1.34 (d, *J* = 7.0 Hz, 3H), 1.01 (d, *J* = 6.6 Hz, 3H), 0.86 (d, *J* = 6.6 Hz, 3H). LCMS (ESI) m/z: [M+H]⁺ = 871.20.

### Preparation of (2S,4R)-4-hydroxy-1-[(2R)-2-[3-(4-{5-[3-(2-hydroxyphenyl)-5-methylthieno[2,3-c]pyridazin-6-yl]pyrimidin-2-yl}piperazin-1-yl)-1,2-oxazol-5-yl]-3-methylbutanoyl]-N-[(1S)-1-[4-(4-methyl-1,3-thiazol-5-yl)phenyl]ethyl]pyrrolidine-2-carboxamide (Compound 31)

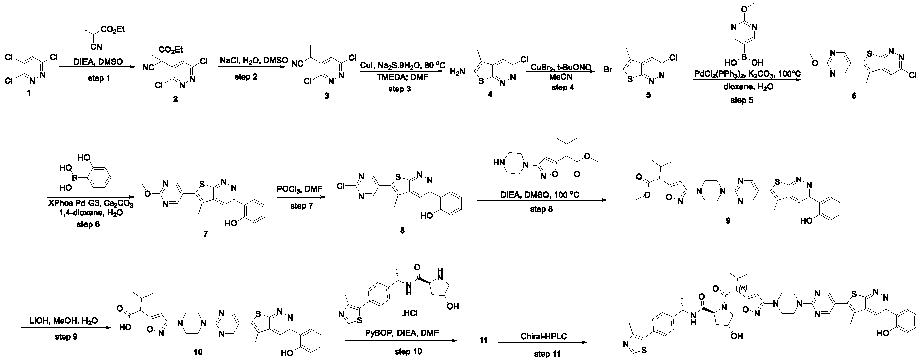

### tep 1: Preparation of ethyl 2-cyano-2-(3,6-dichloropyridazin-4-yl)-2-methylacetate (Intermediate 2).

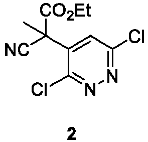

To a solution of 3,4,6-trichloropyridazine (10 g, 54.520 mmol, 1 equiv) and ethyl 2-cyanopropionate (6.93 g, 54.520 mmol, 1 equiv) in DMSO (80 mL) was added DIEA (21.14 g, 163.560 mmol, 3 equiv), the resulting solution was stirred at 100 degrees C for 3 hours. The mixture was diluted with EtOAc (200 mL) and washed with water (200 mL x 3). The organic layer was dried over anhydrous sodium sulfate, filtered and concentrated to give a crude product. The crude product was purified by flash C18 chromatography, elution gradient 0 to 35% ACN in H₂O to give Intermediate **2** (7.3 g, 48.85%) as a brown oil. LCMS (ESI) m/z: [M+H]⁺ = 274.

### Step 2: Preparation of 2-(3,6-dichloropyridazin-4-yl)propanenitrile (Intermediate 3).

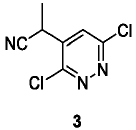

To a solution of Intermediate **2** (7.3 g, 26.633 mmol, 1 equiv) and NaCl (3.11 g, 53.266 mmol, 2 equiv) in DMSO (50 mL) and H₂O (10 mL), the resulting solution was stirred at 120 degrees C for 16 hours. The mixture was diluted with EtOAc (100 mL) and washed with water (100 mL x 3). The organic layer was dried over anhydrous sodium sulfate, filtered and concentrated to give a crude product. The crude product was purified by flash C18 chromatography, elution gradient 0 to 42% ACN in H₂O to give Intermediate 3 (2.8 g, 52.04%) as a brown oil. LCMS (ESI) m/z: [M+H]⁺ = 202.

### Step 3: Preparation of 3-chloro-5-methylthieno[2,3-c]pyridazin-6-amine (Intermediate 4).

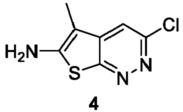

To a solution of Intermediate **3** (2.8 g, 12.374 mmol, 1 equiv) and Cul (235.66 mg, 1.237 mmol, 0.1 equiv) in DMF (25 mL) were added TMEDA (287.59 mg, 2.475 mmol, 0.20 equiv) and Na2S.9H2O (8.92 g, 37.122 mmol, 3 equiv), the resulting solution was stirred at 80 degrees C for 2 hours (under N2 atmosphere). The resulting mixture was filtered, the filter cake was washed with EtOAc (3x20 mL). The filtrate was concentrated under reduced pressure. The crude product was purified by flash C18 chromatography, elution gradient 0 to 42% MeOH in H₂O to give Intermediate **4** (1.1 g, 44.52%) as a yellow solid. LCMS (ESI) m/z: [M+H]⁺ = 200.

### Step 4: Preparation of 6-bromo-3-chloro-5-methylthieno[2,3-c]pyridazine (Intermediate 5).

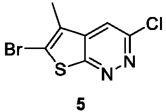

To a solution of Intermediate **4** (1.1 g, 5.509 mmol, 1 equiv) and CuBr2 (1.48 g, 6.611 mmol, 1.2 equiv) in MeCN (10 mL) was added 2-methyl-2-propylnitrit (681.76 mg, 6.611 mmol, 1.2 equiv), the resulting solution was stirred at 25 degrees C for 2 hours. The mixture was diluted with EtOAc (200 mL) and washed with water (200 mL x 3). The organic layer was dried over anhydrous sodium sulfate, filtered and concentrated to give a crude product. The crude product was purified by flash C18 chromatography, elution gradient 0 to 64% ACN in H₂O to give Intermediate **5** (693 mg, 47.73%) as a yellow solid. LCMS (ESI) m/z: [M+H]⁺ = 263.

### Step 5: Preparation of 5-{3-chloro-5-methylthieno[2,3-c]pyridazin-6-yl}-2-methoxypyrimidine (Intermediate 6).

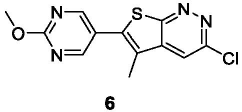

To a solution of Intermediate **5** (693 mg, 2.630 mmol, 1 equiv) and 2-methoxypyrimidin-5-ylboronic acid (404.77 mg, 2.630 mmol, 1 equiv) in dioxane (6 mL) and H₂O (1.5 mL) were added dichloropalladium; bis(triphenylphosphane) (184.57 mg, 0.263 mmol, 0.1 equiv) and K₃PO₄ (1.12 g, 5.260 mmol, 2 equiv), the resulting solution was stirred at 80 degrees C for 16 hours (under N2 atmosphere). The mixture was diluted with EtOAc (100 mL) and washed with water (100 mL x 3). The organic layer was dried over anhydrous sodium sulfate, filtered and concentrated to give a crude product. The crude product was purified by flash C18 chromatography, elution gradient 0 to 67% ACN in H₂O to give Intermediate **6** (253 mg, 32.87%) as a brown solid. LCMS (ESI) m/z: [M+H]⁺ = 293.

### Step 6: Preparation of 2-[6-(2-methoxypyrimidin-5-yl)-5-methylthieno[2,3-c]pyridazin-3-yl]phenol (Intermediate 7).

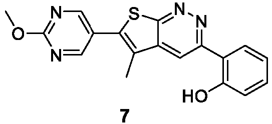

To a solution of Intermediate **6** (271 mg, 0.926 mmol, 1 equiv) and 2-hydroxyphenylboronic acid (191.53 mg, 1.389 mmol, 1.5 equiv) in dioxane (6 mL) and H₂O (1.5 mL) were added XPhos Pd G3 (78.36 mg, 0.093 mmol, 0.1 equiv) and Cs₂CO₃ (603.24 mg, 1.852 mmol, 2 equiv), the resulting solution was stirred at 80 degrees C for 2 hours (under N2 atmosphere). The mixture was diluted with EtOAc (80 mL) and washed with water (80 mL x 3). The organic layer was dried over anhydrous sodium sulfate, filtered and concentrated to give a crude product. The crude product was purified by flash Silica gel column chromatography, elution gradient 0 to 51% EA in PE to give Intermediate **7** (234 mg, 72.14%) as a yellow solid. LCMS (ESI) m/z: [M+H]⁺ = 351.

### Step 7: Preparation of 2-[6-(2-chloropyrimidin-5-yl)-5-methylthieno[2,3-c]pyridazin-3-yl]phenol (Intermediate 8).

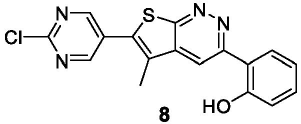

A solution of Intermediate **7**(323 mg, 0.922 mmol, 1 equiv) in POCl₃ (0.5 mL) was added DMF (3 mL) dropwise at 0°C, the resulting solution was stirred at 60 degrees C for 2 hours. The reaction was quenched with Water at 0°C. The mixture was diluted with EtOAc (100 mL) and washed with water (100 mL x 3). The organic layer was dried over anhydrous sodium sulfate, filtered and concentrated to give a crude product. The crude product was purified by flash C18 chromatography, elution gradient 0 to 62% ACN in H₂O to give Intermediate **8** (108 mg, 33.02%) as a yellow solid. LCMS (ESI) m/z: [M+H]⁺ = 355.

### Step 8: Preparation of methyl 2-[3-(4-{5-[3-(2-hydroxyphenyl)-5-methylthieno[2,3-c]pyridazin-6-yl]pyrimidin-2-yl)piperazin-1-yl)-1,2-oxazol-5-yl]-3-methylbutanoate (Intermediate 9).

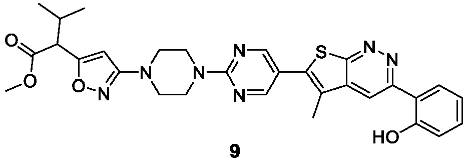

To a solution of Intermediate **8** (103 mg, 0.290 mmol, 1 equiv) and methyl 3-methyl-2-[3-(piperazin-1-yl)-1,2-oxazol-5-yl]butanoate (93.13 mg, 0.348 mmol, 1.2 equiv) in DMSO (3 mL) was added DIEA (112.56 mg, 0.870 mmol, 3 equiv), the resulting solution was stirred at 100 degrees C for 4 hours. The reaction was purified by flash C18 chromatography, elution gradient 0 to 71% ACN in H₂O to give Intermediate **9** (96 mg, 56.46%) as a yellow solid. LCMS (ESI) m/z: [M+H]⁺ = 586.

### Step 9: Preparation of 2-[3-(4-{5-[3-(2-hydroxyphenyl)-5-methylthieno[2,3-c]pyridazin-6-yl]pyrimidin-2-yl}piperazin-1-yl)-1,2-oxazol-5-yl]-3-methylbutanoic acid (Intermediate 10).

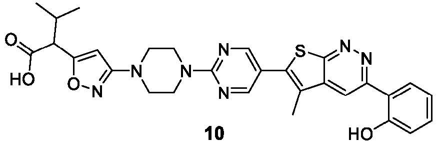

To a solution of methyl Intermediate **9** (91 mg, 0.155 mmol, 1 equiv) and LiOH (7.44 mg, 0.310 mmol, 2 equiv) in MeOH (4 mL) and H₂O (1 mL). The resulting solution was stirred at 25 degrees C for 16 hours. The mixture was diluted with EtOAc (50 mL) and washed with water (50 mL x 3). The organic layer was dried over anhydrous sodium sulfate, filtered and concentrated to give Intermediate 10 (92 mg, crude) that was used in the next step directly without further purification. LCMS (ESI) m/z: [M+H]⁺ = 572.#

### Step 10: Preparation of (2S,4R)-4-hydroxy-1-{2-[3-(4-{5-[3-(2-hydroxyphenyl)-5-methylthieno[2,3-c]pyridazin-6-yl]pyrimidin-2-yl)piperazin-1-yl)-1,2-oxazol-5-yl]-3-methylbutanoyl}-N-[(1S)-1-[4-(4-methyl-1,3-thiazol-5-yl)phenyl]ethyl]pyrrolidine-2-carboxamide (Intermediate 11).

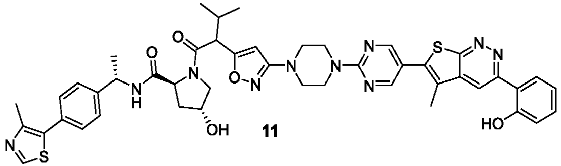

To a solution of Intermediate **10** (96 mg, 0.168 mmol, 1 equiv), (2S,4R)-4-hydroxy-N-[(1S)-1-[4-(4-methyl-1,3-thiazol-5-yl)phenyl]ethyl]pyrrolidine-2-carboxamide (83.49 mg, 0.252 mmol, 1.5 equiv) and PyBOP (174.78 mg, 0.336 mmol, 2 equiv) in DMF (3 mL), the resulting solution was stirred at 25 degrees C for 10 minutes, then DIEA (108.52 mg, 0.840 mmol, 5 equiv) was added to the mixture. The resulting solution was stirred at 25 degrees C for 2 hours. The reaction was purified by flash C18 chromatography, elution gradient 0 to 91% ACN in H₂O to give Intermediate **11** (94 mg, 61.59%) as a yellow solid. LCMS (ESI) m/z: [M+H]⁺ = 885.

### Step 11: Preparation of (2S,4R)-4-hydroxy-1-[(2R)-2-[3-(4-{5-[3-(2-hydroxyphenyl)-5-methylthieno[2,3-c]pyridazin-6-yl]pyrimidin-2-yl)piperazin-1-yl)-1,2-oxazol-5-yl]-3-methylbutanoyl]-N-[(1S)-1-[4-(4-methyl-1,3-thiazol-5-yl)phenyl]ethyl]pyrrolidine-2-carboxamide

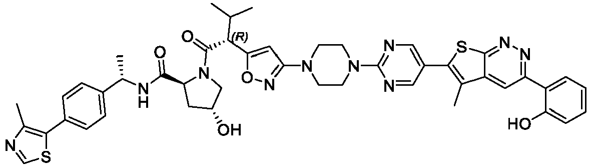

The Intermediate **11** (94 mg) was purified by CHIRAL_HPLC with the following conditions (Column: CHIRALPAK ID, 2*25 cm, 5 µm; Mobile Phase A: MtBE(10mM NH3-MeOH), Mobile Phase B: EtOH--HPLC; Flow rate: 20 mL/min; Gradient: 50% B to 50% B in 25 min; Wave Length: 210/270 nm; RT1(min): 9.5; RT2(min): 17.4; Sample Solvent: MeOH--HPLC; Injection Volume: 1.5 mL; Number Of Runs: 5 to afford title compound (second peak) (33.5 mg, 22.95%) as a yellow solid. The resulting solid was dried by lyophilization. ¹H NMR (300 MHz, DMSO-d6) δ 12.69 (d, J = 3.1 Hz, 1H), 8.99 (s, 1H), 8.78 (d, J = 1.3 Hz, 3H), 8.50 - 8.15 (m, 2H), 7.52 - 7.30 (m, 5H), 7.10 - 6.98 (m, 2H), 6.26 (s, 1H), 5.13 (d, J = 3.6 Hz, 1H), 4.93 (t, J = 7.2 Hz, 1H), 4.39 (t, J = 7.8 Hz, 1H), 4.30 (s, 1H), 3.99 - 3.90 (m, 4H), 3.80 - 3.69 (m, 1H), 3.62 (d, J = 9.8 Hz, 1H), 3.48 - 3.41 (m, 1H), 3.40 - 3.36 (m, 4H), 2.57 (s, 3H), 2.47 (s, 3H), 2.30 - 2.21 (m, 1H), 2.10 - 1.98 (m, 1H), 1.82 - 1.73 (m, 1H), 1.51 - 1.32 (m, 3H), 0.98 (d, J = 6.4 Hz, 3H), 0.90 - 0.79 (m, 3H). LCMS (ESI) m/z: [M+H]⁺ = 885.30.

The compound in Table 6 was prepared using a procedure similar to the one used above for the preparation of (2S,4R)-4-hydroxy-1-[(2R)-2-[3-(4-{5-[3-(2-hydroxyphenyl)-5-methylthieno[2,3-c]pyridazin-6-yl]pyrimidin-2-yl}piperazin-1-yl)-1,2-oxazol-5-yl]-3-methylbutanoyl]-N-[(1S)-1-[4-(4-methyl-1,3-thiazol-5-yl)phenyl]ethyl]pyrrolidine-2-carboxamide (Compound 31) using 2-[6-(2-chloropyrimidin-5-yl)-5-methylthieno[2,3-c]pyridazin-3-yl]phenol and the appropriate amine.

**Table 6.**

| **No.** | **Name** | **LCMS (ESI) m/z** | **¹H NMR** |
|---|---|---|---|
| 32 | (2S,4R)-4-hydroxy-1-((R)-2-(3-(1-(5-(3-(2-hydroxyphenyl)-5-methylthieno[2,3-c]pyridazin-6-yl)pyrimidin-2-yl)piperidin-4-yl)isoxazol-5-yl)-3-methylbutanoyl)-N-((S)-1-(4-(4-methylthiazol-5-yl)phenyl)ethyl)pyrrolidine-2-carboxamide | 884.25 | 1H NMR (400 MHz, Methanol-d4) δ 8.92 (d, J = 14.2 Hz, 1H), 8.79 - 8.57 (m, 3H), 7.98 (d, J = 7.4 Hz, 1H), 7.60 - 7.25 (m, 5H), 7.08 (t, J = 8.2 Hz, 2H), 6.34 (s, 1H), 5.03 (d, J = 6.9 Hz, 1H), 4.50 (t, J = 8.2 Hz, 1H), 4.44 (s, 1H), 3.91 - 3.77 (m, 2H), 3.62 (d, J = 8.9 Hz, 2H), 3.16 (s, 2H), 2.60 (d, J = 3.8 Hz, 3H), 2.47 (d, J = 7.5 Hz, 3H), 2.15 (s, 1H), 2.08 (d, J = 12.9 Hz, 2H), 1.94 (d, J = 18.1 Hz, 1H), 1.74 (d, J = 12.6 Hz, 2H), 1.55 (dd, J = 26.2, 7.0 Hz, 3H), 1.07 (d, J = 6.6 Hz, 3H), 0.96 - 0.79 (m, 3H). |

### Preparation of 2-[5-methyl-6-(piperazin-1-yl)thieno[2,3-c]pyridazin-3-yl]phenol (1-10)

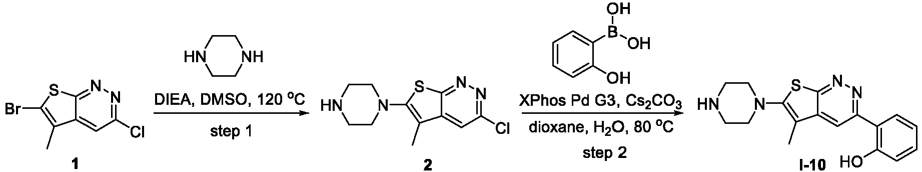

### Step 1: Preparation of 1-{3-chloro-5-methylthieno[2,3-c]pyridazin-6-yl}piperazine (intermediate 2)

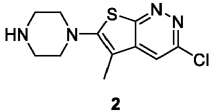

To a solution of 6-bromo-3-chloro-5-methylthieno[2,3-c]pyridazine (160 mg, 0.607 mmol, 1 equiv) and piperazine (104.59 mg, 1.214 mmol, 2 equiv) in DMSO (3 mL) was added DIEA (156.94 mg, 1.214 mmol, 2 equiv), the resulting solution was stirred at 120 degrees C for 2 hours. The residue was purified by flash C18 chromatography, elution gradient 0 to 30% ACN in H₂O to give intermediate 2 (81 mg, 49.64%) as a brown solid. LCMS (ESI) m/z: [M+H]⁺ =269.

### Step 2: Preparation of 2-[5-methyl-6-(piperazin-1-yl)thieno[2,3-c]pyridazin-3-yl]phenol (I-10)

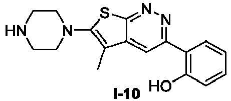

To a solution of intermediate **2** (80 mg, 0.298 mmol, 1 equiv) and 2-hydroxyphenylboronic acid (61.59 mg, 0.447 mmol, 1.5 equiv) in dioxane (2 mL) and H₂O (0.5 mL) were added XPhos Pd G3 (25.20 mg, 0.030 mmol, 0.1 equiv), Cs₂CO₃ (193.97 mg, 0.596 mmol, 2 equiv), the resulting solution was stirred at 80 degrees C for 3 hours. The mixture was diluted with EtOAc (200 mL) and washed with water (200 mL x 3). The organic layer was dried over anhydrous sodium sulfate, filtered and concentrated to give a crude product. The crude product was purified by flash C18 chromatography, elution gradient 0 to 43% ACN in H₂O to give **I-10** (70 mg, 72.04%) as a brown solid. LCMS (ESI) m/z: [M+H]⁺ =327.

### Preparation of (2S,4R)-4-hydroxy-1-[(2R*)-2-{3-[(4-{4-[3-(2-hydroxyphenyl)-5-methylthieno[2,3-c]pyridazin-6-yl]piperidin-1-yl}pyrimidin-2-yl)oxy]-1,2-oxazol-5-yl}-3-methylbutanoyl]-N-[(1S)-1-[4-(4-methyl-1,3-thiazol-5-yl)phenyl]ethyl]pyrrolidine-2-carboxamide (Compound 61)

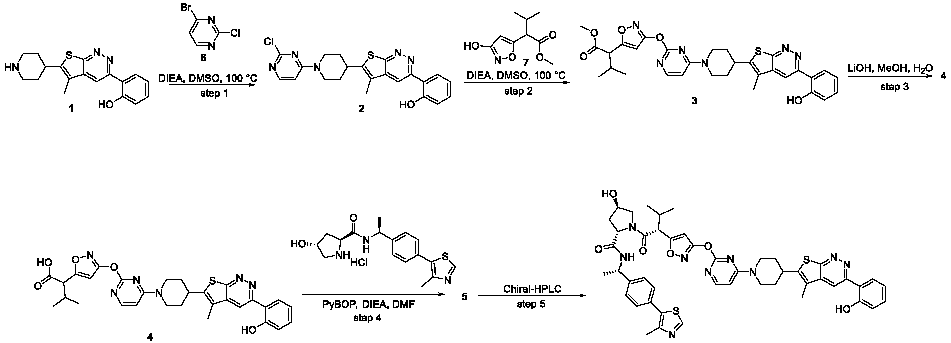

### Step 1: Preparation of 2-{6-[1-(2-chloropyrimidin-4-yl)piperidin-4-yl]-5-methylthieno[2,3-c]pyridazin-3-yl}phenol (Intermediate 2).

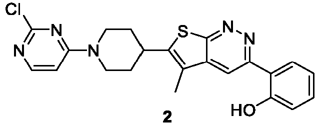

A mixture of 2-[5-methyl-6-(piperidin-4-yl)thieno[2,3-c]pyridazin-3-yl]phenol (700 mg, 2.151 mmol, 1 equiv); DIEA (1.39 g, 10.755 mmol, 5 equiv) and 4-bromo-2-chloropyrimidine (416.07 mg, 2.151 mmol, 1 equiv) in DMF (7 mL) was stirred for 3 h at 100°C. The reaction was monitored by LCMS. Desired product could be detected by LCMS. The residue was purified by reverse flash chromatography with the following conditions: column, silica gel; mobile phase, MeCN in water, 0 to 100% gradient in 25 min; detector, UV 254 nm. to afford Intermediate **2** (356 mg, 37.79%) as a light yellow solid. LCMS (ESI) m/z: [M+H]⁺ = 438.

### Step 2: Preparation of methyl 2-{3-[(4-{4-[3-(2-hydroxyphenyl)-5-methylthieno[2,3-c]pyridazin-6-yl]piperidin-1-yl)pyrimidin-2-yl)oxy]-1,2-oxazol-5-yl}-3-methylbutanoate (Intermediate 3).

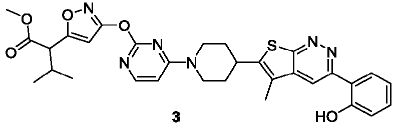

A mixture of Intermediate 2 (336 mg, 0.767 mmol, 1 equiv), DIEA (495.80 mg, 3.835 mmol, 5 equiv) and methyl 2-(3-hydroxy-1,2-oxazol-5-yl)-3-methylbutanoate (458.50 mg, 2.301 mmol, 3 equiv) in DMSO (5 mL) was stirred for 3 h at 100°C. Desired product could be detected by LCMS. The residue was purified by reverse flash chromatography with the following conditions: column, silica gel; mobile phase, MeCN in water, 0 to 100% gradient in 25 min; detector, UV 254 nm. to afford Intermediate 3 (99 mg, 21.48%) as a light yellow solid. LCMS (ESI) m/z: [M+H]⁺ = 601.

### Step 3: Preparation of 2-{3-[(4-{4-[3-(2-hydroxyphenyl)-5-methylthieno[2,3-c]pyridazin-6-yl]piperidin-1-yl}pyrimidin-2-yl)oxy]-1,2-oxazol-5-yl}-3-methylbutanoic acid (Intermediate 4).

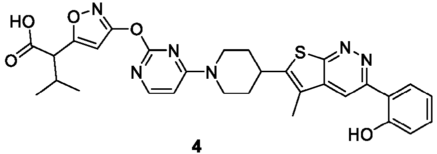

A mixture of Intermediate **3** (94 mg, 0.156 mmol, 1 equiv) and LiOH.H₂O (19.70 mg, 0.468 mmol, 3 equiv) in MeOH (2 mL) and H₂O (1 mL) was stirred for 1 h at room temperature. The reaction was monitored by LCMS. Desired product could be detected by LCMS. The mixture was acidified to pH 6 with HCl (1 mol/L). The residue was purified by reverse flash chromatography with the following conditions: column, silica gel; mobile phase, MeCN in water, 0 to 100% gradient in 30 min; detector, UV 254 nm. to afford Intermediate **4** (51 mg, 55.55%) as a light yellow solid. LCMS (ESI) m/z: [M+H]⁺ = 587.

### Step 4: Preparation of (2S,4R)-4-hydroxy-1-{2-[3-(6-{4-[3-(2-hydroxyphenyl)-5-methylthieno[2,3-c]pyridazin-6-yl]piperidin-1-yl}pyridazin-3-yl)-1,2-oxazol-5-yl]-3-methylbutanoyl}-N-[(1S)-1-[4-(4-methyl-1,3-thiazol-5-yl)phenyl]ethyl]pyrrolidine-2-carboxamide (Intermediate 5).

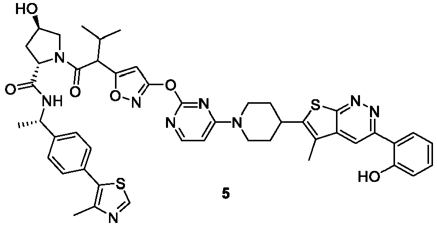

A mixture of Intermediate **4** (51 mg, 0.087 mmol, 1 equiv), PyBOP (67.86 mg, 0.131 mmol, 1.5 equiv); DIEA (0.05 mL, 0.261 mmol, 3 equiv) and (2S,4R)-4-hydroxy-N-[(1S)-1-[4-(4-methyl-1,3-thiazol-5-yl)phenyl]ethyl]pyrrolidine-2-carboxamide (43.22 mg, 0.131 mmol, 1.5 equiv) in DMF (2 mL) was stirred for 2 h at room temperature. The reaction was monitored by LCMS. Desired product could be detected by LCMS. The crude product (41.5mg) was purified by Prep-HPLC with the following conditions (Column: XBridge Shield RP18 OBD Column, 19*150 mm, 5 µm; Mobile Phase A: water (10 mmol/L NH₄HCO₃), Mobile Phase B: ACN; Flow rate: 25 mL/min; Gradient: 55% B to 80% B in 7 min, 80% B; Wave Length: 254/220 nm; RT1(min): 5.18; to afford Intermediate **5** (35 mg, 40.26%) as an off-white solid. The resulting solid was dried by lyophilization. LCMS (ESI) m/z: [M+H]⁺ = 900.

### Step 5: Preparation of 2S,4R)-4-hydroxy-1-[(2R*)-2-{3-[(4-{4-[3-(2-hydroxyphenyl)-5-methylthieno[2,3-c]pyridazin-6-yl]piperidin-1-yl}pyrimidin-2-yl)oxy]-1,2-oxazol-5-yl}-3-methylbutanoyl]-N-[(1S)-1-[4-(4-methyl-1,3-thiazol-5-yl)phenyl]ethyl]pyrrolidine-2-carboxamide

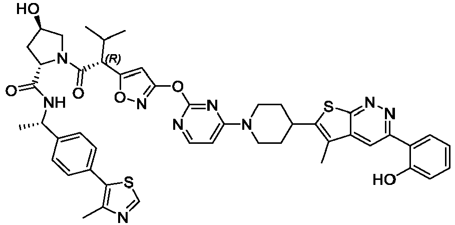

The Intermediate **5** (35 mg) was purified by Chiral-HPLC with the following conditions (Column: CHIRALPAK IE-3, 4.6*50mm, 3µm; Mobile Phase A: MtBE(0.1%DEA): MeOH=70: 30; Flow rate: 1 mL/min; Gradient: 0% B to 0% B; Injection Volume: 5ul mL) to afford title compound (10.8 mg, 30.64%) as an off-white solid. The resulting solid was dried by lyophilization. ¹H NMR (400 MHz, DMSO-d6) δ 12.78 - 12.73 (m, 1H), 9.00 - 8.94 (m, 1H), 8.73 - 8.67 (m, 1H), 8.40 (d, J = 7.7 Hz, 1H), 8.19 - 8.09 (m, 2H), 7.47 - 7.30 (m, 5H), 7.06 - 6.97 (m, 2H), 6.82 - 6.76 (m, 1H), 6.51 (s, 1H), 5.11 (d, J = 3.7 Hz, 1H), 4.88 (t, J = 7.2 Hz, 1H), 4.50 (s, 2H), 4.39 (t, J = 7.8 Hz, 1H), 4.29 (s, 1H), 3.79 (d, J = 9.6 Hz, 1H), 3.76 - 3.54 (m, 2H), 3.53 - 3.46 (m, 1H), 3.13 (s, 2H), 2.50 - 2.41 (m, 5H), 2.33 - 2.23 (m, 2H), 2.06 - 2.01 (m, 3H), 1.84 - 1.73 (m, 1H), 1.68 - 1.60 (m, 2H), 1.48 - 1.28 (m, 3H), 1.02 - 0.95 (m, 3H), 0.90 - 0.81 (m, 3H). LCMS (ESI) m/z: [M+H]⁺ = 900.20.

### Preparation of (2S,4R)-4-hydroxy-1-((R)-2-(3-((2-(4-(3-(2-hydroxyphenyl)-5-methylthieno[2,3-c]pyridazin-6-yl)piperidin-1-yl)pyrimidin-5-yl)oxy)isoxazol-5-yl)-3-methylbutanoyl)-N-((S)-1-(4-(4-methylthiazol-5-yl)phenyl)ethyl)pyrrolidine-2-carboxamide (Compound 51)

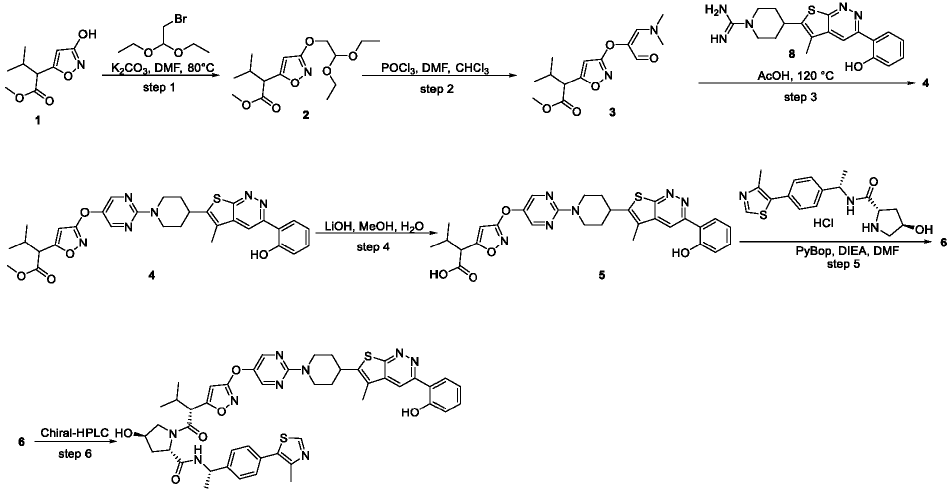

### Step 1: Preparation of methyl 2-(3-(2,2-diethoxyethoxy)isoxazol-5-yl)-3-methylbutanoate (Intermediate 2).

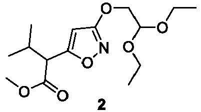

A mixture of methyl 2-(3-hydroxy-1,2-oxazol-5-yl)-3-methylbutanoate (2.00 g, 10.040 mmol, 1.00 equiv), 2-bromo-1,1-diethoxyethane (3.96 g, 20.080 mmol, 2.00 equiv) and K₂CO₃ (4.16 g, 30.120 mmol, 3.00 equiv) in DMF (20 mL) was stirred for overnight at 80 °C. Desired product could be detected by LCMS. The residue was purified by reverse flash chromatography with the following conditions: column, C18 silica gel; mobile phase, MeCN in Water (0.1% FA), 0% to 80% gradient in 10 min; detector, UV 254 and 220 nm. The resulting mixture was concentrated under vacuum to afford intermediate 2 (2.4 g, 75.80%) as an off-white oil. LCMS (ESI) m/z: [M+H]⁺ = 316.

### Step 2: Preparation of methyl (E)-2-(3-((1-(dimethylamino)-3-oxoprop-1-en-2-yl)oxy)isoxazol-5-yl)-3-methylbutanoate (Intermediate 3).

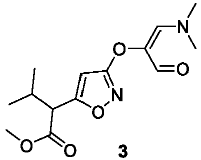

A mixture of POCl₃ (3.40 g, 22.195 mmol, 5.00 equiv) in DMF (1.62 g, 22.195 mmol, 5.00 equiv) was stirred for 45 min at 50 °C under nitrogen atmosphere and cool down to room temperature. To a solution of intermediate **2** (1.4 g, 4.439 mmol, 1.00 equiv) in CHCl₃ (15 mL) was added in above mixture at 78 degrees C. The mixture was stirred for 3 h. The reaction mixture was quenched by KOH (aq). desired product could be detected by LCMS. The resulting mixture was concentrated under reduced pressure. The residue was purified by reverse flash chromatography with the following conditions: column, C18 silica gel; mobile phase, MeCN in Water (10 mmol/L NH₄HCO₃), 0% to 50% gradient in 20 min; detector, UV 254 nm and 220 nm. The resulting mixture was concentrated under vacuum to afford intermediate **3** (335 mg, 28.03%) as an off-white solid. LCMS (ESI) m/z: [M+H]⁺ = 297.

### Step 3: Preparation of methyl 2-(3-((2-(4-(3-(2-hydroxyphenyl)-5-methylthieno[2,3-c]pyridazin-6-yl)piperidin-1-yl)pyrimidin-5-yl)oxy)isoxazol-5-yl)-3-methylbutanoate (Intermediate 4).

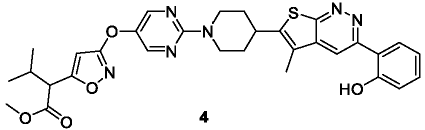

A mixture of intermediate **3** (330.0 mg, 1.337 mmol, 1.00 equiv) and 4-[3-(2-hydroxyphenyl)-5-methylthieno[2,3-c]pyridazin-6-yl]piperidine-1-carboximidamide (736.9 mg, 2.006 mmol, 1.50 equiv) in AcOH (4 mL) was stirred for overnight at 120°C. Desired product could be detected by LCMS. The resulting mixture was concentrated under reduced pressure. The resulting mixture was diluted with DMF (3 mL). The residue was purified by reverse flash chromatography with the following conditions: column, C18 silica gel; mobile phase, MeCN in Water (0.1% FA), 50% to 100% gradient in 10 min; detector, UV 254 and 220 nm. The resulting mixture was concentrated under reduced pressure to afford intermediate **4** (81 mg, 10.09%) as a light yellow solid. LCMS (ESI) m/z: [M+H]⁺ = 601.

### Step 4: Preparation of 2-(3-((2-(4-(3-(2-hydroxyphenyl)-5-methylthieno[2,3-c]pyridazin-6-yl)piperidin-1-yl)pyrimidin-5-yl)oxy)isoxazol-5-yl)-3-methylbutanoic acid (Intermediate 5).

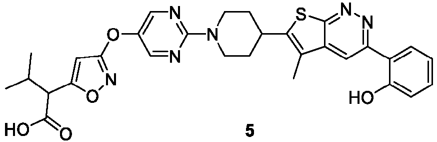

A mixture of intermediate **4** (81.0 mg, 0.135 mmol, 1.00 equiv) and LiOH (9.7 mg, 0.405 mmol, 3.00 equiv) in MeOH (2 mL) and H₂O (2 mL) was stirred for overnight at room temperature. Desired product could be detected by LCMS. The residue was purified by reverse flash chromatography with the following conditions: column, C18 silica gel; mobile phase, MeCN in Water (0.1% FA), 50% to 100% gradient in 20 min; detector, UV 254 and 220 nm. The resulting mixture was concentrated under reduced pressure to afford intermediate **5** (38 mg, 48.03%) as a light yellow solid. LCMS (ESI) m/z: [M+H]⁺ = 587.

### Step 5: Preparation of (2S,4R)-4-hydroxy-1-(2-(3-((2-(4-(3-(2-hydroxyphenyl)-5-methylthieno[2,3-c]pyridazin-6-yl)piperidin-1-yl)pyrimidin-5-yl)oxy)isoxazol-5-yl)-3-methylbutanoyl)-N-((S)-1-(4-(4-methylthiazol-5-yl)phenyl)ethyl)pyrrolidine-2-carboxamide (Intermediate 6).

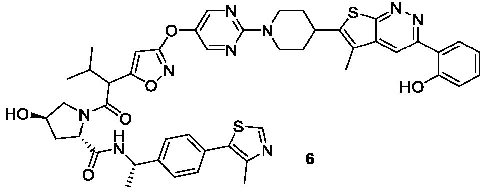

A mixture of intermediate 5 (38.0 mg, 0.065 mmol, 1.00 equiv), PyBop (67.4 mg, 0.130 mmol, 2.00 equiv), DIEA (25.1 mg, 0.195 mmol, 3.00 equiv) and (2S,4R)-4-hydroxy-N-[(1S)-1-[4-(4-methyl-1,3-thiazol-5-yl)phenyl]ethyl]pyrrolidine-2-carboxamide hydrochloride (35.74 mg, 0.098 mmol, 1.5 equiv) in DMF (3 mL) was stirred for 3 h at room temperature. Desired product could be detected by LCMS. The residue was purified by reverse flash chromatography with the following conditions: column, C18 silica gel; mobile phase, MeCN in Water (0.1% FA), 50% to 100% gradient in 20 min; detector, UV 254 and 220 nm. The resulting mixture was concentrated under vacuum to afford intermediate **6** (17 mg, 29.16%) as an off-white solid. LCMS (ESI) m/z: [M+H]⁺ = 900.

### Step 6: Preparation of (2S,4R)-1-[(2R)-2-[3-(1,3-dioxolan-2-ylmethyl)-1,2-oxazol-5-yl]-3-methylbutanoyl]-4-hydroxy-N-[(1S)-1-[4-(4-methyl-1,3-thiazol-5-yl)phenyl]ethylpyrrolidine-2-carboxamide

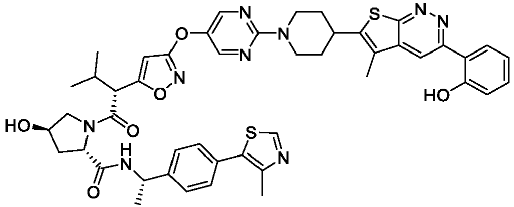

The Intermediate **6** (17.0 mg) was purified by Chiral-HPLC. This resulted in title compound (second peak) (8.6 mg) as a white solid. ¹H NMR (400 MHz, Methanol-d4) δ 8.87 (s, 1H), 8.56 - 8.51 (m, 1H), 8.42 - 8.35 (m, 2H), 8.06 (dd, J = 8.4, 1.8 Hz, 1H), 7.48 - 7.39 (m, 4H), 7.38 - 7.12 (m, 1H), 7.06 - 6.98 (m, 2H), 6.21 (s, 1H), 5.12 - 5.01 (m, 1H), 5.00 - 4.95 (m, 1H), 4.95 - 4.91 (m, 1H), 4.53 (t, J = 8.3 Hz, 1H), 4.48 - 4.39 (m, 1H), 3.84 (dd, J = 10.9, 4.1 Hz, 1H), 3.78 - 3.71 (m, 1H), 3.68 - 3.58 (m, 2H), 3.19 - 3.08 (m, 2H), 2.52 (d, J = 2.9 Hz, 3H), 2.48 (s, 3H), 2.43 - 2.33 (m, 1H), 2.24 - 2.15 (m, 1H), 2.10 (d, J = 12.8 Hz, 2H), 2.00 - 1.92 (m, 1H), 1.84 - 1.70 (m, 2H), 1.54 (dd, J = 13.4, 7.0 Hz, 3H), 1.07 (d, J = 6.6 Hz, 3H), 0.93 (t, J = 6.3 Hz, 3H). LCMS (ESI) m/z: [M+H]⁺ = 900.25.

### Preparation of (2S,4R)-1-[(2R)-2-(3-{6-[5-cyclopropyl-3-(2-hydroxyphenyl)thieno[2,3-c]pyridazin-6-yl]-2-azaspiro[3.3]heptan-2-yl}-1,2-oxazol-5-yl)-3-methylbutanoyl]-4-hydroxy-N-[(1S)-1-[4-(4-methyl-1,3-thiazol-5-yl)phenyl]ethyl]pyrrolidine-2-carboxamide (Compound 87)

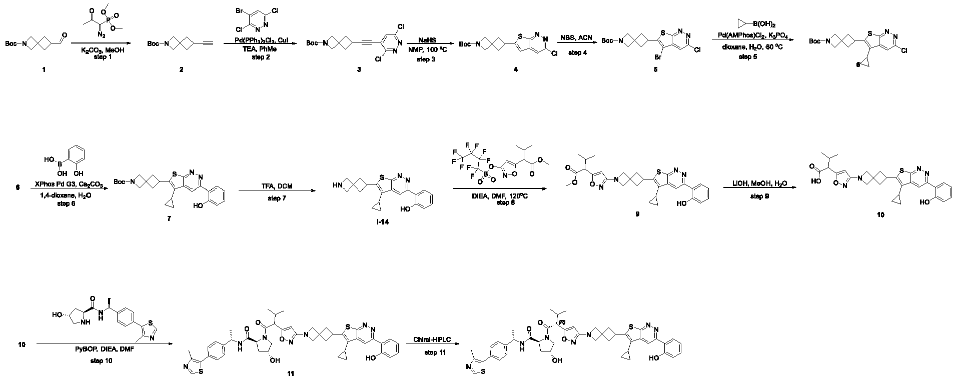

### Step 1: Preparation of tert-butyl 6-ethynyl-2-azaspiro[3.3]heptane-2-carboxylate (Intermediate 2)

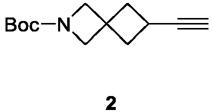

To a stirred mixture of tert-butyl 6-formyl-2-azaspiro[3.3]heptane-2-carboxylate (5 g, 22.194 mmol, 1 equiv) and K₂CO₃ (9.20 g, 66.582 mmol, 3 equiv) in MeOH was added seyferth-gilbert homologation (5.12 g, 26.633 mmol, 1.2 equiv) in portions at 0°C under air atmosphere. The reaction was monitored by LCMS. Desired product could be detected by LCMS. The aqueous layer was extracted with EtOAc (3x100 mL). The resulting solid was dried in an oven under reduced pressure. This resulted in Intermediate 2 (5.0 g, 101.80%) as a brown solid. LCMS (ESI) m/z: [M+H]⁺ = 222.

### Step 2: Preparation of tert-butyl 6-[2-(3,6-dichloropyridazin-4-yl)ethynyl]-2-azaspiro[3.3]heptane-2-carboxylate (Intermediate 3)

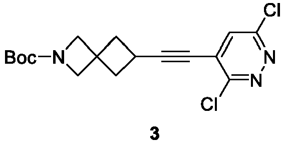

To a stirred mixture of intermediate **2** (3 g, 13.556 mmol, 1 equiv) and 4-bromo-3,6-dichloropyridazine (3.71 g, 16.267 mmol, 1.2 equiv) in toluene (30 mL) were added Cul (1.29 g, 6.778 mmol, 0.5 equiv) and Et₃N (5.65 mL, 40.668 mmol, 3 equiv) dropwise at room temperature under nitrogen atmosphere. The resulting mixture was stirred for additional 2 h at room temperature. Desired product could be detected by LCMS. The resulting mixture was concentrated under reduced pressure. The residue was purified by silica gel column

chromatography, eluted with PE / EA (4:1) to afford intermediate **3** (2.9 g, 58.09%) as a yellow solid. LCMS (ESI) m/z: [M+H]⁺ = 368.

### Step 3: Preparation of tert-butyl 6-{3-chlorothieno[2,3-c]pyridazin-6-yl}-2-azaspiro[3.3]heptane-2-carboxylate (Intermediate 4)

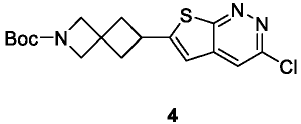

A mixture of intermediate **3** (3 g, 8.146 mmol, 1 equiv) and sodium hydrosulfide (685.03 mg, 12.219 mmol, 1.5 equiv) in NMP (45 mL) was stirred for 15 min at 100 °C under nitrogen atmosphere. The resulting mixture was diluted with EtOAc (150 mL). The resulting mixture was washed with 3x100 mL of brine, dried over anhydrous Na₂SO₄. After filtration, the filtrate was concentrated under reduced pressure. The residue was purified by silica gel column chromatography, eluted with PE / EA (2:1) to afford intermediate **4** (1.5 g, 50.33%) as a red solid. LCMS (ESI) m/z: [M+H]⁺ = 366.

### Step 4: Preparation of tert-butyl 6-{5-bromo-3-chlorothieno[2,3-c]pyridazin-6-yl}-2-azaspiro[3.3]heptane-2-carboxylate (Intermediate 5)

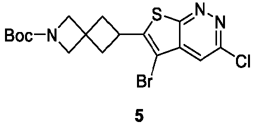

A mixture of intermediate **4** (1.6 g, 4.373 mmol, 1 equiv) and NBS (3.89 g, 21.865 mmol, 5 equiv) in ACN (20 mL) was stirred for 2 h at room temperature under nitrogen atmosphere. The resulting mixture was concentrated under reduced pressure. The residue was purified by silica gel column chromatography, eluted with PE / EA (4:1) to afford intermediate **5** (1.73 g, 88.95%) as a off-white solid. LCMS (ESI) m/z: [M+H]⁺ = 444.

### Step 5: Preparation of tert-butyl 6-{3-chloro-5-cyclopropylthieno[2,3-c]pyridazin-6-yl}-2-azaspiro[3.3]heptane-2-carboxylate (Intermediate 6)

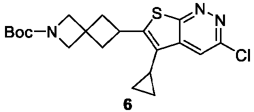

To a stirred mixture of intermediate **5** (1.7 g, 3.822 mmol, 1 equiv) and cyclopropylboronic acid (984.98 mg, 11.466 mmol, 3 equiv) in dioxane (20 mL) and H₂O (2 mL) were added K₃PO₄ (2.43 g, 11.466 mmol, 3 equiv) and Pd(amphos)₂Cl₂ (541.28 mg, 0.764 mmol, 0.2 equiv) in portions at room temperature under nitrogen atmosphere. The resulting mixture was stirred for additional 1 h at 60 °C. The resulting mixture was concentrated under reduced pressure. The residue was purified by silica gel column chromatography, eluted with PE / EA (4:1) to afford intermediate **6** (1.37 g, 88.30%) as a yellow solid. LCMS (ESI) m/z: [M+H]⁺ = 406.

### Step 6: Preparation of tert-butyl 6-[5-cyclopropyl-3-(2-hydroxyphenyl)thieno[2,3-c]pyridazin-6-yl]-2-azaspiro[3.3]heptane-2-carboxylate (Intermediate 7)

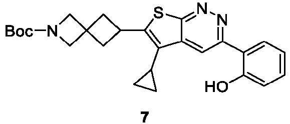

To a stirred mixture of intermediate **6** (1.35 g, 3.326 mmol, 1 equiv) and 2-hydroxyphenylboronic acid (1.38 g, 9.978 mmol, 3 equiv) in dioxane (10 mL) and H₂O (2 mL) were added XPhos Pd G3 (563.00 mg, 0.665 mmol, 0.2 equiv) and Cs₂CO₃ (3.25 g, 9.978 mmol, 3 equiv) in portions at room temperature under nitrogen atmosphere. The resulting mixture was stirred for additional 1 h at 80 °C. The resulting mixture was concentrated under reduced pressure. The residue was purified by silica gel column chromatography, eluted with PE / EA (4:1) to afford intermediate **7** (1 g, 64.86%) as a yellow solid. LCMS (ESI) m/z [M+H]⁺ = 464.

### Step 7: Preparation of 2-(6-{2-azaspiro[3.3]heptan-6-yl}-5-cyclopropylthieno[2,3-c]pyridazin-3-yl)phenol (I-14)

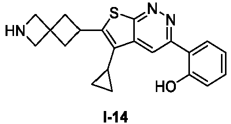

A mixture of intermediate **7** (1.06 g, 2.286 mmol, 1 equiv) in TFA (3 mL) and DCM (9 mL) was stirred for 1 h at room temperature under nitrogen atmosphere. The resulting mixture was concentrated under reduced pressure. The resulting solid was dried by lyophilization. This resulted in 1-14 (800 mg, 96.26%) as a yellow solid. LCMS (ESI) m/z: [M+H]⁺ = 364.

### Step 8: Preparation of methyl 2-(3-{6-[5-cyclopropyl-3-(2-hydroxyphenyl)thieno[2,3-c]pyridazin-6-yl]-2-azaspiro[3.3]heptan-2-yl)-1,2-oxazol-5-yl)-3-methylbutanoate (Intermediate 9)

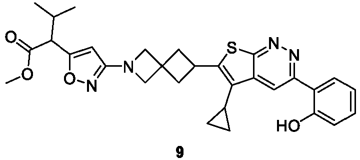

To a stirred mixture of **I-14** (300 mg, 0.825 mmol, 1 equiv) and methyl 3-methyl-2-{3-[(1,1,2,2,3,3,4,4,4-nonafluorobutanesulfonyl)oxy]-1,2-oxazol-5-yl}butanoate (397.24 mg, 0.825 mmol, 1 equiv) in DMF (10 mL) was added DIEA (320.02 mg, 2.475 mmol, 3 equiv) dropwise at 120 °C under nitrogen atmosphere. Desired product could be detected by LCMS. The residue was purified by reverse flash chromatography with the following conditions: column, C18 silica gel; mobile phase, MeCN in Water (0.1% FA), 10% to 90% gradient in 40 min; detector, UV 254 nm.This resulted in intermediate **9** (213 mg, 47.38%) as a reddish solid. LCMS (ESI) m/z [M+H]⁺ = 545.

### Step 9: Preparation of 2-(3-[6-[5-cyclopropyl-3-(2-hydroxyphenyl)thieno[2,3-c]pyridazin-6-yl]-2-azaspiro[3.3]heptan-2-yl)-1,2-oxazol-5-yl)-3-methylbutanoic acid (Intermediate 10)

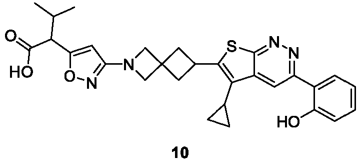

A mixture of intermediate **9** (170 mg, 0.312 mmol, 1 equiv) and LiOH.H₂O (130.96 mg, 3.120 mmol, 10 equiv) in MeOH (2 mL) and H₂O (2 mL) was stirred for 1 h at room temperature under nitrogen atmosphere. The mixture was acidified to pH 6 with HCl (aq.). The resulting mixture was diluted with DCM (20 mL). The resulting mixture was washed with 2x20 mL of water, dried over anhydrous Na₂SO₄. After filtration, the filtrate was concentrated under reduced pressure. This resulted in intermediate **10** (130 mg, 78.49%) as a light brown solid. LCMS (ESI) m/z [M+H]⁺ = 531.

### Step 10: Preparation of (2S,4R)-1-[2-(3-{6-[5-cyclopropyl-3-(2-hydroxyphenyl)thieno[2,3-c]pyridazin-6-yl]-2-azaspiro[3.3]heptan-2-yl}-1,2-oxazol-5-yl)-3-methylbutanoyl]-4-hydroxy-N-[(1S)-1-[4-(4-methyl-1,3-thiazol-5-yl)phenyl]ethyl]pyrrolidine-2-carboxamide (Intermediate 11)

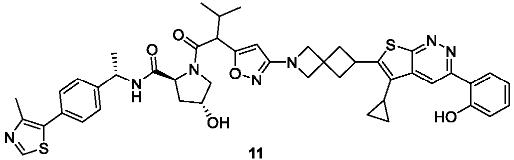

To a stirred mixture of intermediate **10** (131 mg, 0.247 mmol, 1 equiv) and (2S,4R)-4-hydroxy-N-[(1S)-1-[4-(4-methyl-1,3-thiazol-5-yl)phenyl]ethyl]pyrrolidine-2-carboxamide (122.73 mg, 0.370 mmol, 1.5 equiv) in DMF (0.4 mL) were added PyBOP (256.94 mg, 0.494 mmol, 2 equiv) and DIEA (95.72 mg, 0.741 mmol, 3 equiv) in portions at room temperature under nitrogen atmosphere. The residue was purified by reverse flash chromatography with the following conditions: column, C18 silica gel; mobile phase, MeCN in Water (10mmol/L NH₄HCO₃), 10% to 90% gradient in 35 min; detector, UV 254 nm. This resulted in intermediate **11** (75.6 mg, 36.28%) as a white solid. LCMS (ESI) m/z: [M+H]⁺ = 844.

### Step 11: Preparation of (2S,4R)-1-[(2R)-2-(3-{6-[5-cyclopropyl-3-(2-hydroxyphenyl)thieno[2,3-c]pyridazin-6-yl]-2-azaspiro[3.3]heptan-2-yl)-1,2-oxazol-5-yl)-3-methylbutanoyl]-4-hydroxy-N-[(1S)-1-[4-(4-methyl-1,3-thiazol-5-yl)phenyl]ethyl]pyrrolidine-2-carboxamide (Compound 87)

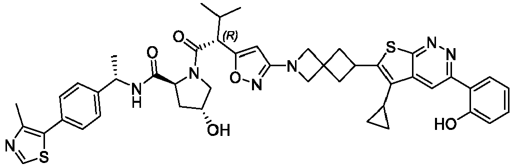

Intermediate **11** was purified by Prep Chiral-HPLC with the following conditions Column: CHIRALPAK ID, 2*25 cm, 5 µm; Mobile Phase A: MtBE(10mM NH₃-MeOH), Mobile Phase B: MeOH--HPLC; Flow rate: 20 mL/min; Gradient: 50% B to 50% B in 14 min; Wave Length: 208/266 nm; RT1(min): 6; RT2(min): 8.705; Sample Solvent: MeOH: DCM=1: 1--HPLC; Injection Volume: 0.45 mL to afford Compound **87** (34.7 mg, 45.20%) as a light-yellow solid. ¹H NMR (400 MHz, DMSO-d6) δ 12.18 (d, *J* = 3.9 Hz, 1H), 9.00 - 8.75 (m, 1H), 8.54 (s, 1H), 8.39 (d, *J* = 7.7 Hz, 1H), 8.09 - 8.02 (m, 1H), 7.49 - 7.32 (m, 5H), 7.02 (dd, *J* = 8.5, 7.4 Hz, 2H), 5.82 (d, *J* = 42.5 Hz, 1H), 5.04 (dd, *J* = 39.8, 3.3 Hz, 1H), 4.91 (q, *J* = 7.2 Hz, 1H), 4.37 (t, *J* = 7.9 Hz, 1H), 4.29 (s, 1H), 4.14 (p, *J* = 8.7 Hz, 1H), 4.06 (s, 2H), 3.88 (s, 2H), 3.71 (dd, *J* = 10.6, 4.5 Hz, 1H), 3.59 (d, *J* = 9.7 Hz, 1H), 3.52 - 3.38 (m, 1H), 2.86 (t, *J* = 10.5 Hz, 2H), 2.46 (d, *J* = 2.9 Hz, 5H), 2.22 (dq, J = 15.7, 6.4 Hz, 1H), 2.02 (t, *J* = 9.1 Hz, 1H), 1.95 - 1.73 (m, 2H), 1.42 (dd, J = 30.7, 7.0 Hz, 3H), 1.13 - 1.02 (m, 2H), 0.96 (t, *J* = 6.4 Hz, 3H), 0.81 (dd, J = 14.3, 6.6 Hz, 3H), 0.71 - 0.63 (m, 2H). LCMS (ESI) m/z: [M+H]⁺ = 844.15.

### Preparation of (2S,4R)-1-((R)-2-(3-(6-(5-cyclopropyl-3-(2-hydroxyphenyl)thieno[2,3-c]pyridazin-6-yl)-2,6-diazaspiro[3.3]heptan-2-yl)isoxazol-5-yl)-3-methylbutanoyl)-4-hydroxy-N-((S)-1-(4-(4-methylthiazol-5-yl)phenyl)ethyl)pyrrolidine-2-carboxamide (Compound 71)

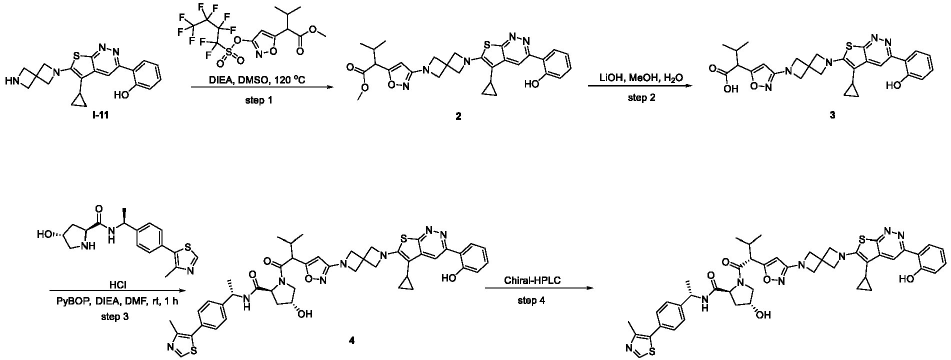

### Step 1: methyl 2-(3-(6-(5-cyclopropyl-3-(2-hydroxyphenyl)thieno[2,3-c]pyridazin-6-yl)-2,6-diazaspiro[3.3]heptan-2-yl)isoxazol-5-yl)-3-methylbutanoate (Intermediate 2).

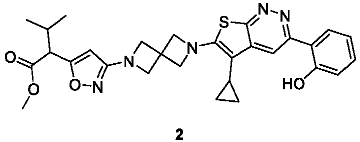

To a solution of **I-11** (200.0 mg, 0.549 mmol, 1 equiv) and methyl 3-methyl-2-{3-[(1,1,2,2,3,3,4,4,4-nonafluorobutanesulfonyl)oxy]-1,2-oxazol-5-yl}butanoate (528.2 mg, 1.098 mmol, 2 equiv) in DMSO (2 mL) was added DIEA (212.77 mg, 1.647 mmol, 3 equiv). After stirring for 2 hrs at 100 degress C, the resulting mixture was concentrated under reduced pressure. The residue was purified by reversed-phase flash chromatography with the following conditions: column, C18 silica gel; mobile phase, ACN in water, 10% to 50% gradient in 10 min; detector, UV 254 nm to afford intermediate 2 (50.0 mg, 15.03%) as a yellow solid. LCMS (ESI) m/z: [M+H]⁺ =546.

### Step 3: Preparation of 2-(3-(6-(5-cyclopropyl-3-(2-hydroxyphenyl)thieno[2,3-c]pyridazin-6-yl)-2,6-diazaspiro[3.3]heptan-2-yl)isoxazol-5-yl)-3-methylbutanoic acid (Intermediate 3).

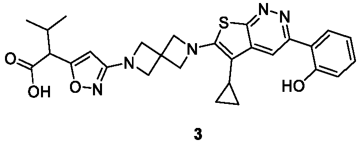

To a solution of intermediate **2** (50.0 mg, 0.092 mmol, 1 equiv) in THF (2 mL) and H₂O (0.5 mL) was added LiOH.H₂O (38.4 mg, 0.920 mmol, 10 equiv). After stirring for 2 hrs at 60 degress C, the mixture was acidified to pH=7 with 1N HCl, the resulting mixture was concentrated under reduced pressure. The residue was purified by reversed-phase flash chromatography with the following conditions: column, C18 silica gel; mobile phase, ACN in water, 10% to 50% gradient in 10 min; detector, UV 254 nm to afford intermediate 3 (45.0 mg, 83.1%) as a yellow solid.LCMS (ESI) m/z: [M+H]⁺ = 532.

### Step 4: Preparation of (2S,4R)-1-(2-(3-(6-(5-cyclopropyl-3-(2-hydroxyphenyl)thieno[2,3-c]pyridazin-6-yl)-2,6-diazaspiro[3.3]heptan-2-yl)isoxazol-5-yl)-3-methylbutanoyl)-4-hydroxy-N-((S)-1-(4-(4-methylthiazol-5-yl)phenyl)ethyl)pyrrolidine-2-carboxamide (Intermediate 4).

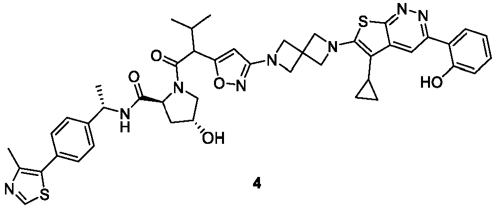

To a solution of intermediate **3** (45.0 mg, 0.085 mmol, 1 equiv) and (2S,4R)-4-hydroxy-N-[(1S)-1-[4-(4-methyl-1,3-thiazol-5-yl) phenyl]ethyl] pyrrolidine-2-carboxamide (42.08 mg, 0.128 mmol, 1.5 equiv) in DMF (1 mL) was added DIEA (32.8 mg, 0.255 mmol, 3 equiv). After stirring for an hour at room temperature, the residue was purified by reversed-phase flash chromatography with the following conditions: column, C₁₈ silica gel; mobile phase, ACN in water, 10% to 50% gradient in 10 min; detector, UV 254 nm to afford intermediate 4 (60.0 mg, 75.4%) as a yellow solid. LCMS (ESI) m/z: [M+H]⁺ = 845.

### Step 5: Preparation of (2S,4R)-1-((R)-2-(3-(6-(5-cyclopropyl-3-(2-hydroxyphenyl)thieno[2,3-c]pyridazin-6-yl)-2,6-diazaspiro[3.3]heptan-2-yl)isoxazol-5-yl)-3-methylbutanoyl)-4-hydroxy-N-((S)-1-(4-(4-methylthiazol-5-yl)phenyl)ethyl)pyrrolidine-2-carboxamide (Compound 71).

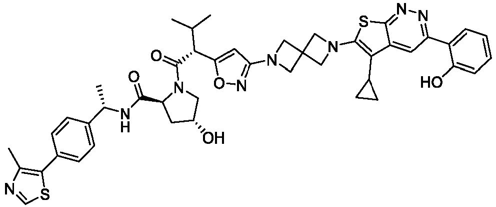

Intermediate **4** (60.0 mg, 0.071 mmol, 1 equiv) was separated by chiral-HPLC with follow conditions: Column: CHIRALPAK ID, 2*25 cm, 5 µm; Mobile Phase A: MtBE(10mM NH3-MeOH), Mobile Phase B: MeOH--HPLC; Flow rate: 20 mL/min; Gradient: 30% B to 30% B in 13 min; Wave Length: 266/210 nm; RT1(min): 6.5; RT2(min): 15; Sample Solvent: MeOH: DCM=1: 2; Injection Volume: 0.38 mL; Number Of Runs: 4 to afford compound 71 (23.5 mg, 39.0%) as a light-yellow solid. LCMS (ESI) m/z: [M+H]⁺ = 845.30 . ¹H NMR (400 MHz, DMSO-d6) δ 13.59 (s, 1H), 9.02 - 8.97 (m, 1H), 8.42 (d, J = 7.7 Hz, 1H), 8.03 (d, J = 8.8 Hz, 2H), 7.51 - 7.41 (m, 2H), 7.41 - 7.30 (m, 3H), 7.02 - 6.94 (m, 2H), 5.92 (s, 1H), 5.12 (d, J = 3.5 Hz, 1H), 4.92 (t, J = 7.2 Hz, 1H), 4.61 (s, 4H), 4.36 (t, J = 7.9 Hz, 1H), 4.29 (s, 1H), 4.13 (s, 4H), 3.71 (dd, J = 10.6, 4.4 Hz, 1H), 3.60 (d, J = 9.7 Hz, 1H), 3.43 (d, J = 11.0 Hz, 1H), 2.46 (s, 3H), 2.27 - 2.17 (m, 1H), 2.05 - 1.98 (m, 1H), 1.84 - 1.72 (m, 2H), 1.49 - 1.35 (m, 3H), 1.03 - 0.92 (m, 5H), 0.86 - 0.76 (m, 3H), 0.58 - 0.52 (m, 2H).

Compounds in Table 7 were prepared using procedures similar to the one used above for the preparation of (2S,4R)-1-((R)-2-(3-(6-(5-cyclopropyl-3-(2-hydroxyphenyl)thieno[2,3-c]pyridazin-6-yl)-2,6-diazaspiro[3.3]heptan-2-yl)isoxazol-5-yl)-3-methylbutanoyl)-4-hydroxy-N-((S)-1-(4-(4-methylthiazol-5-yl)phenyl)ethyl)pyrrolidine-2-carboxamide (Compound 71) using the appropriate amines.

**Table 7.**

| **No.** | **Name** | **LCMS (ESI) m/z** | **¹H NMR** |
|---|---|---|---|
| **68** | (2S,4R)-4-hydroxy-1-((R)-2-(3-(6-(3-(2-hydroxyphenyl)thieno[2,3-c]pyridazin-6-yl)-2,6-diazaspiro[3.3]heptan-2-yl)isoxazol-5-yl)-3-methylbutanoyl)-N-((S)-1-(4-(4-methylthiazol-5-yl)phenyl)ethyl)pyrrolidine-2-carboxamide | 805.1 | 1H NMR (400 MHz, DMSO-d6) δ 13.69 (s, 1H), 8.99 (s, 1H), 8.41 (d, J = 7.7 Hz, 1H), 8.19 (s, 1H), 8.01 - 7.94 (m, 1H), 7.51 - 7.41 (m, 2H), 7.45 - 7.29 (m, 3H), 7.00 - 6.92 (m, 2H), 5.92 (d, J = 8.0 Hz, 2H), 5.11 (d, J = 3.5 Hz, 1H), 4.96 - 4.86 (m, 1H), 4.45 - 4.33 (m, 4H), 4.32 - 4.25 (m, 1H), 4.12 (s, 4H), 3.71 (dd, J = 10.6, 4.5 Hz, 1H), 3.60 (d, J = 9.8 Hz, 1H), 3.43 (d, J = 10.8 Hz, 1H), 3.30 (s, 1H), 2.47 - 2.45 (m, 3H), 2.26 - 2.14 (m, 1H), 2.06 - 1.98 (m, 1H), 1.84 - 1.73 (m, 1H), 1.38 (d, J = 7.0 Hz, 3H), 0.99 - 0.93 (m, 3H), 0.86 - 0.76 (m, 3H). |
| **79** | (2S,4R)-4-hydroxy-1-((R)-2-(3-(6-(3-(2-hydroxyphenyl)-5-methylthieno[2,3-c]pyridazin-6-yl)-2,6-diazaspiro[3.3]heptan-2-yl)isoxazol-5-yl)-3-methylbutanoyl)-N-((S)-1-(4-(4-methylthiazol-5-yl)phenyl)ethyl)pyrrolidine-2-carboxamide | 819.35 | 1H NMR (300 MHz, Methanol-d4) δ 8.88 (d, J = 2.8 Hz, 1H), 8.02 - 7.92 (m, 2H), 7.48 - 7.34 (m, 4H), 7.33 - 7.18 (m, 2H), 7.02 - 6.93 (m, 2H), 5.92 (s, 1H), 4.53 (s, 4H), 4.44 (s, 1H), 4.18 (s, 3H), 3.87 - 3.80 (m, 1H), 3.68 - 3.56 (m, 2H), 2.48 (d, J = 2.1 Hz, 3H), 2.38 (s, 1H), 2.26 (s, 3H), 2.18 (s, 1H), 1.98 (dd, J = 8.7, 4.4 Hz, 1H), 1.59 - 1.47 (m, 3H), 1.05 (d, J = 6.6 Hz, 3H), 0.89 (d, J = 6.7 Hz, 3H). |
| **80** | (2S,4R)-4-hydroxy-1-((R)-2-(3-(4-(3-(2-hydroxyphenyl)-5-methylthieno[2,3-c]pyridazin-6-yl)piperazin-1-yl)isoxazol-5-yl)-3-methylbutanoyl)-N-((S)-1-(4-(4-methylthiazol-5-yl)phenyl)ethyl)pyrrolidine-2-carboxamide | 807.25 | 1H NMR (400 MHz, DMSO-d6) δ 13.41 - 13.35 (m, 1H), 8.99 (s, 1H), 8.47 (s, 1H), 8.42 (d, J = 7.6 Hz, 1H), 8.18 (d, J = 8.0 Hz, 1H), 7.50 - 7.41 (m, 2H), 7.41 - 7.32 (m, 3H), 7.03 - 6.96 (m, 2H), 6.26 (s, 1H), 5.12 (d, J = 3.7 Hz, 1H), 4.96 - 4.88 (m, 1H), 4.42 - 4.34 (m, 1H), 4.30 (s, 1H), 3.77 - 3.70 (m, 1H), 3.62 (d, J = 9.9 Hz, 1H),3.46 - 3.34 (m, 9H), 2.46 (s, 3H), 2.36 (s, 3H), 2.27 - 2.22 (m, 1H), 2.05 - 2.01 (m, 1H), 1.47 (d, J = 7.0 Hz, 1H), 1.39 (d, J = 7.1 Hz, 3H), 1.02 - 0.94 (m, 3H), 0.88 - 0.79 (m, 3H). |
| **67** | (2S,4R)-4-hydroxy-1-(2-(3-(6-(3-(2-hydroxyphenyl)thieno[3,2-c]pyridazin-6-yl)-2,6-diazaspiro[3.3]heptan-2-yl)isoxazol-5-yl)-3-methylbutanoyl)-N-((S)-1-(4-(4-methylthiazol-5-yl)phenyl)ethyl)pyrrolidine-2-carboxamide | 805.25 | 1H NMR (400 MHz, DMSO-d6) δ 9.02 - 8.96 (m, 1H), 8.88 (s, 1H), 8.40 (d, J = 7.7 Hz, 1H), 7.96 - 7.89 (m, 1H), 7.52 - 7.26 (m, 5H), 6.97 (t, J = 7.6 Hz, 2H), 6.28 (d, J = 4.1 Hz, 1H), 5.92 (d, J = 6.9 Hz, 1H), 5.10 (dd, J = 3.7, 1.8 Hz, 1H), 4.96 - 4.88 (m, 1H), 4.45 - 4.24 (m, 6H), 4.10 (d, J = 11.9 Hz, 4H), 3.73 - 3.41 (m, 3H), 2.46 (d, J = 2.7 Hz, 5H), 2.03 (d, J = 8.4 Hz, 1H), 1.85 - 1.74 (m, 1H), 1.46 (d, J = 7.0 Hz, 1H), 1.37 (t, J = 7.3 Hz, 2H), 0.99 - 0.92 (m, 3H), 0.87 - 0.74 (m, 3H). |

### Preparation of (2S,4R)-4-hydroxy-1-((R)-2-(3-(2-((4-(3-(2-hydroxyphenyl)thieno[2,3-c]pyridazin-6-yl)piperidin-1-yl)methyl)pyrimidin-5-yl)isoxazol-5-yl)-3-methylbutanoyl)-N-((S)-1-(4-(4-methylthiazol-5-yl)phenyl)ethyl)pyrrolidine-2-carboxamide (Compound 99)

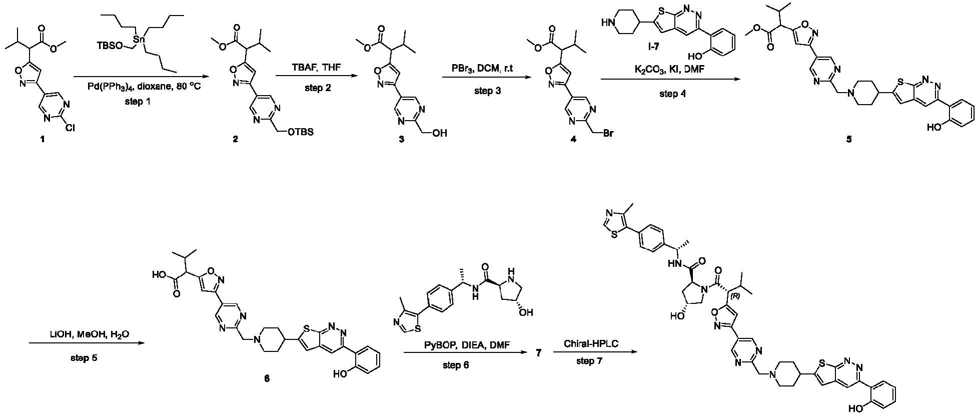

### Step 1: Preparation of methyl 2-[3-(2-{[(tert-butyldimethylsilyl)oxy]methyl}pyrimidin-5-yl)-1,2-oxazol-5-yl]-3-methylbutanoate (Intermediate 2)

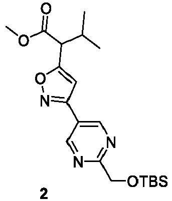

To a stirred solution of methyl 2-[3-(2-chloropyrimidin-5-yl)-1,2-oxazol-5-yl]-3-methylbutanoate (1.0 g, 3.382 mmol, 1 equiv) and tert-butyldimethyl[(tributylstannyl)methoxy]silane (2.94 g, 6.764 mmol, 2.0 equiv) in dioxane (10 mL) was added Pd(PPh₃)₄ (0.39 g, 0.338 mmol, 0.1 equiv) in portions at room temperature under nitrogen atmosphere. The resulting mixture was stirred for 2h at 80 °C under nitrogen atmosphere. The residue was purified by silica gel column chromatography, eluted with PE / EA (2:1) to afford Intermediate 2 (450 mg, 32.81%) as a light yellow solid. LCMS (ESI) m/z: [M+H]⁺ = 406.

### Step 2: Preparation of methyl 2-{3-[2-(hydroxymethyl)pyrimidin-5-yl]-1,2-oxazol-5-yl}-3-methylbutanoate (Intermediate 3)

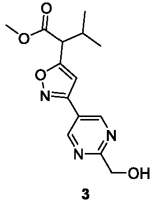

To a stirred solution of intermediate 2 (450 mg, 1.110 mmol, 1 equiv) in THF (10 mL) was added TBAF (870.32 mg, 3.330 mmol, 3.0 equiv) at room temperature. The resulting mixture was stirred for 1h at room temperature. The resulting mixture was concentrated under reduced pressure. The residue was purified by silica gel column chromatography, eluted with PE / EA (1:1) to afford intermediate 3 (230 mg, 71.16%) as an off-white solid. LCMS (ESI) m/z [M+H]⁺ = 292.

### Step 3: Preparation of methyl 2-{3-[2-(bromomethyl)pyrimidin-5-yl}-1,2-oxazol-5-yl}-3-methylbutanoate (Intermediate 4)

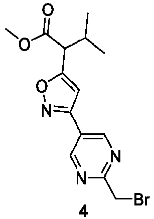

To a stirred solution of intermediate 3 (230 mg, 0.790 mmol, 1 equiv) in DCM (10 mL) was added PBr₃ (641.16 mg, 2.370 mmol, 3.0 equiv) at room temperature. The resulting mixture was stirred for 2h at room temperature. The residue was purified by silica gel column chromatography, eluted with PE / EA (2:1) to afford intermediate 4 (125 mg, 44.70%) as an off-white solid. LCMS (ESI) m/z [M+H]⁺ = 354/356.

### Step 4: Preparation of methyl 2-{3-[2-({4-[3-(2-hydroxyphenyl)thieno[2,3-c]pyridazin-6-yl]piperidin-1-yl}methyl)pyrimidin-5-yl]-1,2-oxazol-5-yl}-3-methylbutanoate (Intermediate 5)

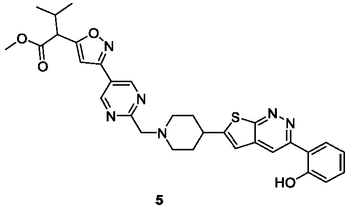

To a stirred solution of intermediate 4 (125 mg, 0.353 mmol, 1 equiv) and I-7 (131.87 mg, 0.424 mmol, 1.2 equiv) in DMF (5 mL) were added K₂CO₃ (146.32 mg, 1.059 mmol, 3.0 equiv) and KI (29.29 mg, 0.176 mmol, 0.5 equiv) at room temperature under nitrogen atmosphere. The resulting mixture was stirred for 1h at 60 °C under nitrogen atmosphere. The residue was purified by reversed-phase flash chromatography with the following conditions: column, C18 silica gel; mobile phase, MeCN in Water (0.1% FA), 0% to 100% gradient in 30 min; detector, UV 254 nm. This resulted in intermediate 5 (80 mg, 38.77%) as an off-white solid. LCMS (ESI) m/z [M+H]⁺ = 585.

### Step 5: Preparation of 2-{3-[2-({4-[3-(2-hydroxyphenyl)thieno[2,3-c]pyridazin-6-yl]piperidin-1-yl}methyl)pyrimidin-5-yl]-1,2-oxazol-5-yl}-3-methylbutanoic acid (Intermediate 6)

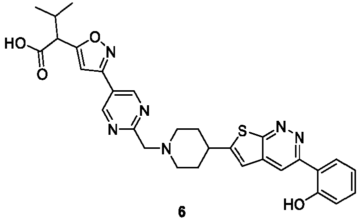

To a stirred solution of intermediate 5 (80 mg, 0.137 mmol, 1 equiv) in MeOH (5 mL) and H₂O (2 mL) was added LiOH (16.38 mg, 0.685 mmol, 5.0 equiv) at room temperature. The resulting mixture was stirred for 2h at room temperature. The mixture was acidified to pH 5 with HCl (aq.). The resulting mixture was extracted with EtOAc (3 x 20 mL). The combined organic layers were washed with brine (1x30 mL), dried over anhydrous Na₂SO₄. After filtration, the filtrate was concentrated under reduced pressure. This resulted in intermediate 6 (100 mg, crude) as an off-white solid. LCMS (ESI) m/z [M+H]⁺ = 571.

### Step 6: Preparation of (2S,4R)-4-hydroxy-1-(2-{3-[2-({4-[3-(2-hydroxyphenyl)thieno[2,3-c]pyridazin-6-yl]piperidin-1-yl}methyl)pyrimidin-5-yl]-1,2-oxazol-5-yl}-3-methylbutanoyl)-N-[(1S)-1-[4-(4-methyl-1,3-thiazol-5-yl)phenyl]ethyl]pyrrolidine-2-carboxamide (Intermediate 7)

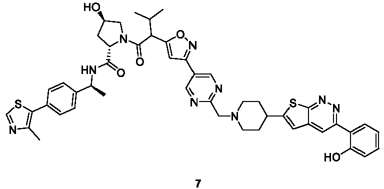

To a stirred solution of intermediate 6 (100 mg, 0.175 mmol, 1 equiv) and (2S,4R)-4-hydroxy-N-[(1S)-1-[4-(4-methyl-1,3-thiazol-5-yl) phenyl] ethyl] pyrrolidine-2-carboxamide (69.69 mg, 0.210 mmol, 1.2 equiv) in DMF (2.5 mL) were added PyBOP (182.38 mg, 0.350 mmol, 2.0 equiv) and DIEA (67.94 mg, 0.525 mmol, 3.0 equiv) at room temperature. The resulting mixture was stirred for 1 h at room temperature under nitrogen atmosphere. The residue was purified by reversed-phase flash chromatography with the following conditions: column, C18 silica gel; mobile phase, MeCN in Water (0.1% FA), 0% to 100% gradient in 25 min; detector, UV 254 nm. This resulted in intermediate 7 (10 mg, 6.45%) as an off-white solid. LCMS (ESI) m/z [M+H]⁺ = 884.

### Step 7: Preparation of (2S,4R)-4-hydroxy-1-((R)-2-(3-(2-((4-(3-(2-hydroxyphenyl)thieno[2,3-c]pyridazin-6-yl)piperidin-1-yl)methyl)pyrimidin-5-yl)isoxazol-5-yl)-3-methylbutanoyl)-N-((S)-1-(4-(4-methylthiazol-5-yl)phenyl)ethyl)pyrrolidine-2-carboxamide (Compound 99)

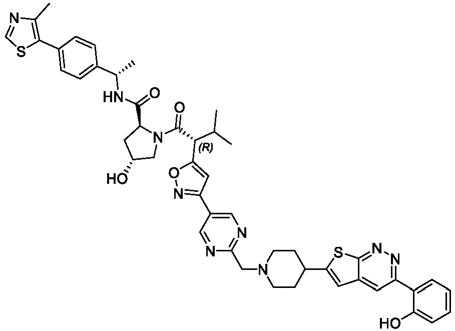

Intermediate 7 (10 mg) was purified by Chiral-Prep-HPLC with the following conditions: Column, CHIRALPAK ID, 2*25 cm, 5 um; mobile phase, MTBE:DCM=1:1(10 mMNH₃) and MeOH- (hold 50% MeOH- in 16 min); Detector, UV 254 nm. This resulted in Compound 99 (3.8 mg, 37.62%) (second peak) as an off-white solid. ¹H NMR (400 MHz, Methanol-d4) δ 9.13 (s, 2H), 8.76 (d, J = 6.1 Hz, 1H), 8.50 (s, 1H), 7.90 - 7.83 (m, 1H), 7.38 - 7.19 (m, 6H), 6.95 - 6.85 (m, 3H), 4.95 (d, J = 7.1 Hz, 1H), 4.44 (t, J = 8.2 Hz, 1H), 4.37 (s, 1H), 3.84 (s, 3H), 3.79 (dd, J = 11.0, 4.2 Hz, 1H), 3.72 - 3.52 (m, 3H), 3.11 = 2.97 (m, 3H), 2.47 - 2.30 (m, 4H), 2.15 - 2.02 (m, 3H), 1.98 - 1.81 (m, 3H), 1.43 (d, J = 7.0 Hz, 3H), 1.02 (d, J = 6.6 Hz, 3H), 0.84 (d, J = 6.6 Hz, 3H). LCMS (ESI) m/z: [M+H]⁺ = 884.40.

### Preparation of (2S,4R)-4-hydroxy-1-[(2R)-2-{3-[1-(2-{4-[3-(2-hydroxyphenyl)-5-methylthieno[2,3-c]pyridazin-6-yl]piperidin-1-yl}pyrimidin-5-yl)piperidin-4-yl]-1,2-oxazol-5-yl}-3-methylbutanoyl]-N-[(1S)-1-[4-(4-methyl-1,3-thiazol-5-yl)phenyl]ethyl]pyrrolidine-2-carboxamide (Compound 100)

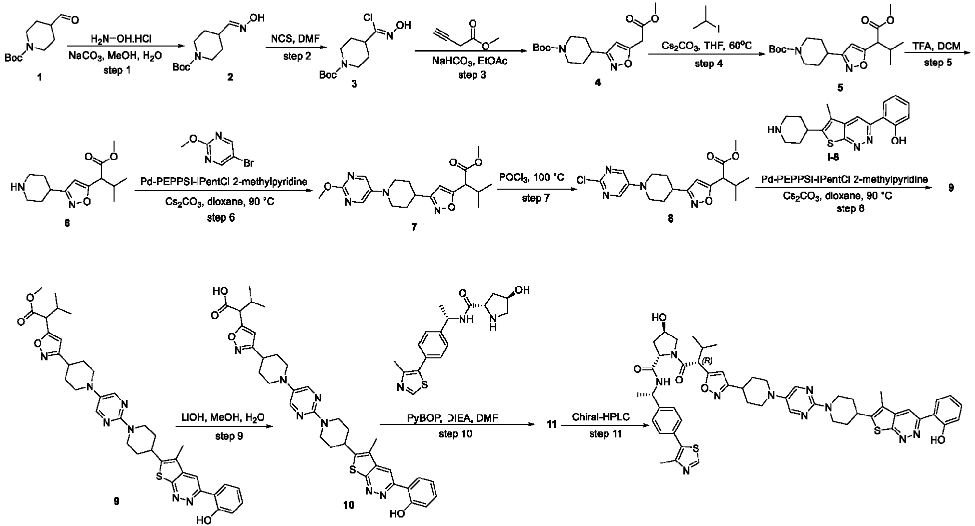

### Step 1: Preparation of tert-butyl 4-[(1E)-(hydroxyimino)methyl]piperidine-1-carboxylate (intermediate 2)

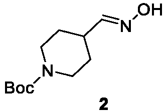

To a solution of tert-butyl 4-formylpiperidine-1-carboxylate (10 g, 46.887 mmol, 1 equiv) and hydroxylamine hydrochloride (6.52 g, 93.774 mmol, 2 equiv) in methanol (30 mL) were added Na₂CO₃ (14.91 g, 140.661 mmol, 3 equiv) and water (30 mL). The resulting mixture was stirred overnight at room temperature. Desired product could be detected by LCMS. The mixture was diluted with ethyl acetate (2000 mL) and washed with water (1000 mL x 3). The organic layer was dried over anhydrous sodium sulfate, filtered and concentrated to give intermediate 2 (10.2 g, crude) as a white solid. LCMS (ESI) m/z: [M+H]⁺ =229.

### Step 2: Preparation of tert-butyl 4-[(1Z)-chloro(hydroxyimino)methyl]piperidine-1-carboxylate (intermediate 3)

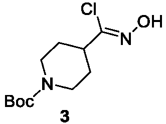

To a solution of intermediate 2 (10.2 g, 44.680 mmol, 1 equiv) in DMF (50 mL) was added NCS (8.95 g, 67.020 mmol, 1.5 equiv). The resulting solution was stirred at 25 degrees C for 2h. Desired product could be detected by LCMS. The resulting mixture was diluted with ethyl acetate (2000 mL) and washed with saturated brine (2 x 1000 mL). The organic layer was dried over anhydrous Na₂SO₄. After filtration, the filtrate was concentrated under reduced pressure. This resulted in intermediate 3 (14 g, crude) as a white solid. LCMS (ESI) m/z: [M+H]⁺ =263.

### Step 3: Preparation of tert-butyl 4-[5-(2-methoxy-2-oxoethyl)-1,2-oxazol-3-yl] piperidine-1-carboxylate (intermediate 4)

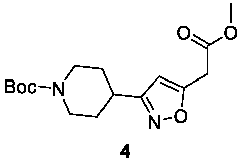

To a solution of intermediate 3 (14 g, 53.287 mmol, 1 equiv), methyl but-3-ynoate (5.23 g, 53.287 mmol, 1 equiv) in ethyl acetate (60 mL) was added NaHCO₃ (13.43 g, 159.861 mmol, 3 equiv). The resulting solution was stirred at 25 degrees C for overnight. Desired product could be detected by LCMS. The resulting mixture was diluted with EtOAc (2000 mL) and washed with saturated brine (2x1000 mL). The organic layer was dried over anhydrous Na₂SO₄. After filtration, the filtrate was concentrated under reduced pressure. The residue was purified by reverse flash chromatography with the following conditions: column, C18 silica gel; mobile phase, acetonitrile in water, 0 to 33% gradient in 30 min; detector, UV 254/220 nm. This resulted in intermediate 4 (7 g, 40.50%) as yellow oil. LCMS (ESI) m/z: [M+H]⁺ =325.

### Step 4: Preparation of tert-butyl 4-[5-(1-methoxy-3-methyl-1-oxobutan-2-yl)-1,2-oxazol-3-yl]piperidine-1-carboxylate (intermediate 5)

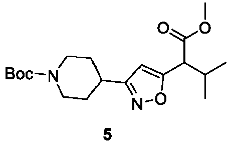

To a solution of intermediate 4 (7 g, 21.580 mmol, 1 equiv) in THF (70 mL, 863.994 mmol, 40.04 equiv) was added Cs₂CO₃ (14.06 g, 43.160 mmol, 2 equiv) and 2-iodopropane (7.34 g, 43.160 mmol, 2 equiv. The resulting solution was stirred at 60 degrees C for overnight. Desired product could be detected by LCMS. The mixture was diluted with ethyl acetate (500 mL) and washed with water (1000 mL x 3). The organic layer was dried over anhydrous sodium sulfate, filtered and concentrated to give a crude product. The crude product was purified by reverse flash chromatography with the following conditions: column, C18 silica gel; mobile phase, acetonitrile in water, 0% to 66% gradient in 30 min to give intermediate **5** (5.8 g, 73.34%) as a yellow oil. LCMS (ESI) m/z: [M+H]⁺ =367.

### Step 5: Preparation of methyl 3-methyl-2-[3-(piperidin-4-yl)-1,2-oxazol-5-yl] butanoate (intermediate 6)

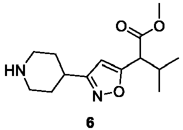

To a solution of intermediate **5** (5.8 g, 15.827 mmol, 1 equiv) in DCM (9 mL) was added TFA (3 mL). The resulting solution was stirred at 25 degrees C for 2h. Desired product could be detected by LCMS. After filtration, the filtrate was concentrated under reduced pressure. This resulted in intermediate 6 (8.3 g, crude). LCMS (ESI) m/z: [M+H]⁺ =267.

### Step 6: Preparation of methyl 2-{3-[1-(2-methoxypyrimidin-5-yl) piperidin-4-yl]-1,2-oxazol-5-yl}-3-methylbutanoate (intermediate 7)

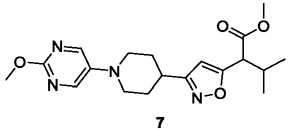

To a solution of intermediate **6** (3 g, 11.264 mmol, 1 equiv), 5-bromo-2-methoxypyrimidine (4.26 g, 22.528 mmol, 2 equiv) in dioxane (30 mL) were added Pd-PEPPSI-IPentCI 2-methylpyridine (o-picoline) (947 mg, 1.123 mmol, 0.1 equiv) and Cs₂CO₃ (7.34 g, 22.528 mmol, 2 equiv). The resulting solution was stirred at 90 degrees C for 2h. Desired product could be detected by LCMS. The mixture was diluted with ethyl acetate (300 mL) and washed with water (300 mL x 3). The organic layer was dried over anhydrous sodium sulfate, filtered and concentrated to give a crude product. The crude product was purified by flash C18 chromatography, elution gradient 0 to 22% petroleum ether in ethyl acetate to give intermediate 7 (800 mg, 18.97%) as a white solid. LCMS (ESI) m/z: [M+H]⁺ =375.

### Step 7: Preparation of methyl 2-{3-[1-(2-chloropyrimidin-5-yl) piperidin-4-yl]-1,2-oxazol-5-yl}-3-methylbutanoate (intermediate 8)

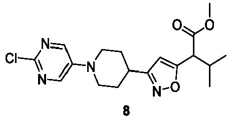

A solution of intermediate 7 (800 mg, 2.13 mmol, 1 equiv) in POCl₃ (8 mL) was stirred at 100 degrees C for 18h. Desired product could be detected by LCMS. The reaction mixture was quenched with water (200 mL) and concentrated under reduced pressure. The residue was purified by reverse flash chromatography with the following conditions: column, C18 silica gel; mobile phase, acetonitrile in water, 0% to 66% gradient in 30 min; detector, UV 254 nm to give intermediate 8 (400 mg, 27.45%) as a yellow solid. LCMS (ESI) m/z: [M+H]⁺ =379.

### Step 8: Preparation of methyl 2-{3-[1-(2-{4-[3-(2-hydroxyphenyl)-5-methylthieno[2,3-c] pyridazin-6-yl]piperidin-1-yl}pyrimidin-5-yl)piperidin-4-yl]-1,2-oxazol-5-yl}-3-methylbutanoate (intermediate 9)

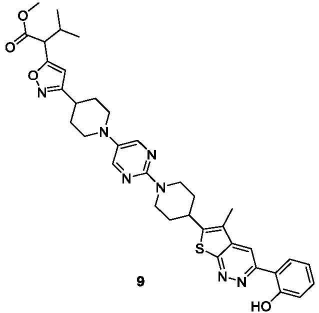

To a solution of intermediate **8** (400 mg, 1.056 mmol, 1 equiv) in and **I-8** (515.38 mg, 1.584 mmol, 1.5 equiv) in dioxane (5 mL) were added Cs₂CO₃ (688.00 mg, 2.112 mmol, 2 equiv) and Pd-PEPPSI-IPentCI 2-methylpyridine (o-picoline) (177.62 mg, 0.211 mmol, 0.2 equiv). The resulting solution was stirred at 90 degrees C overnight. Desired product could be detected by LCMS. The mixture was diluted with ethyl acetate (200 mL) and washed with water (200 mL x 3). The organic layer was dried over anhydrous sodium sulfate, filtered and concentrated to give a crude product. The crude product was purified by flash C18 chromatography, elution gradient 0 to 55% petroleum ether in ethyl acetate to give intermediate **9** (470 mg, 66.66%) as a white solid. LCMS (ESI) m/z: [M+H]⁺ =668.

### Step 9: Preparation of 2-{3-[1-(2-{ 4-[3-(2-hydroxyphenyl)-5-methylthieno[2, 3-c]pyridazin-6-yl]piperidin-1-yl}pyrimidin-5-yl)piperidin-4-yl]-1,2-oxazol-5-yl}-3-methylbutanoic acid (intermediate 10)

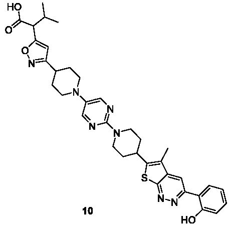

To a solution of intermediate 9 (470 mg, 0.704 mmol, 1 equiv) in methanol (4 mL), THF (4 mL) and water (2 mL) was added LiOH (84.28 mg, 3.520 mmol, 5 equiv). The resulting solution was stirred at 25 degrees C for 2h. Desired product could be detected by LCMS. The mixture was acidified to pH 5 with HCl (aq, 1 mol/L). The resulting mixture was extracted with ethyl acetate (2 x 200 mL). The combined organic layers were dried over anhydrous Na₂SO₄. After filtration, the filtrate was concentrated under reduced pressure. This resulted in intermediate **10** (600 mg, crude) as a yellow solid. LCMS (ESI) m/z: [M+H]⁺ =654.

### Step 10: Preparation of (2S,4R)-4-hydroxy-1-[(2)-2-{3-[1-(2-{4-[3-(2-hydroxyphenyl)-5-methylthieno[2,3-c] pyridazin-6-yl] piperidin-1-yl} pyrimidin-5-yl) piperidin-4-yl]-1,2-oxazol-5-yl}-3-methylbutanoyl]-N-[(1S)-1-[4-(4-methyl-1,3-thiazol-5-yl)phenyl]ethyl]pyrrolidine-2-carboxamide (intermediate 11)

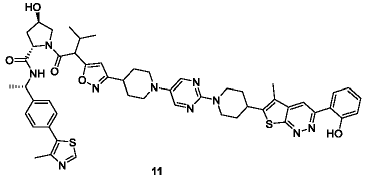

To a solution of intermediate **10** (300 mg, 0.459 mmol, 1 equiv), (2S,4R)-4-hydroxy-N-[(1S)-1-[4-(4-methyl-1,3-thiazol-5-yl)phenyl]ethyl]pyrrolidine-2-carboxamide (304.16 mg, 0.918 mmol, 2 equiv) in DMF (3 mL) were added PyBOP (477.58 mg, 0.918 mmol, 2 equiv) and DIEA (296.53 mg, 2.295 mmol, 5 equiv). The resulting solution was stirred at 25 degrees C for 2h. Desired product could be detected by LCMS. The reaction mixture was purified by reverse flash chromatography with the following conditions: Column: YMC-Actus Triart C18 ExRS, 30*150 mm, 5µm; Mobile Phase A: Water (10 mmol/L NH₄HCO₃), Mobile Phase B: ACN; Flow rate: 60 mL/min; Gradient: 60% B to 83% B in 8 min; Wave Length: 254/220 nm; RT1(min): 8.92. The result in intermediate **11** (136.0 mg, 9.58%) as white solid. LCMS (ESI) m/z: [M+H]⁺ =967.

### Step 11: Preparation of (2S,4R)-4-hydroxy-1-[(2R)-2-{3-[1-(2-{4-[3-(2-hydroxyphenyl)-5-methylthieno[2,3-c] pyridazin-6-yl] piperidin-1-yl} pyrimidin-5-yl) piperidin-4-yl]-1,2-oxazol-5-yl}-3-methylbutanoyl]-N-[(1S)-1-[4-(4-methyl-1,3-thiazol-5-yl)phenyl]ethyl]pyrrolidine-2-carboxamide (Compound 100).

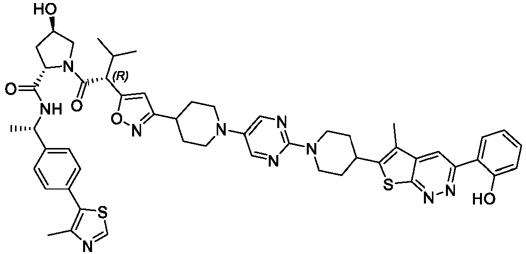

Intermediate 11 (136 mg) was purified by Chiral-Prep-HPLC with the following conditions: Column: CHIRAL ART Cellulose-SB, 4.6*100mm, 3.0um; Mobile Phase A: (MtBE: Hex=1: 1) (0.1%DEA): EtOH=50: 50; Flow rate: 1 mL/min; Gradient: isocratic; Injection Volume: 5ul mL. Compound 100 (42.5 mg, 51.62%) was obtained as a white solid. ¹H NMR (400 MHz, DMSO-d6) δ 12.78 (d, J = 2.9 Hz, 1H), 8.98 (s, 1H), 8.70 (s, 1H), 8.40 (d, J = 7.7 Hz, 1H), 8.25 (s, 2H), 8.20 - 8.13 (m, 1H), 7.50 - 7.41 (m, 2H), 7.37 (dd, J = 8.7, 2.3 Hz, 3H), 7.02 (dd, J = 7.3, 6.5, 1.2 Hz, 2H), 6.37 (s, 1H), 5.09 (d, J = 3.7 Hz, 1H), 4.91 (q, J = 7.1 Hz, 1H), 4.74 (d, J = 13.1 Hz, 2H), 4.38 (t, J = 7.9 Hz, 1H), 4.30 (s, 1H), 3.74 (dd, J = 15.9, 8.0 Hz, 2H), 3.65 - 3.44 (m, 4H), 3.02 (t, J = 12.3 Hz, 2H), 2.87 - 2.71 (m, 3H), 2.65 - 2.54 (m, 3H), 2.46 (s, 3H), 2.23 (s, 1H), 2.02 (d, J = 13.3 Hz, 5H), 1.97 (s, 3H), 1.78 (tt, J = 12.1, 7.1 Hz, 2H), 1.69 - 1.56 (m, 3H), 0.99 (dd, J = 6.5, 4.0 Hz, 3H), 0.81 (dd, J = 11.2, 6.7 Hz, 3H). LCMS (ESI) m/z: [M+H]⁺ =967.25.

### Example 2. Degradation of BRM and BRG1 by Compounds of the Invention

This example demonstrates the ability of the compounds of the disclosure to degrade a HiBit-BRM or HiBit-BRG1 fusion protein in a cell-based degradation assay.

**Procedure:** A stable HeLa cell line expressing HiBiT-BRM was generated. On day 0, 5000 cells were seeded in 40 µL of media into each well of 384-well cell culture plates. On day 1, cells were treated with 120 nL DMSO or 120 nL of 3-fold serially DMSO-diluted compounds (10 points in duplicate with 30 µM as final top dose). Subsequently plates were incubated for 24 h in a standard tissue culture incubator and equilibrated at room temperature for 15 minutes. Nano-Glo HiBiT Lytic Detection System (Promega N3050) reagent was freshly prepared and 20 ul was added to each well. Upon addition of this LgBit-containing reagent, the HiBiT and LgBiT proteins associate to form the luminescent NanoBiT luciferase. The plates were shaken for 10 minutes at room temperature and the bioluminescence read using an EnVision plate reader (PerkinElmer).

For measurement of BRG1 degradation, a stable HeLa cell line expressing HiBit-BRG1 and LgBit was generated. The same protocol as above was then followed.

The degradation% was calculated using the following formula: % degradation = 100%-100% x (Lum_{Sample} - Lum_{LC}) / (Lum_{HC} -Lum_{LC}). DMSO treated cells are employed as High Control (HC) and 2 µM of a known BRM/BRG1 degrader standard treated cells are employed as Low Control (LC). The data was fit to a four parameter, non-linear curve fit to calculate IC₅₀ (µM) values as shown in Table 8.

**Results:** As shown in Table 8 below, the compounds of the invention degraded both BRM and BRG1.

**Table 8.**

| **Compound No.** | **BRM HiBit Degradation IC₅₀ (nM)** | **BRM HiBit Degradation Maximum (%)** | **BRMG1 HiBit Degradation IC₅₀ (nM)** | **BRG1 HiBit Degradation Maximum (%)** |
|---|---|---|---|---|
| 1 | ++++ | A | +++ | A |
| 2 | +++ | A | + | C |
| 3 | +++ | A | + | C |
| 4 | ++++ | A | + | C |
| 5 | ++++ | A | + | C |
| 6 | ++++ | A | + | C |
| 7 | + | C | + | C |
| 8 | +++ | A | + | C |
| 9 | ++++ | A | +++ | A |
| 10 | +++ | A | +++ | A |
| 11 | ++++ | A | +++ | B |
| 12 | ++++ | A | ++ | B |
| 13 | + | C | + | C |
| 14 | ++++ | A | +++ | B |
| 15 | ++ | B | + | C |
| 16 | ++++ | A | +++ | B |
| 17 | ++++ | A | +++ | A |
| 18 | ++++ | A | + | C |
| 19 | + | 2 | + | C |
| 20 | +++ | A | + | C |
| 21 | +++ | A | ++ | B |
| 22 | ++++ | A | + | C |
| 23 | ++++ | A | + | C |
| 24 | +++ | A | ++ | B |
| 25 | +++ | A | + | C |
| 26 | ++++ | A | + | C |
| 27 | ++++ | A | + | C |
| 28 | ++++ | A | + | C |
| 29 | + | C | + | C |
| 30 | ++++ | A | + | C |
| 31 | +++ | B | + | C |
| 32 | + | C | + | C |
| 33 | +++ | A | + | C |
| 34 | ++++ | A | + | C |
| 35 | ++++ | A | + | C |
| 36 | +++ | A | + | C |
| 37 | ++ | A | + | B |
| 38 | ++++ | A | + | C |
| 39 | +++ | A | + | C |
| 40 | ++++ | A | ++ | C |
| 41 | ++++ | A | + | C |
| 42 | ++++ | A | +++ | B |
| 43 | ++++ | A | + | C |
| 44 | ++++ | A | +++ | A |
| 45 | +++ | A | + | C |
| 46 | ++++ | A | + | C |
| 47 | +++ | A | ++++ | A |
| 48 | +++ | A | + | C |
| 49 | +++ | B | + | C |
| 50 | +++ | A | + | C |
| 51 | +++ | A | + | C |
| 52 | +++ | B | + | C |
| 53 | ++++ | A | +++ | A |
| 54 | ++++ | A | + | C |
| 55 | ++++ | A | + | C |
| 56 | +++ | B | + | C |
| 57 | ++++ | A | + | C |
| 58 | ++++ | A | +++ | A |
| 59 | ++++ | A | + | C |
| 60 | ++++ | A | +++ | B |
| 61 | + | C | + | C |
| 62 | + | C | + | C |
| 63 | ++++ | A | + | C |
| 64 | ++++ | A | +++ | A |
| 65 | +++ | A | + | C |
| 66 | ++++ | A | + | C |
| 67 | ++++ | A | NC | C |
| 68 | ++++ | A | NC | C |
| 69 | ++++ | A | ++++ | A |
| 70 | ++++ | B | NC | C |
| 71 | ++++ | A | NC | C |
| 72 | ++++ | A | NC | C |
| 73 | ++++ | A | NC | C |
| 74 | ++++ | A | +++ | B |
| 75 | +++ | B | NC | C |
| 76 | ++++ | A | NC | C |
| 77 | ++++ | A | ++ | B |
| 78 | ++++ | A | +++ | B |
| 79 | ++++ | A | NC | B |
| 80 | +++ | A | NC | C |
| 81 | +++ | A | NC | C |
| 82 | ++++ | A | +++ | B |
| 83 | +++ | A | NC | C |
| 84 | +++ | B | NC | C |
| 85 | +++ | A | NC | C |
| 86 | ++ | A | NC | C |
| 87 | ++++ | A | NC | C |
| 88 | ++++ | A | +++ | A |
| 89 | ++++ | A | +++ | A |
| 90 | ++++ | A | NC | C |
| 91 | ++++ | A | NC | B |
| 92 | ++++ | A | +++ | A |
| 93 | ++++ | A | NC | B |
| 94 | +++ | A | NC | C |
| 95 | ++++ | A | NC | C |
| 96 | +++ | B | NC | C |
| 97 | +++ | A | NC | C |
| 98 | +++ | A | ++ | A |
| 99 | +++ | B | NC | C |
| 100 | ++++ | A | +++ | B |
| 101 | +++ | A | NC | C |
| 102 | ++++ | A | NC | C |
| 103 | ++++ | A | NC | C |

| | | | | |
|---|---|---|---|---|
| "+" indicates inhibitory effect of ≥ 1000 nM; "++" indicates inhibitory effect of ≥ 100 nM; "+++" indicates inhibitory effect of ≥ 10 nM; "++++" indicates inhibitory effect of < 10 nM; "NT" indicates not tested; "A" indicates maximum degradation ≥ 75%; "B" indicates maximum degradation ≥ 50%; and "C" indicates maximum degradation < 50%. | | | | |

### Other Embodiments

In an embodiment of the compound or salt of the invention at least one of f, h, i, and k is 1.

**In** an embodiment of compound or salt of the invention each of B¹, B², B³, and B⁴ is, independently, O, ethynyl, optionally substituted C₂-C₉ heteroaryl, optionally substituted C₂-C₉ heterocyclyl, optionally substituted C₃-C₁₀ cycloalkyl, or optionally substituted C₆-C₁₀ aryl.

In an embodiment of the compound or salt of the invention each of B¹ and B⁴ is, independently,

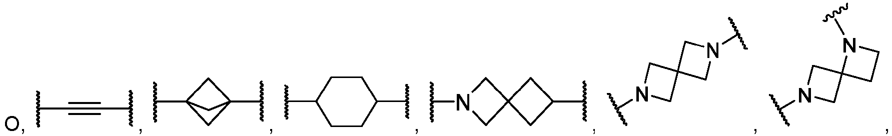

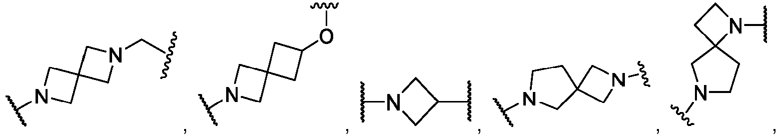

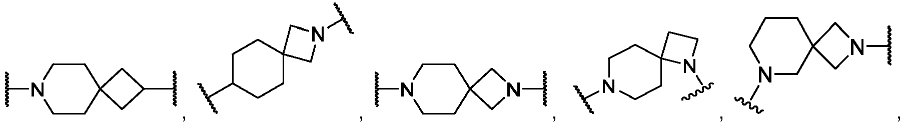

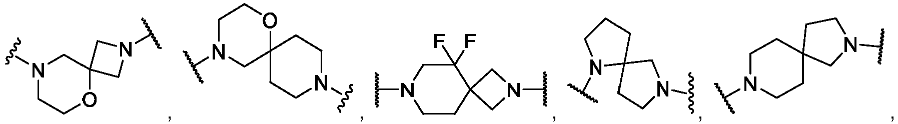

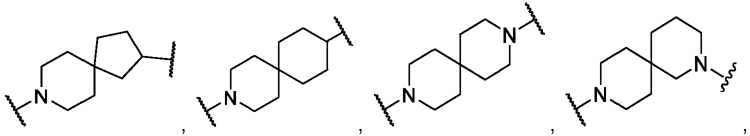

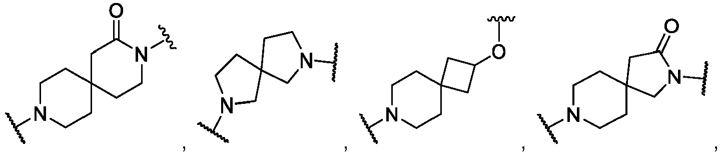

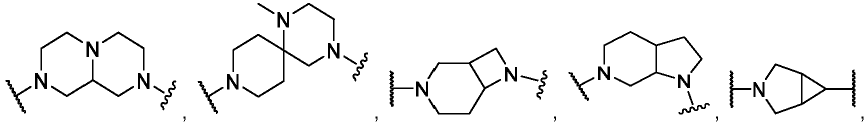

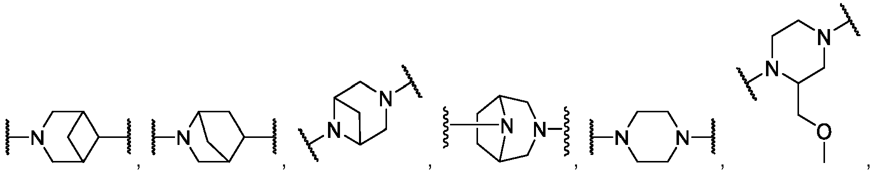

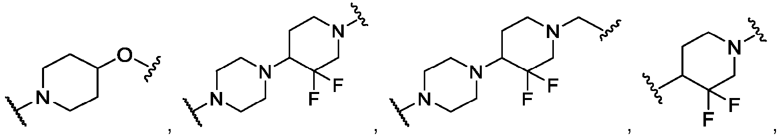

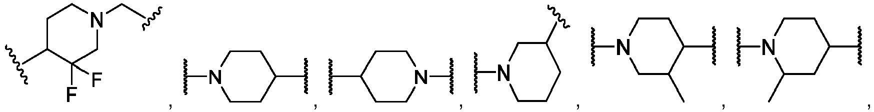

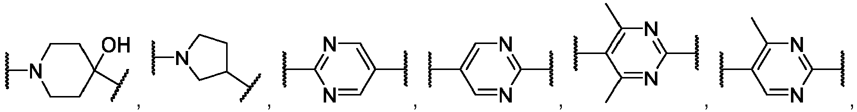

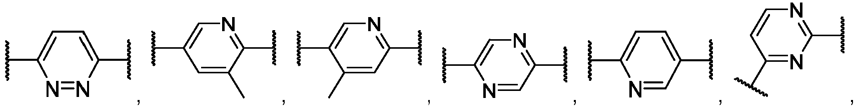

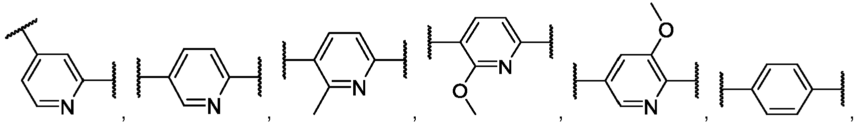

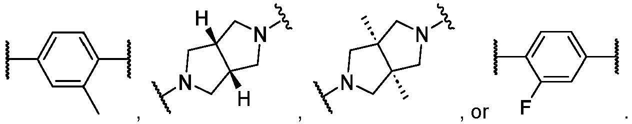

In an embodiment of the compound or salt of the invention B¹ is 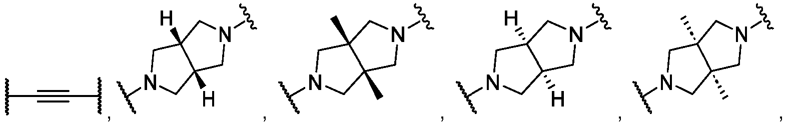 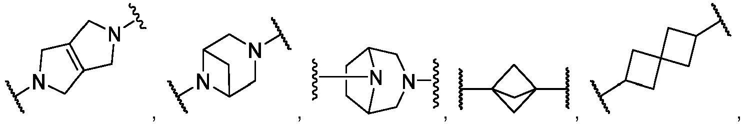 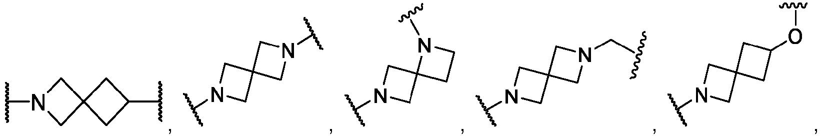 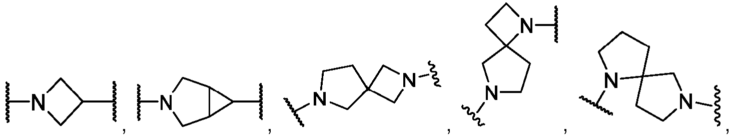 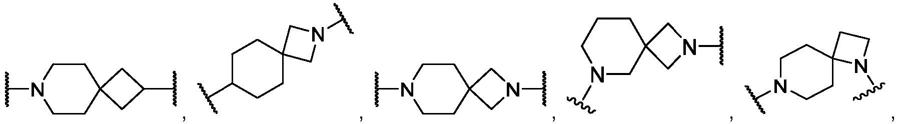 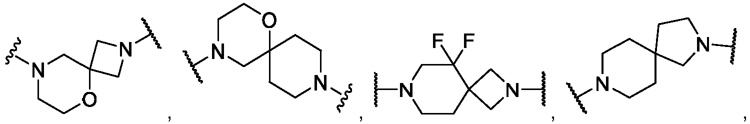 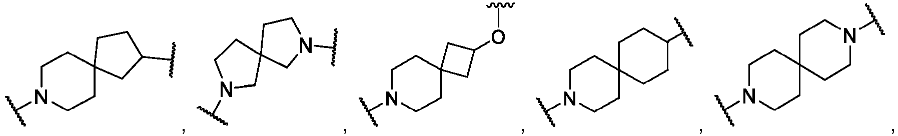 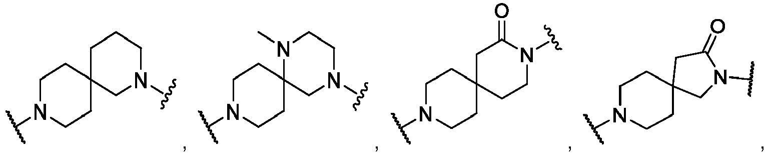 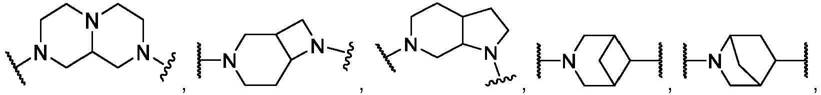 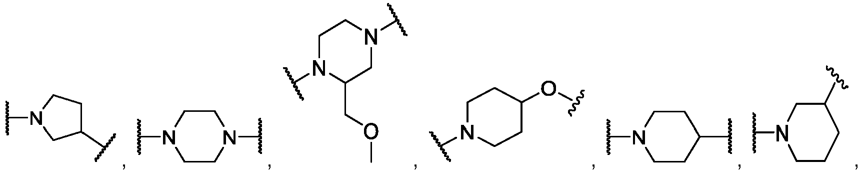 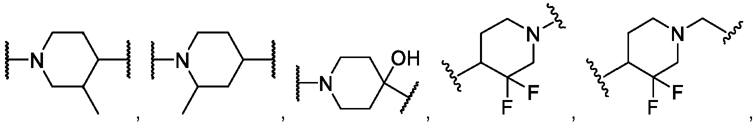 or

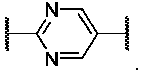

In an embodiment of the compound or salt of the invention B⁴ is 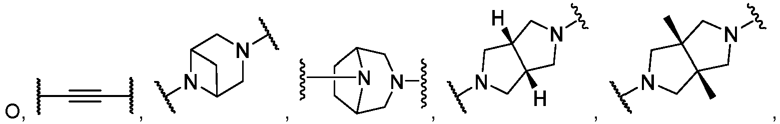 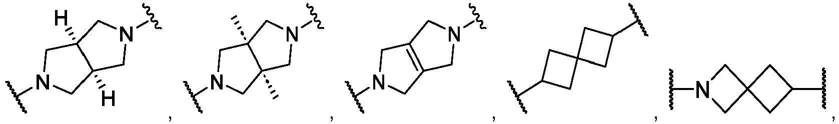 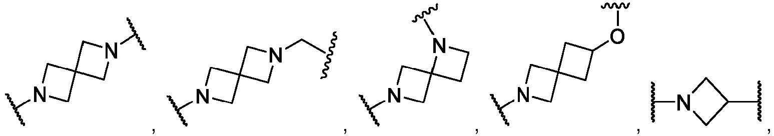 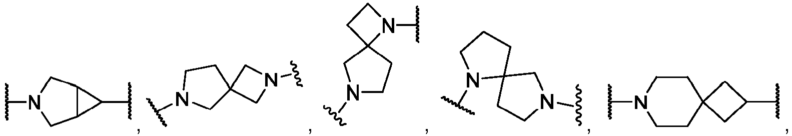 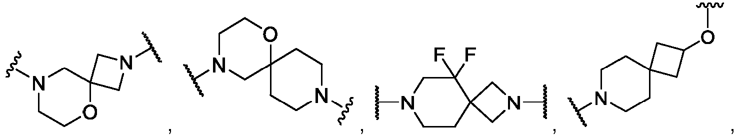 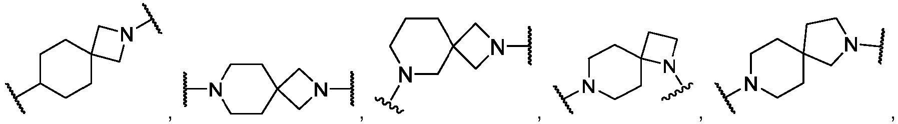 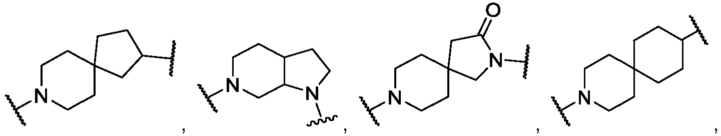 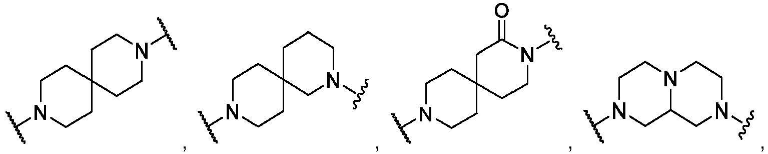 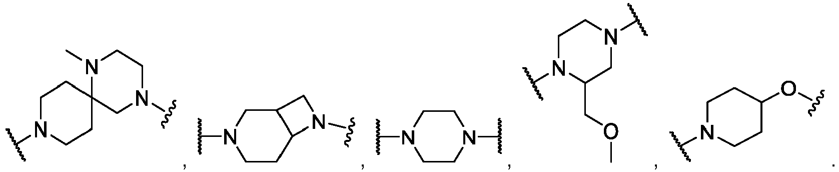 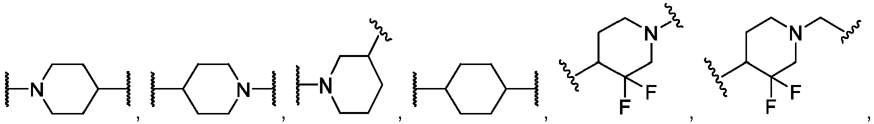 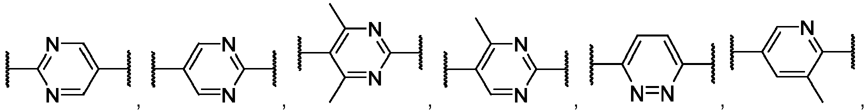 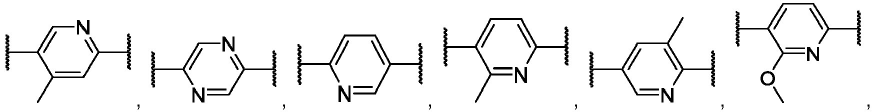 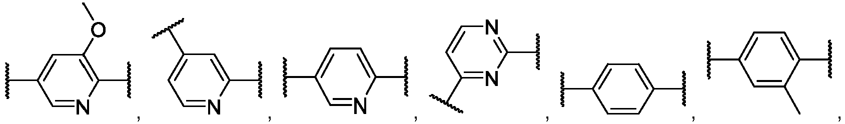 or

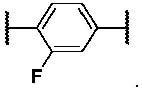

In an embodiment of the compound or salt of the invention B² is NH,

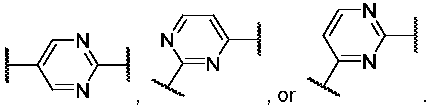

In an embodiment of the compound or salt of the invention L⁴ is -N(R^{B1})(R^{B2}), 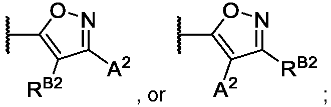
R^{B1} is **H,** A², optionally substituted C₁-C₆ alkyl, or optionally substituted C₁-C₆ heteroalkyl;
R^{B2} is **H,** optionally substituted C₁-C₆ alkyl, or optionally substituted C₁-C₆ heteroalkyl;
R^{B3} is A², optionally substituted C₁-C₆ alkyl, optionally substituted C₁-C₆ heteroalkyl, optionally substituted C₃-C₁₀ carbocyclyl, optionally substituted C₆-C₁₀ aryl, optionally substituted C₁-C₆ alkyl C₃-C₁₀ carbocyclyl, or optionally substituted C₁-C₆ alkyl C₆-C₁₀ aryl;
R^{B4} is **H,** optionally substituted C₁-C₆ alkyl, optionally substituted C₃-C₁₀ carbocyclyl, optionally substituted C₆-C₁₀ aryl, optionally substituted C₁-C₆ alkyl C₃-C₁₀ carbocyclyl, or optionally substituted C₁-C₆ alkyl C₆-C₁₀ aryl;
R^{B5} is **H,** optionally substituted C₁-C₆ alkyl, or optionally substituted C₁-C₆ heteroalkyl;
v2 is 0, 1, 2, 3, or 4;
each R^{B6} is, independently, A², halogen, optionally substituted C₁-C₆ alkyl, optionally substituted C₁-C₆ heteroalkyl, optionally substituted C₃-C₁₀ carbocyclyl, optionally substituted C₂-C₉ heterocyclyl, optionally substituted C₆-C₁₀ aryl, optionally substituted C₂-C₉ heteroaryl, optionally substituted C₂-C₆ alkenyl, optionally substituted C₂-C₆ heteroalkenyl, hydroxy, thiol, or optionally substituted amino;
each of R^{B7} and R^{B8} is, independently, H, halogen, optionally substituted C₁-C₆ alkyl, or optionally substituted C₆-C₁₀ aryl;
R^{B9} is H or optionally substituted C₁-C₆ alkyl; and
A² is a bond between the degradation moiety and the linker;
wherein one and only one of R^{B1}, R^{B3}, and R^{B6} is A²,
or a pharmaceutically acceptable salt thereof.

In an embodiment of the compound or salt of the invention the degradation moiety is

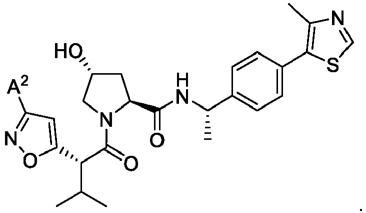

In an embodiment of the compound or salt of the invention the linker has the structure of

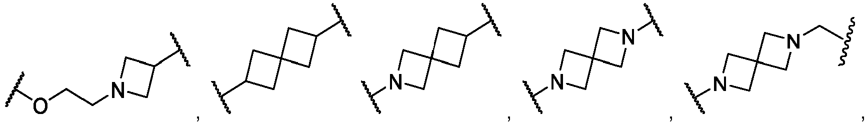

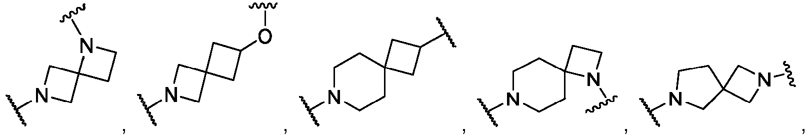

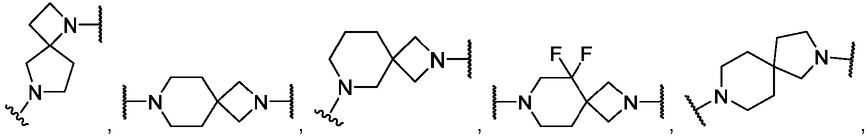

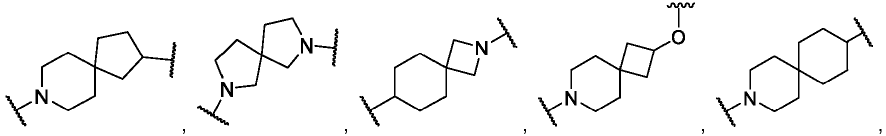

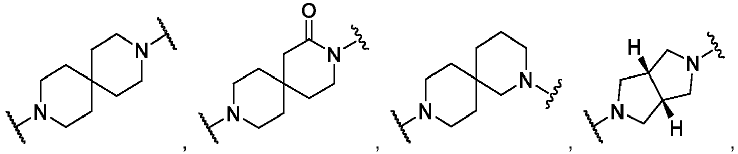

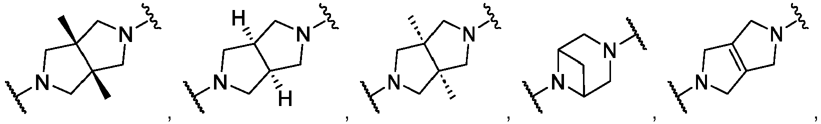

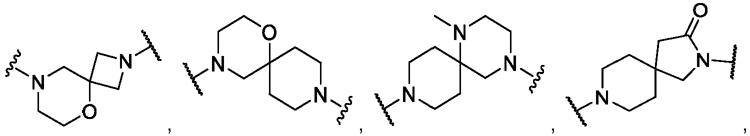

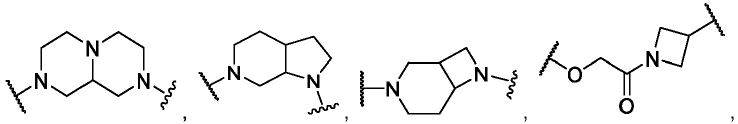

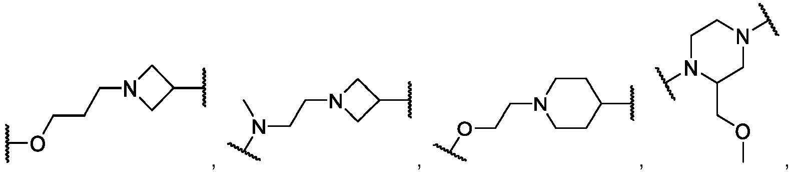

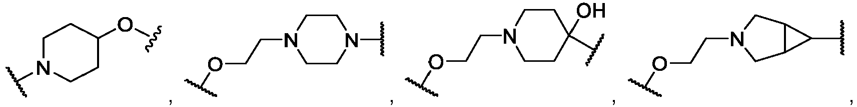

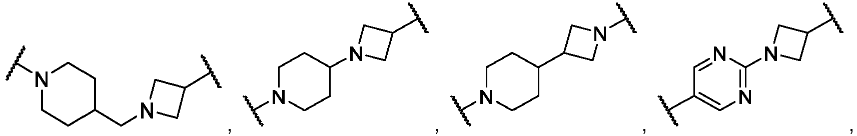

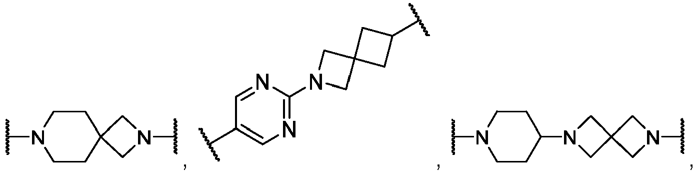

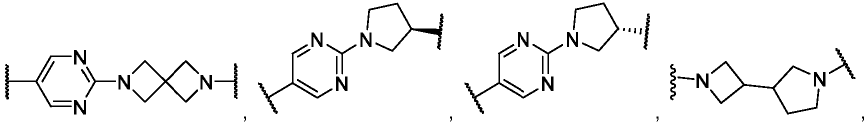

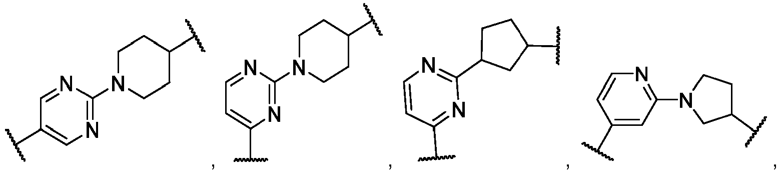

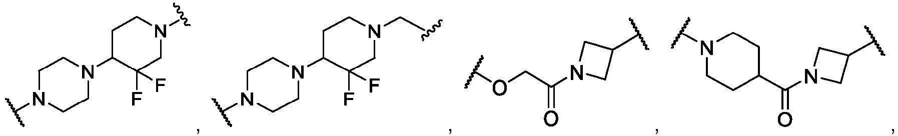

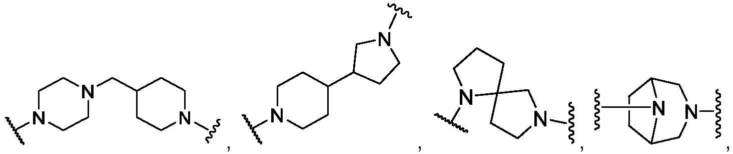

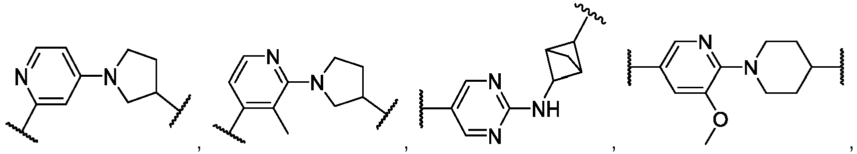

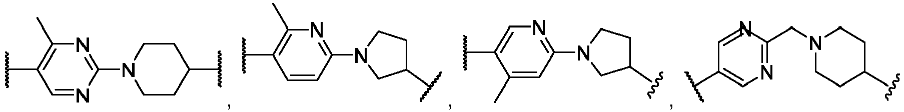

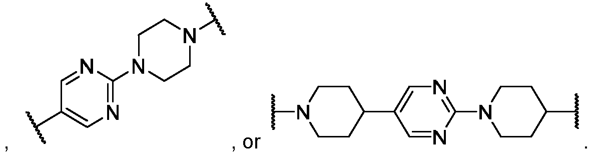

In an embodiment of the compound or salt of the invention the linker has the structure of

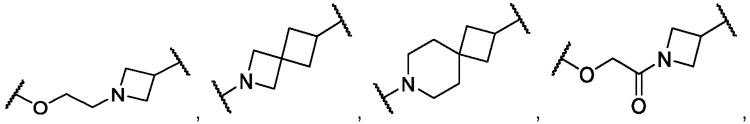

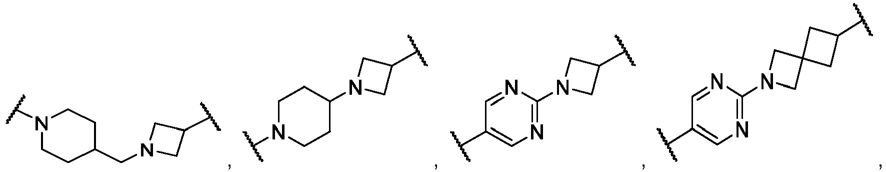

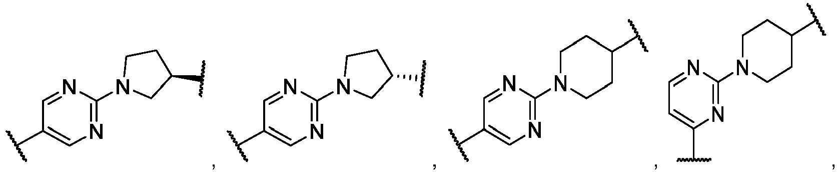

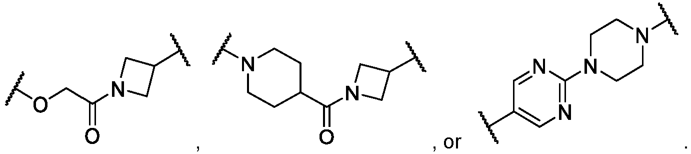

## Claims

1. A compound, or a pharmaceutically acceptable salt thereof, having the structure of Formula I or **II:** 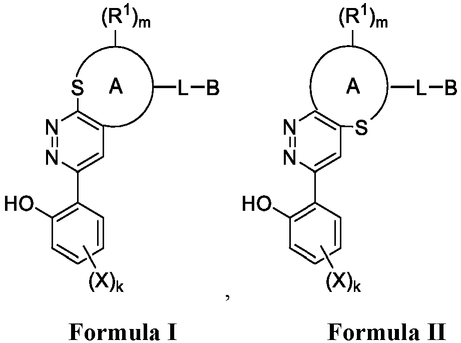 wherein
ring system A is a 5 to 9-membered heterocyclyl or heteroaryl;
m is 0, 1, 2, or 3;
k is 0, 1, or 2;
each R¹ is, independently, halo, optionally substituted C₁-C₆ alkyl, optionally substituted C₁-C₆ heteroalkyl, optionally substituted C₂-C₉ heterocyclyl, optionally substituted C₃-C₈ cycloalkyl or optionally substituted CH₂-C₃-C₈ cycloalkyl;
each X is, independently, halo;
L is a linker of Formula III:
A¹-(B¹)_{f}-(C¹)_{g}-(B²)ₕ-(B³)ᵢ-(C²)ⱼ-(B⁴)ₖ-A², **Formula III**
wherein
A¹ is a bond between the linker and ring system A;
A² is a bond between the degradation moiety and the linker;
each of B¹, B², B³, and B⁴ is, independently, optionally substituted ethynyl, optionally substituted C₆-C₁₀ aryl, optionally substituted C₃-C₁₀ cycloalkyl, optionally substituted C₂-C₉ heterocyclyl, optionally substituted C₂-C₉ heteroaryl, O, S, S(O)₂, or NR^{N};
each R^{N} is, independently, H, optionally substituted C₁₋₄ alkyl, optionally substituted C₂₋₄ alkenyl, optionally substituted C₂₋₄ alkynyl, optionally substituted C₂₋₆ heterocyclyl, optionally substituted C₆₋₁₂ aryl, or optionally substituted C₁₋₇ heteroalkyl;
each of C¹ and C² is, independently, carbonyl, thiocarbonyl, sulphonyl, or phosphoryl; and
each of f, g, h, i, j, and k is, independently, 0 or 1; and
B is a degradation moiety, wherein the degradation moiety has the structure of Formula C: 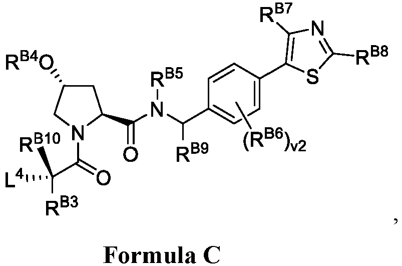
wherein
L⁴ is -N(R^{B1})(R^{B2}), 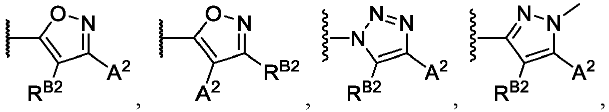 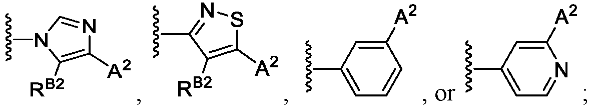
R^{B1} is H, A², optionally substituted C₁-C₆ alkyl, or optionally substituted C₁-C₆ heteroalkyl;
R^{B2} is H, optionally substituted C₁-C₆ alkyl, or optionally substituted C₁-C₆ heteroalkyl;
R^{B3} is A², optionally substituted C₁-C₆ alkyl, optionally substituted C₁-C₆ heteroalkyl, optionally substituted C₃-C₁₀ carbocyclyl, optionally substituted C₆-C₁₀ aryl, optionally substituted C₁-C₆ alkyl C₃-C₁₀ carbocyclyl, or optionally substituted C₁-C₆ alkyl C₆-C₁₀ aryl;
R^{B4} is H, optionally substituted C₁-C₆ alkyl, optionally substituted C₃-C₁₀ carbocyclyl, optionally substituted C₆-C₁₀ aryl, optionally substituted C₁-C₆ alkyl C₃-C₁₀ carbocyclyl, or optionally substituted C₁-C₆ alkyl C₆-C₁₀ aryl;
R^{B5} is H, optionally substituted C₁-C₆ alkyl, or optionally substituted C₁-C₆ heteroalkyl;
v2 is 0, 1, 2, 3, or 4;
each R^{B6} is, independently, A², halogen, optionally substituted C₁-C₆ alkyl, optionally substituted C₂-C₆ alkynyl, optionally substituted C₁-C₆ heteroalkyl, optionally substituted C₃-C₁₀ carbocyclyl, optionally substituted C₂-C₉ heterocyclyl, optionally substituted C₆-C₁₀ aryl, optionally substituted C₂-C₉ heteroaryl, optionally substituted C₂-C₆ alkenyl, optionally substituted C₂-C₆ heteroalkenyl, hydroxy, thiol, cyano, or optionally substituted amino;
each of R^{B7} and R^{B8} is, independently, H, halogen, optionally substituted C₁-C₆ alkyl, or optionally substituted C₆-C₁₀ aryl;
R^{B9} is H or optionally substituted C₁-C₆ alkyl;
R^{B10} is H or F; and
A² is a bond between the degradation moiety and the linker;
wherein one and only one of R^{B1}, R^{B3}, and R^{B6} is A²,
or a pharmaceutically acceptable salt thereof.

2. The compound of claim 1, or a pharmaceutically acceptable salt thereof, wherein the compound has the structure of **Formula I-A or II-A:** 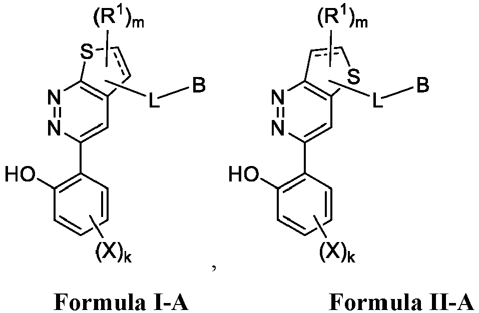 wherein the dashed bond represents a single or double bond.

3. The compound of claim 1, or a pharmaceutically acceptable salt thereof, wherein the compound has the structure **of Formula I-H or II-H:**

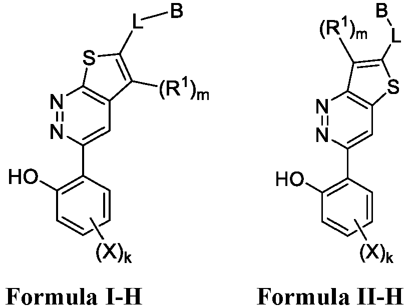

4. The compound of any one of claims 1 to 3, or a pharmaceutically acceptable salt thereof, wherein m is 0 or 1.

5. The compound of any one of claims 1 to 4, or a pharmaceutically acceptable salt thereof, wherein when m is 1, R¹ is halo, optionally substituted C₁-C₆ alkyl or optionally substituted C₃-C₈ cycloalkyl.

6. The compound of any one of claims 1 to 5, or a pharmaceutically acceptable salt thereof, wherein k is 1 and X is Cl.

7. The compound of any one of claims 1 to 6, or a pharmaceutically acceptable salt thereof, wherein k is 0.

8. The compound of any one of claims 1 to 7, or a pharmaceutically acceptable salt thereof, wherein at least one of f, h, i, and k is 1.

9. The compound of any one of claims 1 to 8 or a pharmaceutically acceptable salt thereof, wherein each of B¹, B², B³, and B⁴ is, independently, O, ethynyl, optionally substituted C₂-C₉ heteroaryl, optionally substituted C₂-C₉ heterocyclyl, optionally substituted C₃-C₁₀ cycloalkyl, or optionally substituted C₆-C₁₀ aryl.

10. The compound of any one of claims 1 to 9, or a pharmaceutically acceptable salt thereof, wherein each of B¹ and B⁴ is, independently, 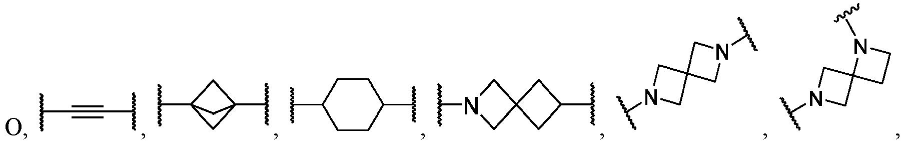 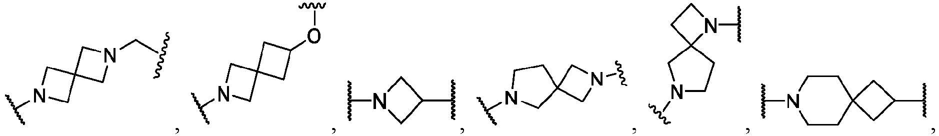 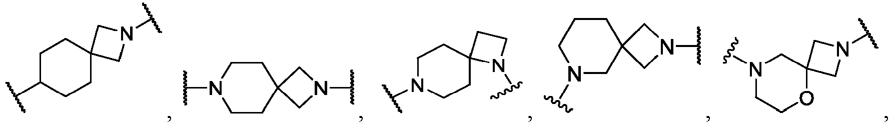 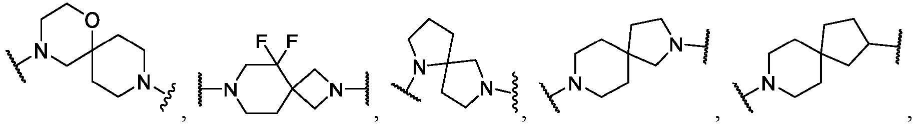 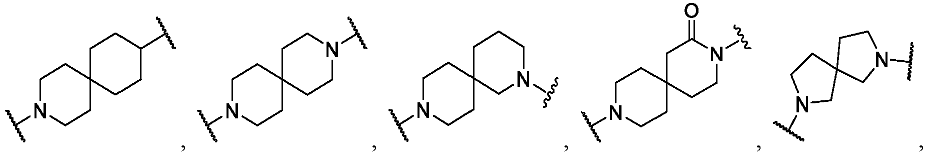 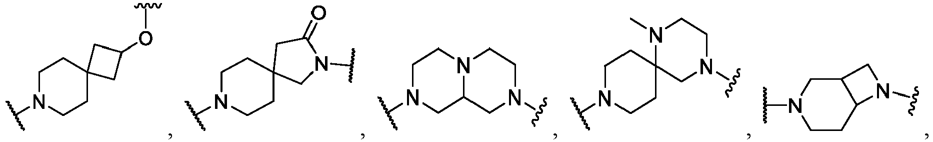 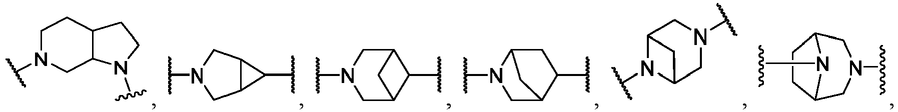 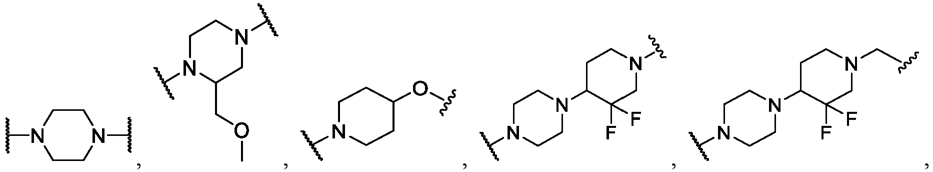 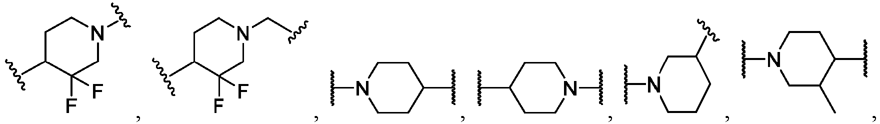 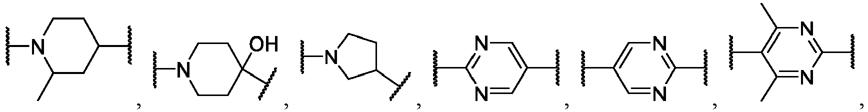 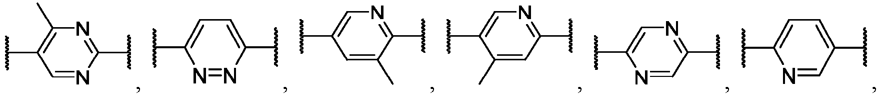 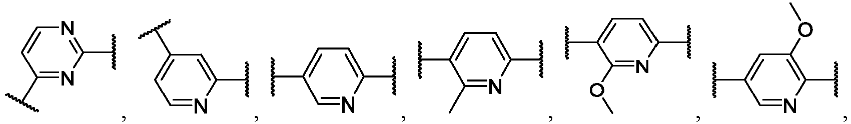 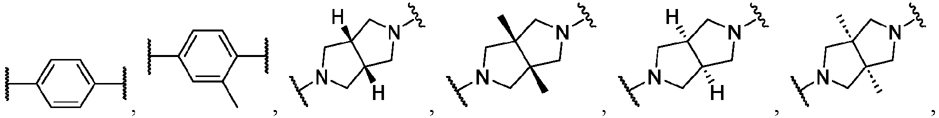 or

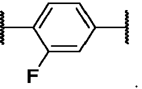

11. The compound of claim 10, or a pharmaceutically acceptable salt thereof, wherein B¹ is

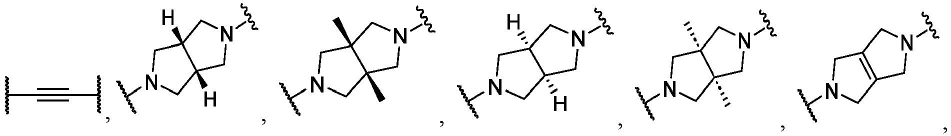

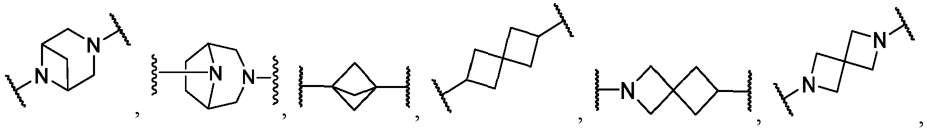

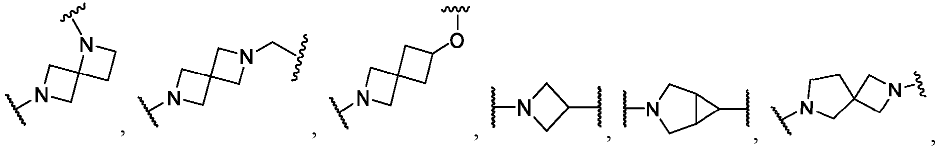

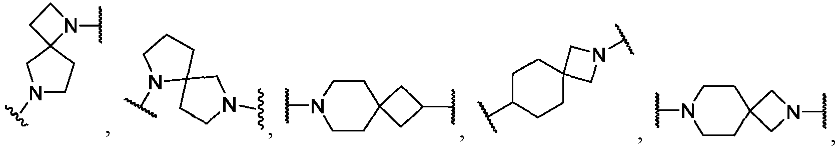

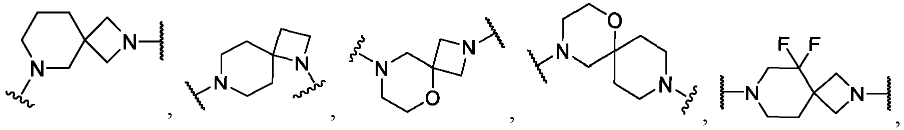

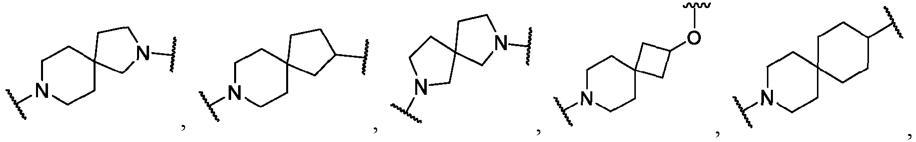

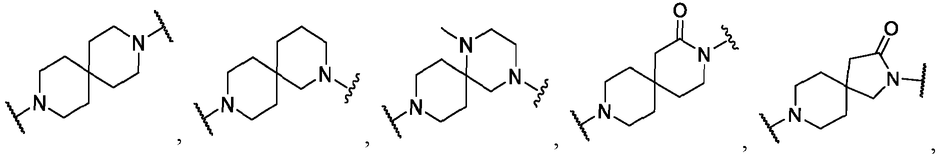

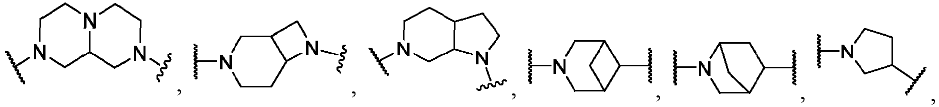

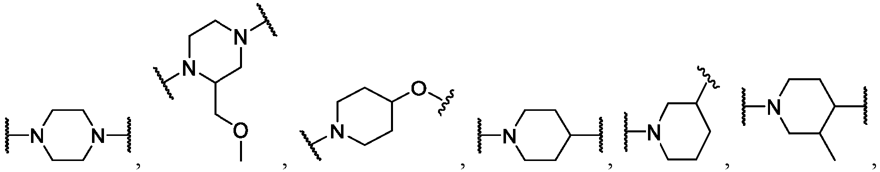

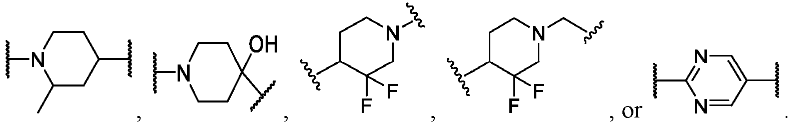

12. The compound of claim 10 or 11, or a pharmaceutically acceptable salt thereof, wherein B⁴ is O, 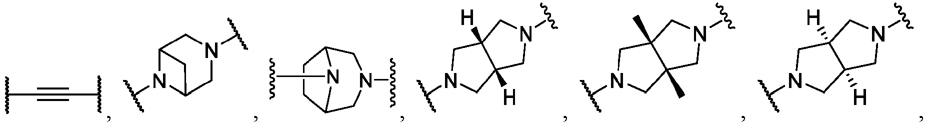 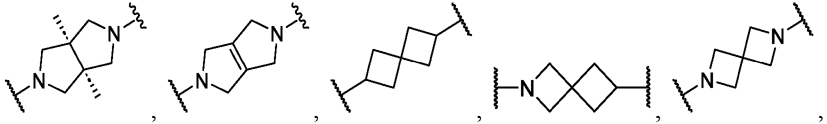 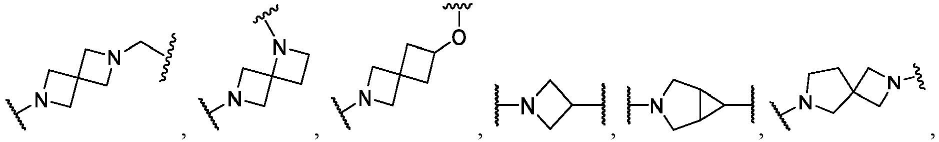 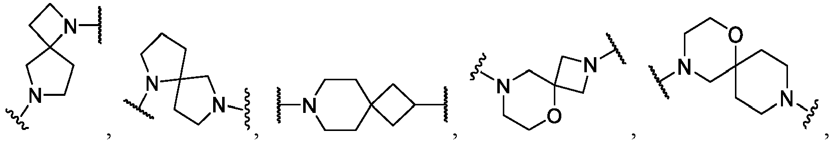 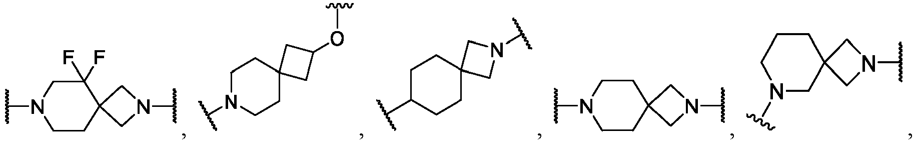 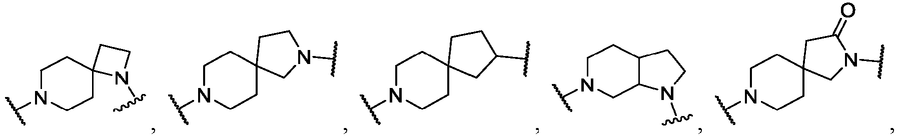 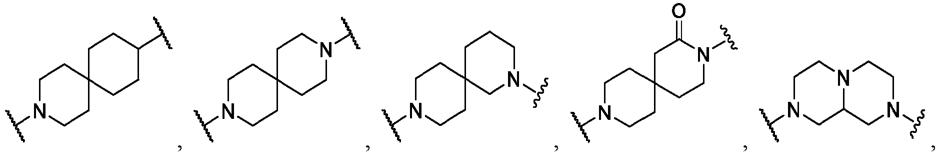 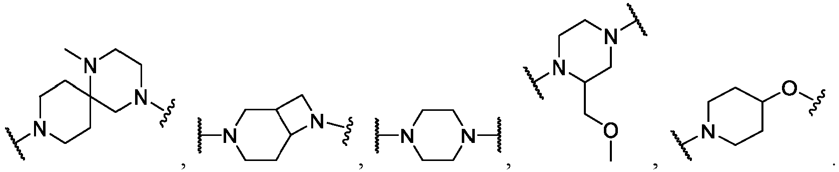 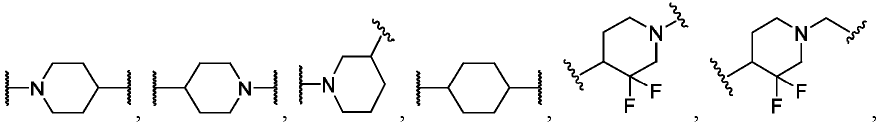 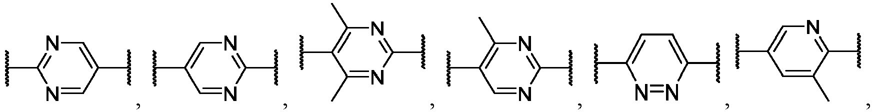 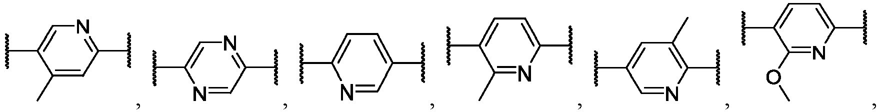 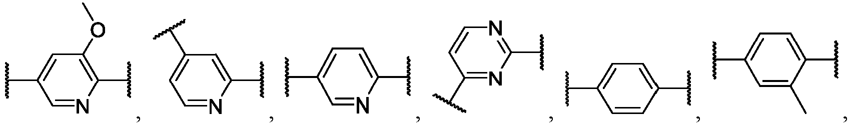 or

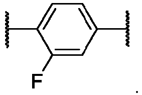

13. The compound of any one of claims 1 to 7, or a pharmaceutically acceptable salt thereof, wherein B² is NH,

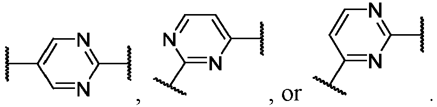

14. The compound of any one of claims 1 to 13, or a pharmaceutically acceptable salt thereof, wherein
L⁴ is -N(R^{B1})(R^{B2}), 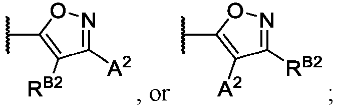
R^{B1} is H, A², optionally substituted C₁-C₆ alkyl, or optionally substituted C₁-C₆ heteroalkyl;
R^{B2} is H, optionally substituted C₁-C₆ alkyl, or optionally substituted C₁-C₆ heteroalkyl;
R^{B3} is A², optionally substituted C₁-C₆ alkyl, optionally substituted C₁-C₆ heteroalkyl, optionally substituted C₃-C₁₀ carbocyclyl, optionally substituted C₆-C₁₀ aryl, optionally substituted C₁-C₆ alkyl C₃-C₁₀ carbocyclyl, or optionally substituted C₁-C₆ alkyl C₆-C₁₀ aryl;
R^{B4} is H, optionally substituted C₁-C₆ alkyl, optionally substituted C₃-C₁₀ carbocyclyl, optionally substituted C₆-C₁₀ aryl, optionally substituted C₁-C₆ alkyl C₃-C₁₀ carbocyclyl, or optionally substituted C₁-C₆ alkyl C₆-C₁₀ aryl;
R^{B5} is H, optionally substituted C₁-C₆ alkyl, or optionally substituted C₁-C₆ heteroalkyl;
v2 is 0, **1, 2,** 3, or 4;
each R^{B6} is, independently, A², halogen, optionally substituted C₁-C₆ alkyl, optionally substituted C₁-C₆ heteroalkyl, optionally substituted C₃-C₁₀ carbocyclyl, optionally substituted C₂-C₉ heterocyclyl, optionally substituted C₆-C₁₀ aryl, optionally substituted C₂-C₉ heteroaryl, optionally substituted C₂-C₆ alkenyl, optionally substituted C₂-C₆ heteroalkenyl, hydroxy, thiol, or optionally substituted amino;
each of R^{B7} and R^{B8} is, independently, H, halogen, optionally substituted C₁-C₆ alkyl, or optionally substituted C₆-C₁₀ aryl;
R^{B9} is H or optionally substituted C₁-C₆ alkyl; and
A² is a bond between the degradation moiety and the linker;
wherein one and only one of R^{B1}, R^{B3}, and R^{B6} is A²,
or a pharmaceutically acceptable salt thereof.

15. The compound of claim 14, or a pharmaceutically acceptable salt thereof, wherein the degradation moiety is

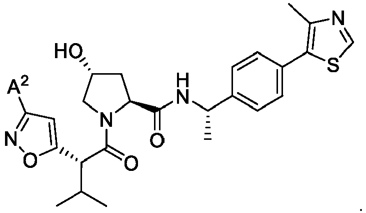

16. The compound of any one of claims 1 to 15, or a pharmaceutically acceptable salt thereof, wherein the linker has the structure of 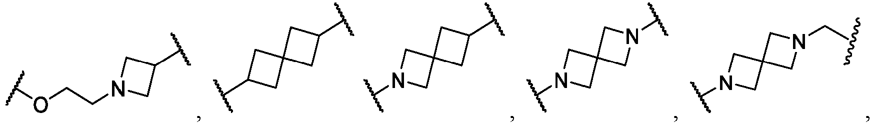 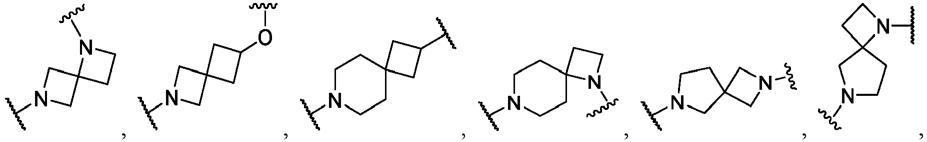 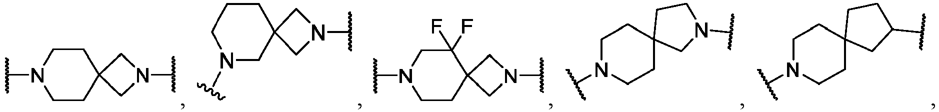 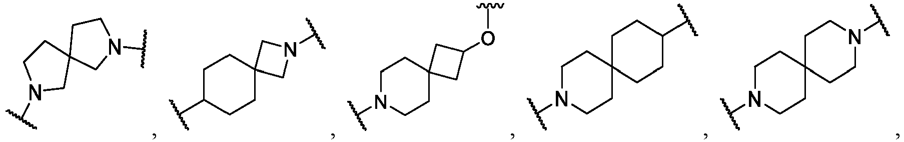 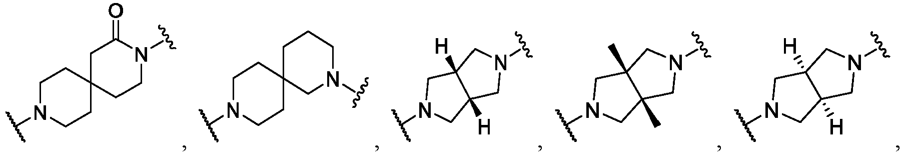 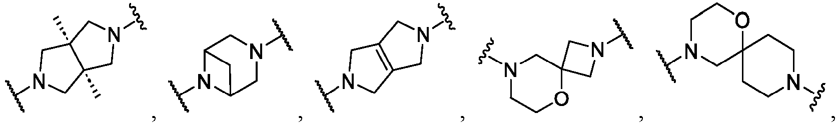 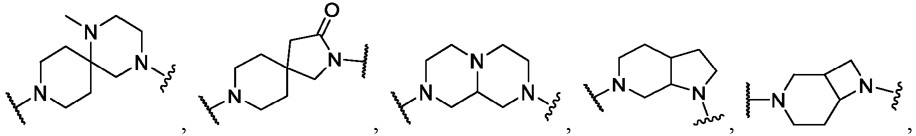 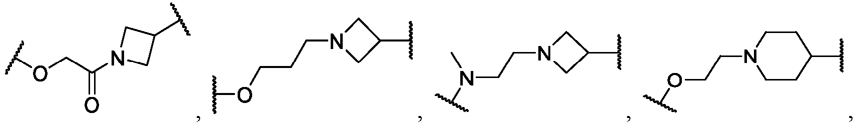 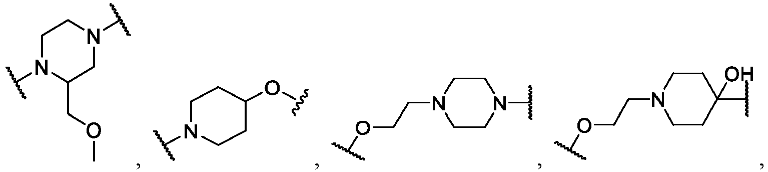 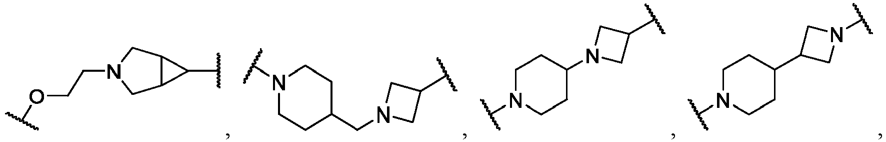 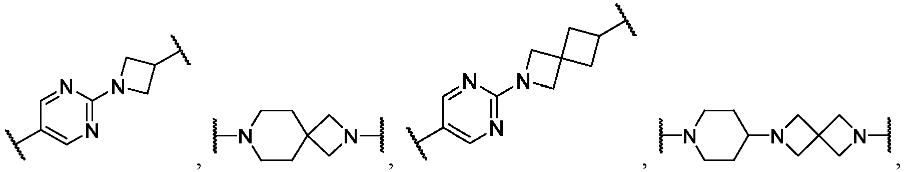 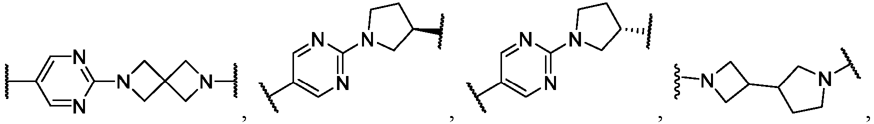 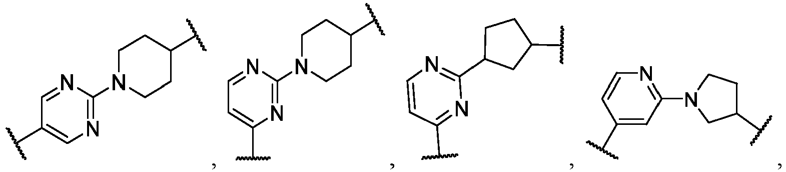 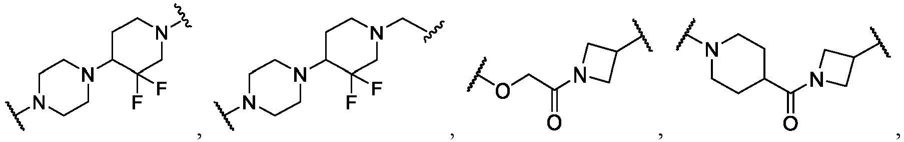 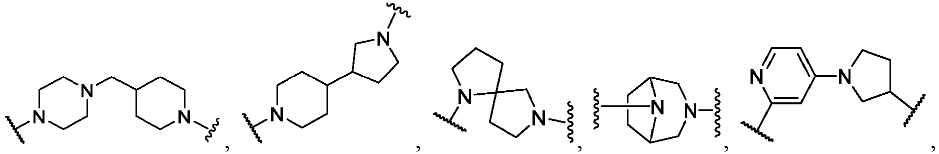 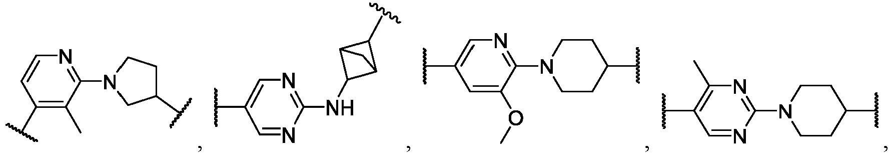 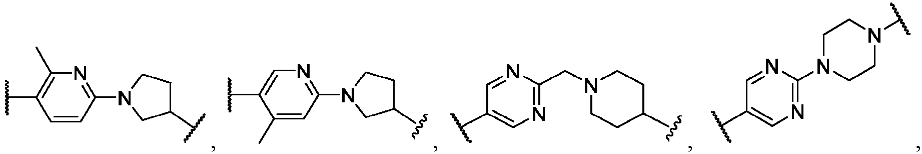 or

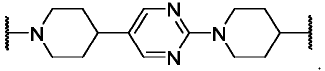

17. The compound of any one of claims 1 to 15, or a pharmaceutically acceptable salt thereof, wherein the linker has the structure of

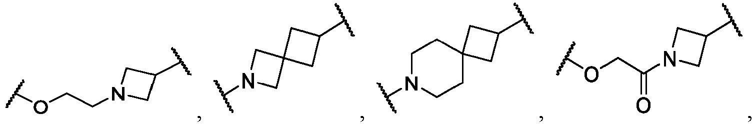

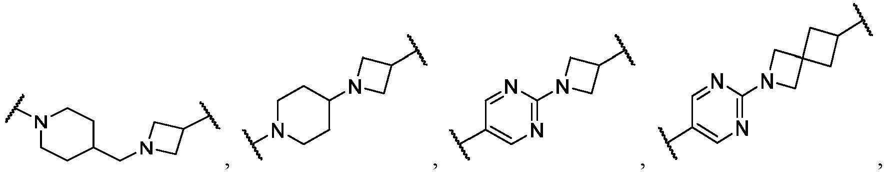

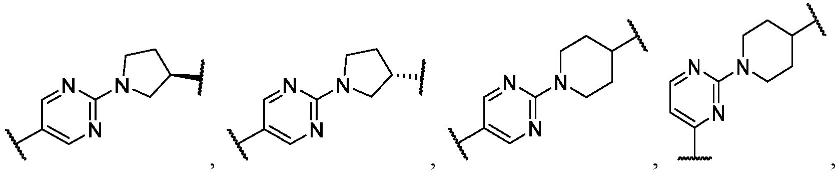

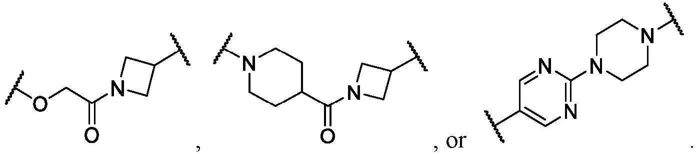

18. A pharmaceutical composition comprising a compound of any one of claims 1 to 17 and a pharmaceutically acceptable excipient.

19. A compound of any one of claims 1 to 17 or a pharmaceutically acceptable salt thereof, or a pharmaceutical composition of claim 18, for use in therapy.

20. A compound of any one of claims 1 to 17, or a pharmaceutically acceptable salt thereof, or a pharmaceutical composition of claim 18, for use in treating cancer.

21. The compound, or the pharmaceutically acceptable salt thereof, or the pharmaceutical composition for use according to claim 20, wherein the cancer is non-small cell lung cancer, colorectal cancer, bladder cancer, cancer of unknown primary, glioma, breast cancer, melanoma, non-melanoma skin cancer, endometrial cancer, esophagogastric cancer, pancreatic cancer, hepatobiliary cancer, soft tissue sarcoma, ovarian cancer, head and neck cancer, renal cell carcinoma, bone cancer, non-Hodgkin lymphoma, small-cell lung cancer, prostate cancer, embryonal tumor, germ cell tumor, cervical cancer, thyroid cancer, salivary gland cancer, gastrointestinal neuroendocrine tumor, uterine sarcoma, gastrointestinal stromal tumor, CNS cancer, thymic tumor, Adrenocortical carcinoma, appendiceal cancer, small bowel cancer, or penile cancer.

## Patentansprüche

1. Verbindung oder ein pharmazeutisch verträgliches Salz davon, aufweisend die Struktur der **Formel I oder II:** 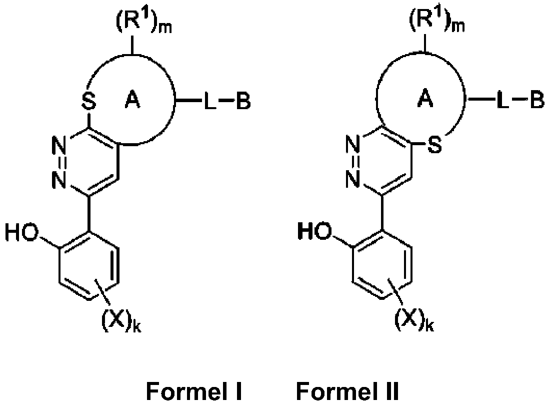 worin
Ringsystem A ein 5- bis 9-gliedriges Heterocyclyl oder Heteroaryl ist;
m 0, 1, 2 oder 3 ist;
k 0, 1 oder 2 ist;
jedes R¹ unabhängig Halogen, gegebenenfalls substituiertes C₁-C₆-Alkyl, gegebenenfalls substituiertes C₁-C₆-Heteroalkyl, gegebenenfalls substituiertes C₂-C₉-Heterocyclyl, gegebenenfalls substituiertes C₃-C₈-Cycloalkyl oder gegebenenfalls substituiertes CH₂-C₃-C₈-Cycloalkyl ist;
jedes X unabhängig Halogen ist;
L ein Linker der Formel III ist:
A¹-(B¹)_{f}-(C¹)_{g}-(B²)ₕ-(B³)ᵢ-(C²)ⱼ-(B⁴)ₖ-A², **Formel III**
worin
A¹ eine Bindung zwischen dem Linker und Ringsystem A ist;
A² eine Bindung zwischen dem Abbaurest und dem Linker ist;
jedes von B¹, B², B³ und B⁴ unabhängig voneinander gegebenenfalls substituiertes Ethinyl, gegebenenfalls substituiertes C₆-C₁₀-Aryl, gegebenenfalls substituiertes C₃-C₁₀-Cycloalkyl, gegebenenfalls substituiertes C₂-C₉-Heterocyclyl, gegebenenfalls substituiertes C₂-C₉-Heteroaryl, O, S, S(O)₂ oder NR^{N};
jedes Rⁿ unabhängig H, gegebenenfalls substituiertes C₁₋₄-Alkyl, gegebenenfalls substituiertes C₂₋₄-Alkenyl, gegebenenfalls substituiertes C₂₋₄-Alkinyl, gegebenenfalls substituiertes C₂₋₆-Heterocyclyl, gegebenenfalls substituiertes C₆₋₁₂-Aryl oder gegebenenfalls substituiertes C₁₋₇-Heteroalkyl ist;
jedes von C¹ und C² unabhängig voneinander Carbonyl, Thiocarbonyl, Sulfonyl oder Phosphoryl ist; und
jedes von f, g, h, i, j und k unabhängig voneinander 0 oder 1 ist; und
B ein Abbaurest ist, wobei der Abbaurest die Struktur der Formel C aufweist: 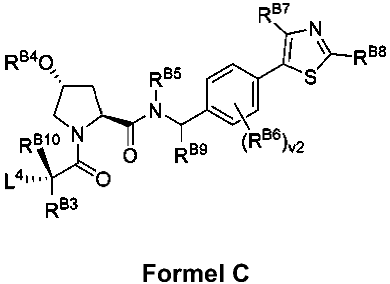
worin
L⁴ is -N(R^{B1})(R^{B2}), 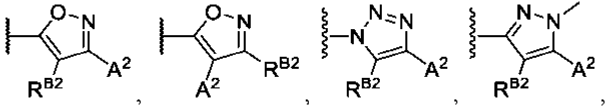 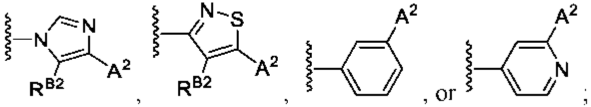
R^{B1} H, A², gegebenenfalls substituiertes C₁-C₆-Alkyl oder gegebenenfalls substituiertes C₁-C₆-Heteroalkyl ist;
R^{B2} H, gegebenenfalls substituiertes C₁-C₆-Alkyl oder gegebenenfalls substituiertes C₁-C₆-Heteroalkyl ist;
R^{B3} A², gegebenenfalls substituiertes C₁-C₆-Alkyl, gegebenenfalls substituiertes C₁-C₆-Heteroalkyl, gegebenenfalls substituiertes C₃-C₁₀-Carbocyclyl, gegebenenfalls substituiertes C₆-C₁₀-Aryl, gegebenenfalls substituiertes C₁-C₆-Alkyl-C₃-C₁₀-carbocyclyl oder gegebenenfalls substituiertes C₁-C₆-Alkyl-C₆-C₁₀-aryl ist;
R^{B4} H, gegebenenfalls substituiertes C₁-C₆-Alkyl, gegebenenfalls substituiertes C₃-C₁₀-Carbocyclyl, gegebenenfalls substituiertes C₆-C₁₀-Aryl, gegebenenfalls substituiertes C₁-C₆-Alkyl-C₃-C₁₀-carbocyclyl oder gegebenenfalls substituiertes C₁-C₆-Alkyl-C₆-C₁₀-aryl ist;
R^{B5} H, gegebenenfalls substituiertes C₁-C₆-Alkyl oder gegebenenfalls substituiertes C₁-C₆-Heteroalkyl ist;
v2 0, 1, 2, 3 oder 4 ist;
jedes R^{B6} unabhängig A², Halogen, gegebenenfalls substituiertes C₁-C₆-Alkyl, gegebenenfalls substituiertes C₂-C₆-Alkinyl, gegebenenfalls substituiertes C₁-C₆-Heteroalkyl, gegebenenfalls substituiertes C₃-C₁₀-Carbocyclyl, gegebenenfalls substituiertes C₂-C₉-Heterocyclyl, gegebenenfalls substituiertes C₆-C₁₀-Aryl, gegebenenfalls substituiertes C₂-C₉-Heteroaryl, gegebenenfalls substituiertes C₂-C₆-Alkenyl, gegebenenfalls substituiertes C₂-C₆-Heteroalkenyl, Hydroxy, Thiol, Cyano oder gegebenenfalls substituiertes Amino ist;
R^{B7} und R^{B8} jeweils unabhängig voneinander H, Halogen, gegebenenfalls substituiertes C₁-C₆-Alkyl oder gegebenenfalls substituiertes C₆-C₁₀-Aryl sind;
R^{B9} H oder gegebenenfalls substituiertes C₁-C₆-Alkyl ist;
R^{B10} H oder F ist; und
A² eine Bindung zwischen dem Abbaurest und dem Linker ist;
wobei genau eines von R^{B1}, R^{B3} und R^{B6} A² ist,
oder ein pharmazeutisch verträgliches Salz davon.

2. Verbindung nach Anspruch 1 oder ein pharmazeutisch verträgliches Salz davon, wobei die Verbindung die Struktur von **Formel I-A oder II-A** aufweist: 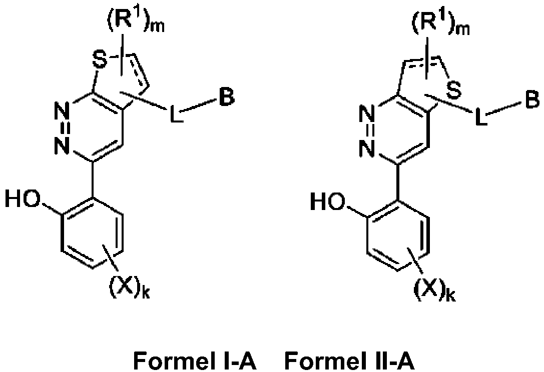 worin die gestrichelte Bindung eine Einfach- oder Doppelbindung darstellt.

3. Verbindung nach Anspruch 1 oder ein pharmazeutisch verträgliches Salz davon, wobei die Verbindung die Struktur von **Formel I-H oder II-H** aufweist:

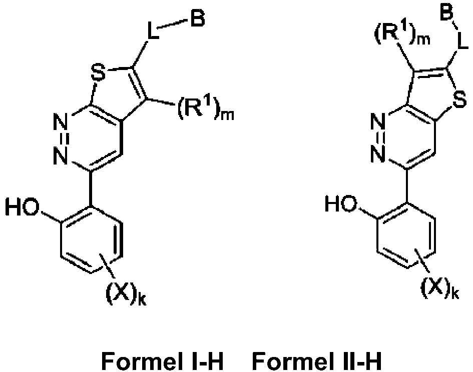

4. Verbindung nach einem der Ansprüche 1 bis 3 oder ein pharmazeutisch verträgliches Salz davon, wobei m 0 oder 1 ist.

5. Verbindung nach einem der Ansprüche 1 bis 4 oder ein pharmazeutisch verträgliches Salz davon, wobei wenn m 1 ist, R¹ Halogen, gegebenenfalls substituiertes C₁-C₆-Alkyl oder gegebenenfalls substituiertes C₃-C₈-Cycloalkyl ist.

6. Verbindung nach einem der Ansprüche 1 bis 5 oder ein pharmazeutisch verträgliches Salz davon, wobei k 1 und X Cl ist.

7. Verbindung nach einem der Ansprüche 1 bis 6 oder ein pharmazeutisch verträgliches Salz davon, wobei k 0 ist.

8. Verbindung nach einem der Ansprüche 1 bis 7 oder ein pharmazeutisch verträgliches Salz davon, wobei mindestens eines von f, h, i, und k 1 ist.

9. Verbindung nach einem der Ansprüche 1 bis 8 oder ein pharmazeutisch verträgliches Salz davon, wobei jedes von B¹, B², B³ und B⁴ unabhängig voneinander O, Ethinyl, gegebenenfalls substituiertes C₂-C₉-Heteroaryl, gegebenenfalls substituiertes C₂-C₉-Heterocyclyl, gegebenenfalls substituiertes C₃-C₁₀-Cycloalkyl oder gegebenenfalls substituiertes C₆-C₁₀-Aryl ist.

10. Verbindung nach einem der Ansprüche 1 bis 9 oder ein pharmazeutisch verträgliches Salz davon, wobei jedes von B¹ und B⁴ unabhängig voneinander 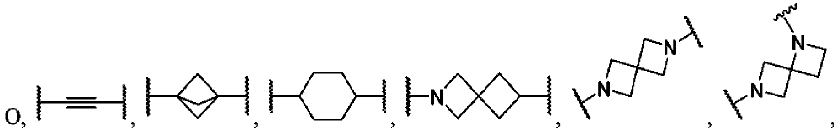 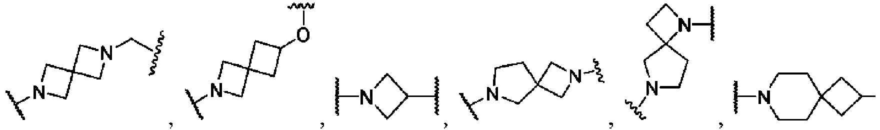 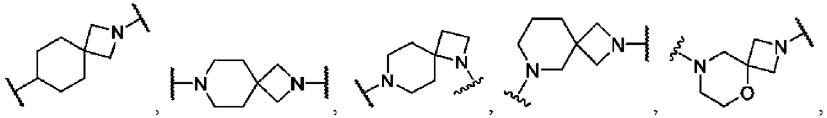 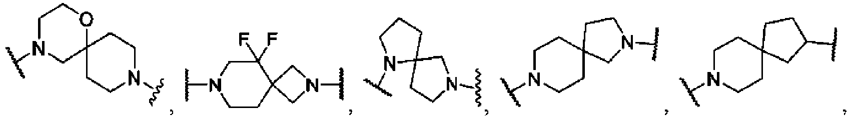 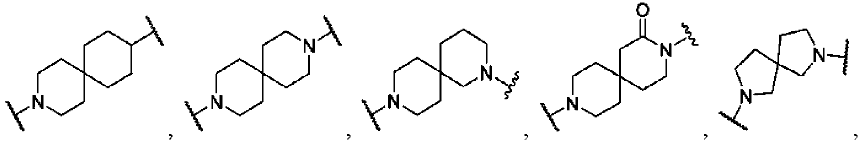 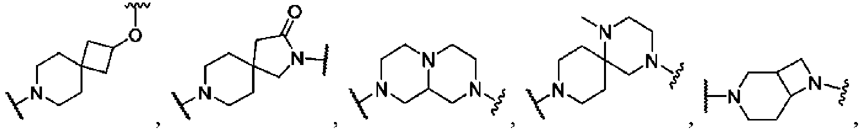 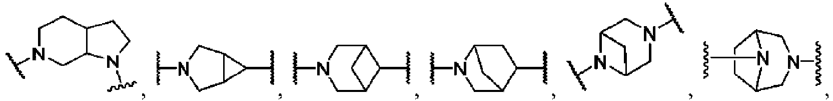 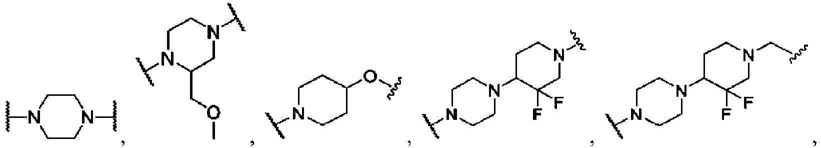 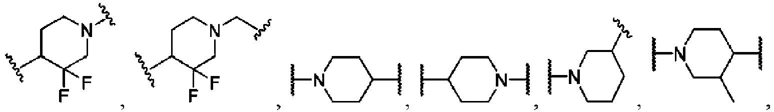 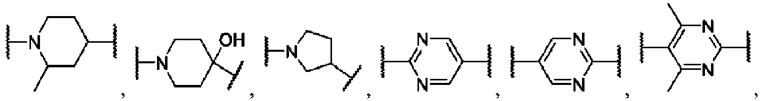 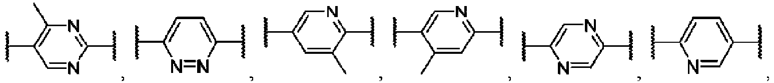 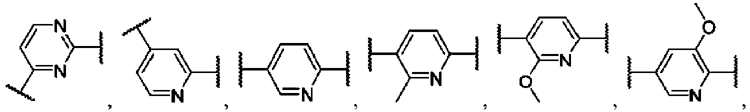 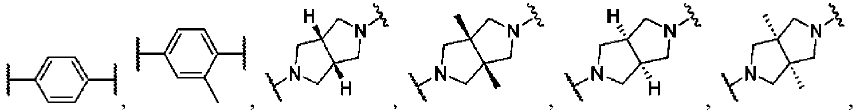 oder 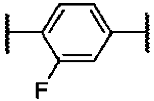 ist.

11. Verbindung nach Anspruch 10 oder ein pharmazeutisch verträgliches Salz davon, wobei B¹ 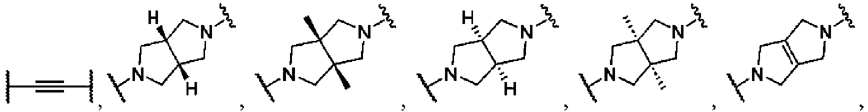 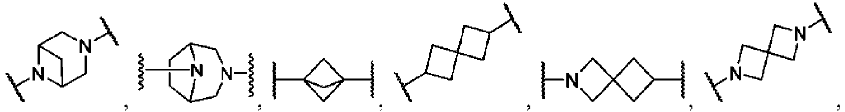 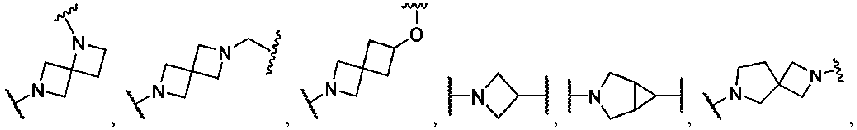 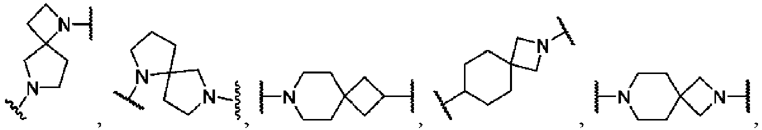 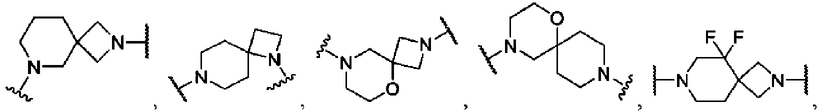 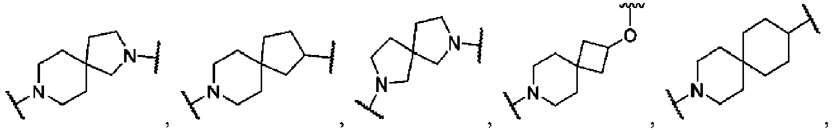 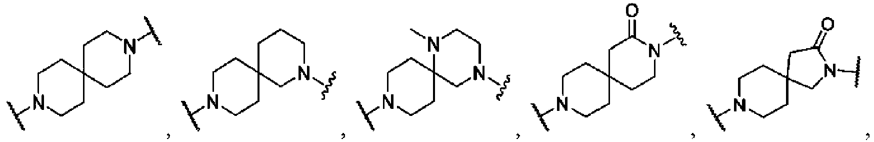 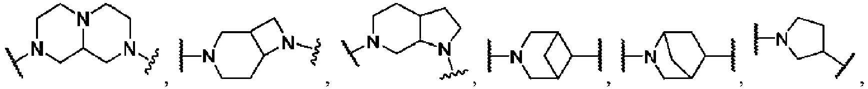 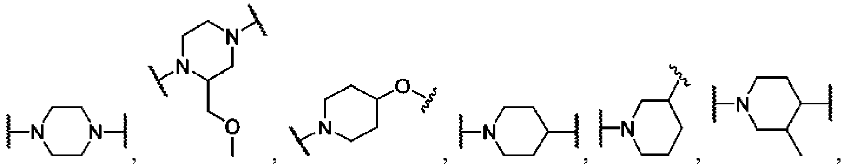 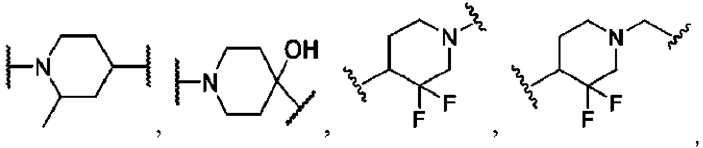 oder 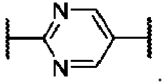 ist.

12. Verbindung nach Anspruch 10 oder 11, oder ein pharmazeutisch verträgliches Salz davon, wobei B⁴
O, 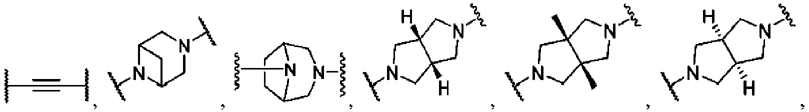 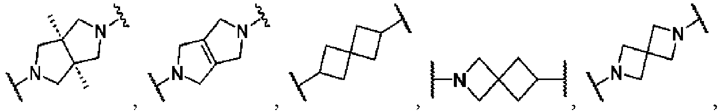 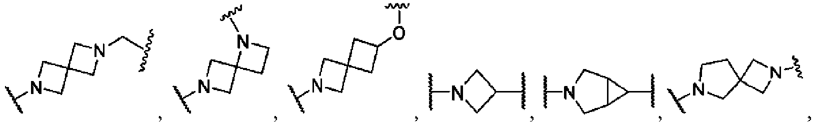 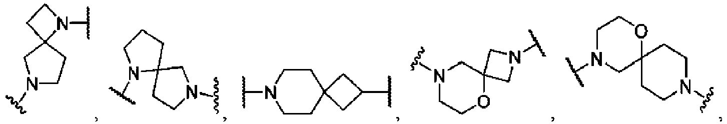 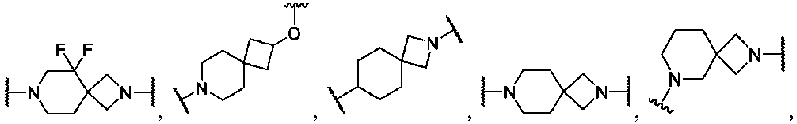 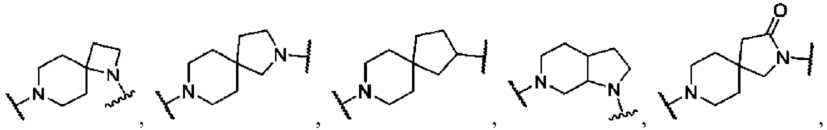 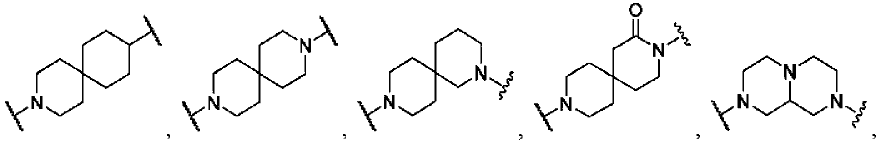 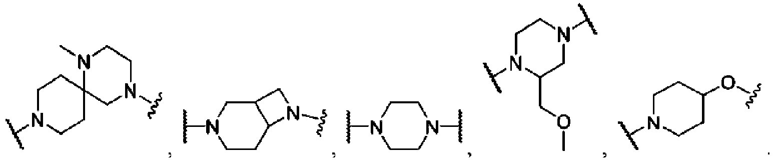 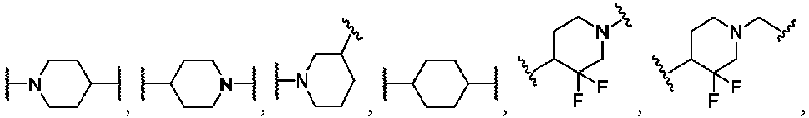 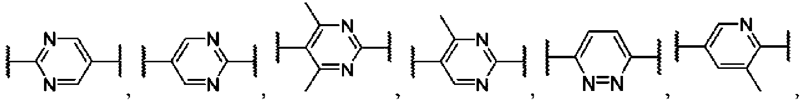 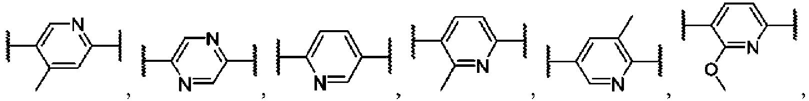 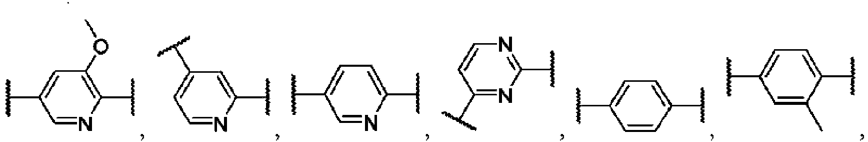 oder 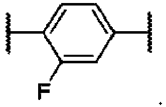 ist.

13. Verbindung nach einem der Ansprüche 1 bis 7, oder ein pharmazeutisch verträgliches Salz davon, wobei B² NH, 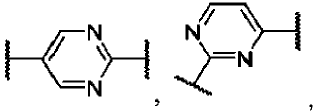 oder 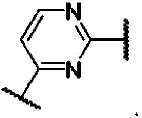 ist.

14. Verbindung nach einem der Ansprüche 1 bis 13 oder ein pharmazeutisch verträgliches Salz davon, wobei
L⁴ -N(R^{B1})(R^{B2}), 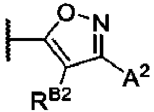 oder 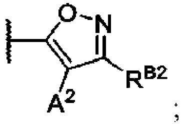 ist;
R^{B1} H, A², gegebenenfalls substituiertes C₁-C₆-Alkyl oder gegebenenfalls substituiertes C₁-C₆-Heteroalkyl ist;
R^{B2} H, gegebenenfalls substituiertes C₁-C₆-Alkyl oder gegebenenfalls substituiertes C₁-C₆-Heteroalkyl ist;
R^{B3} A², gegebenenfalls substituiertes C₁-C₆-Alkyl, gegebenenfalls substituiertes C₁-C₆-Heteroalkyl, gegebenenfalls substituiertes C₃-C₁₀-Carbocyclyl, gegebenenfalls substituiertes C₆-C₁₀-Aryl, gegebenenfalls substituiertes C₁-C₆-Alkyl-C₃-C₁₀-carbocyclyl oder gegebenenfalls substituiertes C₁-C₆-Alkyl-C₆-C₁₀-aryl ist;
R^{B4} H, gegebenenfalls substituiertes C₁-C₆-Alkyl, gegebenenfalls substituiertes C₃-C₁₀-Carbocyclyl, gegebenenfalls substituiertes C₆-C₁₀-Aryl, gegebenenfalls substituiertes C₁-C₆-Alkyl-C₃-C₁₀-carbocyclyl oder gegebenenfalls substituiertes C₁-C₆-Alkyl-C₆-C₁₀-aryl ist;
R^{B5} H, gegebenenfalls substituiertes C₁-C₆-Alkyl oder gegebenenfalls substituiertes C₁-C₆-Heteroalkyl ist;
v2 0, 1, 2, 3 oder 4 ist;
jedes R^{B6} unabhängig A², Halogen, gegebenenfalls substituiertes C₁-C₆-Alkyl, gegebenenfalls substituiertes C₁-C₆-Heteroalkyl, gegebenenfalls substituiertes C₃-C₁₀-Carbocyclyl, gegebenenfalls substituiertes C₂-C₉-Heterocyclyl, gegebenenfalls substituiertes C₆-C₁₀-Aryl, gegebenenfalls substituiertes C₂-C₉-Heteroaryl, gegebenenfalls substituiertes C₂-C₆-Alkenyl, gegebenenfalls substituiertes C₂-C₆-Heteroalkenyl, Hydroxy, Thiol oder gegebenenfalls substituiertes Amino ist;
R^{B7} und R^{B8} jeweils unabhängig voneinander H, Halogen, gegebenenfalls substituiertes C₁-C₆-Alkyl oder gegebenenfalls substituiertes C₆-C₁₀-Aryl sind;
R^{B9} H oder gegebenenfalls substituiertes C₁-C₆-Alkyl ist; und
A² eine Bindung zwischen dem Abbaurest und dem Linker ist;
wobei genau eines von R^{B1}, R^{B3} und R^{B6} A² ist,
oder ein pharmazeutisch verträgliches Salz davon.

15. Verbindung nach Anspruch 14 oder ein pharmazeutisch verträgliches Salz davon, wobei der Abbaurest Folgender ist

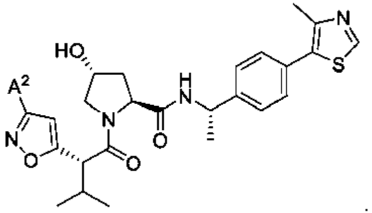

16. Verbindung nach einem der Ansprüche 1 bis 15 oder ein pharmazeutisch verträgliches Salz davon, wobei der Linker die folgende Struktur aufweist 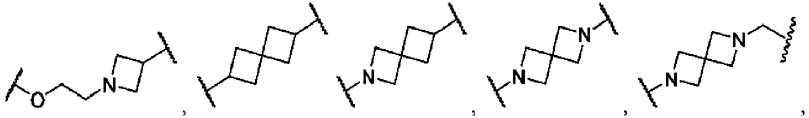 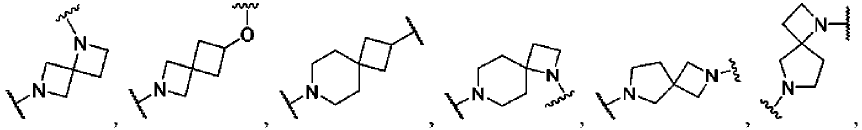 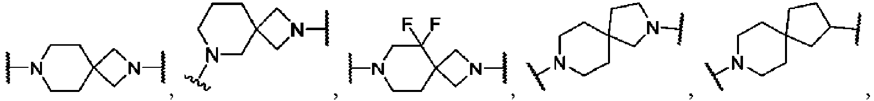 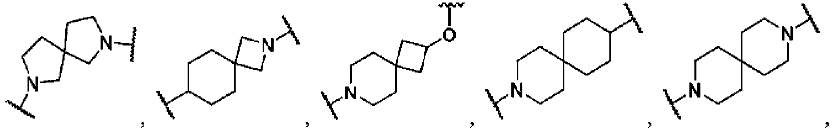 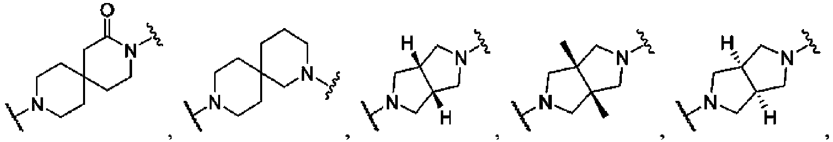 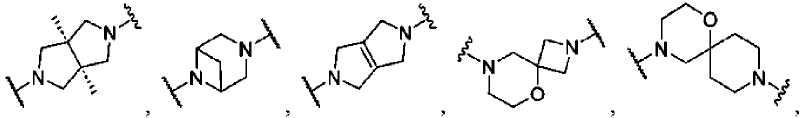 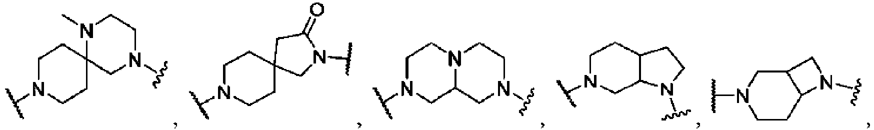 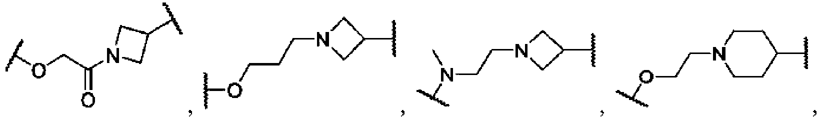 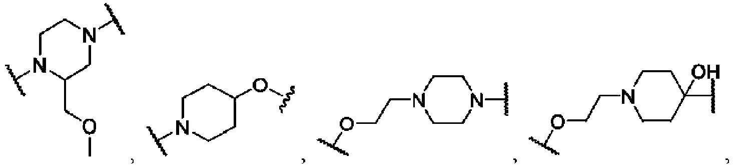 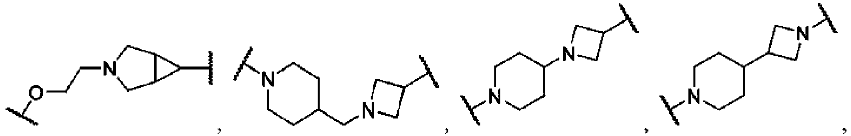 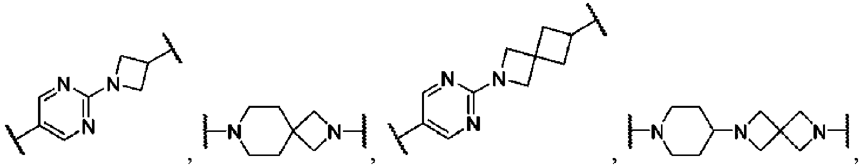 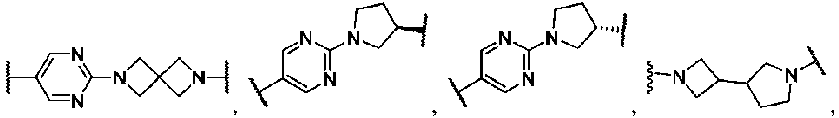 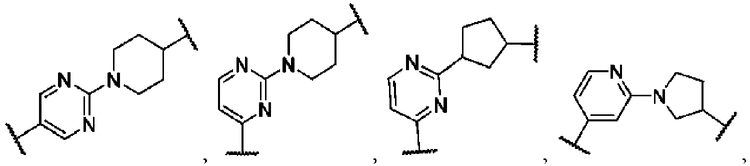 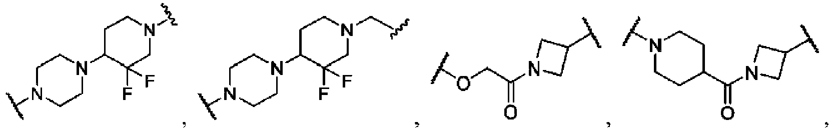 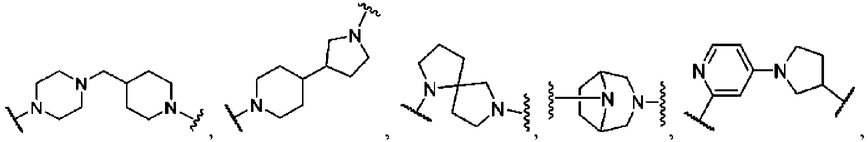 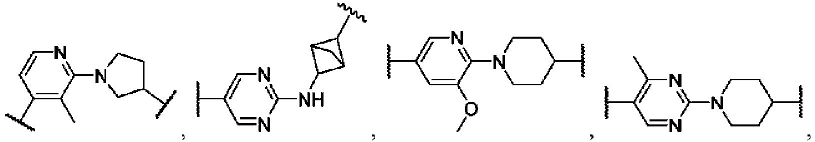 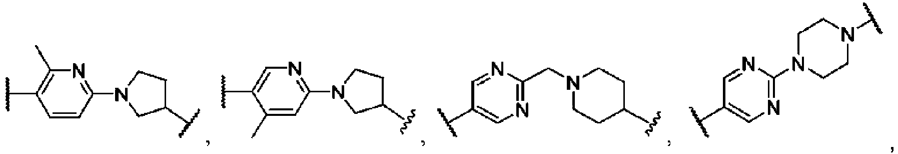 oder

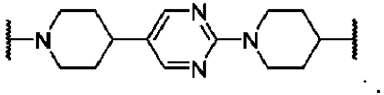

17. Verbindung nach einem der Ansprüche 1 bis 15 oder ein pharmazeutisch verträgliches Salz davon, wobei der Linker die folgende Struktur aufweist 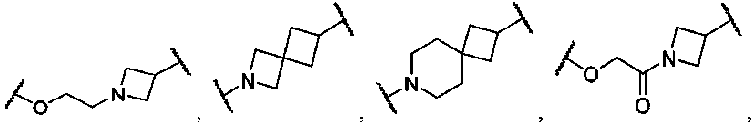 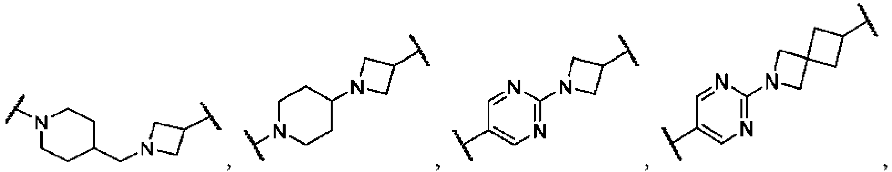 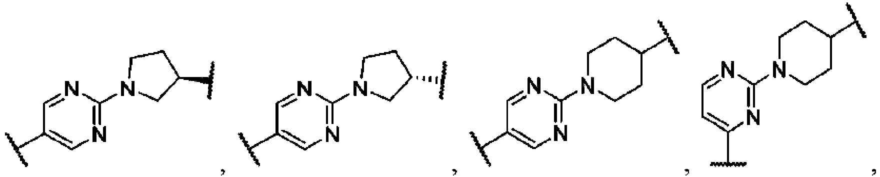 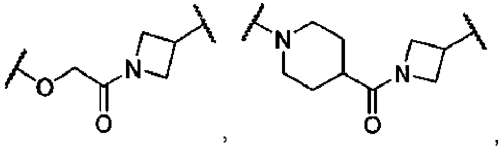 oder

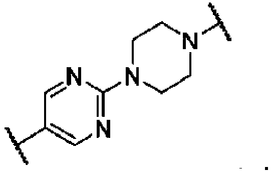

18. Pharmazeutische Zusammensetzung, umfassend eine Verbindung nach einem der Ansprüche 1 bis 17 und einen pharmazeutisch verträglichen Trägerstoff.

19. Verbindung nach einem der Ansprüche 1 bis 17 oder ein pharmazeutisch verträgliches Salz davon, oder eine pharmazeutische Zusammensetzung nach Anspruch 18 für eine Verwendung bei einer Therapie.

20. Verbindung nach einem der Ansprüche 1 bis 17 oder ein pharmazeutisch verträgliches Salz davon oder eine pharmazeutische Zusammensetzung nach Anspruch 18 für eine Verwendung beim Behandeln von Krebs.

21. Verbindung oder pharmazeutisch verträgliches Salz davon oder die pharmazeutische Zusammensetzung zur Verwendung nach Anspruch 20, wobei der Krebs nichtkleinzelliger Lungenkrebs, kolorektaler Krebs, Blasenkrebs, Krebs unbekannten Primärtumors, Gliom, Brustkrebs, Melanom, Nicht-Melanom-Hautkrebs, Endometriumkrebs, ösophagogastrischer Krebs, Bauchspeicheldrüsenkrebs, hepatobiliärer Krebs, Weichgewebesarkom, Eierstockkrebs, Kopf- und Halskrebs, Nierenzellkarzinom, Knochenkrebs, Non-Hodgkin-Lymphom, kleinzelliger Lungenkrebs, Prostatakrebs, embryonaler Tumor, Keimzelltumor, Gebärmutterhalskrebs, Schilddrüsenkrebs, Speicheldrüsenkrebs, gastrointestinaler neuroendokriner Tumor, Uterussarkom, gastrointestinaler Stromatumor, ZNS-Krebs, Thymustumor, Nebennierenrindenkarzinom, Appendixkrebs, Dünndarmkrebs oder Peniskrebs ist.

## Revendications

1. Composé, ou sel pharmaceutiquement acceptable de celui-ci, ayant la structure de **Formule I ou II :** 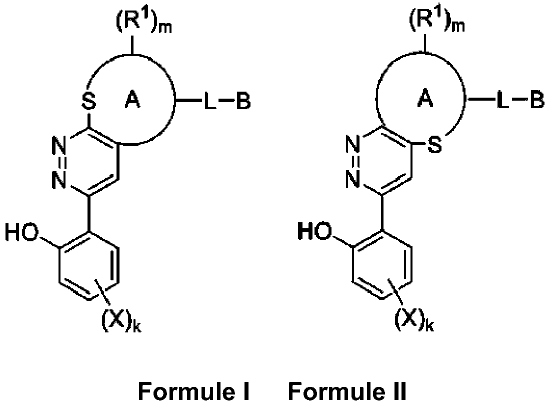 dans lequel
le système cyclique A est un hétérocyclyle de 5 à 9 chaînons ou un hétéroaryle ;
m vaut 0, 1, 2, ou 3 ;
k vaut 0, 1 ou 2 ;
chaque R¹ est, indépendamment, halo, alkyle en C₁ à C₆ facultativement substitué, hétéroalkyle en C₁ à C₆ facultativement substitué, hétérocyclyle en C₂ à C₉ facultativement substitué, cycloalkyle en C₃ à C₈ facultativement substitué ou CH₂-cycloalkyle en C₃ à C₈ facultativement substitué ;
chaque X est, indépendamment, halo ;
L est un lieur de Formule III :
A¹-(B¹)_{f}-(C¹)_{g}-(B²)ₕ-(B³)ᵢ-(C²)ⱼ-(B⁴)ₖ-A², **Formule III**
dans lequel
A¹ est une liaison entre le lieur et le système cyclique A ;
A² est une liaison entre le fragment de dégradation et le lieur ;
chacun de B¹, B², B³ et B⁴ est, indépendamment, éthynyle facultativement substitué, aryle en C₆ à C₁₀ facultativement substitué, cycloalkyle en C₃ à C₁₀ facultativement substitué, hétérocyclyle en C₂ à C₉ facultativement substitué, hétéroaryle en C₂ à C₉ facultativement substitué, O, S, S(O)₂ ou NR^{N} ;
chaque R^{N} est, indépendamment, H, alkyle en C₁ à ₄ facultativement substitué, alcényle en C₂ à ₄ facultativement substitué, alcynyle en C₂ à ₄ facultativement substitué, hétérocyclyle en C₂ à ₆ facultativement substitué, aryle en C₆ à ₁₂ facultativement substitué, ou hétéroalkyle en C₁ à ₇ facultativement substitué ;
chacun de C¹ et de C² est, indépendamment, carbonyle, thiocarbonyle, sulfonyle ou phosphoryle ; et
chacun de f, g, h, i, j et k vaut, indépendamment, 0 ou 1 ; et
B est un fragment de dégradation, dans lequel le fragment de dégradation a la structure de Formule C : 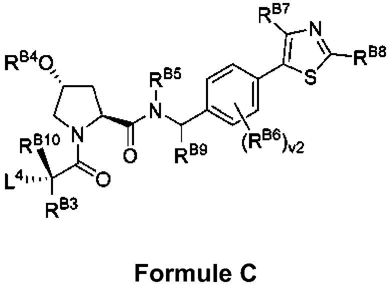
dans lequel
L⁴ is -N(R^{B1})(R^{B2}), 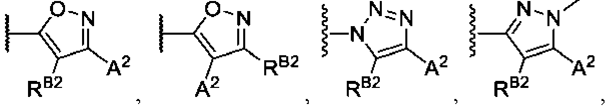 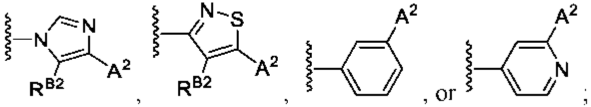
R^{B1} est H, A², alkyle en C₁ à C₆ facultativement substitué ou hétéroalkyle en C₁ à C₆ facultativement substitué ;
R^{B2} est H, alkyle en C₁ à C₆ facultativement substitué ou hétéroalkyle en C₁ à C₆ facultativement substitué ;
R^{B3} est A², alkyle en C₁ à C₆ facultativement substitué, hétéroalkyle en C₁ à C₆ facultativement substitué, carbocyclyle en C₃ à C₁₀ facultativement substitué, aryle en C₆ à C₁₀ facultativement substitué, alkyl en C₁ à C₆-carbocyclyle en C₃ à C₁₀ facultativement substitué, ou alkyl en C₁ à C₆-aryle en C₆ à C₁₀ facultativement substitué ;
R^{B4} est H, alkyle en C₁ à C₆ facultativement substitué, carbocyclyle en C₃ à C₁₀ facultativement substitué, aryle en C₆ à C₁₀ facultativement substitué, alkyl en C₁ à C₆-carbocyclyle en C₃ à C₁₀ facultativement substitué, ou alkyl en C₁ à C₆-aryle en C₆ à C₁₀ facultativement substitué ;
R^{B5} est H, alkyle en C₁ à C₆ facultativement substitué ou hétéroalkyle en C₁ à C₆ facultativement substitué ;
v2 vaut 0, 1, 2, 3 ou 4 ;
chaque R^{B6} est, indépendamment, A², halogène, alkyle en C₁ à C₆ facultativement substitué, alcynyle en C₂ à C₆ facultativement substitué, hétéroalkyle en C₁ à C₆ facultativement substitué, carbocyclyle en C₃ à C₁₀ facultativement substitué, hétérocyclyle en C₂ à C₉ facultativement substitué, aryle en C₆ à C₁₀ facultativement substitué, hétéroaryle en C₂ à C₉ facultativement substitué, alcényle en C₂ à C₆ facultativement substitué, hétéroalcényle en C₂ à C₆ facultativement substitué, hydroxy, thiol, cyano, ou amino facultativement substitué ;
chacun de R^{B7} et R^{B8} est, indépendamment, H, halogène, alkyle en C₁ à C₆ facultativement substitué ou aryle en C₆ à C₁₀ facultativement substitué ;
R^{B9} est H ou alkyle en C₁ à C₆ facultativement substitué ;
R^{B10} est H ou F ; et
A² est une liaison entre le fragment de dégradation et le lieur ;
dans lequel un et seulement un parmi R^{B1}, R^{B3} et R^{B6} est A²,
ou sel pharmaceutiquement acceptable de celui-ci.

2. Composé selon la revendication 1, ou sel pharmaceutiquement acceptable de celui-ci, dans lequel le composé a la structure de **Formule I-A ou II-A :** 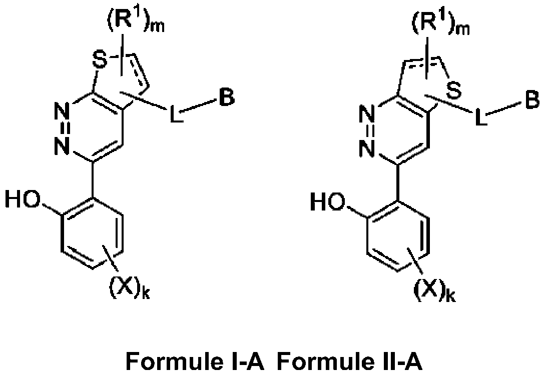 dans lequel la liaison en pointillés représente une liaison simple ou double.

3. Composé selon la revendication 1, ou sel pharmaceutiquement acceptable de celui-ci, dans lequel le composé a la structure de **Formule I-H ou II-H :**

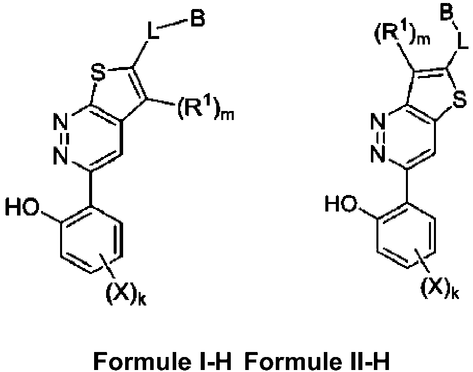

4. Composé selon l'une quelconque des revendications 1 à 3, ou sel pharmaceutiquement acceptable de celui-ci, dans lequel m vaut 0 ou 1.

5. Composé selon l'une quelconque des revendications 1 à 4, ou sel pharmaceutiquement acceptable de celui-ci, dans lequel lorsque m vaut 1, R¹ est halo, alkyle en C₁ à C₆ facultativement substitué ou cycloalkyle en C₃ à C₈ facultativement substitué.

6. Composé selon l'une quelconque des revendications 1 à 5, ou sel pharmaceutiquement acceptable de celui-ci, dans lequel k vaut 1 et X est Cl.

7. Composé selon l'une quelconque des revendications 1 à 6, ou sel pharmaceutiquement acceptable de celui-ci, dans lequel k vaut 0.

8. Composé selon l'une quelconque des revendications 1 à 7, ou sel pharmaceutiquement acceptable de celui-ci, dans lequel au moins l'un parmi f, h, i et k vaut 1.

9. Composé selon l'une quelconque des revendications 1 à 8 ou sel pharmaceutiquement acceptable de celui-ci, dans lequel chacun de B¹, B², B³ et B⁴ est, indépendamment, O, éthynyle, hétéroaryle en C₂ à C₉ facultativement substitué, hétérocyclyle en C₂ à C₉ facultativement substitué, cycloalkyle en C₃ à C₁₀ facultativement substitué, ou aryle en C₆ à C₁₀ facultativement substitué.

10. Composé selon l'une quelconque des revendications 1 à 9, ou sel pharmaceutiquement acceptable de celui-ci, dans lequel chacun de B¹ et B⁴ est, indépendamment,
O, 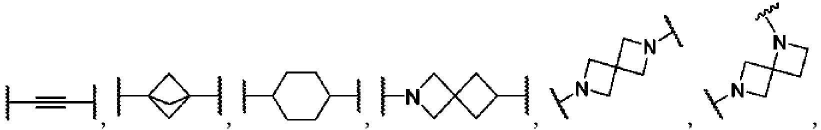 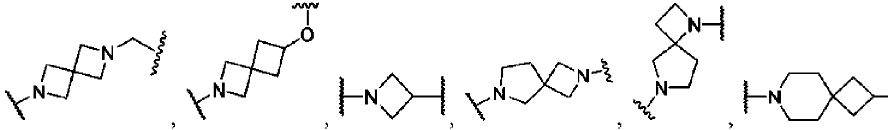 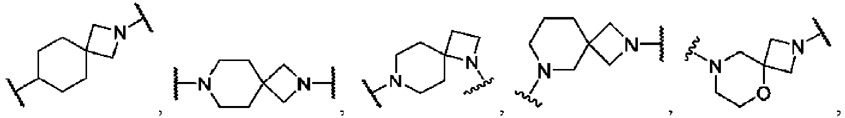 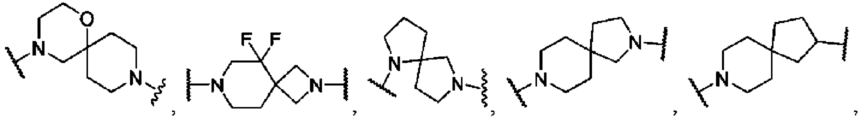 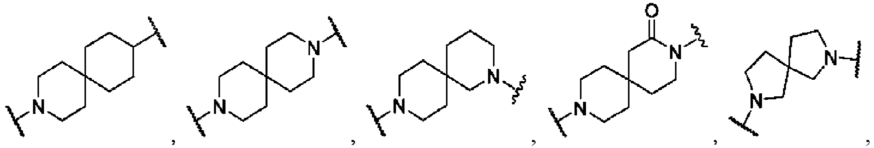 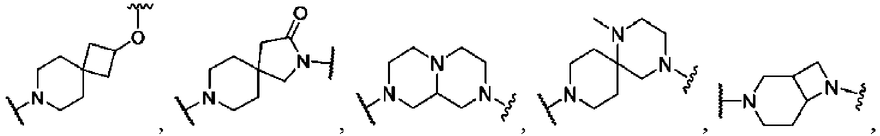 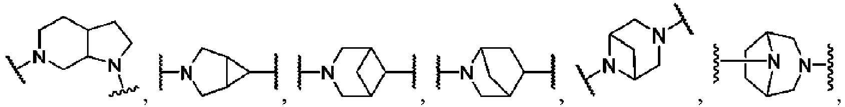 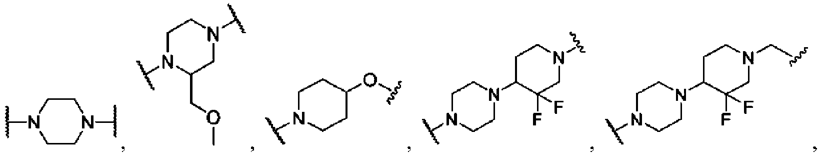 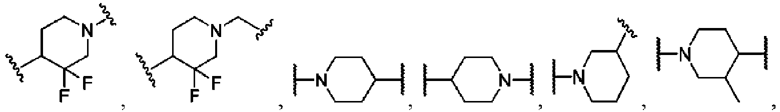 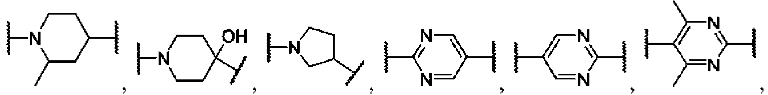 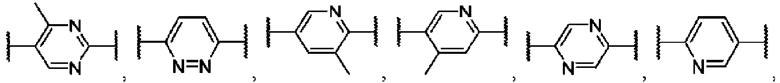 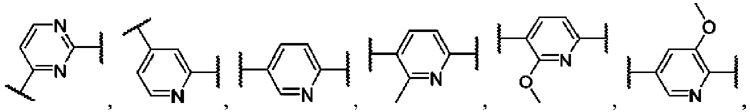 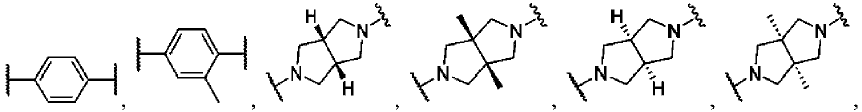 ou

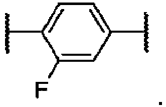

11. Composé selon la revendication 10, ou sel pharmaceutiquement acceptable de celui-ci, dans lequel B¹ est

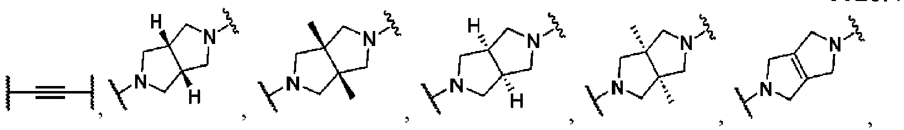

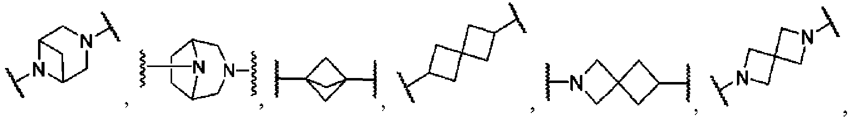

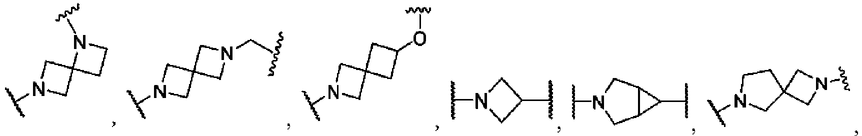

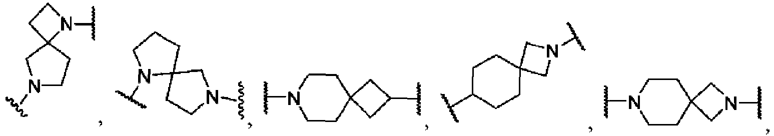

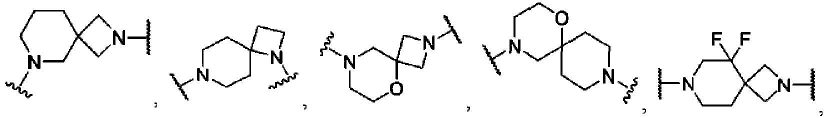

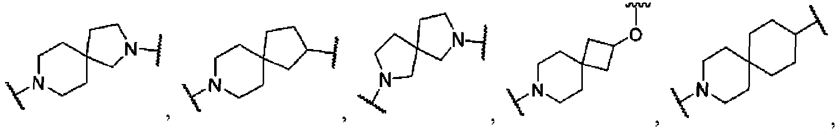

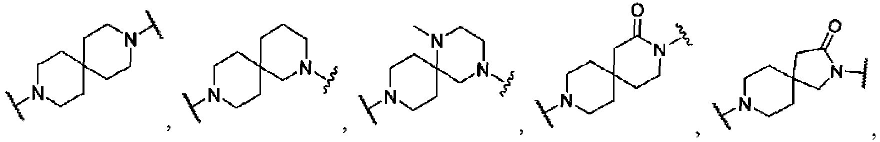

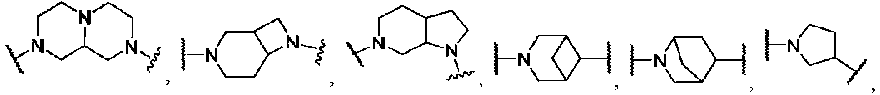

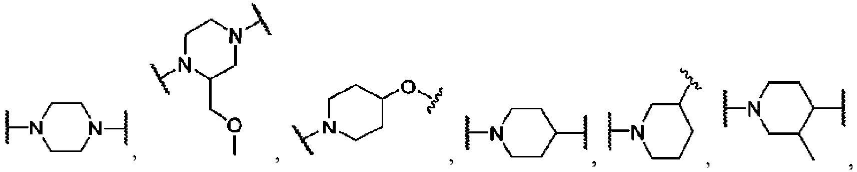

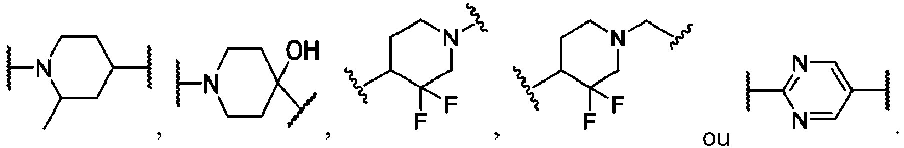

12. Composé selon la revendication 10 ou 11, ou sel pharmaceutiquement acceptable de celui-ci, dans lequel B⁴ est
O, 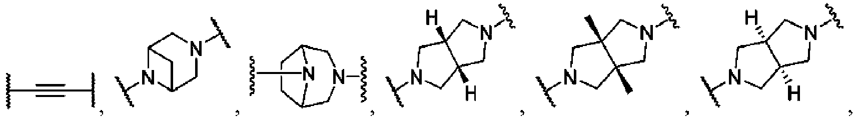 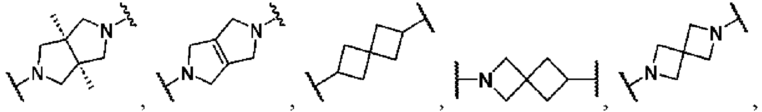 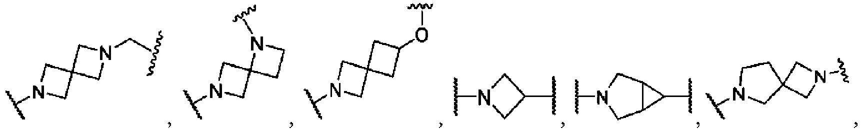 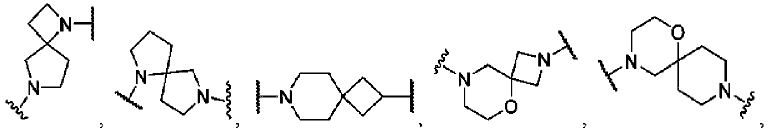 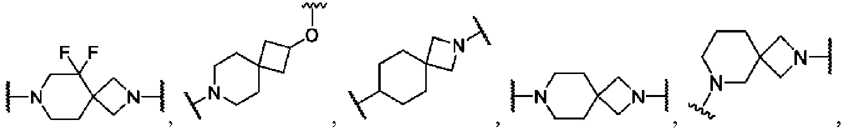 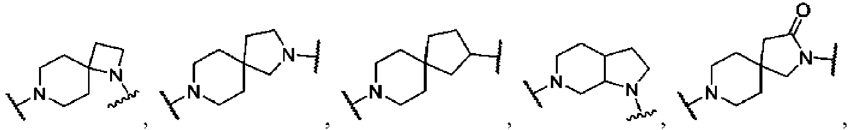 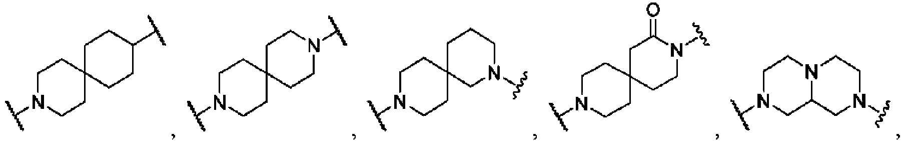 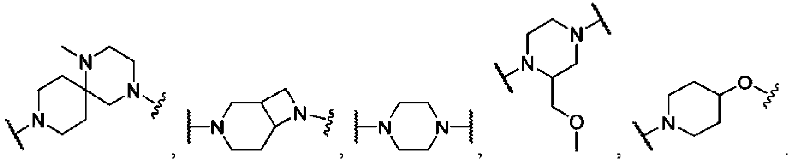 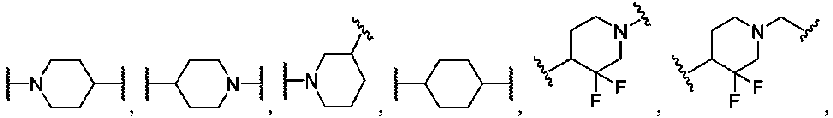 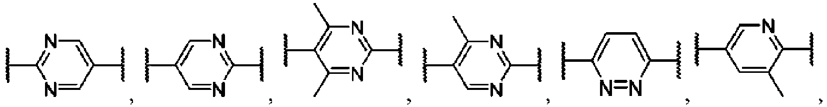 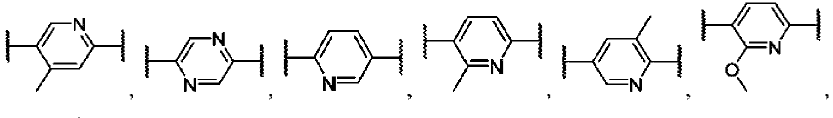 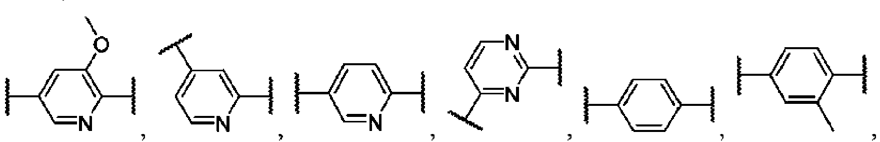 ou

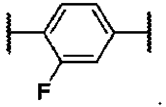

13. Composé selon l'une quelconque des revendications 1 à 7, ou sel pharmaceutiquement acceptable de celui-ci, dans lequel B² est NH,

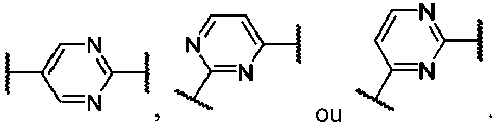

14. Composé selon l'une quelconque des revendications 1 à 13, ou sel pharmaceutiquement acceptable de celui-ci, dans lequel
L⁴ est -N(R^{B1})(R^{B2}), 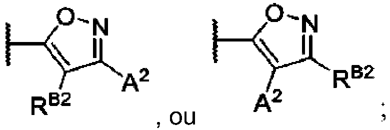
R^{B1} est H, A², alkyle en C₁ à C₆ facultativement substitué ou hétéroalkyle en C₁ à C₆ facultativement substitué ;
R^{B2} est H, alkyle en C₁ à C₆ facultativement substitué ou hétéroalkyle en C₁ à C₆ facultativement substitué ;
R^{B3} est A², alkyle en C₁ à C₆ facultativement substitué, hétéroalkyle en C₁ à C₆ facultativement substitué, carbocyclyle en C₃ à C₁₀ facultativement substitué, aryle en C₆ à C₁₀ facultativement substitué, alkyl en C₁ à C₆-carbocyclyle en C₃ à C₁₀ facultativement substitué, ou alkyl en C₁ à C₆-aryle en C₆ à C₁₀ facultativement substitué ;
R^{B4} est H, alkyle en C₁ à C₆ facultativement substitué, carbocyclyle en C₃ à C₁₀ facultativement substitué, aryle en C₆ à C₁₀ facultativement substitué, alkyl en C₁ à C₆-carbocyclyle en C₃ à C₁₀ facultativement substitué, ou alkyl en C₁ à C₆-aryle en C₆ à C₁₀ facultativement substitué ;
R^{B5} est H, alkyle en C₁ à C₆ facultativement substitué ou hétéroalkyle en C₁ à C₆ facultativement substitué ;
v2 vaut 0, 1, 2, 3 ou 4 ;
chaque R^{B6} est, indépendamment, A², halogène, alkyle en C₁ à C₆ facultativement substitué, hétéroalkyle en C₁ à C₆ facultativement substitué, carbocyclyle en C₃ à C₁₀ facultativement substitué, hétérocyclyle en C₂ à C₉ facultativement substitué, aryle en C₆ à C₁₀ facultativement substitué, hétéroaryle en C₂ à C₉ facultativement substitué, alcényle en C₂ à C₆ facultativement substitué, hétéroalcényle en C₂ à C₆ facultativement substitué, hydroxy, thiol, ou amino facultativement substitué ;
chacun de R^{B7} et R^{B8} est, indépendamment, H, halogène, alkyle en C₁ à C₆ facultativement substitué ou aryle en C₆ à C₁₀ facultativement substitué ;
R^{B9} est H ou alkyle en C₁ à C₆ facultativement substitué ; et
A² est une liaison entre le fragment de dégradation et le lieur ;
dans lequel un et seulement un parmi R^{B1}, R^{B3} et R^{B6} est A²,
ou sel pharmaceutiquement acceptable de celui-ci.

15. Composé selon la revendication 14, ou sel pharmaceutiquement acceptable de celui-ci, dans lequel le fragment de dégradation est

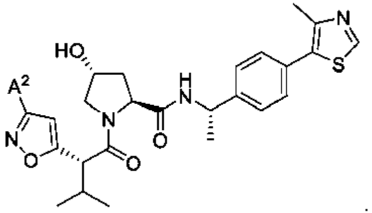

16. Composé selon l'une quelconque des revendications 1 à 15, ou sel pharmaceutiquement acceptable de celui-ci, dans lequel le lieur a la structure de 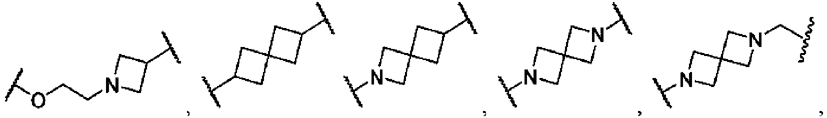 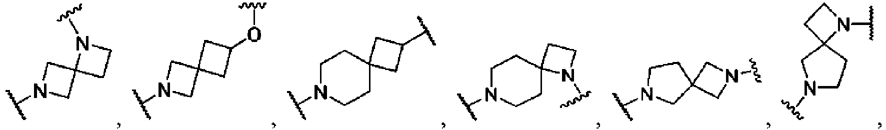 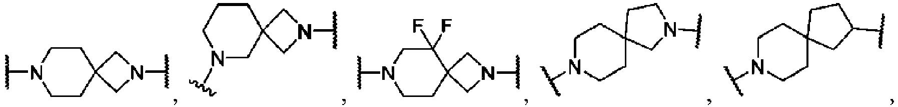 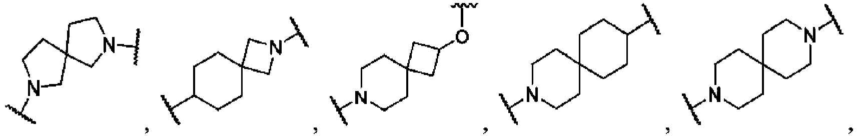 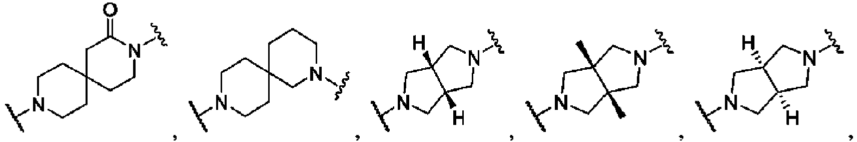 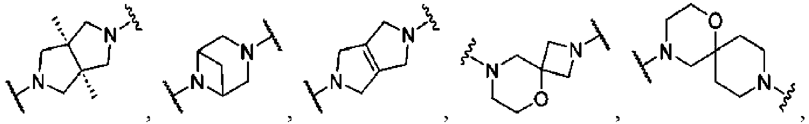 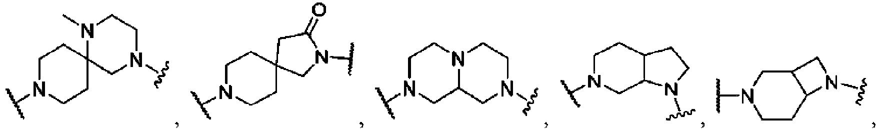 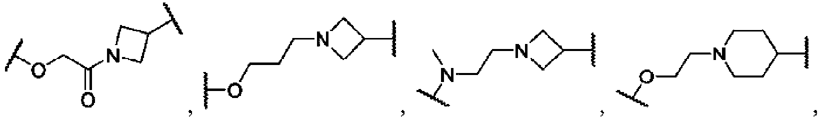 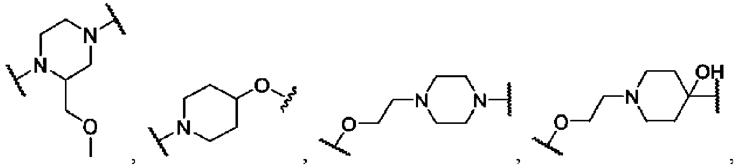 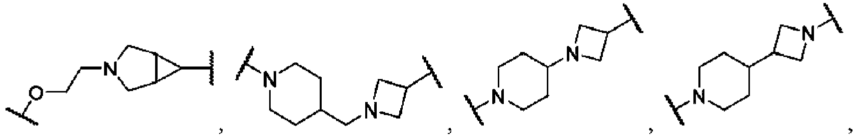 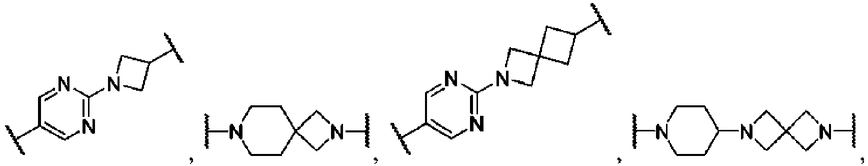 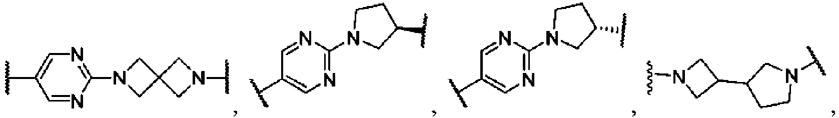 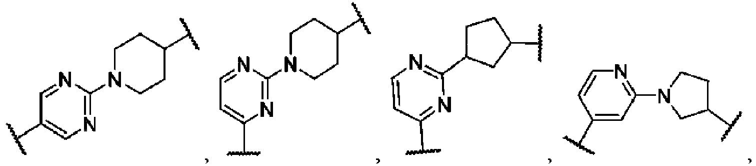 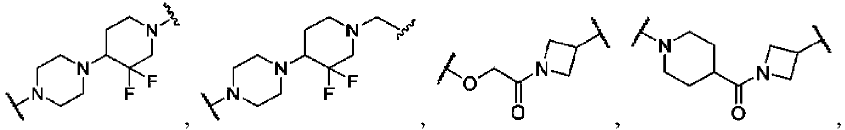 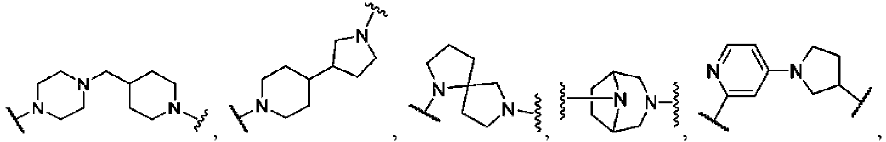 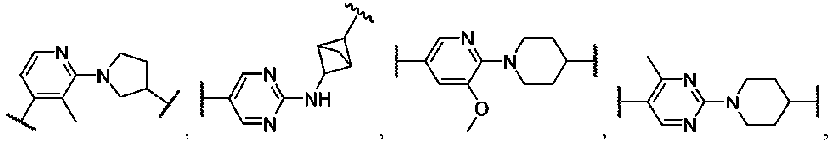 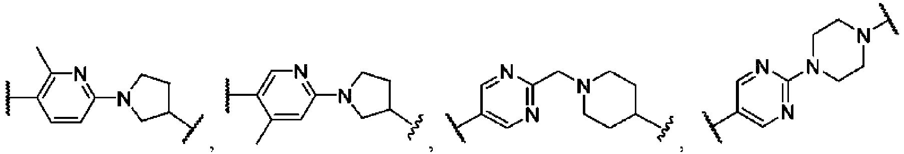 ou

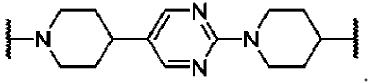

17. Composé selon l'une quelconque des revendications 1 à 15, ou sel pharmaceutiquement acceptable de celui-ci, dans lequel le lieur a la structure de

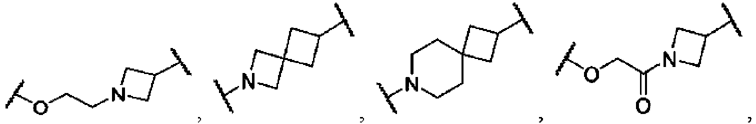

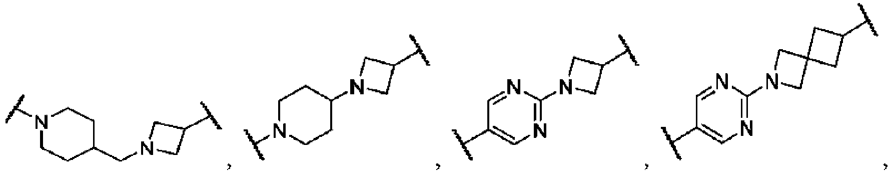

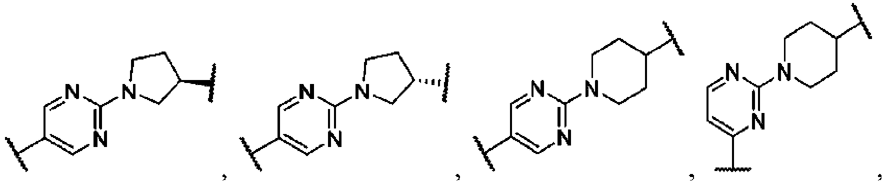

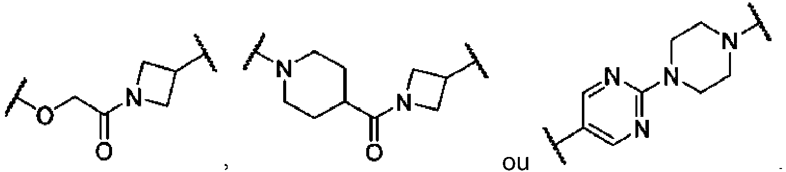

18. Composition pharmaceutique comprenant un composé selon l'une quelconque des revendications 1 à 17 et un excipient pharmaceutiquement acceptable.

19. Composé selon l'une quelconque des revendications 1 à 17, ou sel pharmaceutiquement acceptable de celui-ci, ou composition pharmaceutique selon la revendication 18, pour une utilisation en thérapie.

20. Composé selon l'une quelconque des revendications 1 à 17, ou sel pharmaceutiquement acceptable de celui-ci, ou composition pharmaceutique selon la revendication 18, pour utilisation dans le traitement d'un cancer.

21. Composé, ou sel pharmaceutiquement acceptable de celui-ci, ou composition pharmaceutique pour utilisation selon la revendication 20, dans lequel le cancer est un cancer du poumon non à petites cellules, un cancer colorectal, un cancer de la vessie, un cancer d'origine inconnue, un gliome, un cancer du sein, un mélanome, un cancer de la peau autre que mélanome, un cancer de l'endomètre, un cancer œsophagien et gastrique, un cancer du pancréas, un cancer hépatobiliaire, un sarcome des tissus mous, un cancer de l'ovaire, un cancer de la tête et du cou, un carcinome rénal, un cancer des os, un lymphome non hodgkinien, un cancer du poumon à petites cellules, un cancer de la prostate, une tumeur embryonnaire, une tumeur germinale, un cancer du col de l'utérus, un cancer de la thyroïde, un cancer des glandes salivaires, une tumeur neuroendocrine gastro-intestinale, un sarcome utérin, une tumeur stromale gastro-intestinale, un cancer du système nerveux central, une tumeur thymique, un carcinome corticosurrénalien, un cancer de l'appendice, un cancer de l'intestin grêle ou un cancer du pénis.
